# EUROPEAN PATENT APPLICATION

(11) **EP 1 623 710 A1**
(43) Date of publication of application: **08.02.2006**
(21) Application number: 04727766.0
(22) Date of filing: 15.04.2004
(51) Int. Cl.: A61K 31/5513, A61K 31/553, A61K 31/5377, A61P 43/00, A61P 25/04, A61P 37/06, A61P 35/00, A61P 15/00, A61P 9/10, A61P 7/02, A61P 1/14, A61P 25/28, A61P 21/00, A61P 25/08, A61P 25/14, A61P 7/00, A61P 5/24, A61P 3/10, A61P 13/02, A61P 3/06, A61P 17/00

(54) **RECEPTOR ANTAGONIST**

(30) Priority: 18.04.2003 JP 2003114313
(71) Applicant: Takeda Pharmaceutical Company Limited, Osaka-shi, Osaka 541-0045 (JP)
(72) Inventor: ITOH, Fumio, 10, Wadai Tsukuba-shi, Ibaraki (JP); HINUMA, Shuji, 10, Wadai Tsukuba-shi, Ibaraki (JP); KANZAKI, N. c/o Takeda Pharmaceutical Company Ltd., Osaka-shi, Osaka (JP); MABUCHI, H. c/o Takeda Pharmaceutical Company Ltd., Osaka-shi, Osaka (JP); YOSHIDA, Hiromi, 10, Wadai Tsukuba-shi, Ibaraki (JP); MATSUMOTO, Hirokazu, 10, Wadai Tsukuba-shi, Ibaraki (JP); WAKABAYASHI, T. Takeda Pharmaceutical Company Ltd., Osaka-shi, Osaka (JP)
(74) Representative: Lewin, John Harvey
(86) International application number: PCT/JP2004/005406
(87) International publication number: WO 2004/091628

(57) **Abstract**

An agent for modulating the function of an RFRP receptor, **characterized by** containing either a compound represented by the formula (I) 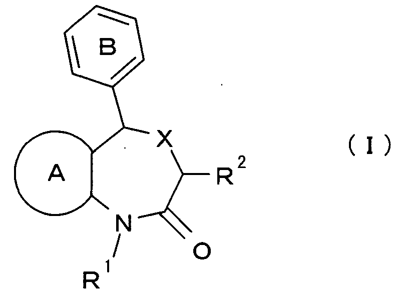 [wherein ring A represents an optionally substituted aromatic ring; ring B represents an optionally substituted benzene ring; X represents oxygen, S(O)ₙ (n is an integer of 0 to 2), or NR³ (R³ represents hydrogen, an optionally substituted hydrocarbon group, or an optionally substituted heterocyclic group); and R¹ and R² each represents hydrogen, an optionally substituted hydrocarbon group, or an optionally substituted heterocyclic group] or a salt of the compound.

SEQUENCE LISTING
<110> Takeda Chemical Industries, Ltd.
<120> RFRP Receptor Antagonist
<130> 3169WOOP
<150> JP 2003-114313
<151> 2003-04-18
<160> 4
<210> 1
<211> 430
<212> PRT
<213> Homo sapiens
<400> 1
Met Glu Gly Glu Pro Ser Gln Pro Pro Asn Ser Ser Trp Pro Leu Ser
1               5                   10                  15
Gln Asn Gly Thr Asn Thr Glu Ala Thr Pro Ala Thr Asn Leu Thr Phe
            20                  25                  30
Ser Ser Tyr Tyr Gln His Thr Ser Pro Val Ala Ala Met Phe Ile Val
        35                  40                  45
Ala Tyr Ala Leu Ile Phe Leu Leu Cys Met Val Gly Asn Thr Leu Val
    50                  55                  60
Cys Phe Ile Val Leu Lys Asn Arg His Met His Thr Val Thr Asn Met
65                  70                  75                  80
Phe Ile Leu Asn Leu Ala Val Ser Asp Leu Leu Val Gly Ile Phe Cys
                85                  90                  95
Met Pro Thr Thr Leu Val Asp Asn Leu Ile Thr Gly Trp Pro Phe Asp
            100                 105                 110
Asn Ala Thr Cys Lys Met Ser Gly Leu Val Gln Gly Met Ser Val Ser
        115                 120                 125
Ala Ser Val Phe Thr Leu Val Ala Ile Ala Val Glu Arg Phe Arg Cys
    130                 135                 140
Ile Val His Pro Phe Arg Glu Lys Leu Thr Leu Arg Lys Ala Leu Val
145                 150                 155                 160
Thr Ile Ala Val Ile Trp Ala Leu Ala Leu Leu Ile Met Cys Pro Ser
                165                 170                 175
Ala Val Thr Leu Thr Val Thr Arg Glu Glu His His Phe Met Val Asp
            180                 185                 190
Ala Arg Asn Arg Ser Tyr Pro Leu Tyr Ser Cys Trp Glu Ala Trp Pro
        195                 200                 205
Glu Lys Gly Met Arg Arg Val Tyr Thr Thr Val Leu Phe Ser His Ile
    210                 215                 220
Tyr Leu Ala Pro Leu Ala Leu Ile Val Val Met Tyr Ala Arg Ile Ala
225                 230                 235                 240
Arg Lys Leu Cys Gln Ala Pro Gly Pro Ala Pro Gly Gly Glu Glu Ala
                245                 250                 255
Ala Asp Pro Arg Ala Ser Arg Arg Arg Ala Arg Val Val His Met Leu
            260                 265                 270
Val Met Val Ala Leu Phe Phe Thr Leu Ser Trp Leu Pro Leu Trp Ala
        275                 280                 285
Leu Leu Leu Leu Ile Asp Tyr Gly Gln Leu Ser Ala Pro Gln Leu His
    290                 295                 300
Leu Val Thr Val Tyr Ala Phe Pro Phe Ala His Trp Leu Ala Phe Phe
305                 310                 315                 320
Asn Ser Ser Ala Asn Pro Ile Ile Tyr Gly Tyr Phe Asn Glu Asn Phe
                325                 330                 335
Arg Arg Gly Phe Gln Ala Ala Phe Arg Ala Arg Leu Cys Pro Arg Pro
            340                 345                 350
Ser Gly Ser His Lys Glu Ala Tyr Ser Glu Arg Pro Gly Gly Leu Leu
        355                 360                 365
His Arg Arg Val Phe Val Val Val Arg Pro Ser Asp Ser Gly Leu Pro
    370                 375                 380
Ser Glu Ser Gly Pro Ser Ser Gly Ala Pro Arg Pro Gly Arg Leu Pro
385                 390                 395                 400
Leu Arg Asn Gly Arg Val Ala His His Gly Leu Pro Arg Glu Gly Pro
                405                 410                 415
Gly Cys Ser His Leu Pro Leu Thr Ile Pro Ala Trp Asp Ile
            420                 425                 430
<210> 2
<211> 432

<212> PRT
<213> Rattus norvegicus
<400> 2
Met Glu Ala Glu Pro Ser Gln Pro Pro Asn Gly Ser Trp Pro Leu Gly
                5                   10                  15
Gln Asn Gly Ser Asp Val Glu Thr Ser Met Ala Thr Ser Leu Thr Phe
            20                  25                  30
Ser Ser Tyr Tyr Gln His Ser Ser Pro Val Ala Ala Met Phe Ile Ala
        35                  40                  45
Ala Tyr Val Leu Ile Phe Leu Leu Cys Met Val Gly Asn Thr Leu Val
    50                  55                  60
Cys Phe Ile Val Leu Lys Asn Arg His Met Arg Thr Val Thr Asn Met
65                  70                  75                  80
Phe Ile Leu Asn Leu Ala Val Ser Asp Leu Leu Val Gly Ile Phe Cys
                85                  90                  95
Met Pro Thr Thr Leu Val Asp Asn Leu Ile Thr Gly Trp Pro Phe Asp
            100                 105                 110
Asn Ala Thr Cys Lys Met Ser Gly Leu Val Gln Gly Met Ser Val Ser
        115                 120                 125
Ala Ser Val Phe Thr Leu Val Ala Ile Ala Val Glu Arg Phe Arg Cys
    130                 135                 140
Ile Val His Pro Phe Arg Glu Lys Leu Thr Leu Arg Lys Ala Leu Phe
145                 150                 155                 160
Thr Ile Ala Val Ile Trp Ala Leu Ala Leu Leu Ile Met Cys Pro Ser
                165                 170                 175
Ala Val Thr Leu Thr Val Thr Arg Glu Glu His His Phe Met Leu Asp
            180                 185                 190
Ala Arg Asn Arg Ser Tyr Pro Leu Tyr Ser Cys Trp Glu Ala Trp Pro
        195                 200                 205
Glu Lys Gly Met Arg Lys Val Tyr Thr Ala Val Leu Phe Ala His Ile
    210                 215                 220
Tyr Leu Val Pro Leu Ala Leu Ile Val Val Met Tyr Val Arg Ile Ala
225                 230                 235                 240
Arg Lys Leu Cys Gln Ala Pro Gly Pro Ala Arg Asp Thr Glu Glu Ala
                245                 250                 255
Val Ala Glu Gly Gly Arg Thr Ser Arg Arg Arg Ala Arg Val Val His
            260                 265                 270
Met Leu Val Met Val Ala Leu Phe Phe Thr Leu Ser Trp Leu Pro Leu
        275                 280                 285
Trp Val Leu Leu Leu Leu Ile Asp Tyr Gly Glu Leu Ser Glu Leu Gln
    290                 295                 300
Leu His Leu Leu Ser Val Tyr Ala Phe Pro Leu Ala His Trp Leu Ala
305                 310                 315                 320
Phe Phe His Ser Ser Ala Asn Pro Ile Ile Tyr Gly Tyr Phe Asn Glu
                325                 330                 335
Asn Phe Arg Arg Gly Phe Gln Ala Ala Phe Arg Ala Gln Leu Cys Trp
            340                 345                 350
Pro Pro Trp Ala Ala His Lys Gln Ala Tyr Ser Glu Arg Pro Asn Arg
        355                 360                 365
Leu Leu Arg Arg Arg Val Val Val Asp Val Gln Pro Ser Asp Ser Gly
    370                 375                 380
Leu Pro Ser Glu Ser Gly Pro Ser Ser Gly Val Pro Gly Pro Gly Arg
385                 390                 395                 400
Leu Pro Leu Arg Asn Gly Arg Val Ala His Gln Asp Gly Pro Gly Glu
                405                 410                 415
Gly Pro Gly Cys Asn His Met Pro Leu Thr Ile Pro Ala Trp Asn Ile
            420                 425                 430
<210> 3
<211> 432
<212> PRT
<213> Mus musculus
<400> 3
Met Glu Ala Glu Pro Ser Gln Pro Pro Asn Gly Ser Trp Pro Pro Ser
                5                   10                  15
Leu Asn Glu Ser Asp Ala Glu Thr Ala Pro Val Ala Ser Leu Thr Phe
            20                  25                  30
Ser Ser Tyr Tyr Gln His Ser Ser Pro Val Ala Ala Met Phe Ile Ala
        35                  40                  45

Ala Tyr Ala Leu Ile Phe Leu Leu Cys Met Val Gly Asn Thr Leu Val
        50                  55                  60
Cys Phe Ile Val Leu Lys Asn Arg His Met Arg Thr Val Thr Asn Met
 65                  70                  75                  80
Phe Ile Leu Asn Leu Ala Val Ser Asp Leu Leu Val Gly Ile Phe Cys
                 85                  90                  95
Met Pro Thr Thr Leu Val Asp Asn Leu Ile Thr Gly Trp Pro Phe Asp
            100                 105                 110
Asn Ala Thr Cys Lys Met Ser Gly Leu Val Gln Gly Met Ser Val Ser
        115                 120                 125
Ala Ser Val Phe Thr Leu Val Ala Ile Ala Val Glu Arg Phe Arg Cys
    130                 135                 140
Ile Val His Pro Phe Arg Glu Lys Leu Thr Leu Arg Lys Ala Leu Leu
145                 150                 155                 160
Thr Ile Ala Val Ile Trp Ala Leu Ala Leu Leu Ile Met Cys Pro Ser
                165                 170                 175
Ala Val Thr Leu Thr Val Thr Arg Glu Glu His His Phe Met Leu Asp
            180                 185                 190
Ala Arg Asn Arg Ser Tyr Pro Leu Tyr Ser Cys Trp Glu Ala Trp Pro
        195                 200                 205
Glu Lys Gly Met Arg Lys Val Tyr Thr Ala Val Leu Phe Ala His Ile
    210                 215                 220
Tyr Leu Ala Pro Leu Ala Leu Ile Val Val Met Tyr Ala Arg Ile Ala
225                 230                 235                 240
Arg Lys Leu Cys Gln Ala Pro Gly Pro Ala Arg Asp Ala Glu Glu Ala
                245                 250                 255
Val Ala Glu Gly Gly Arg Ala Ser Arg Arg Arg Ala Arg Val Val His
            260                 265                 270
Met Leu Val Met Val Ala Leu Phe Phe Thr Leu Ser Trp Leu Pro Leu
        275                 280                 285
Trp Val Leu Leu Leu Leu Ile Asp Tyr Gly Glu Leu Ser Glu Leu Gln
    290                 295                 300
Leu His Leu Leu Ser Val Tyr Ala Phe Pro Leu Ala His Trp Leu Ala
305                 310                 315                 320
Phe Phe His Ser Ser Ala Asn Pro Ile Ile Tyr Gly Tyr Phe Asn Glu
                325                 330                 335
Asn Phe Arg Arg Gly Phe Gln Ala Ala Phe Arg Ala Gln Leu Cys Trp
            340                 345                 350
Leu Pro Trp Ala Ala His Lys Gln Ala Tyr Ser Glu Arg Pro Gly Arg
        355                 360                 365
Leu Leu Arg Arg Arg Val Val Val Asp Val Gln Pro Ser Asp Ser Gly
    370                 375                 380
Leu Pro Ser Glu Ser Gly Pro Ser Ser Gly Val Pro Gly Pro Asn Arg
385                 390                 395                 400
Leu Pro Leu Arg Asn Gly Arg Val Ala His Gln Asp Gly Pro Arg Glu
                405                 410                 415
Gly Pro Gly Cys Asn His Met Pro Leu Thr Ile Pro Ala Trp Asn Ile
            420                 425                 430
<210> 4
<211> 180
<212> PRT
<213> Homo sapiens
<400> 4
Met Glu Ile Ile Ser Ser Lys Leu Phe Ile Leu Leu Thr Leu Ala Thr
 1               5                  10                  15
Ser Ser Leu Leu Thr Ser Asn Ile Phe Cys Ala Asp Glu Leu Val Met
            20                  25                  30
Ser Asn Leu His Ser Lys Glu Asn Tyr Asp Lys Tyr Ser Glu Pro Arg
        35                  40                  45
Gly Tyr Pro Lys Gly Glu Arg Ser Leu Asn Phe Glu Glu Leu Lys Asp
    50                  55                  60
Trp Gly Pro Lys Asn Val Ile Lys Met Ser Thr Pro Ala Val Asn Lys
65                  70                  75                  80
Met Pro His Ser Phe Ala Asn Leu Pro Leu Arg Phe Gly Arg Asn Val
                85                  90                  95
Gln Glu Glu Arg Ser Ala Gly Ala Thr Ala Asn Leu Pro Leu Arg Ser
            100                 105                 110
Gly Arg Asn Met Glu Val Ser Leu Val Arg Arg Val Pro Asn Leu Pro

115                 120                 125
Gln Arg Phe Gly Arg Thr Thr Thr Ala Lys Ser Val Cys Arg Met Leu
    130                 135                 140
Ser Asp Leu Cys Gln Gly Ser Met His Ser Pro Cys Ala Asn Asp Leu
145                 150                 155                 160
Phe Tyr Ser Met Thr Cys Gln His Gln Glu Ile Gln Asn Pro Asp Gln
                165                 170                 175
Lys Gln Ser Arg
            180

## Description

### Technical Field

The present invention relates to an agent for modulating the function of an RFRP receptor having a fused 7-membered ring skeleton, a representative of which is benzoxazepine, that is useful as a medicament such as an analgesic.

### Background Art

Secretion peptides called RFRP-1, RFRP-2 and RFRP-3, and a G protein conjugated-type receptor protein OT7T022 (hereinafter, abbreviated as RFRP receptor) to which the peptide is bound are known (WO 00/29441).

It is known that RFRP-1, RFRP-2 and RFRP-3 have prolactin secretion modulating activity (WO 01/66134).

It is known that RFRP-1 inhibits analgesic activity of morphine (Journal of Biological Chemistry, vol. 276, No. 40, p36961-36969, 2001).

It is known that a benzoxazepine derivative has somatostatin receptor agonist activity, and is useful as a diabetic drug (WO 98/47882), has squalene synthesis inhibiting activity (EP567029, WO 97/10224), has ataractic activity (JP 57-35576 A), and has osteoporosis treating effect (WO 93/17129), but it is not known that a benzoxazepine derivative is bound to an RFRP receptor.

An object of the present invention is to provide a synthetic compound having excellent antagonism for an RFRP receptor.

### Disclosure of the Invention

In order to solve the aforementioned object, the present inventors intensively continued to study and, as a result, found that a compound having a fused 7-memberd ring skeleton or a salt thereof, a representative of which is benzoxazepine, has unexpectedly excellent RFRP receptor antagonism based on its peculiar chemical structure, further has excellent nature in physical properties as a medicament such as stability, and becomes a drug which is safe and useful as an analgesic, resulting in completion of the present invention based on these findings.

That is, the present invention relates to:
(1) an agent for modulating the function of an RFRP receptor, which comprises a compound represented by the formula: 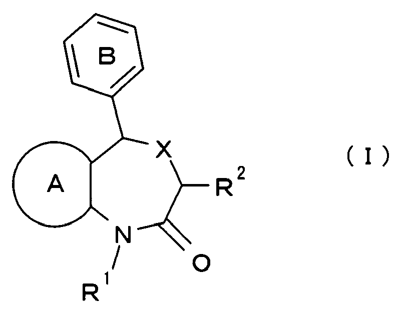
   wherein a ring A represents an optionally substituted aromatic ring, a ring B represents an optionally substituted benzene ring, X represents O, S(O)n (n represents an integer of 0 to 2) or NR³ (R³ represents a hydrogen atom, an optionally substituted hydrocarbon group, or an optionally substituted heterocyclic group), and R¹ and R² each represents a hydrogen atom, an optionally substituted hydrocarbon group, or an optionally substituted heterocyclic group, or a salt thereof, or a prodrug thereof,
(2) the agent according to the above (1), which comprises a compound represented by the formula: 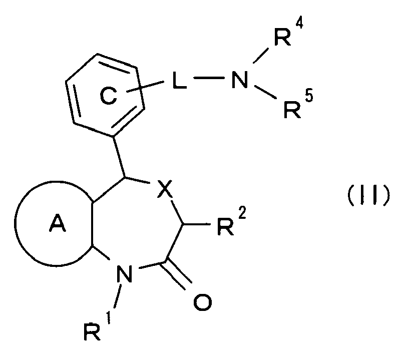
   wherein L represents a linker, R⁴ and R⁵ each represents a hydrogen atom, an optionally substituted hydrocarbon group, an acyl group, an optionally substituted carbamoyl group, an esterified carboxyl group, or an optionally substituted heterocyclic group, R⁴ and R⁵ may be taken together to form a ring, or a R⁴ or R⁵ may be taken together with a linker represented by L to form a ring, a ring C represents an optionally further substituted benzene ring, and the other symbols are as defined in the above (1), or a salt thereof, or a prodrug thereof,
(3) the agent according to the above (1), which comprises a compound represented by the formula: 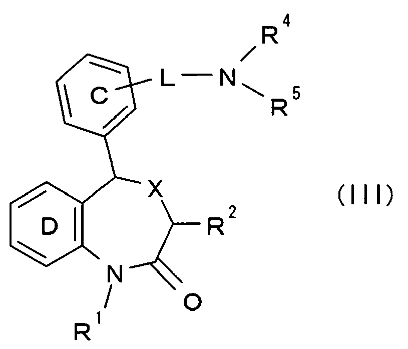
   wherein a ring D represents an optionally substituted benzene ring, L represents a linker, R⁴ and R⁵ each represents a hydrogen atom, an optionally substituted hydrocarbon group, an acyl group, an optionally substituted carbamoyl group, an esterified carboxyl group, or an optionally substituted heterocyclic group, R⁴ and R⁵ may be taken together to form a ring, or R⁴ or R⁵ may be taken together with a linker represented by L to form a ring, a ring C represents an optionally further substituted benzene ring, and the other symbols are as defined in the above (1), or a salt thereof, or a prodrug thereof,
(4) the agent according to the above (1), which comprises a compound represented by the formula: 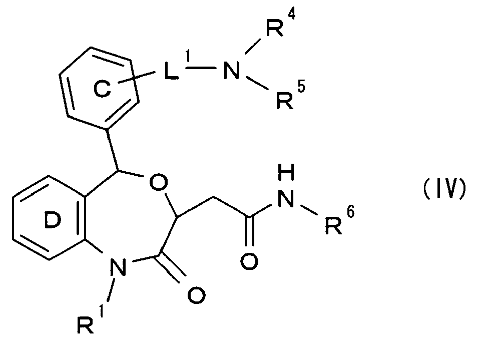
   wherein a ring D represents an optionally substituted benzene ring, L¹ represents a linker represented by optionally substituted -Y-(CH₂)m- (Y represents a bond, -O-, -S(O)n¹- (n¹ represents an integer of 0 to 2) or -NR⁷ - (R⁷ represents a hydrogen atom, an optionally substituted hydrocarbon group, or an optionally substituted heterocyclic group), and m represents an integer of 0 to 6), R⁴ and R⁵ each represents a hydrogen atom, an optionally substituted hydrocarbon group, an acyl group, an optionally substituted carbamoyl group, an esterified carboxyl group or an optionally substituted heterocyclic group, R⁴ and R⁵ may be taken together to form a ring, or R⁴ or R⁵ may be taken together with a linker represented by L¹ to form a ring, a ring C represents an optionally further substituted benzene ring, R⁶ represents an optionally substituted hydrocarbon group, or an optionally substituted heterocyclic group, and the other symbols are as defined in the above (1), or a salt thereof, or a prodrug thereof,
(5) the agent according to the above (1), which is an analgesic, an agent for promoting analgesic activity of another analgesic drug, or an agent for avoiding resistance due to another analgesic drug,
(6) the agent according to the above (1), which is an agent for modulating the prolactin secretion,
(7) the agent according to the above (1), which is an agent for preventing or treating hyperprolactinemia, pituitary gland tumor, diencephalons tumor, emmeniopathy, stress, autoimmune disease, prolactinoma, infertility, impotence, amenorrhea, galactic leakage, acromegaly, Chiari-Frommel syndrome, Argonz-del Castilo syndrome, Forbes-Albright syndrome, breast cancer lymphoma, Sheehan's syndrome or spermatogenesis abnormality,
(8) the agent according to the above (1), which is an agent for suppressing the pancreatic glucagon secretion, an agent for lowering a blood glucose or an agent for suppressing the urine production,
(9) the agent according to the above (1), which is an agent for preventing or treating diabetes, glucose tolerance disorder, ketosis, acidosis, diabetic neuropathy, diabetic nephropathy, diabetic retinopathy, pollakiuria, nocturnal enarusis, hyperlipemia, sexual function disorder, skin disease, arthritis, osteopenia, arteriosclerosis, thrombotic disease, maldigestion or memory and learning disabilities,
(10) the agent according to the above (1), which is an agent for suppressing the bladder constriction,
(11) the agent according to the above (1), which is an agent for preventing or treating urine incontinence, lower uropathy, urge micturition due to excessive active bladder, or hypotonic bladder accompanied with excessive active bladder,
(12) a compound represented by the formula: 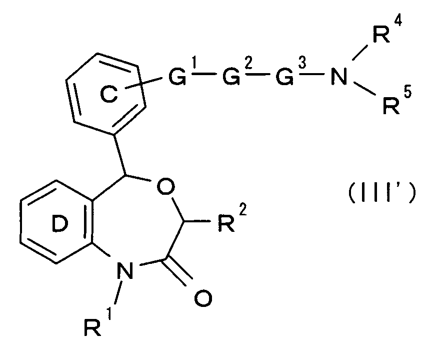
   wherein a ring D represents an optionally substituted benzene ring, G¹ represents a bond, or an optionally substituted divalent hydrocarbon group, G² represents -O-, -NR⁸- (R⁸ represents a hydrogen atom, an optionally substituted hydrocarbon group, or an optionally substituted heterocyclic group) or -S(O)n²- (n² represents an integer of 0 to 2), G³ represents an optionally substituted divalent hydrocarbon group, R⁴and R⁵ each represents a hydrogen atom, an optionally substituted hydrocarbon group, an acyl group, an optionally substituted carbamoly group, an esterified carboxyl group, or an optionally substituted heterocyclic group, a ring C represents an optionally further substituted benzene ring, R¹ and R² each represents a hydrogen atom, an optionally substituted hydrocarbon group, or an optionally substituted heterocyclic group, R⁴ may be taken together with G³ or R⁵to form a ring and, when G² is -NR⁸-, R⁴ and R⁸ may be taken together to form a ring, provided that 3,5-trans-N-(2-fluorobenzyl)-5-[3-(3-tert-butoxycarbonylaminopropyl)aminomethylphenyl]-7-chloro-1-neopentyl-2-oxo-1,2,3,5-tetrahydro-4,1-benzooxazepine-3-acetamide, 3,5-trans-N-(2-fluorobenzyl)-5-[3-(3-aminopropyl)aminomethylphenyl]-7-chloro-1-neopentyl-2-oxo-1,2,3,5-tetrahydro-4,1-benzooxazepine-3-acetamide, 3,5-trans-N-(2-fluorobenzyl)-5-(3-aminoacetylaminomethylphenyl)-1-benzyl-7-chloro-2-oxo-1,2,3,5-tetrahydro-4,1-benzooxazepine-3-acetamide, 3,5-trans-N-(2-fluorobenzyl)-1-(4-biphenylmethyl)-7-chloro-2-oxo-5-[3-[(piperidin-4-yl)carbonylaminomethyl]phenyl]-1,2,3,5-tetrahydro-4,1-benzooxazepine-3-acetamide, 3,5-trans-N-(2-fluorobenzyl)-5-[2-(3-aminopropyloxy)phenyl]-7-chloro-1-isobutyl-2-oxo-1,2,3,5-tetrahydro-4,1-benzooxazepine-3-acetamide, 3,5-trans-N-(2-fluorobenzyl)-5-[4-(3-aminopropyloxy)-2-methoxyphenyl]-7-chloro-1-neopentyl-2-oxo-1,2,3,5-tetrahydro-4,1-benzooxazepine-3-acetamide, 7-chloro-5-[2-[3-[[(1,1-dimethylethoxy)carbonyl]amino]propoxy]phenyl]-1,2,3,5-tetrahydro-1-(2-methylpropyl)-2-oxo-4,1-benzooxazepin-3-ylacetic acid ethyl ester, and 7-chloro-5-[4-[3-[[(1,1-dimethylethoxy)carbonyl]amino]propoxy]-2-methoxyphenyl]-1-(2,2-dimethylpropyl)]-1,2,3,5-tetrahydro-2-oxo-4,1-benzooxazepin-3-ylacetic acid ethyl ester are excluded], or a salt thereof,
(13) the compound according to the above (12), which is represented by the formula: 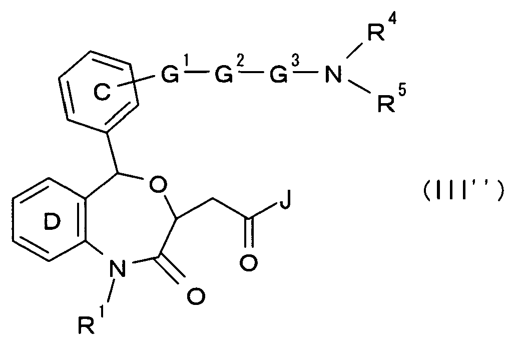
   wherein J represents an optionally substituted hydroxyl group, or an optionally substituted amino group, and the other symbols are as defined in the above (12),
(14) the compound according to the above (12), which is represented by the formula: 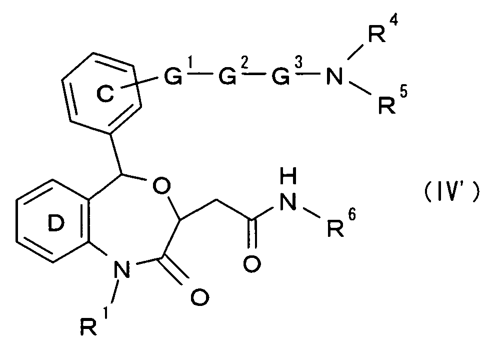
   wherein R⁶ represents an optionally substituted hydrocarbon group, or an optionally substituted heterocyclic group, and the other symbols are as defined in the above (12),
(15) the compound according to the above (12), wherein G¹ is a bond, or an optionally substituted C₁₋₃ alkylene group,
(16) the compound according to the above (12), wherein G³ is an optionally substituted C₂₋₆ alkylene group,
(17) the compound according to the above (12), wherein G¹ is a bond, and G² is -O-,
(18) the compound according to the above (12), wherein R¹ is an optionally substituted hydrocarbon group,
(19) the compound according to the above (12), wherein R¹ is an optionally substituted C₁₋₈ alkyl group, or an optionally substituted C₇₋₁₆ aralkyl group,
(20) the compound according to the above (12), wherein R⁴ is a hydrogen atom,
(21) the compound according to the above (12), wherein R⁵ is an optionally substituted C₁₋₆ alkyl group, or an optionally substituted C₇₋₁₆ aralkyl group,
(22) the compound according to the above (13), wherein J is a hydroxyl group, an optionally substituted lower alkoxy group, an amino group optionally substituted with an optionally substituted alkyl group, or an optionally substituted cyclic amino group,
(23) the compound according to the above (13), wherein J is an optionally substituted 5- to 8- membered cyclic amino group,
(24) the compound according to the above (14), wherein R⁶ is an optionally substituted benzyl group, or an optionally substituted phenyl group,
(25) the compound according to the above (12), wherein G³ is an optionally substituted divalent hydrocarbon group other than a carbonyl group and, when R⁴ is a hydrogen atom, R⁵ is not a hydrogen atom or a tert-butyloxycarbonyl group,
(26) a compound represented by the formula: 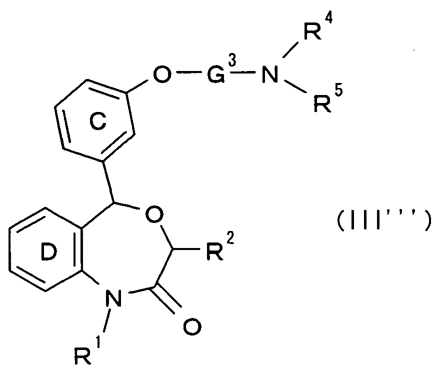
   wherein a ring D represents an optionally substituted benzene ring, G³ represents an optionally substituted divalent hydrocarbon group, R⁴ and R⁵ each represents a hydrogen atom, an optionally substituted hydrocarbon group, an acyl group, an optionally substituted carbamoyl group, an esterified carboxyl group, or an optionally substituted heterocyclic group, a ring C represents an optionally further substituted benzene ring, R¹ and R² each represents an hydrogen atom, an optionally substituted hydrocarbon group, or an optionally substituted heterocyclic group, and R⁴ may be taken together with G³ or R⁵ to form a ring, or a salt thereof,
(27) 2-(3,5-trans-7-Chloro-1-(2,2-dimethylpropyl)-5-{2-methoxy-3-[3-(3-phenylpropylamino)propoxy]phenyl}-2-oxo-1,2,3,5-tetrahydro-4,1-benzooxazepin-3-yl)-N-(2-fluorobenzyl)acetamide, 2-{3,5-trans-7-chloro-1-(2,2-dimethylpropyl)-5-[2-methoxy-3-(3-(pentylamino)propoxy)phenyl]-2-oxo-1,2,3,5-tetrahydro-4,1-benzooxazepin-3-yl}-N-(2-fluorobenzyl)acetamide, trans-2-{7-chloro-5-[3-(3-{[3-(2-chlorophenyl)propyl]amino}propoxy)-2-methoxyphenyl]-1-(2,2-dimethylpropyl)-2-oxo-1,2,3,5-tetrahydro-4,1-benzooxazepin-3-yl}-N-(2-fluorobenzyl)acetamide, trans-2-[7-chloro-1-(2,2-dimethylpropyl)-5-[2-methoxy-3-(3-{[(2E)-3-phenyl-2-propenyl]amino}propoxy)phenyl]-2-oxo-1,2,3,5-tetrahydro-4,1-benzooxazepin-3-yl]-N-(2-fluorobenzyl)acetamide, trans-2-[7-chloro-1-(2,2-dimethylpropyl)-5-(2-methoxy-3-{3-[(3-phenylpropyl)amino]propoxy}phenyl)-2-oxo-1,2,3,5-tetrahydro-4,1-benzooxazepin-3-yl]-N-propylacetamide, trans-7-chloro-1-(2,2-dimethylpropyl)-5-(2-methoxy-3-{3-[(3-phenylpropyl)amino]propoxy}phenyl)-3-[2-oxo-2-(1-piperazinyl)ethyl]-1,5-dihydro-4,1-benzooxazepine-2(3H)-one, trans-7-chloro-1-(2,2-dimethylpropyl)-3-[2-(4-hydroxypiperidin-1-yl)-2-oxoethyl]-5-(2-methoxy-3-{3-[(3-phenylpropyl)amino]propoxy}phenyl)-1,5-dihydro-4,1-benzooxazepine-2(3H)-one or 4-{[3,5-trans-7-chloro-1-(2,2-dimethylpropyl)-5-(2-methoxy-3-{3-[(3-phenylpropyl)amino]propoxy}phenyl)-2-oxo-1,2,3,5-tetrahydro-4,1-benzooxazepin-3-yl]acetyl}piperazine-2-carboxylic acid, or a salt thereof,
(28) a prodrug of the compound according to the above (12) or (26),
(29) a drug containing the compound according to the above (12) or (26) or a prodrug thereof,
(30) the drug according to the above (29), which is an agent for preventing or treating RFRP-associated with morbid state or a disease involved in RFRP,
(31) a method of modulating the function of an RFRP receptor, which comprises administering an effective amount of a compound represented by the formula: 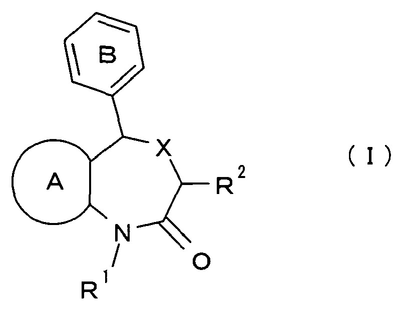
   wherein a ring A represents an optionally substituted aromatic ring, a ring B represents an optionally substituted benzene ring, X represents O, S(O)n (n represents an integer of 0 to 2) or NR³ (R³ represents a hydrogen atom, an optionally substituted hydrocarbon group, or an optionally substituted heterocyclic group), and R¹ and R ² each represents a hydrogen atom, an optionally substituted hydrocarbon group, or an optionally substituted heterocyclic ring, or a salt thereof, or a prodrug thereof to a mammal, and
(32) use of a compound represented by the formula: 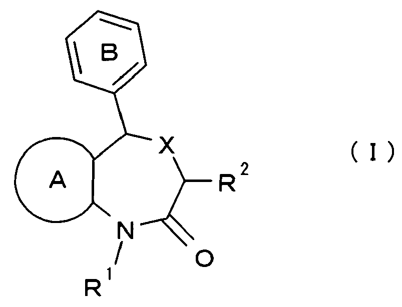
   wherein a ring A represents an optionally substituted aromatic ring, a ring B represents an optionally substituted benzene ring, X represents O, S(O)n (n represents an integer of 0 to 2) or NR³ (R³ represents a hydrogen atom, an optionally substituted hydrocarbon group, or an optionally substituted heterocyclic group), and R¹ and R² each represents a hydrogen atom, an optionally substituted hydrocarbon group, or an optionally substituted heterocyclic group, or a salt thereof, or a prodrug thereof for preparing an agent for modulating the function of an RFRP receptor.
   Further, the present invention relates to:
(33) the agent according to the above (1), wherein the ring A is (i) an aromatic hydrocarbon ring of a carbon number of 6 to 14, or (ii) a 5- to 14- membered aromatic heterocyclic containing 1 to 4 of 1 or 2 kinds of heteroatoms selected from a nitrogen atom, a sulfur atom and an oxygen atom in addition to a carbon atom, which may be substituted with a substituent selected from a substituent A group,
   the substituent A group is a group consisting of:
   (i) a halogen atom,
   (ii) a nitro group,
   (iii) a cyano group
   (iv) a straight or branched C₁₋₁₅ alkyl group, a C₃₋₁₀ cycloalkyl group, a C₂₋₁₈ alkenyl group, a C₃₋₁₀ cycloalkenyl group, a C₂₋₈ alkynyl group, a C₇₋₁₆ aralkyl group, a C₆₋₁₄ aryl group, a biphenyl group, a trityl group or a tolyl group, which may be substituted with a substituent selected from a substituent B group [a nitro group, a hydroxyl group, an oxo group, a cyano group, a carbamoyl group, a mono- or di-C₁₋₆ alkyl-carbamoyl group (the alkyl group may be substituted with a halogen atom, a hydroxyl group, or a C₁₋₆ alkoxy group), a mono- or di-C₂₋₆ alkenyl-carbamoyl group (the alkenyl group may be substituted with a halogen atom, a hydroxyl group, or a C₁₋₆ alkoxy group), a mono- or di-C₆₋14 aryl-carbamoyl group, a mono- or di-C₇₋₁₆ aralkyl-carbamoyl group, a C₁₋₆ alkoxy-carbonyl-carbamoyl group, a C₁₋₆ alkylsulfonyl-carbamoyl group, a C₁₋₆ alkoxy-carbamoyl group, an amino-carbamoyl group, a mono- or di-C₁₋₆ alkylamino-carbamoyl group, a mono- or di-C₆₋₁₄ arylamino-carbamoyl group, a carboxyl group, a C₁₋₆ alkoxy -carbonyl group, a sulfo group, a halogen atom, an optionally halogenated C₁₋₆ alkoxy group, a C₁₋₆ alkoxy group optionally substituted with a hydroxyl group, a C₁₋₆ alkoxy group optionally substituted with a carboxyl group, a C₁₋₆ alkoxy group optionally substituted with a C₁₋₆ alkoxy-carbonyl group, a C₁₋₆ alkoxy-C₁₋₆ alkoxy group, a C₁₋₆ alkoxy-C₁₋₆ alkoxy-C₁₋₆ alkoxy group, a C₆₋₁₄ aryloxy group, a C₆₋₁₄ aryloxy-C₁₋₆ alkyl group, a C₆₋₁₄ aryloxy-C₁₋₆ alkoxy group, a C₁₋₆ alkylcarbonyl-oxy group, a carbamoyloxy group, a mono or -di-C₁₋₆ alkyl-carbamoyloxy group, an optionally halogenated C₆₋₁₄ aryl group (e.g. a phenyl group, 1- or 2-naphthyl), an optionally halogenated C₆₋₁₄ aryl-C₁₋₆ alkyl group, an optionally halogenated C₆₋₁₄ aryl-C₂₋₆ alkenyl group, an optionally halogenated C₆₋₁₄ aryloxy group, a 5-to 10- membered heterocyclic-oxy group containing at least one 1 to 3 kinds of heteroatoms selected from an oxygen atom, a sulfur atom and a nitrogen atom (the heterocyclic group may be substituted with a C₁₋₆ alkyl group), a C₃₋₁₀ cycloalkyl group, a C₃₋₁₀ cycloalkyl-C₁₋₆ alkoxy group, a C₃₋₁₀ cycloalkyl-C₁₋₆ alkyl group, an optionally halogenated C₁₋₆ alkyl group, an optionally halogenated C₂₋₆ alkenyl group, an optionally halogenated C₁₋₆ alkylthio group, a C₁₋₆ alkyl group optionally substituted with a hydroxyl group, a C₁₋₆ alkylthio group optionally substituted with a hydroxy group, a mercapto group, a thioxo group, a C₇₋₁₆ aralkyloxy group (optionally substituted with a substituent selected from a halogen atom, a carboxyl group and a C₁₋₆ alkoxy-carbonyl group) or a C₇₋₁₆ aralkylthio group (optionally substituted with a substituent selected from a halogen atom, a carboxyl group and a C₁₋₆ alkoxy-carbonyl group), an optionally halogenated C₆₋₁₄ arylthio group, a 5- to 10-membered heterocyclic-thio group containing at lease one 1 to 3 kinds of heteroatoms selected from an oxygen atom, a sulfur atom, and a nitrogen atom (the heterocyclic group may be substituted with a C₁₋₆ alkyl group), a C₆₋₁₄ arylthio-C₁₋₆ alkyl group, a 5- to 10-membered heterocyclic-thio-C₁₋₆ alkyl group containing at least one 1 to 3 kinds of heteroatoms selected from an oxygen atom, a sulfur atom and a nitrogen atom (the heterocyclic group may be substituted with a C₁₋₆ alkyl group), an optionally halogenated C₁₋₆ alkylsulfinyl group, a C₆₋₁₄ arylsulfinyl group, a C₆₋₁₄ arylsulfinyl-C₁₋₆ alkyl group, an optionally halogenated C₁₋₆ alkylsulfonyl group, a C₆₋₁₄ arylsulfonyl group, a C₆₋₁₄ arylsulfonyl-C₁₋₆ alkyl group, an amino group, an aminosulfonyl group, a mono- or di-C₁₋₆ alkylaminosulfonyl group (the alkyl group may be substituted with a halogen atom, a hydroxy group, or a C₁₋₆ alkoxy group), a C₁₋₁₀ alkanoyl-amino group (the C₁₋₁₀ alkanoyl may be substituted with a halogen atom, a hydroxy group, or a carboxyl group), a C₃₋₁₀ alkenoylamino group (the C₃₋₁₀ alkenoyl may be substituted with a halogen atom, a hydroxy group, or a carboxyl group), a C₃₋₁₀ cycloalkylcarbonylamino group (the C₃₋₁₀ cycloalkylcarbonyl may be substituted with a halogen atom, a hydroxy group, or a carboxyl group), a C₆₋₁₄ arylcarbonylamino group (the C₆₋₁₄ arylcarbonyl may be substituted with a halogen atom, a hydroxy group, or a carboxyl group), a C₁₋₆ alkylsulfonylamino group (the C₁₋₆ alkylsulfonyl may be substituted with a halogen atom, a hydroxy group, or a carboxyl group), a C₆₋₁₄ arylsulfonylamino group (the C₆₋₁₄ arylsulfonyl may be substituted with a halogen atom, a hydroxyl group, or a carboxyl group), a C₇₋₁₄ aralkyloxycarbonylamino group, an optionally halogenated C₁₋₆ alkoxycarbonylamino group, a carbamoylamino group, a mono- or di-C₁₋₆ alkylcarbamoylamino group, a mono- or di-C₁₋₆ alkylamino group (the alkyl group may be substituted with a halogen atom, a hydroxy group, or a C₁₋₆ alkoxy group), a mono- or di-C₁₋₆ alkanoylamino group (the alkanoyl group may be substituted with a halogen atom, a hydroxy group, or a C₁₋₆ alkoxy group), a C₆₋₁₄ arylamino group, a C₇₋₁₆ aralkylamino group, a C₁₋₆ alkyl (C₇₋₁₆ aralkyl) amino group, a C₁₋₆ alkanoyl (C₇₋₁₆ aralkyl) amino group, a 3- to 8-membered cyclic amino group, a 3- to 8-membered cyclic amino-carbonyl group, a 3- to 8-membered cyclic amino carbonyl-oxy group, a 3- to 8-membered cyclic amino-carbonyl-amino group, a 3- to 8-membered cyclic amino-sulfonyl group, a 3- to 8-membered cyclic amino-C₁₋₆ alkyl group, a C₁₋₆ alkanoyl group (optionally substituted with a substituent selected from a halogen atom, amino, a carboxyl group and a C₁₋₆ alkoxy-carbonyl group), a C₆₋₁₄ aryl-carbonyl group (optionally substituted with a substituent selected from a halogen atom, a carboxyl group and a C₁₋₆ alkoxy-carbonyl group), a C₇₋₁₆ aralkyl-carbonyl group (optionally substituted with a substituent selected from a halogen atom, amino, a carboxyl group and a C₁₋₆ alkoxy-carbonyl group), a 5- to 10-membered heterocyclic group containing at least one 1 to 3 kinds of heteroatoms selected from an oxygen atom, a sulfur atom and a nitrogen atom (the heterocyclic group may be substituted with a C₁₋₆ alkyl group), a 5- to 10-membered heterocyclic-carbonyl group containing at least one 1 to 3 kinds of heteroatoms selected from an oxygen atom, a sulfur atom and a nitrogen atom (the heterocyclic group may be substituted with a C₁₋₆ alkyl group), a hydroxyimino group, a C₁₋₆ alkoxyimino group, an optionally halogenated straight or branched C₁₋₄ alkylenedioxy group, an ureido group, a C₁₋₆ alkyl-ureido group, and a C₁₋₆ alkyl group optionally substituted with a halogen atom] (hereinafter, optionally substituted hydrocarbon group),
   (v) a 5- to 16-membered monocyclic to tricyclic aromatic heterocyclic group containing at least one 1 to 3 kinds of heteroatoms selected from an oxygen atom, a sulfur atom, and a nitrogen atom, or a 3- to 8-membered saturated or unsaturated non-aromatic heterocyclic group, which may be substituted with a substituent selected from the substituent B group (hereinafter, optionally substituted heterocyclic group),
   (vi) a hydroxy group optionally substituted with a substituent selected from the group consisting of:
      (a) the aforementioned optionally substituted hydrocarbon group,
      (b) a group represented by R^{A}CO-, R^{A}OCO-, R^{A}SO₂-, R^{A}SO-or R^{A}OPO(OR^{B})-(R^{A}represents (aa) a hydrogen atom, (bb) the aforementioned optionally substituted hydrocarbon group or (cc) the aforementioned optionally substituted heterocyclic group, and R^{B}represents (aa) a hydrogen atom or (bb) the aforementioned optionally substituted hydrocarbon group) (hereinafter, acyl group),
      (c) a group represented by the formula -COOR^{C} (R^{C} represents (aa) a hydrogen atom, (bb) the aforementioned optionally substituted hydrocarbon group or (cc) the aforementioned optionally substituted heterocyclic group) (hereinafter, optionally esterified carboxyl group),
      (d) a carbamoyl group optionally substituted with a substituent selected from the group consisting of:
         (aa) the aforementioned optionally substituted hydrocarbon group,
         (bb) the aforementioned acyl group,
         (cc) the aforementioned optionally esterified carboxyl group,
         (dd) a carbamoyl group optionally substituted with 1 to 2 substituents selected from a C₁₋₆ alkyl group and a C₆₋₁₄ aryl group, and
         (ee) the aforementioned optionally substituted heterocyclic group (hereinafter, optionally substituted carbamoyl group), and
      (e) the aforementioned optionally substituted heterocyclic group
         (hereinafter, optionally substituted hydroxyl group),
   (vii) a thiol group optionally substituted with a substituent selected from the group consisting of:
      (a) the aforementioned optionally substituted hydrocarbon group,
      (b) the aforementioned acyl group,
      (c) the aforementioned optionally esterified carboxyl group,
      (d) the aforementioned optionally substituted carbamoyl group, and
      (e) the aforementioned optionally substituted heterocyclic group
         (hereinafter, optionally substituted thiol group),
   (viii) a sulfinyl group substituted with a substituent selected from the group consisting of:
      (a) the aforementioned optionally substituted hydroxy group,
      (b) the following optionally substituted amino group,
      (c) the aforementioned optionally substituted hydrocarbon group, and
      (d) the aforementioned optionally substituted heterocyclic group
         (hereinafter, substituted sulfinyl group),
   (ix) a sulfonyl group substituted with a substituent selected from the group consisting of:
      (a) the aforementioned optionally substituted hydroxy group,
      (b) the following optionally substituted amino group,
      (c) the aforementioned optionally substituted hydrocarbon group, and
      (d) the aforementioned optionally substituted heterocyclic group
         (hereinafter, substituted sulfonyl group),
   (x) an amino group or a 3- to 8-membered cyclic amino group optionally substituted with a substituent selected from the group consisting of:
      (a) the aforementioned optionally substituted hydrocarbon group,
      (b) the aforementioned acyl group,
      (c) the aforementioned optionally esterified carboxyl group,
      (d) the aforementioned optionally substituted carbamoyl group, and
      (e) the aforementioned optionally substituted heterocyclic group,
         (hereinafter, optionally substituted amino group),
   (xi) the aforementioned acyl group,
   (xii) the aforementioned optionally substituted carbamoyl group,
   (xiii) the aforementioned optionally esterified carboxyl group, and
   (xiv) a C₁₋₃ alkylenedioxy group,
      the ring B is a benzene ring optionally substituted with a substituent selected from the substituent A group,
      X is:
      (i) O,
      (ii) S(O)n (n represents an integer of 0 to 2), or
      (iii) NR³
      (R³ represents:
         (a) a hydrogen atom,
         (b) the aforementioned optionally substituted hydrocarbon group, or
         (c) the aforementioned optionally substituted heterocyclic group),

      R¹ is:
      (i) a hydrogen atom,
      (ii) the aforementioned optionally substituted hydrocarbon group, or
      (iii) the aforementioned optionally substituted heterocyclic group,
      R² is:
      (i) a hydrogen atom
      (ii) a straight or branched C₁₋₁₅ alkyl group, a C₃₋₁₀ cycloalkyl group, a C₂₋₁₈ alkenyl group, a C₃₋₁₀ cycloalkenyl group, a C₂₋₈ alkynyl group, a C₇₋₁₆ aralkyl group, a C₆₋₁₄ aryl group, a biphenyl group or a tolyl group, which may be substituted with a substituent selected from the group consisting of:
         (a) a substituent B group,
         (b) a group represented by the formula -CONR¹²(R¹³)
            (R¹² represents:
            (aa) a hydrogen atom,
            (bb) the aforementioned optionally substituted hydrocarbon group (e.g. a C₁₋₆ alkyl group optionally substituted with a substituent selected from a substituent B group, a C₆₋₁₄ aryl group optionally substituted with a substituent selected from a substituent B group, or a C₇₋₁₆ aralkyl group optionally substituted with a substituent selected from the substituent B group),
            (cc) the aforementioned optionally substituted heterocyclic group,

            (R¹³ represents a hydrogen atom or a C₁₋₆ alkyl group)
         (c) a group represented by the formula -COO-R¹⁴,
            (R¹⁴ is:
            (aa) a hydrogen atom, or
            (bb) a C₁₋₆ alkyl group optionally substituted with a substituent selected from the substituent B group),
         (d) a group represented by the formula -NR¹⁵(R¹⁶)
            (R¹⁵ represents:
            (aa) a hydrogen atom,
            (bb) a C₇₋₁₆ aralkyl group optionally substituted with a substituent selected from the substituent B group, or
            (cc) the aforementioned acyl group,
               (R¹⁶ represents a hydrogen atom or a C₁₋₆ alkyl group),
         (e) a group represented by the formula -CO-Q (Q represents a 5- to 8-membered nitrogen-containing ring group optionally substituted with a substituent selected from the substituent B group),
         (f) a group represented by the formula -NH-CO-NR¹⁹(R²⁰)
            (R¹⁹ represents:
            (aa) a hydrogen atom, or
            (bb) a C₆₋₁₄ aryl group optionally substituted with a substituent selected from the substituent B group,

            (R²⁰ represents:
            (aa) a hydrogen atom, or
            (bb) a C₁₋₆ alkyl group optionally substituted with a substituent selected from the substituent B group),
         (g) a group represented by the formula -CO-J
            (J represents:
            (aa) the aforementioned optionally substituted hydroxy group, or
            (bb) the aforementioned optionally substituted amino group),
      (iii) the aforementioned optionally substituted heterocyclic group,
(34) the compound according to the above (12), wherein the ring D is a benzene ring optionally substituted with a substituent selected from the substituent A group,
   G¹ is:
   (i) a bond, or
   (ii) (a) a C₁₋₆ alkylene group, (b) a C₂₋₆ alkenylene group, (c) a C₂₋₆ alkynylene group, (d) a group obtained by removing a hydrogen atom from two carbon atoms of a C₆₋₁₄ aryl ring, or (e) a group obtained by removing a hydrogen atom of two carbon atoms of a C₃₋₈ cycloalkane, which may be substituted with a substituent selected from the substituent B group,

   G² is:
   (i) -O-,
   (ii) -NR⁸-
      (R⁸ represents:
      (a) a hydrogen atom,
      (b) the aforementioned optionally substituted hydrocarbon group, or
      (c) the aforementioned optionally substituted heterocyclic group), or
   (iii) -S(O)n²- (n² represents an integer of 0 to 2),
      G³ is (a) a C₁₋₆ alkylene group, (b) a C₂₋₆ alkenylene group, (c) a C₂₋₆ alkynylene group, (d) a group obtained by removing a hydrogen atom from two carbon atoms of a C₆₋₁₄ aryl ring, or (e) a group obtained by removing a hydrogen atom from two carbon atoms of C₃₋₈ cycloalkane, which may be substituted with a substituent selected from the substituent B group,

   R⁴ and R⁵ each is:
   (i) a hydrogen atom,
   (ii) the aforementioned optionally substituted hydrocarbon group,
   (iii) the aforementioned acyl group,
   (iv) the aforementioned optionally substituted carbamoyl group,
   (v) an esterified carboxyl group (the aforementioned optionally esterified carboxyl group other than a free carboxyl group), or
   (vi) the aforementioned optionally substituted heterocyclic group,
      the ring C is a benzene ring optionally further substituted with a substituent selected from the substituent A group,

   R¹ and R² each is:
   (i) a hydrogen atom,
   (ii) the aforementioned optionally substituted hydrocarbon group, or
   (iii) the aforementioned optionally substituted heterocyclic group,
      R⁴ may be taken together with G³ or R⁵ to form a 5- to 10-membered nitrogen-containing ring optionally containing 1 to 3 kinds of heteroatoms selected from an oxygen atom, a sulfur atom and a nitrogen atom in addition to carbon atoms and one nitrogen atom, which may be substituted with a substituent selected from the substituent B group,
      when G² is -NR⁸-, R⁴ and R⁸ may be taken together to form a 5- to 10-membered nitrogen-containing ring optionally containing 1 to 3 kinds of heteroatoms selected from an oxygen atom, a sulfur atom and a nitrogen atom in addition to carbon atoms and one nitrogen atom, which may be substituted with a substituent selected from the substituent B group,
(35) the compound according to the above (13), wherein J is the aforementioned optionally substituted hydroxy group or the aforementioned optionally substituted amino group,
(36) the compound according to the above (14), wherein R⁶ is the aforementioned optionally substituted hydrocarbon group or the aforementioned optionally substituted heterocyclic group,
(37) the compound according to the above (26), wherein a ring D is a benzene ring optionally substituted with a substituent selected from the substituent A group,
   G³ is (a) a C₁₋₆ alkylene group, (b) a C₂₋₆ alkenylene group, (c) a C₂₋₆ alkynylene group, (d) a group obtained by removing a hydrogen atom from two carbon atoms of a C₆₋₁₄ aryl ring, or (e) a group obtained by removing a hydrogen atom from two carbon atoms of C₃₋₈ cycloalkane, which may be substituted with a substituent selected from the substituent B group,
   R⁴ and R⁵ each is:
   (i) a hydrogen atom,
   (ii) the aforementioned optionally substituted hydrocarbon group,
   (iii) the aforementioned acyl group,
   (iv) the aforementioned optionally substituted carbamoyl group,
   (v) an esterified carboxyl group (the aforementioned optionally esterified carboxyl group other than a free carboxyl group), or
   (vi) the aforementioned optionally substituted heterocyclic group,
      the ring C is a benzene ring optionally substituted with a substituent selected from the substituent A group,

   R¹ and R² each is:
   (i) a hydrogen atom,
   (ii) the aforementioned optionally substituted hydrocarbon group, or
   (iii) the aforementioned optionally substituted heterocyclic group,

   R⁴ may be taken together with G³ or R⁵ to form a 5- to 10-membered nitrogen-containing ring optionally containing at least one of 3 kinds of heteroatoms selected from an oxygen atom, a sulfur atom, and a nitrogen atom in addition to carbon atoms and one nitrogen atom, which may be substituted with a substituent selected from the substituent B group.

### Brief Description of the Drawings

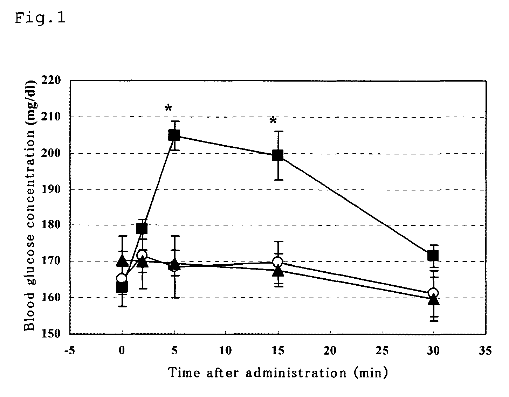

Fig. 1 shows results of investigation of a variation in a blood glucose concentration upon intravenous administration of RFRP-1 to a rat under no anesthesia. In the figure, (-O-) represents a blood glucose concentration of a physiological saline administration group, (-▲-) represents a blood glucose concentration of an RFRP-1 1 nmol/kg administration group, and (-■-) represents a blood concentration of an RFRP-1 10 nmol/kg administration group. A value indicates mean ±standard deviation (mean ± SE) (n = 4). * indicates that a P value is 0.05 or less as compared with a physiological saline administration group.

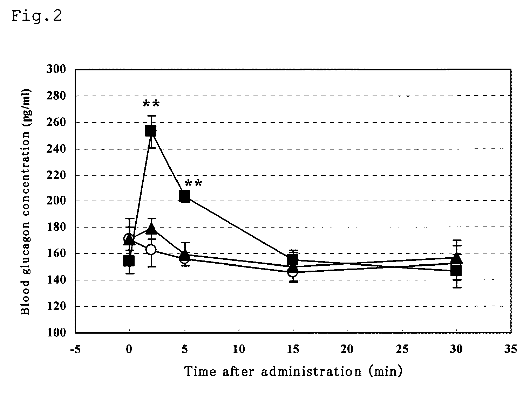

Fig. 2 shows results of investigation of a variation in a blood glucagon concentration upon oral administration of RFRP-1 to a rat under no anesthesia. In the figure, (-o-) represents a blood glucagon concentration of a physiological saline administration group, (-▲-) represents a blood glucagon concentration of an RFRP-1 1 nmol/kg administration group, and (-■-) represents a blood glucagon concentration of an RFRP-1 10 nmol/kg administration group. A value indicates mean ± standard deviation (mean ± SE) (n = 4). ** indicates that a P value is 0.01 or less as compared with a physiological saline administration group.

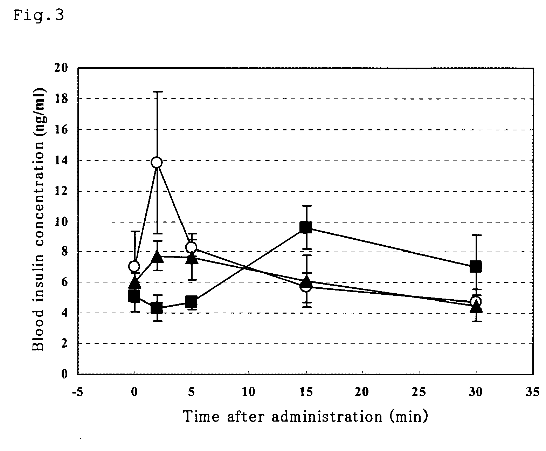

Fig. 3 shows results of investigation of a variation in a blood insulin concentration upon intravenous administration of RFRP-1 to a rat under no anesthesia. In the figure, (-o-) represents a blood insulin concentration of a physiological saline administration group, (-▲-) represents a blood insulin concentration of an RFRP-1 1 nmol/kg administration group, and (-■-) represents a blood insulin concentration of an RFRP-1 10 nmol/kg administration group. A value indicates mean ± standard deviation (mean ± SE) (n = 4).

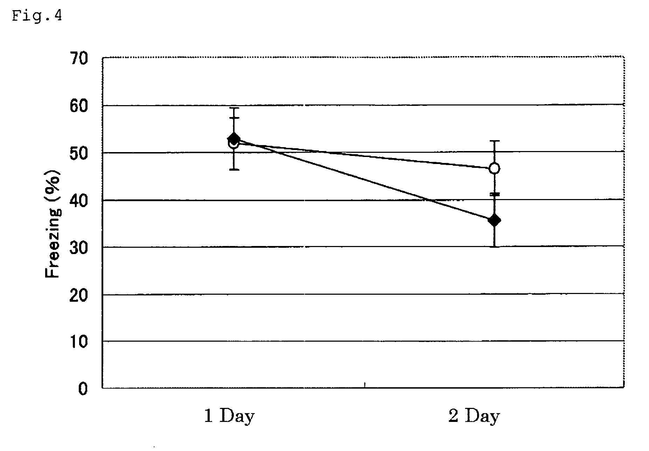

Fig. 4 indicates a ratio of freezing in a sound clue when RFRP-1 (-□-) and a physiological saline (-○-) are administered in a ventricle. An ordinate axis indicates freezing (%) of one day and two day after administration, respectively, expressed by mean ± standard error.

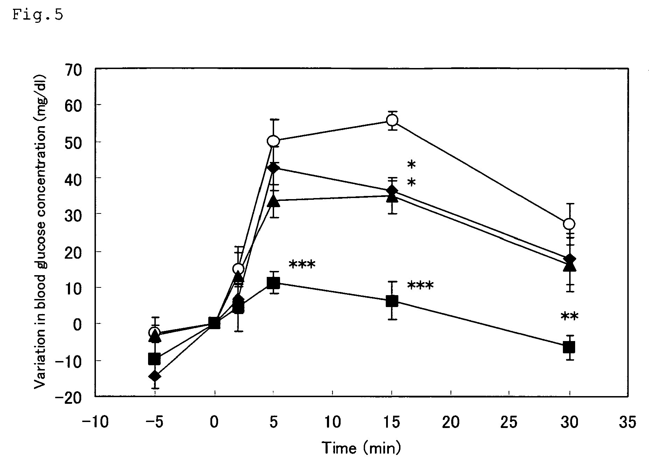

Fig. 5 shows results of investigation of a variation in a blood glucose concentration when a compound of Example 50 is intravenously administered to a rat under no anesthesia and, after five minutes, RFRP-1 is intravenously administered. In the figure, (-o-) represents a blood glucose concentration of a physiological saline administration group, (-□-) represents a blood glucose concentration of an Example 50 compound 0.2 mg/kg administration group, (-▲-) represents a blood glucose concentration of an Example 50 compound 1 mg/kg administration group, and (-■-) represents a blood glucose concentration of an Example 50 compound 5 mg/kg administration group, as expressed by a variation value from at a time point of administration of RFRP-1. A value indicates mean ± standard deviation (mean ± SE) (n = 4). * indicates that a P value is 0.05 or less as compared with a physiological saline administration group, ** indicates that a P value is 0.01 or less as compared with a physiological saline administration group, and *** indicates that a P value is 0.001 or less as compared with a physiological saline administration group.

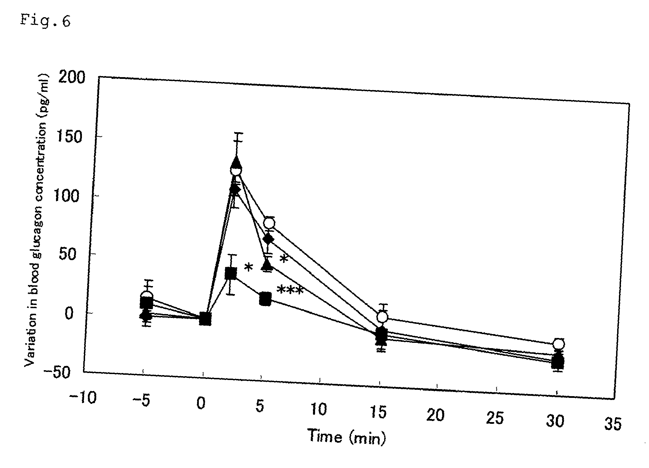

Fig. 6 shows results of investigation of a variation in a blood glucagon concentration when a compound of Example 50 is intravenously administered to a rat under no anesthesia and, after five minutes, RFRP-1 is intravenously administered. In the figure, (-o-) represents a blood glucagon concentration of a physiological saline administration group, (-□-) represents a blood glucagon concentration of an Example 50 compound 0.2 mg/kg administration group, (-▲-) represents a blood glucagon concentration of an Example 50 compound (1 mg/kg) administration group, and (-■-) represents a blood glucagon concentration of an Example 50 compound 5 mg/kg administration group, as expressed by a variation value from a time point of administration of RFRP-1. A value indicates mean ± standard deviation (mean ± SE) (n = 4). * indicates that a P value is 0.05 or less as compared with a physiological saline administration group, and *** indicates that a value is 0.001 or less as compared with a physiological saline administration group.

### Best Mode for Carrying Out the Invention

In the aforementioned formulas, the ring A represents an optionally substituted aromatic ring.

As the aromatic ring represented by the ring A, an aromatic hydrocarbon ring or an aromatic heterocyclic ring is used.

As the aromatic hydrocarbon ring, an aromatic hydrocarbon ring of a carbon number of 6 to 14 such as a benzene ring, and a naphthalene ring is used and, inter alia, a benzene ring is preferably used.

As the aromatic heterocyclic ring, for example, a 5-to 14-membered (monocyclic, dicyclic or tricyclic), preferably 5- to 10-membered, more preferably 5- to 6-membered aromatic heterocyclic ring containing 1 to 4 of 1 or 2 kinds of heteroatoms selected from a nitrogen atom, a sulfur atom and an oxygen atom in addition to carbon atoms is used. As the "5- to 14-membered (preferably 5- to 10-membered) aromatic heterocyclic ring", an aromatic heterocyclic ring such as thiophene, furan, oxazole, benzo[b]thiophene, benzo[b]furan, benzimidazole, benzoxazole, benzothiazole, benzisothiazole, naphtha[2,3-b]thiophene, pyrrole, imidazole, pyrazole, pyridine, pyrazine, pyrimidine, pyridazine, indole, isoindole, 1H-indazole, purine, 4H-quinolizine, isoquinoline, quinoline, phthalazine, naphthyridine, quinoxaline, quinazoline, cinnoline, carbazole, β-carboline, phenanthridine, acridine, phenazine, thiazole, isothiazole, phenothiazine, isooxazole, furazane, and phenoxazine, or a ring formed by fusing these rings (preferably monocycle) with one to plural (preferably 1 or 2) aromatic rings (e.g. benzene ring etc.) is used. Inter alia, a monocyclic aromatic heterocyclic ring is preferable and, for example, thiophene, pyrazole, imidazole, pyridine, pyrazine, pyrimidine, pyridazine, pyrrole, furan, thiazole, isothiazole, and isooxazole are used.

The ring B represents an optionally substituted benzene ring.

Examples of the substituent which may be possessed by the ring A include a halogen atom (e.g. fluorine, chlorine, bromine, iodine), a nitro group, a cyano group, an optionally substituted hydrocarbon group, an optionally substituted heterocyclic group, an optionally substituted hydroxy group, an optionally substituted thiol group, a substituted sulfinyl group, a substituted sulfonyl group, an optionally substituted amino group, an acyl group, an optionally substituted carbamoyl group, an optionally esterified carboxyl group, and a C₁₋₃ alkylenedioxy group (hereinafter, substituent A group).

Examples of the substituent which may be possessed by the ring B include a halogen atom (e.g. fluorine, chlorine, bromine, iodine), a nitro group, a cyano group, an optionally substituted hydrocarbon group, an optionally substituted heterocyclic group, an optionally substituted hydroxy group, an optionally substituted thiol group, a substituted sulfinyl group, a substituted sulfonyl group, an optionally substituted amino group, an acyl group, an optionally substituted carbamoyl group, an optionally esterified carboxyl group, a C₁₋₃ alkylenedioxy group, and a group represented by the formula: 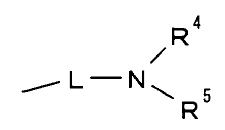 described hereinafter.

Examples of the "hydrocarbon group" of the "optionally substituted hydrocarbon group" as the substituent which may be possessed by the ring A or the ring B include an alkyl group, a cycloalkyl group, an alkenyl group, a cycloalkenyl group, an alkynyl group, an aralkyl group, an aryl group.

As the "alkyl group", a "straight or branched C₁₋₁₅ alkyl group" such as methyl, ethyl, propyl, isopropyl, butyl, isobutyl, sec-butyl, tert-butyl, pentyl, neopentyl, hexyl, heptyl, octyl, nonyl, decyl, undecyl, tridecyl, tetradecyl, and pentadecyl, preferably, a C₁₋₈ alkyl group is used. More preferably, a C₁₋₆ alkyl group is used. Further preferably, a C₁₋₄ alkyl group is used.

As the "cycloalkyl group", a "C₃₋₁₀ cycloalkyl group" such as cyclopropyl, cyclobutyl, cyclopentyl, cyclohexyl, cycloheptyl, cyclooctyl, and adamantyl is used. More preferably, a C₃₋₈ cycloalkyl group is used. Further preferably, a C₅₋₇ cycloalkyl group is used.

As the "alkenyl group", a "C₂₋₁₈ alkenyl group" such as vinyl, allyl, isopropenyl, 3-butenyl, 3-octenyl, and 9-octadecenyl is used. More preferably, a C₂₋₆ alkenyl group is used. Further preferably, a C₂₋₄ alkenyl group is used.

As the "cycloalkenyl group", a "C₃₋₁₀ cycloalkenyl group" such as cyclopropenyl, cyclobutenyl, cyclopentenyl, cyclohexenyl, cycloheptenyl, and cyclooctenyl is used. More preferably, a C₃₋₈ cycloalkenyl group is used. Further preferably, a C₅₋₇ cycloalkenyl group is used.

As the "alkynyl group", a "C₂₋₈ alkynyl group" such as ethynyl, 1-propynyl, propargyl, 1-butynyl, 2-butynyl, 1-pentynyl, 2-pentynyl, and 3-pentynyl is used. More preferably, a C₂₋₆ alkynyl group is used. Further preferably, a C₂₋₄ alkynyl group is used.

As the "aralkyl group", a C₇₋₁₄ aralkyl group is used. Specifically, a phenyl-C₁₋₆ alkyl group such as benzyl, phenethyl, 3-phenylpropyl, and 4-phenylbutyl, and a naphthyl-C₁₋₆ alkyl group such as (1-naphthyl)methyl, 2-(1-naphthyl)ethyl, and 2-(2-naphthyl)ethyl are used.

As the "aryl group", an aromatic monocyclic, dicyclic or tricyclic C₆₋₁₄ aryl group such as phenyl, 1-naphthyl, 2-naphthyl, phenthryl, and anthryl, a biphenyl group, and a tolyl group are used. Preferably, a C₆₋₁₀ aryl group such as phenyl and naphthyl is used. More preferably, phenyl is used.

As the substituent which may be possessed by the "hydrocarbon group" in the "optionally substituted hydrocarbon group" as the substituent which may be possessed by the ring A or the ring B, for example, (i) a nitro group, (ii) a hydroxy group, an oxo group, (iii) a cyano group, (iv) a carbamoyl group, (v) a mono- or di-C₁₋₆ alkyl-carbamoyl group (e.g. N-methylcarbamoyl, N-ethylcarbamoyl, N,N-dimethylcarbamoyl, N,N-diethylcarbamoyl etc.; the alkyl group may be substituted with a halogen atom, a hydroxy group, or a C₁₋₆ alkoxy group), a mono- or di-C₂₋₄ alkenyl-carbamoyl group (e.g. N-allylcarbamoyl etc.; the alkenyl group may be substituted with a halogen atom, a hydroxy group, a C₁₋₆ alkoxy group etc.), a mono- or di-C₆₋₁₄ aryl-carbamoyl group (the C₆₋₁₄ aryl group may be substituted with a halogen atom, a C₁₋₆ alkyl optionally substituted with a halogen atom, a C₁₋₆ alkoxy group etc.), a mono- or di-C₇₋₁₆ aralkyl-carbamoyl group (the C₇₋₁₆ aralkyl group (e.g. benzyl group) may be substituted with a halogen atom, a C₁₋₆ alkyl optionally substituted with a halogen atom, a C₁₋₆ alkoxy group etc.), a C₁₋₆ alkoxy-carbonyl-carbamoyl group, a C₁₋₆ alkylsulfonyl-carbamoyl group, a C₁₋₆ alkoxy-carbamoyl group, an amino-carbamoyl group, a mono- or di-C₁₋₆ alkylamino-carbamoyl group, a mono- or di-C₆₋₁₄ arylamino-carbamoyl group, (vi) a carboxyl group, (vii) a C₁₋₆ alkoxy-carbonyl group (e.g. methoxycarbonyl, ethoxycarbonyl, propoxycarbonyl, isopropoxycarbonyl etc.), (viii) a sulfo group, (ix) a halogen atom (e.g. fluorine, chlorine, bromine, iodine), (x) an optionally halogenated C₁₋₆ alkoxy group (e.g. methoxy, ethoxy, propoxy, isopropoxy etc.), a C₁₋₆ alkoxy group optionally substituted with a hydroxy group, a C₁₋₆ alkoxy group optionally substituted with a carboxyl group, a C₁₋₆ alkoxy group optionally substituted with a C₁₋₆ alkoxy-carbonyl group, a C₁₋₆ alkoxy-C₁₋₆ alkoxy group, a C₁₋₆ alkoxy-C₁₋₆ alkoxy-C₁₋₆ alkoxy group, (xi) a C₆₋₁₄ aryloxy group, a C₆₋₁₄ aryloxy-C₁₋₆ alkyl group, a C₆₋₁₄ aryloxy-C₁₋₆ alkoxy group, a C₁₋₆ alkylcarbonyl-oxy group, a carbamoyloxy group, a mono- or di-C₁₋₆ alkyl-carbamoyloxy group, (xii) an optionally halogenated C₆₋₁₄ aryl group (e.g. a phenyl group, 1- or 2-naphthyl), an optionally halogenated C₆₋₁₄ aryl-C₁₋₆ alkyl group, an optionally halogenated C₆₋₁₄ aryl-C₂₋₄ alkenyl group, an optionally halogenated C₆₋₁₄ aryloxy group (e.g. o-, m- or p-chlorophenoxy, o-, m- or p-bromophenoxy etc.), a 5- to 10-membered heterocyclic-oxy group containing at least one (preferably 1 to 4, further preferably 1 or 2) of 1 to 3 kinds (preferably 1 or 2 kinds) of heteroatoms selected from an oxygen atom, a sulfur atom, and a nitrogen atom (e.g. pyridyloxy; the heterocyclic ring may be substituted with C₁₋₆ alkyl), a C₃₋₁₀ cycloalkyl group, a C₃₋₁₀ cycloalkyl-C₁₋₆ alkoxy group, a C₃₋₁₀ cycloalkyl-C₁₋₆ alkyl group, (xiii) an optionally halogenated C₁₋₆ alkyl group (e.g. methyl, ethyl, propyl, isopropyl, butyl etc.), an optionally halogenated C₂₋₆ alkenyl group (e.g. vinyl, allyl, 2-butenyl, 3-butenyl etc.), an optionally halogenated C₁₋₆ alkylthio group (e.g. methylthio, ethylthio, n-propylthio, isopropylthio, n-butylthio etc.), a C₁₋₆ alkyl group optionally substituted with a hydroxy group, a C₁₋₆ alkylthio group optionally substituted with a hydroxy group, (xiv) a mercapto group, a thioxo group, (xv) a C₇₋₁₆ aralkyloxy group (e.g. benzyloxy) or a C₇₋₁₆ aralkylthio group (e.g. benzylthio), each optionally substituted with a substituent selected from a halogen atom, a carboxyl group and a C₁₋₆ alkoxy-carbonyl group, (xvi) an optionally halogenated C₆₋₁₄ arylthio group, a 5- to 10-membered heterocyclic-thio group containing at least one (preferably 1 to 4, further preferably 1 to 2) of 1 to 3 kinds (preferably 1 to 2 kinds) of heteroatoms selected from an oxygen atom, a sulfur atom and a nitrogen atom (e.g. pyridylthio; the heterocyclic ring may be substituted with C₁₋₆ alkyl), a C₆₋₁₄ arylthio-C₁₋₆ alkyl group, a 5- to 10-membered heterocyclic-thio-C₁₋₆ alkyl group containing at least one (preferably 1 to 4, further preferably 1 to 2) of 1 to 3 kinds (preferably 1 to 2 kinds) of heteroatoms selected from an oxygen atom, a sulfur atom, and a nitrogen atom (e.g. pyridylthio-C₁₋₆ alkyl; the heterocyclic ring may be substituted with C₁₋₆ alkyl), (xvii) an optionally halogenated C₁₋₆ alkylsulfinyl group (e.g. methylsulfinyl, ethylsulfinyl etc.), a C₆₋₁₄ arylsulfinyl group, a C₆₋₁₄ arylsulfinyl-C₁₋₆ alkyl group, (xviii) an optionally halogenated C₁₋₆ alkylsulfonyl group (e.g. methylsulfonyl, ethylsulfonyl etc.), a C₆₋₁₄ arylsulfonyl group, a C₆₋₁₄ arylsulfonyl-C₁₋₆ alkyl group, (xix) an amino group, an aminosulfonyl group, a mono- or di-C₁₋₆ alkylaminosulfonyl group (e.g. methylaminosulfonyl, ethylaminosulfonyl, N,N-dimethylaminosulfonyl, N,N-diethylaminosulfonyl etc.; the alkyl group may be substituted with a halogen atom, a hydroxy group, a C₁₋₆ alkoxy group etc.), (xx) a C₁₋₁₅ acyl-amino group [e.g. a C₁₋₁₀ alkanoylamino group (e.g. formylamino, acetylamino, trifluoroacetylamino, propionylamino, pivaloylamino etc.), a C₃₋₁₀ alkenoylamino group (e.g. acryloylamino, methacryloylamino etc.), a C₃₋₁₀ cycloalkylcarbonylamino group (e.g. cyclopropylcarbonylamino, cyclopentylcarbonylamino etc.), C₆₋₁₄ arylcarbonylamino (e.g. benzoylamino), C₁₋₆ alkylsulfonylamino (e.g. methanesulfonylamino, trifluoromethanesulfonylamino etc.), C₆₋₁₄ arylsulfonylamino (e.g. benzenesulfonylamino, toluenesulfonylamino etc.); C₁₋₁₅ acyl may be substituted with a halogen atom, a hydroxy group, a carboxyl group etc.], C₇₋₁₆ aralkyloxycarbonylamino (e.g. benzyloxycarbonylamino), optionally halogenated C₁₋₆ alkoxycarbonylamino, a carbamoylamino group, a mono- or di-C₁₋₆ alkylcarbamoylamino group, (xxi) a mono- or di-C₁₋₆ alkylamino group (e.g. methylamino, ethylamino, dimethylamino, diethylamino etc.; the alkyl group may be substituted with a halogen atom, a hydroxy group, a C₁₋₆ alkoxy group etc.), a mono- or di-C₁₋₆ alkanoylamino group (e.g. formylamino, acetylamino etc.; the alkanoyl group may be substituted with a halogen atom, a hydroxy group, a C₁₋₆ alkoxy group etc.), C₆₋₁₄ arylamino, C₇₋₁₆ aralkylamino (e.g. benzylamino), a C₁₋₆ alkyl (C₇₋₁₆ aralkyl)amino group (e.g. C₁₋₆ alkyl (benzyl) amino etc.), a C₁₋₆ alkanoyl (C₇₋₁₆ aralkyl)amino group (e.g. C₁₋₆ alkanoyl(benzyl)amino etc.), (xxii) a 3- to 8-membered cyclic amino group (e.g. 1-azetidinyl, 1-pyrrolidinyl, piperidino, morpholino, thiomorpholino, 1-piperazinyl etc., preferably a 5- or 6-membered cyclic amino group), a 3- to 8-membered cyclic amino-carbonyl group (e.g. 1-azetidinylcarbonyl, 1-pyrrolidinylcarbonyl, piperidinocarbonyl, morpholinocarbonyl, thiomorpholinocarbonyl, 1-piperazinylcarbonyl etc., preferably a 5- or 6-membered cyclic amino-carbonyl group), a 3- to 8-membered cyclic amino-carbonyl-oxy group (e.g. 1-pyrrolidinylcarbonyloxy, piperidinocarbonyloxy, morpholinocarbonyloxy, thiomorpholinocarbonyloxy, 1-piperazinylcarbonyloxy etc., preferably a 5- or 6-membered cyclic amino-carbonyl group), a 3- to 8-membered cyclic amino-carbonyl-amino group (e.g. 1-pyrrolidinylcarbonylamino, piperidinocarbonylamino, morpholinocarbonylamino, thiomorpholinocarbonylamino, 1-piperazinylcarbonylamino etc., preferably a 5- or 6-membered cyclic amino-carbonyl-amino group), a 3- to 8-membered cyclic amino-sulfonyl group (e.g. 1-pyrrolidinylsulfonyl, piperidinosulfonyl, morpholinosulfonyl, thiomorpholinosulfonyl, 1-piperazinylsulfonyl etc., preferably a 5- or 6-membered cyclic amino-sulfonyl group), a 3- to 8-membered cyclic amino-C₁₋₆ alkyl group (preferably a 5- or 6-membered cyclic amino-C₁₋₆ alkyl group), (xxiii) a C₁₋₆ alkanoyl group (e.g. formyl, acetyl etc.) or a C₆₋₁₄ aryl-carbonyl group (e.g. a benzoyl group), each optionally substituted with a substituent selected from a halogen atom, amino, a carboxyl group and a C₁₋₆ alkoxy-carbonyl group, (xxiv) a C₇₋₁₆ aralkyl-carbonyl (e.g. a benzylcarbonyl group) optionally substituted with a substituent selected from a halogen atom, amino, a carboxyl group and a C₁₋₆ alkoxy-carbonyl group, (xxv) a 5- to 10-membered heterocyclic group containing at least one (preferably 1 to 4, further preferably 1 to 2) of 1 to 3 kinds (preferably 1 or 2 kinds) of heteroatoms selected from an oxygen atom, a sulfur atom and a nitrogen atom (e.g. 2- or 3-thienyl, 2- or 3-furyl, 3-, 4- or 5-pyrazolyl, 2-, 4- or 5-thiazolyl, 3-, 4- or 5-isothiazolyl, 2-, 4- or 5-oxazolyl, 1,2,3- or 1,2,4-triazolyl, 1H- or 2H-tetrazolyl, 2-, 3- or 4-pyridyl, 2-, 4- or 5-pyrimidyl, 3-or 4-pyridazinyl, quinolyl, isoquinolyl, indolyl etc.; the heterocyclic group may be substituted with a C₁₋₆ alkyl group), (xxvi) a 5- to 10-membered heterocyclic-carbonyl group containing at least one (preferably 1 to 4, further preferably 1 to 2) of 1 to 3 kinds (preferably 1 or 2 kinds) of heteroatoms selected from an oxygen atom, a sulfur atom and a nitrogen atom (e.g. 2- or 3-thienylcarbonyl, 2- or 3-furylcarbonyl, 3-, 4- or 5-pyrazolylcarbonyl, 2-, 4- or 5-thiazolylcarbonyl, 3-, 4- or 5-isothiazolylcarbonyl, 2-, 4- or 5-oxazolylcarbonyl, 1,2,3- or 1,2,4-triazolylcarbonyl, 1H- or 2H-tetrazolylcarbonyl, 2-, 3- or 4-pyridylcarbonyl, 2-, 4- or 5-pyrimidylcarbonyl, 3- or 4-pyridazinylcarbonyl, quinolylcarbonyl, isoquinolylcarbonyl, indolylcarbonyl etc., particularly a 5- to 10-membered aromatic heterocyclic-carbonyl group; the heterocyclic group may be substituted with a C₁₋₆ alkyl group), (xxvii) a hydroxyimino group, a C₁₋₆ alkoxyimino group, (xxviii) an optionally halogenated straight or branched C₁₋₆ alkylenedioxy group (e.g. methylenedioxy, ethylenedioxy, propylenedioxy, tetrafluoroethylenedioxy etc.), (xxiv) an ureido group, (xxx) a C₁₋₆ alkyl-ureido group (e.g. methylureido, ethylureido etc.), and (xxxi) a C₁₋₆ alkyl group optionally substituted with a halogen atom (forgoing, substituent B group) are used. The "hydrocarbon group" may have 1 to 5 of these substituents at any possible positions and, when it has 2 or more substituents, they may be the same or different.

Examples of the "heterocyclic group" of the "optionally substituted heterocyclic group" as the substituent which may be possessed by the ring A or the ring B include a 5- to 16-membered monocyclic to tricyclic aromatic heterocyclic group, and a 3- to 8-membered saturated or unsaturated non-aromatic heterocyclic group (aliphatic heterocyclic group), containing at least one (preferably 1 to 4, further preferably 1 to 2) of 1 to 3 kinds (preferably 1 or 2 kinds) of heteroatoms selected from an oxygen atom, a sulfur atom and a nitrogen atom, as an atom constituting a ring system (ring atom).

Examples of the "aromatic heterocyclic group" include a 5- or 6-membered aromatic monocyclic heterocyclic group such as furyl, thienyl, pyrrolyl, oxazolyl, isooxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, and triazinyl, and a 8- to 16-membered (preferably 8- to 12-membered) aromatic fused heterocyclic group (preferably a heterocyclic ring in which 1 to 2 (preferably 1) of the aforementioned 5- or 6-membered aromatic monocyclic heterocyclic groups are fused with 1 to 2 (preferably 1) of benzene rings are fused, or a heterocyclic ring in which 2 to 3 (preferably 2) of the same or different heterocyclic rings of the aforementioned 5- or 6-membered aromatic monocyclic heterocyclic groups are fused, more preferably a heterocyclic ring in which the aforementioned 5- or 6-membered aromatic monocyclic heterocyclic group is fused with a benzene ring) such as benzofuranyl, isobenzofuranyl, benzo[b]thienyl, indolyl, isoindolyl, 1H-indazolyl, benzimidazolyl, benzooxazolyl, 1,2-benzoisooxazolyl, benzothiazolyl, benzopyranyl, 1,2-benzoisothiazolyl, 1H-benzotriazolyl, quinolyl, isoquinolyl, cinnolinyl, quinazolinyl, quinoxalinyl, phthalazinyl, naphthyridyl, purinyl, pteridinyl, carbazolyl, α-carbolinyl, β-carbolinyl, γ-carbolinyl, acridinyl, phenoxazinyl, phenothiazinyl, phenazinyl, phenoxathiyl, thianthrenyl, phenatrizinyl, phenathrolinyl, indolizinyl, pyrrolo[1,2-b]pyridazinyl, pyrazolo[1,5-a]pyridyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl, 1,2,4-triazolo[4,3-b]pyridazinyl, benzo[1,2,5]thiadiazolyl, and benzo[1,2,5]oxadiazolyl.

Examples of the "non-aromatic heterocyclic group" include a 3- to 8-membered (preferably 5- to 6-membered) saturated or unsaturated (preferably saturated) non-aromatic monocyclic heterocyclic group (aliphatic monocyclic heterocyclic group) such as oxiranyl, azetidinyl, oxetanyl, thietanyl, pyrrolidinyl (preferably, 1-pyrrolidinyl), tetrahydrofuryl, thiolanyl, piperidinyl (preferably, 1-piperidinyl or 4-piperidinyl), tetrahydropyranyl, morpholinyl, thiomorpholinyl, and piperazinyl, a heterocyclic group in which 1 to 2 (preferably 1) of the aforementioned non-aromatic monocyclic heterocyclic groups are fused with 1 to 2 (preferably 1) of benzene rings such as 2,3-dihydroindolyl, and 1,3-dihydroisoindolyl, a heterocyclic group in which 1 to 2 (preferably 1) of the aforementioned non-aromatic monocyclic heterocyclic groups are fused with 1 to 2 (preferably 1) of the aforementioned 5- to 6-memebred aromatic monocyclic heterocyclic groups, and a non-aromatic heterocyclic group in which a part or all of double bonds of the aforementioned aromatic monocyclic heterocyclic group or aromatic fuse heterocyclic group are saturated such as 1,2,3,4-tetrahydroquinolyl, and 1,2,3,4-tetrahydroisoquinolyl.

As the "heterocyclic group" in the "optionally substituted heterocyclic group", a 5- or 6-membered aromatic monocyclic heterocyclic group is preferable.

As the substituent which may be possessed by the "heterocyclic group", the same number of the same groups as those for the substituent (substituent B group) which may be possessed by the "hydrocarbon group" in the "optionally substituted hydrocarbon group" as the substituent which may be possessed by the ring A are used.

Examples of the "optionally substituted amino group", the "optionally substituted hydroxy group" and the "optionally substituted thiol group" as the substituent which may be possessed by the ring A or the ring B include an "amino group", a "hydroxy group" and a "thiol group", each optionally having a substituent, such as an optionally substituted hydrocarbon group, an acyl group, an optionally esterified carboxyl group, an optionally substituted carbamoyl group, and an optionally substituted heterocyclic group.

As the "hydrocarbon group" in the "optionally substituted hydrocarbon group" and the "heterocyclic group" in the "optionally substituted heterocyclic group", the same groups as the "hydrocarbon group" in the "optionally substituted hydrocarbon group" and the "heterocyclic group" in the "optionally substituted heterocyclic group" as the substituent which may be possessed by the ring A are used, respectively.

In addition, as the "acyl group" and the "optionally esterified carboxyl group" as the substituent, the same groups as the "optionally esterified carboxyl group" and the "acryl group" as the substituent which may be possessed by the ring A as described hereinafter are used, respectively.

As the "optionally substituted carbamoyl group", the same group as the "optionally substituted carbamoyl group" as the substituent which may be possessed by the ring A as described hereinafter is used.

In addition, as the substituent in the "optionally substituted hydrocarbon group" and the "optionally substituted heterocyclic group", the same number of the same groups as those for the substituent (substituent B group) in the "optionally substituted hydrocarbon group" and the "optionally substituted heterocyclic group" as the substituent which may be possessed by the ring A are used, respectively. Inter alia, preferable examples include an "amino group", a "hydroxy group" and a "thiol group" optionally having a substituent such as a lower alkyl (e.g. C₁₋₆ alkyl such as methyl, ethyl, propyl, isopropyl, butyl, isobutyl, tert-butyl, pentyl, hexyl etc.) optionally substituted with a substituent selected from a halogen atom (e.g. fluorine, chlorine, bromine, iodine etc.), optionally halogenated C₁₋₆ alkoxy (e.g. methoxy, ethoxy, trifluoromethoxy, 2,2,2-trifluoroethoxy, trichloromethoxy, 2,2,2-trichloroethoxy etc.), optionally substituted phenyl (preferably, phenyl optionally substituted with a substituent selected from an optionally halogenated C₁₋₆ alkyl group, an optionally halogenated C₁₋₆ alkoxy group, a carboxyl group and a halogen atom), and a 5- to 10-membered heterocyclic group containing at least one (preferably 1 to 4, further preferably 1 or 2) of 1 to 3 kinds (preferably 1 or 2 kinds) of heteroatoms selected from an oxygen atom, a sulfur atom and a nitrogen atom (e.g. 2- or 3-thienyl, 2-or 3-furyl, 3-, 4- or 5-pyrazolyl, 2-, 4- or 5-thiazolyl, 3-, 4- or 5-isothiazolyl, 2-, 4- or 5-oxazolyl, 1,2,3- or 1,2,4-triazolyl, 1H- or 2H-tetrazolyl, 2-, 3- or 4-pyridyl, 2-, 4- or 5-pyrimidyl, 3- or 4-pyridazinyl, quinolyl, isoquinolyl, indolyl etc.; the heterocyclic may be substituted with a C₁₋₄ alkyl group etc.), acyl (C₁₋₆ alkanoyl (e.g. formyl, acetyl, propionyl, pivaloyl etc.), benzoyl, C₁₋₆ alkylsulfonyl (e.g. methanesulfonyl etc.), benzenesulfonyl etc.), optionally halogenated C₁₋₆ alkoxycarbonyl (e.g. methoxycarbonyl, ethoxycarbonyl, trifluoromethoxycarbonyl, 2,2,2-trifluoroethoxycarbonyl, trichloromethoxycarbonyl, 2,2,2-trichloroethoxycarbonyl etc.), C₁₋₆ alkoxycarbonyl optionally substituted with phenyl (e.g. benzyloxycarbonyl etc.), an optionally substituted carbamoyl group (e.g. a carbamoyl group optionally substituted with 1 to 2 substituents such as a lower (C₁₋₆) alkyl group, a C₆₋₁₄ aryl group (e.g. a phenyl group) etc., such as carbamoyl, N-methylcarbamoyl, N,N-dimethylcarbamoyl, phenylcarbamoyl etc.), a heterocyclic group (the same group as the "heterocyclic group" in the "optionally substituted heterocyclic group" as a substituent which may be possessed by a ring A) and the like.

In addition, two substituents in N,N-disubstituted amino may be taken together with a nitrogen atom to form a "cyclic amino group" and, as the "cyclic amino group", a 3-to 8-membered (preferably 5- to 8-membered more preferably 5- or 6-membered) cyclic amino group such as 1-azetidinyl, 1-pyrrolidinyl, piperidino, morpholino, thiomorpholino (a sulfur atom may be oxidized), 1-piperazinyl, and 1,4-diazepanyl is used. The "cyclic amino group" may have a substituent selected from the substituent B group. Specifically, 1-piperazinyl, and 1,4-diazepanyl optionally having lower alkyl (e.g. C₁₋₆ alkyl such as methyl, ethyl, propyl, isopropyl, butyl, tert-butyl, pentyl, hexyl etc.), aralkyl (e.g. C₇₋₁₆ aralkyl such as benzyl, phenethyl etc.), aryl (e.g. C₆₋₁₄ aryl such as phenyl, 1-naphthyl, 2-naphthyl etc.) and the like at a 4-position are used.

The "substituted sulfinyl group" and the "substituted sulfonyl group" as the substituent which may be possessed by the ring A or the ring B represent a sulfinyl group or a sulfonyl group substituted with a substituent such as the "optionally substituted hydroxy group", the "optionally substituted amino group", the "optionally substituted hydrocarbon group" or the "optionally substituted heterocyclic group", respectively.

As the "hydrocarbon group" in the "optionally substituted hydrocarbon group", the same group as the "hydrocarbon group" in the "optionally substituted hydrocarbon group" as the substituent which may be possessed by the ring A is used. As the "heterocyclic group" in the "optionally substituted heterocyclic group", the same group as the "heterocyclic group" in the "optionally substituted heterocyclic group" as the substituent which may be possessed by the ring A is used. As the substituent optionally substituting at the hydroxy group and the amino group which are the substituents of the "substituted sulfinyl group" and the "substituted sulfonyl group", the same group as the substituent which may be possessed by the "hydroxy group" in the "optionally substituted hydroxy group" or by the "amino group" in the "optionally substituted amino group" as the substituent which may be possessed by the ring A is used, and preferable examples include a C₁₋₆ alkyl group, a C₃₋₈ cycloalkyl group, a C₂₋₄ alkenyl group, a C₆₋₁₀ aryl group, an acyl group, an amino group and a heterocyclic group (the same group as the "heterocyclic group" in the "optionally substituted heterocyclic group" as a substituent which may be possessed by the ring A).

In addition, as the substituent in the "optionally substituted hydrocarbon group" and the "optionally substituted heterocyclic group" which are the substituent of the "substituted sulfinyl group" and the "substituted sulfonyl group", the same number of the same groups as those for the substituent (substituent B group) in the "optionally substituted hydrocarbon group" and the "optionally substituted heterocyclic group" as the substituent which may be possessed by the ring A are used.

As the "acyl group" as a substituent which may be possessed by the ring A or the ring B, an acyl group obtained by removing a OH group from carboxylic acid such as R^{A}COOH, sulfonic acid such as R^{A}SO₃H, sulfinic acid such as R^{A}SO₂H, or phosphoric acid such as R^{A}OPO (OR^{B}) OH (R^{A} represents a hydrogen atom, an optionally substituted hydrocarbon group, or an optionally substituted heterocyclic group, and R^{B} represents a hydrogen atom, or an optionally substituted hydrocarbon group) is used and, specifically, R^{A}CO, R^{A}SO₂, R^{A}SO, and R^{A}OPO(OR^{B}) (symbols in the formulas are as defined above) are used.

As the "hydrocarbon group" in the "optionally substituted hydrocarbon group" and the "heterocyclic group" in the "optionally substituted heterocyclic group" represented by R^{A} (and R^{B}), the same groups as the "hydrocarbon group" in the "optionally substituted hydrocarbon group" and the "heterocyclic group" in the "optionally substituted heterocyclic group" as the substituent which may be possessed by the ring A are used, respectively. In addition, as the substituent in the "optionally substituted hydrocarbon group" and the "optionally substituted heterocyclic group", the same number of the same groups as those for the substituent (substituent B group) in the "optionally substituted hydrocarbon group" and the "optionally substituted heterocyclic group" as the substituent which may be possessed by the ring A are used, respectively.

Examples of R^{A}CO include formyl, acetyl, propionyl, butyryl, isobutyryl, valeryl, isovaleryl, pivaloyl, hexanoyl, cyclobutanecarbonyl, cyclopentanecarbonyl, cyclohexanecarbonyl, crotonyl, benzoyl, nicotinoyl, isonicotinoyl, and trifluoroacetyl and, inter alia, R^{A}CO in which R^{A} is a lower (C₁₋₆) alkyl group such as acetyl, propionyl, butyryl, valeryl is more preferable.

Examples of the "optionally substituted carbamoyl group" as the substituent which may be possessed by the ring A or the ring B include N-monosubstituted carbamoyl and N,N-disubstituted carbamoyl in addition to unsubstituted carbamoyl.

Examples of the substituent which may be possessed by the "carbamoyl group" in the "optionally substituted carbamoyl group" include the same group as the substituent of the "amino group" of the "optionally substituted amino group" as the substituent which may be possessed by the ring A ("optionally substituted hydrocarbon group", "acyl group", "optionally substituted alkoxycarbonyl group", "optionally substituted carbamoyl group" (preferably a carbamoyl group optionally substituted with 1 to 2 substituents such as a lower (C₁₋₆) alkyl group, and a C₆₋₁₄ aryl group (e.g. a phenyl group), such as carbamoyl, N-methylcarbamoyl, N,N-dimethylcarbamoyl, phenylcarbamoyl etc.), "optionally substituted heterocyclic group" etc.), and a "carbamoyl group" having the "optionally substituted amino group" (i.e. "optionally substituted carbazoyl group"), and a "carbamoyl group" having the "optionally substituted hydroxy group" (i.e. "optionally substituted N-hydroxycarbamoyl group") may be used. Alternatively, two substituents in N,N-disubstituted carbamoyl may be taken together with a nitrogen atom to form cyclic amino and, as cyclic amino carbonyl in such a case, 3- to 8-membered (preferably 5- to 6-membered) cyclic aminocarbonyl such as 1-azetidinylcarbonyl, 1-pyrrolidinylcarbonyl, piperidinocarbonyl, morpholinocarbonyl, thiomorpholinocarbonyl (a sulfur atom may be oxidized), 1-piperazinylcarbonyl, and 1-piperazinylcarbonyl having lower alkyl (e.g. C₁₋₆ alkyl such as methyl, ethyl, propyl, isopropyl, butyl, tert-butyl, pentyl, hexyl etc.), aralkyl (e.g. C₇₋₁₀ aralkyl such as benzyl, phenethyl etc.), aryl (e.g. C₆₋₁₀ aryl such as phenyl, 1-naphthyl, 2-naphthyl etc.), or an acyl group (e.g. formyl, acetyl, benzoyl, methoxycarbonyl, benzyloxycarbonyl, methylsulfonyl etc.) at a 4-position is used.

Examples of the "optionally esterified carboxyl group" as the substituent which may be possessed by the ring A or the ring B include a group represented by the formula - COOR^{C} (R^{C} represents a hydrogen atom, or an optionally substituted hydrocarbon group, or an optionally substituted heterocyclic group) and, inter alia, free carboxyl, lower alkoxycarbonyl, aryloxycarbonyl, aralkyloxycarbonyl, heterocyclic oxycarbonyl, and heterocyclic methyloxycarbonyl are preferably used.

As "the hydrocarbon group" in the "optionally substituted hydrocarbon group" and the "heterocyclic group" in the "optionally substituted heterocyclic group" represented by R^{c}, the same groups as those for the "hydrocarbon group" in the "optionally substituted hydrocarbon group" and the "heterocyclic group" in the "optionally substituted heterocyclic group" as the substituent which may be possessed by the ring A are used, respectively. In addition, as a substituent which may be possessed by the "hydrocarbon group" or the "heterocyclic group", the same number of the same groups as those for the substituent (substituent B group) which may be possessed by the "hydrocarbon group" in the "optionally substituted hydrocarbon group" and the "heterocyclic group" in the "optionally substituted heterocyclic group" as the substituent which may be possessed by the ring A are used, respectively.

Examples of the "lower alkoxycarbonyl" include C₁₋₆ alkoxycarbonyl such as methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl, isopentyloxycarbonyl, and neopentyloxycarbonyl and, inter alia, C₁₋₃ alkoxycarbonyl such as methoxycarbonyl, ethoxycarbonyl, and propoxycarbonyl is preferable.

The "lower alkoxycarbonyl" may have a substituent at the "lower alkyl" part of the "lower alkoxy" and, as the substituent, the same number of the same groups as those listed as the substituent which may be possessed by the "hydrocarbon group" in the "optionally substituted hydrocarbon group" as the substituent which may be possessed by the ring A are used.

As the "aryloxycarbonyl", C₇₋₁₂aryloxycarbonyl such as phenoxycarbonyl, 1-naphthoxycarbonyl, and 2-naphthoxycarbonyl is preferable.

As the "aralkyloxycarbonyl", C₇₋₁₅ aralkyloxycarbonyl such as benzyloxycarbonyl, and phenethyloxycarbonyl (preferably, C₆₋₁₀ aryl-C₁₋₆ alkoxy-carbonyl etc.) is preferable.

As the heterocyclic in the "heterocyclic oxycarbonyl" and the "heterocyclic methyloxycarbonyl", the same groups as those for the "heterocyclic" in the "optionally substituted heterocyclic group" as the substituent which may be possessed by the ring A are used and, for example, pyridyl, quinolyl, indolyl, piperidinyl, and tetrahydropyranyl are preferably used.

The "aryloxycarbonyl", the "aralkyloxycarbonyl" and the "heterocyclic oxycarbonyl" may have a substituent, respectively. As the substituent thereof, the same number of the same groups as groups listed as the substituent (substituent B group) which may be possessed by the "hydrocarbon group" in the "optionally substituted hydrocarbon group" as the substituent which may be possessed by the ring A are used.

As the "C₁₋₃ alkylenedioxy group" as the substituent which may be possessed by the ring A or the ring B, methylenedioxy, and ethylenedioxy are used.

As the substituent which may be possessed by the ring A, a halogen atom (e.g. fluorine, chlorine, bromine, iodine), and a C₆₋₁₄ aryl group (e.g. phenyl) are preferable, and a chlorine atom, and a phenyl group are particularly preferably used.

As the substituent which may be possessed by the ring B,
(i) a group represented by the formula: 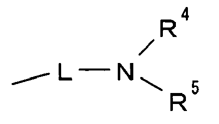 as described hereinafter,
(ii) a halogen atom (e.g. fluorine, chlorine, bromine, iodine)
(iii) a C₁₋₆ alkyl group (e.g. methyl, ethyl, propyl, isopropyl, butyl, tert-butyl etc., particularly methyl) optionally substituted with a substituent selected from:
   (a) a halogen atom (e.g. fluorine etc.) and
   (b) amino optionally substituted with 1 or 2 substituents selected from:
      (aa) C₁₋₆ alkyl (e.g. methyl, ethyl, tert-butyl) optionally having a substituent selected from C₁₋₆ alkoxy-carbonyl-amino (e.g. tert-butoxycarbonylamino), and 5- or 6-membered cyclic amino,
      (bb) trityl,
      (cc) C₇₋₁₆ aralkyl (e.g. phenyl-C₁₋₆ alkyl),
      (dd) C₁₋₆ alkyl-carbonyl (e.g. aminoacetyl) optionally having amino, and
      (ee) 5- to 10-membered heterocyclic-carbonyl group (e.g. 4-piperidinyl-carbonyl) containing at least one of 1 to 3 kinds of heteroatoms selected from an oxygen atom, a sulfur atom and a nitrogen atom,
(iv) a C₁₋₆ alkoxy group optionally substituted with amino (e.g. aminopropoxy),
(v) a 5- to 10-membered heterocyclic-C₁₋₆ alkyl group (e.g. methyl, ethyl, propyl, isopropyl, butyl etc., particularly methyl) containing at least one (preferably 1 to 4, further preferably 1 or 2) of 1 to 3 kinds (preferably 1 or 2 kinds) of heteroatoms selected from an oxygen, a sulfur atom and a nitrogen atom,
(vi) a C₁₋₆ alkoxy-carbonyl-C₂₋₆ alkenyl group (e.g. methoxycarbonylvinyl, ethoxycarbonylvinyl, methoxycarbonylallyl, ethoxycarbonylallyl, methoxycarbonylisopropenyl, ethoxycarbonylisopropenyl, methoxycarbonyl-3-butenyl, ethoxycarbonyl-3-butenyl etc.),
(vii) a hydroxy group,
(viii) C₇₋₁₆ aralkyloxy (e.g. benzyloxy), and
(ix) an amino group;
are preferably used.

In the aforementioned formulas, as the ring A, a benzene ring represented by the formula: 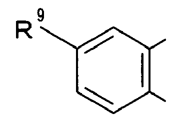
wherein R⁹ represents a hydrogen atom, a halogen atom or a C₆₋₁₄ aryl group, is preferably used. As R⁹, a hydrogen atom, a chlorine atom, and a phenyl group are preferable.

In the aforementioned formulas, as the ring B, a benzene ring represented by the formula: 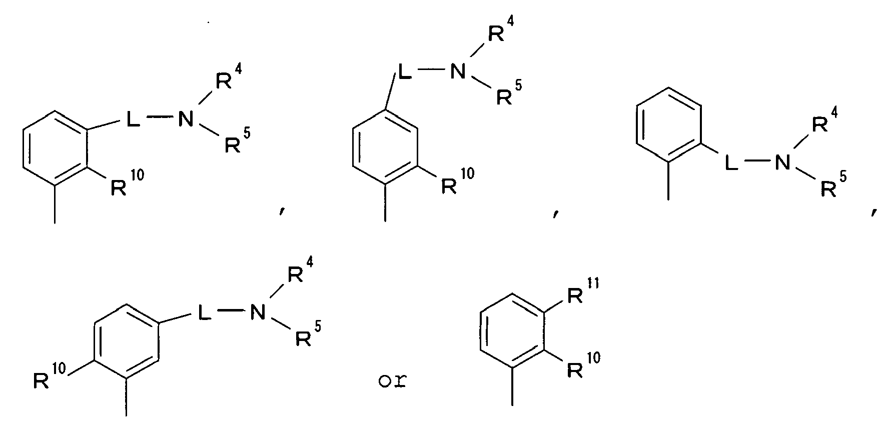
wherein R¹⁰ and R¹¹ each represents (i) a hydrogen atom, (ii) a C₁₋₆ alkyl group optionally substituted with a halogen atom, (iii) a C₁₋₆ alkoxy group, (iv) a 5- to 10-membered heterocyclic group-C₁₋₆ alkyl group containing at least one of 1 to 3 kinds of heteroatoms selected from an oxygen atom, a sulfur atom and a nitrogen atom, or (v) a C₁₋₆ alkoxy-carbonyl-C₂₋₆ alkenyl group) is preferably used.

As R¹⁰, a hydrogen atom or a C₁₋₆ alkoxy group (e.g. methoxy) is preferable.

In the aforementioned formulas, X represents O, S(O)n (n represents an integer of 0 to 2) or NR³ (R³ represents a hydrogen atom, an optionally substituted hydrocarbon group, or an optionally substituted heterocyclic group).

As the "optionally substituted hydrocarbon group" represented by R³, the same group as the "optionally substituted hydrocarbon group" as the substituent which may be possessed by the ring A is used.

As the "optionally substituted heterocyclic group" represented by R³, the same group as the "optionally substituted heterocyclic group" as the substituent which may be possessed by the ring A is used.

The "hydrocarbon group" and the "heterocyclic group" may have 1 to 5 (preferably 1 to 3) substituents at any possible positions, respectively. When 2 or more substituents are possessed, they may be the same or different.

As R³, an optionally substituted alkyl group is preferable and, inter alia, a C₁₋₆ alkyl group such as methyl, ethyl and propyl is preferable.

As X, O is preferable.

In the aforementioned formulas, R¹ and R² each represents a hydrogen atom, an optionally substituted hydrocarbon group or an optionally substituted heterocyclic group.

As the "optionally substituted hydrocarbon group" represented by R¹, the same group as the "optionally substituted hydrocarbon group" as the substituent which may be possessed by the ring A is used.

As the "hydrocarbon group" of the "optionally substituted hydrocarbon group" represented by R², the same group as the "hydrocarbon group" of the "optionally substituted hydrocarbon group" as the substituent which may be possessed by the ring A is used.

As the substituent of the "hydrocarbon group" represented by R², in addition to the "substituent" (substituent B group) of the "optionally substituted hydrocarbon group" as the substituent which may be possessed by the ring A, (i) a group represented by the formula -CONR¹²(R¹³), (ii) a group represented by the formula -COO-R¹⁴, (iii) a group represented by the formula -NR¹⁵R¹⁶), (iv) a group represented by the formula -CO-Q (preferably a group represented by -CO-Q'), and (v) a group represented by the formula -NH-CO-NR¹⁹(R²⁰) described hereinafter and, particularly, a group represented by - CONH-R⁶ as described hereinafter are used. In addition, as the substituent of the "hydrocarbon group" represented by R², a group represented by the formula -CO-J as described hereinafter is preferable.

As the "optionally substituted heterocyclic group" represented by R¹ or R², the same group as the "optionally substituted heterocyclic group" as the substituent which may be possessed by the ring A is used.

The "hydrocarbon group" and the "heterocyclic group" may have 1 to 5 (preferably 1 to 3) substituents at any possible position, respectively. When two or more substituents are possessed, they may be the same or different.

As R¹, an optionally substituted C₁₋₈ alkyl group, and an optionally substituted C₇₋₁₆ aralkyl group are preferable.

As the "optionally substituted C₁₋₈ alkyl group", for example, a C₁₋₈ alkyl group (e.g. methyl, ethyl, propyl, isopropyl, butyl, isobutyl, sec-butyl, tert-butyl, neopentyl etc.) optionally substituted with a halogen atom (e.g. fluorine etc.), carboxyl, or C₁₋₆ alkoxy-carbonyl (e.g. tert-butoxycarbonyl) is used and, inter alia, a branched C₃₋₆ alkyl group such as isobutyl, sec-butyl, tert-butyl, and neopentyl is preferable, and neopentyl is particularly preferable.

As the "optionally substituted C₇₋₁₆ aralkyl group", for example, a C₇₋₁₆ aralkyl optionally substituted with C₁₋₆ alkoxy (e.g. methoxy), or optionally halogenated C₆₋₁₄ aryl (e.g. phenyl) (e.g. phenyl-C₁₋₆ alkyl) is used, and benzyl, 4-biphenylmethyl, and 2,4-dimethoxy-benzyl are particularly preferable.

As R¹, a branched C₃₋₆ alkyl group such as isobutyl, sec-butyl, tert-butyl, and neopentyl is preferable, and neopentyl is particularly preferable.

As R², for example, a C₁₋₆ alkyl group (e.g. methyl) optionally substituted with:
(i) a group represented by the formula -CONR¹² (R¹³) (R¹² represents a hydrogen atom, an optionally substituted hydrocarbon group (e.g. an optionally substituted C₁₋₆ alkyl group, an optionally substituted C₆₋₁₄ aryl group or an optionally substituted C₇₋₁₆ aralkyl group), or an optionally substituted heterocyclic group, and R¹³ represents a hydrogen atom or a C₁₋₆ alkyl group),
(ii) a group represented by the formula -COO-R¹⁴ (R¹⁴ represents a hydrogen atom, or an optionally substituted C₁₋₆ alkyl group),
(iii) a group represented by the formula (-NR¹⁵(R¹⁶)) (R¹⁵ represents a hydrogen atom, an optionally substituted C₇₋₁₆ aralkyl group or an acyl group, and R¹⁶ represents a hydrogen atom or a C₁₋₆ alkyl group),
(iv) a group represented by the formula -CO-Q (Q represents an optionally substituted 5- to 8-membered nitrogen-containing ring group), or
(v) a group represented by the formula -NH-CO-NR¹⁹(R²⁰) (R¹⁹ represents a hydrogen atom or an optionally substituted C₆₋₁₄ aryl group, and R²⁰ represents a hydrogen atom or an optionally substituted C₁₋₆ alkyl group); is preferable, and a C₁₋₆ alkyl group (particularly methyl) optionally substituted with -CONR¹²(R¹³) is particularly preferable.

As R², a group represented by the formula -CO-J (J represents an optionally substituted hydroxy group or an optionally substituted amino group) is also preferable.

As the "optionally substituted hydroxy group" and the "optionally substituted amino group" represented by J, the same groups as the "optionally substituted hydroxy group" and the "optionally substituted amino group" which are the substituent of the ring A are used.

As the "optionally substituted hydroxy group" represented by J, a group represented by the formula -COO-R¹⁴ is preferable.

As the "optionally substituted amino group" represented by the J, (i) a group represented by the formula -CONR¹²(R¹³), and (ii) a group represented by -CO-Q' (Q' represents an optionally substituted 5- to 8-membered cyclic amino group) are preferable.

As the "optionally substituted hydrocarbon group" represented by R¹², the same group as the "optionally substituted hydrocarbon group" as the substituent which may be possessed by the ring A is used.

As the "optionally substituted heterocyclic group" represented by R¹², the same group as the "optionally substituted heterocyclic group" as the substituent which may be possessed by the ring A is used.

As the substituent which may be possessed by the "C₁₋₆ alkyl group", the "C₆₋₁₄ aryl group" or the "C₇₋₁₆ aralkyl group" of the "optionally substituted C₁₋₆ alkyl group", the "optionally substituted C₆₋₁₄ aryl group" or the "optionally substituted C₇₋₁₆ aralkyl group" represented by any of R¹² to R²⁰, the same group as the "substituent" (substituent B group) which may be possessed by the "hydrocarbon group" of the "optionally substituted hydrocarbon group" as the substituent which may be possessed by the ring A is used.

R¹² represents a hydrogen atom, an optionally substituted hydrocarbon group or an optionally substituted heterocyclic group. As R¹², an optionally substituted C₁₋₆ alkyl group, an optionally substituted C₆₋₁₄ aryl group or an optionally substituted C₇₋₁₆ aralkyl group is preferable, inter alia, an optionally substituted benzyl group or an optionally substituted phenyl group is preferable, and an optionally substituted benzyl group is particularly preferable.

As the "optionally substituted C₁₋₆ alkyl group" represented by R¹², for example, a C₁₋₆ alkyl group (e. g. methyl, ethyl, propyl, isopropyl, butyl, isobutyl, sec-butyl, tert-butyl etc., particularly a C₁₋₃ alkyl group such as methyl, ethyl, and propyl) optionally substituted with amino, carboxyl, mono- or di-C₁₋₆ alkylamino (e.g. methylamino, ethylamino, propylamino, butylamino, dimethylamino, diethylamino etc.), mono- or di-C₇₋₁₆ aralkylamino (e.g. benzylamino etc.), cyano, a halogen atom (e.g. fluorine etc.), C₁₋₆ alkoxy (e.g. methoxy, ethoxy etc.), C₃₋₈ cycloalkyl (e.g. cyclopropyl, cyclobutyl, cyclopentyl, cyclohexyl etc.), or a 5- to 10-membered heterocyclic group containing at least one (preferably 1 to 4, further preferably 1 to 2) of 1 to 3 kinds (preferably 1 or 2 kinds) of heteroatoms selected from an oxygen atom, a sulfur atom and a nitrogen atom (e.g. 2-thienyl, 2-thiazolyl, 2-pyridyl, 3-pyridyl, 4-pyridyl, 2-furanyl, 2-tetrahydrofuranyl, 2-tetrahydropyranyl, 4-piperidinyl etc.) is used and, inter alia, a C₁₋₆ alkyl group (e.g. methyl, ethyl, propyl, isopropyl, butyl, isobutyl, sec-butyl, tert-butyl etc., particularly a C₁₋₃ alkyl group such as methyl, ethyl, and propyl) optionally substituted with amino, carboxyl, or a 5- to 10-membered heterocyclic group (e.g. 2-pyridyl, 4-piperidinyl etc.) containing at least one (preferably 1 to 4, further preferably 1 or 2) of 1 to 3 kinds (preferably 1 or 2 kinds) of heteroatoms selected from an oxygen atom, a sulfur atom and a nitrogen atom is preferably used.

As the "optionally substituted C₆₋₁₄ aryl group" represented by R¹², for example, a C₆₋₁₄ aryl group (e.g. phenyl etc.) optionally substituted with a halogen atom (e.g. fluorine etc.), or C₁₋₆ alkoxy (e.g. methoxy, ethoxy etc.) is used.

As the "optionally substituted C₇₋₁₆ aralkyl group" represented by R¹², a C₇₋₁₆ aralkyl group (e.g. benzyl, phenylethyl etc.) optionally substituted with a substituent selected from a halogen atom (e.g. fluorine etc.), a C₁₋₆ alkyl group (e.g. methyl, ethyl, propyl, isopropyl, butyl, isobutyl, sec-butyl, tert-butyl etc.) optionally substituted with a halogen atom (e.g. fluorine etc.), and C₁₋₆ alkoxy (e.g. methoxy, ethoxy, propoxy etc.) is used.

As the "optionally substituted benzyl group" represented by R¹², a benzyl group optionally substituted with a substituent selected from a halogen atom (e.g. fluorine etc.), a C₁₋₆ alkyl group (e.g. methyl, ethyl, propyl, isopropyl, butyl, isobutyl, sec-butyl, tert-butyl etc.) optionally substituted with a halogen atom (e.g. fluorine etc.), and C₁₋₆ alkoxy (e.g. methoxy, ethoxy, propoxy etc.) is preferably used.

In particular, as R¹²,
(i) a C₁₋₆ alkyl group (e.g. methyl, ethyl, propyl, isopropyl, butyl, isobutyl, sec-butyl, tert-butyl etc., particularly a C₁₋₃ alkyl group such as methyl, ethyl, and propyl) optionally substituted with amino, carboxyl, or a 5- to 10-membered heterocyclic group (e.g. 2-pyridyl, 4-piperidinyl etc.) containing at least one (preferably 1 to 4, further preferably 1 or 2) of 1 to 3 kinds (preferably 1 or 2 kinds) of heteroatoms selected from an oxygen atom, a sulfur atom and a nitrogen atom,
(ii) a C₆₋₁₄ aryl group (e.g. phenyl etc.), and
(iii) a C₇₋₁₆ aralkyl group (e.g. benzyl, phenylethyl etc.) optionally substituted with a substituent selected from a halogen atom (e.g. fluorine etc.), and C₁₋₆ alkoxy (e.g. methoxy, ethoxy, propoxy etc.); are preferable.

R¹³ represents a hydrogen atom or a C₁₋₆ alkyl group and, inter alia, a hydrogen atom, methyl, and ethyl are preferable.

R¹⁴ represents a hydrogen atom, or an optionally substituted C₁₋₆ alkyl group, and optionally substituted C₁₋₆ alkyl group is preferable.

As the "optionally substituted C₁₋₆ alkyl group" represented by R¹⁴, an unsubstituted C₁₋₆ alkyl group such as methyl, ethyl, propyl, isopropyl, butyl, isobutyl, sec-butyl, and tert-butyl is preferable, and methyl, and ethyl are particularly preferable.

R¹⁵ represents a hydrogen atom or an optionally substituted C₇₋₁₆ aralkyl group, and an optionally substituted C₇₋₁₆ aralkyl group is preferable.

As the "optionally substituted C₇₋₁₆ aralkyl group" represented by R¹⁵, a C₇₋₁₆ aralkyl group (e.g. benzyl) optionally substituted with a halogen atom (e.g. fluorine etc.) is used.

R¹⁶ represents a hydrogen atom or a C₁₋₆ alkyl group and, inter alia, a hydrogen atom is preferable.

As the "5- to 8-membered nitrogen-containing ring group" of the "optionally substituted 5- to 8-membered nitrogen-containing ring group" represented by Q, a 5- to 8-membered nitrogen-containing ring group optionally having 1 to 3 heteroatoms selected from a nitrogen atom, an oxygen atom and a sulfur atom in addition to one nitrogen atom and carbon atoms is used and, specifically, for example, piperidinyl (e.g. 1-piperidinyl, 4-piperidinyl etc.), piperazino, morpholino, thiomorpholino, and 1,4-diazepanyl are used.

As the "5- to 8-membered nitrogen-containing ring group" represented by Q, a 5- to 8-membered cyclic amino group (Q') is preferable and, for example, 1-piperidinyl, piperazino, morpholino, thiomorpholino, and 1,4-diazepanyl are used.

As the substituent of the "5- to 8-membered nitrogen-containing ring group" or the "5- to 8-membered cyclic amino group", the same substituents as those for the substituent B group are used, inter alia, C₁₋₆ alkyl (e.g. methyl), amino, hydroxy, carboxyl, C₁₋₆ alkoxy-carbonyl (e.g. methoxycarbonyl, ethoxycarbonyl, tert-butoxycarbonyl etc.), C₁₋₆ alkoxycarbonyl-amino (e.g. tert-butoxycarbonylamino etc.), C₁₋₆ alkylcarbonyl-oxy (e.g. acetoxy etc.), and a 5-to 8-membered cyclic amino group (e.g. 1-piperidinyl etc.) are preferable, and C₁₋₆ alkyl (e.g. methyl), amino, hydroxy, carboxyl, C₁₋₆ alkylcarbonyl-oxy (e.g. acetoxy), and a 5- to 8-membered cyclic amino group (e.g. 1-piperidinyl etc.) are preferable.

As the "optionally substituted 5- to 8-membered cyclic amino group", for example, a group represented by the formula: 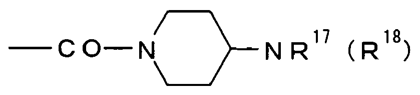
wherein R¹⁷ represents a hydrogen atom or an optionally substituted C₇₋₁₆ aralkyl group, and R¹⁸ represents a hydrogen atom or a C₁₋₆ alkyl group, is also preferable.

R¹⁷ represents a hydrogen atom or an optionally substituted C₇₋₁₆ aralkyl group, and an optionally substituted C₇₋₁₆ aralkyl group is preferable.

As the "optionally substituted C₇₋₁₆ aralkyl group" represented by R¹⁷, a C₇₋₁₆ aralkyl group (e.g. benzyl) optionally substituted with C₁₋₆ alkoxy (e.g. methoxy, ethoxy, propoxy etc.) is used.

R¹⁸ represents a hydrogen atom or a C₁₋₆ alkyl group and, inter alia, a hydrogen atom is preferable.

R¹⁹ represents a hydrogen atom or an optionally substituted C₆₋₁₄ aryl group, and an optionally substituted C₆₋₁₄ aryl group is preferable.

As the "optionally substituted C₆₋₁₄ aryl group" represented by R¹⁹, a C₆₋₁₄ aryl group (e.g. phenyl) optionally substituted with a halogen atom (e.g. fluorine etc.) is used.

R²⁰ represents a hydrogen atom or a C₁₋₆ alkyl group and, inter alia, a hydrogen atom is preferable.

As the compound represented by the formula (I), for example,
(1) a compound represented by the formula: 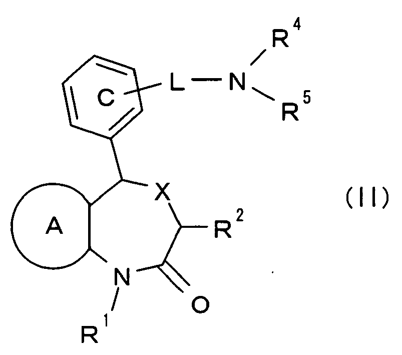
   wherein L represents a linker, R⁴ and R⁵ each represents a hydrogen atom, an optionally substituted hydrocarbon group, an acyl group, an optionally substituted carbamoyl group, an esterified carboxyl group, or an optionally substituted heterocyclic group, R⁴ and R⁵ may be taken together to form a ring, or R⁴ or R⁵ may be taken together with a linker represented by L to form a ring, a ring C represents an optionally further substituted benzene ring, and the other symbols are as defined above,
(2) a compound represented by the formula: 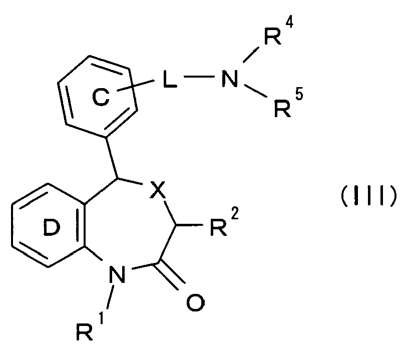
   wherein a ring D represents an optionally substituted benzene ring, and the other symbols are as defined above, and
(3) a compound represented by the formula:

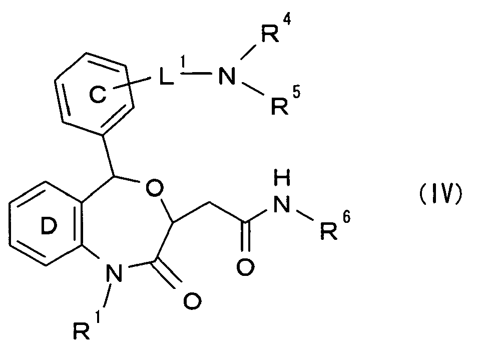

wherein L¹ represents a linker represented by -Y-(CH₂)m- (Y represents a bond, -O-, -S(O)n¹- (n¹ represents an integer of 0 to 2) or -NR⁷- (R⁷ represents a hydrogen atom, an optionally substituted hydrocarbon group, or an optionally substituted heterocyclic group), and m represents an integer of 0 to 6), R⁴ and R⁵ each represents a hydrogen atom, an optionally substituted hydrocarbon group, an acyl group, an optionally substituted carbamoyl group, an esterified carboxyl group, or an optionally substituted heterocyclic group, R⁴ and R⁵ may be taken together to form a ring, or R⁴ or R⁵ may be taken together with a linker represented by L¹ to form a ring, R⁶ represents an optionally substituted hydrocarbon group, or an optionally substituted heterocyclic group, and the other symbols are as defined above, are preferably used.

L represents a linker.

As the "linker", (i) an optionally substituted divalent hydrocarbon group (preferably, an alkylene group), (ii) -O-, (iii) -S(O)n¹- (n¹ represents an integer of 0 to 2), (iv) -NR⁷- (R⁷ represents a hydrogen atom, an optionally substituted hydrocarbon group, or an optionally substituted heterocyclic group), or (v) a group of a combination of two or more (e.g. 2 to 5, preferably 2 to 3) of them is used.

As the "divalent hydrocarbon group" of the "optionally substituted divalent hydrocarbon group", for example, a group obtained by removing a hydrogen atom from two carbon atoms of an alkylene group, an alkenylene group, an alkynylene group, or a cyclic hydrocarbon is used.

As the alkylene group, a C₁₋₆ alkylene group such as methylene, ethylene, and propylene is used and, inter alia, methylene is preferable.

As the alkenylene group, a C₂₋₆ alkenylene group such as -CH = CH-, -CH = CH-CH₂-, -CH₂-CH = CH-CH₂-, -(CH₂)₂-CH = CH-CH₂-, -(CH₂)₂-CH = CH-(CH₂)₂-, and -(CH₂)₃-CH = CH-CH₂- is used.

As the alkynylene group, a C₂₋₆ alkynylene group such as -C≡C-, -C≡C-CH₂-, -CH₂-C≡C-CH₂-, -(CH₂)₂-C≡C-CH₂-, - (CH₂)₂-C≡C-(CH₂)₂-, and - (CH₂) ₃-C≡C-CH₂- is used.

As the group obtained by removing a hydrogen atom from two carbon atoms of a cyclic hydrocarbon, a group obtained by removing a hydrogen atom from two carbon atoms of a C₆₋₁₄ aryl ring (e.g. benzene, naphthalene etc.), or C₃₋₈ cycloalkane (e.g. cyclopropane, cyclobutane, cyclopentane, cyclohexane etc.), such as 1,4-phenylene, and 1,4-cyclohexynylene is used.

As the alkylene group of the "optionally substituted alkylene group", a C₁₋₆ alkylene group such as methylene, ethylene, and propylene is used and, inter alia, methylene, ethylene, and propylene are preferable.

As the substituent of the "divalent hydrocarbon group" or the "alkylene group", the same groups as those for the "substituent" (substituent B group) of the "optionally substituted hydrocarbon group" as the substituent which may be possessed by the ring A are used and, inter alia, an oxo group is preferable.

As the "optionally substituted hydrocarbon group" represented by R⁷, the same group as the "optionally substituted hydrocarbon group" as the substituent which may be possessed by the ring A is used.

As the "optionally substituted heterocyclic group" represented by R⁷, the same group as the "optionally substituted heterocyclic group" as the substituent which may be possessed by the ring A is used.

As n¹, 0 is preferable.

As a linker represented by L, a group represented by the formula -G¹-G²-G³- (G¹ represents a bond or an optionally substituted divalent hydrocarbon group, G² represents -O-, -NR⁸- (R⁸ represents a hydrogen atom, an optionally substituted hydrocarbon group or an optionally substituted heterocyclic group) or -S(O)n² - (n² represents an integer of 0 to 2), and G³ represents an optionally substituted divalent hydrocarbon group) is preferable.

As the "optionally substituted hydrocarbon group" represented by R⁸, the same group as the "optionally substituted hydrocarbon group" as the substituent which may be possessed by the ring A is used.

As the "optionally substituted heterocyclic group" represented by R⁸, the same group as the "optionally substituted heterocyclic group" as the substituent which may be possessed by the ring A is used.

As the "optionally substituted divalent hydrocarbon group" represented by G¹ or G³, the same group as the "optionally substituted divalent hydrocarbon group" exemplified as the linker is used. As the "optionally substituted divalent hydrocarbon group" represented by G¹ or G³ an "optionally substituted alkylene group" is preferable, inter alia, a C₁₋₆ alkylene group such as methylene, ethylene, and propylene is preferably used, and a C₁₋₃ alkylene group such as methylene, and ethylene is particularly preferable.

As G¹, a bond or an optionally substituted C₁₋₃ alkylene group (e.g. methylene) is preferable, inter alia, a bond or a C₁₋₃ alkylene group (e.g. methylene) is preferable, and a bond is particularly preferable.

As G³, an optionally substituted C₂₋₆ alkylene group (e.g. ethylene, propylene, butylene) is preferable and, inter alia, ethylene, and propylene are preferable.

As R⁸, a hydrogen atom is preferable.

As n², 0 is preferable.

As G², -O-, and -NH- are preferable, and -O- is particularly preferable.

In addition, as the linker represented by L, (i) a linker represented by the formula -(CH₂)m¹-W-(CH₂)m²- (m¹ and m² each represents an integer of 0 to 6, and W represents a bond, -O-, -S(O)n¹- (n¹ represents an integer of 0 to 2), -NR⁷-, -CO-, -CO-N(R⁷)- or -N(R⁷)-CO-), and (ii) a linker represented by the formula -W¹-(CH₂)m³-W²-(CH₂)m²⁻(m³ represents an integer of 1 to 6, W¹ and W² each represents a bond, -O-, or -S(O)n¹- (n¹ represents an integer of 0 to 2), and other symbols are as defined above) are preferable.

As m¹, an integer of 0 to 3 is preferable, 0 or 1 is particularly preferable, and 0 is particularly preferable.

As m², an integer of 1 to 6 is preferable, and an integer of 1 to 3 is preferable.

As m³, an integer of 0 to 3 is preferable.

As the combination of m¹ and m², when m¹ is 0 or 1, the case where m² is an integer of 1 to 6 is preferable and, particularly, when m¹ is 0, the case where m² is an integer of 1 to 6 is preferable.

As the combination of m³ and m², when m³ is an integer of 1 to 3, the case where m² is an integer of 0 to 3 is preferable and, particularly, when m³ is an integer of 1 to 3, the case where m² is 0 is preferable.

As R⁷, a hydrogen atom is preferable.

As the linker represented by L, particularly, "optionally substituted -Y-(CH₂)m- (Y represents a bond, - O-, -S(O)n¹- (n¹ represents an integer of 0 to 2) or -NR⁷⁻(R⁷ is as defined above), and m represents an integer of 0 to 6)" represented by L¹ is preferably used.

As the substituent of -Y-(CH₂)m-, the same group as the "substituent" (substituent B group) which may be possessed by the "hydrocarbon group" of the "optionally substituted hydrocarbon group" as the substituent which may be possessed by the ring A is used, but unsubstituted is preferable.

As R⁷, a hydrogen atom is preferable.

As m, an integer of 1 to 3 is preferable, and 3 is particularly suitable.

Among the forgoing, as L, -(CH₂)m³-, -O- (CH₂) m³-, - (CH₂)m⁴-NH-(CH₂)m³-, -(CH₂)m⁴-NHCO-(CH₂)m³-, and -O-(CH₂)m³⁻CO- (m³ represents an integer of 1 to 6, preferably an integer of 1 to 3, and m⁴ represents an integer of 1 to 6, preferably an integer of 1 to 3, and particularly preferably 1) are preferable.

R⁴ and R⁵ each represents a hydrogen atom, an optionally substituted hydrocarbon group, an acyl group, an optionally substituted carbamoyl group, an esterified carboxyl group or an optionally substituted heterocyclic group, R⁴ and R⁵ may be taken together to form a ring, or R⁴ or R⁵ may be taken together with the linker represented by L or L¹ to form a ring.

As the "optionally substituted hydrocarbon group" represented by R⁴ or R⁵, the same group as the "optionally substituted hydrocarbon group" as the substituent which may be possessed by the ring A is used.

As the "acyl group" represented by R⁴ or R⁵, the same group as the "acyl group" as the substituent which may be possessed by the ring A is used.

As the "optionally substituted carbamoyl group" represented by R⁴ or R⁵, the same group as the "optionally substituted carbamoyl group" as the substituent which may be possessed by the ring A is used.

As the "esterified carboxyl group" represented by R⁴ or R⁵, the same group as the "optionally esterified carboxyl group" as the substituent which may be possessed by the ring A other than a free carboxyl group is used.

As the "optionally substituted heterocyclic group" represented by R⁴ or R⁵, the same group as the "optionally substituted heterocyclic group" as the substituent which may be possessed by the ring A is used.

As R⁴, a hydrogen atom, a C₁₋₆ alkyl group (e.g. methyl), a C₇₋₁₆ aralkyl group (e.g. a phenyl-C₁₋₃ alkyl group), and a C₁₋₆ alkoxycarbonyl group (e.g. tert-butoxycarbonyl) are preferable, inter alia, a hydrogen atom, and a C₁₋₆ alkyl group are preferable, and a hydrogen atom is particularly preferable.

As R⁵, a hydrogen atom, an optionally substituted C₁₋₆ alkyl group, an optionally substituted C₂₋₆ alkenyl group, an optionally substituted C₇₋₁₆ aralkyl group, and an esterified carboxyl group are preferable.

As the "optionally substituted C₁₋₆ alkyl group", for example, a C₁₋₆ alkyl group optionally substituted with a substituent selected from the substituent B group is used and, inter alia, a C₁₋₆ alkyl group (e.g. a C₁₋₃ alkyl group such as methyl, and ethyl) optionally substituted with a substituent selected from phenoxy, and a 5- to 10-membered heterocyclic group (e.g. furyl, thienyl, pyridyl, and indolyl) containing at least one of 1 to 3 kinds of heteroatoms selected from an oxygen atom, a sulfur atom and a nitrogen atom is preferable.

As the "optionally substituted C₂₋₆ alkenyl group", for example, a C₂₋₆ alkenyl group optionally substituted with a substituent selected from the substituent B group is used and, inter alia, a C₂₋₆ alkenyl group optionally substituted with C₆₋₁₄ aryl (e.g. phenyl) is preferable.

As the "optionally substituted C₇₋₁₆ aralkyl group", for example, a C₇₋₁₆ aralkyl group (e.g. phenyl-C₁₋₆ alkyl, naphthyl-C₁₋₆ alkyl etc.) optionally substituted with a substituent selected from the substituent B group is used and, inter alia, a C₇₋₁₆ aralkyl group optionally substituted with a substituent selected from a halogen atom (e.g. chlorine atom etc.) and C₁₋₆ alkoxy (e.g. methoxy etc.) is preferable.

As the "esterified carboxyl group", a C₁₋₆ alkoxycarbonyl group such as tert-butoxycarbonyl is preferable.

Inter alia, as R⁵, a hydrogen atom, an optionally substituted C₁₋₆ alkyl group, an optionally substituted C₂₋₆ alkenyl group, and an optionally substituted C₇₋₁₆ aralkyl group are preferable, and a C₇₋₁₆ aralkyl group (e.g. a phenyl-C₁₋₃ alkyl group) optionally substituted with a halogen atom (e.g. chlorine atom), a C₁₋₆ alkyl group, and a phenyl-C₂₋₆ alkenyl group are preferable.

As the ring which R⁴ and R⁵ are taken together to form, the ring which R⁴ or R⁵ is taken together with the linker represented by L or L¹ to form, the ring which R⁴ and G³ are taken together to form, and the ring which R⁴ and R⁸ are taken together to form when G² is -NR⁸-, a 5- to 10-membered nitrogen-containing ring optionally containing at least one (preferably 1 to 4, further preferably 1 to 2) of 1 to 3 kinds (preferably 1 or 2 kinds) of heteroatoms selected from an oxygen atom, a sulfur atom and a nitrogen atom in addition to carbon atoms and one nitrogen atom is used. Specifically, pyrrole, imidazole, indole, isoindole, benzimidazole, indolizine, isoindolizine, azetidine, pyrrolidine, piperidine, azepine, morpholine, thiomorpholine, piperazine, 1,2,3,4-tetrahydroquinoline, and 1,2,3,4-tetrahydroisoquinoline are used and, inter alia, a 5- or 6-membered nitrogen-containing ring such as pyrrole, and piperazine is preferable.

In particular, as the ring which R⁹ and R⁵ are taken together to form, a 5- to 8-membered, preferably 5- or 6-membered cyclic amino group (e.g. pyrrole, piperazine) optionally containing at least one (preferably 1 to 4, further preferably 1 to 2) of 1 to 3 kinds (preferably 1 or 2 kinds) of heteroatoms selected from an oxygen atom, a sulfur atom, and a nitrogen atom in addition to carbon atoms and one nitrogen atom is preferable.

On the other hand, as the ring which R⁴ or R⁵ is taken together with the linker represented by L or L¹ to form, the ring which R⁴ and G³ are taken together to form, and the ring which R⁴ and R⁸ are taken together to form when G² is -NR⁸-, particularly, a 5- to 8-membered non-aromatic nitrogen-containing ring (e.g. 4-piperidine, piperazine) optionally containing at least one (preferably 1 to 4, further preferably 1 or 2) of 1 to 3 kinds (preferably 1 or 2 kinds) of heteroatoms selected from an oxygen atom, a sulfur atom, and a nitrogen atom in addition to carbon atoms and one nitrogen atom is preferable.

As the substituent which may be possessed by the ring which R⁴ and R⁵ are taken together to form, the same group as the "substituent" (substituent B group) which may be possessed by the "hydrocarbon group" of the "optionally substituted hydrocarbon group" as the substituent which may be possessed by the ring A is used and, inter alia, a C₁₋₆ alkyl group (e.g. methyl) is preferable.

As the substituent which may be possessed by the ring which R⁴ or R⁵ is taken together with the linker represented by L or L¹ to form, the ring which R⁴ and G³ are taken together to form, or the ring which R⁴ and R⁸ are taken together to form when G² is -NR⁸-, in addition to R⁵, the same group as the "substituent" (substituent B group) which may be possessed by the "hydrocarbon group" of the "optionally substituted hydrocarbon group" as the substituent which may be possessed by the ring A is used. As R⁵ in this case, a C₁₋₆ alkyl group (e.g. methyl) is preferable.

The ring C represents a benzene ring which may be further substituted in addition to with a group represented by the formula:

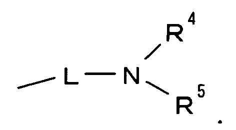

The ring D represents an optionally substituted benzene ring.

As the substituent which may be further possessed by the benzene ring represented by the ring C, the same group as the substituent which may be possessed by the ring A is used, inter alia, a halogen atom, and a C₁₋₆ alkoxy group (e.g. methoxy) are preferable, and a C₁₋₆ alkoxy group is particularly preferable.

As the substituent which may be possessed by the benzene ring represented by the ring D, the same group as the substituent which may be possessed by the ring A is used, inter alia, a halogen atom (e.g. a chlorine atom), a C₁₋₆ alkoxy group, and a C₆₋₁₄ aryl group (e.g. phenyl) are preferable, and a halogen atom (e.g. a chlorine atom), and a C₆₋₁₄ aryl group (e.g. phenyl) are particularly preferable.

R⁶ represents an optionally substituted hydrocarbon group, or an optionally substituted heterocyclic group.

As the "optionally substituted hydrocarbon group" represented by R⁶, the same group as the "optionally substituted hydrocarbon group" as the substituent which may be possessed by the ring A is used.

As the "optionally substituted heterocyclic group" represented by R⁶, the same group as the "optionally substituted heterocyclic group" as the substituent which may be possessed by the ring A is used.

As R⁶, for example, an "optionally substituted C₁₋₆ alkyl group", an "optionally substituted C₆₋₁₄ aryl group", and an "optionally substituted C₇₋₁₆ aralkyl group" are preferable, inter alia, an "optionally substituted C₁₋₆ alkyl group", and an "optionally substituted C₇₋₁₆ aralkyl group" are preferable, and an "optionally substituted C₁₋₆ alkyl group" and an "optionally substituted benzyl group" are particularly preferable.

As the "optionally substituted C₁₋₆ alkyl group" represented by R⁶, for example, a C₁₋₆ alkyl group (e.g. methyl, ethyl, propyl, isopropyl, butyl, isobutyl, sec-butyl, tert-butyl etc., particularly a C₁₋₃ alkyl group such as methyl, ethyl, and propyl) optionally substituted with amino, carboxyl, mono or di-C₁₋₆ alkylamino (e.g. methylamino, ethylamino, propylamino, butylamino, dimethylamino, diethylamino etc.), mono- or di-C₇₋₁₆ aralkylamino (e.g. benzylamino etc.), cyano, halogen atom (e.g. fluorine etc.), C₁₋₆ alkoxy (e.g. methoxy, ethoxy etc.), C₃₋₈ cycloalkyl (e.g. cyclopropyl, cyclobutyl, cyclopentyl, cyclohexyl etc.), or a 5- to 10-membered heterocyclic group containing at least one (preferably 1 to 4, further preferably 1 to 2) of 1 to 3 kinds (preferably 1 or 2 kinds) of heteroatoms selected from an oxygen atom, a sulfur atom and a nitrogen atom (e.g. 2-thienyl, 2-thiazolyl, 2-pyridyl, 3-pyridyl, 4-pyridyl, 2-furanyl, 2-tetraydrofuranyl, 2-tetrahydropyranyl, 4-piperidyl, etc.) is used, inter alia, a C₁₋₆ alkyl group (e.g. methyl, ethyl, propyl, isopropyl, butyl, isobutyl, sec-butyl, tert-butyl, etc., particularly a C₁₋₃ alkyl group such as methyl, ethyl, and propyl) optionally substituted with amino, carboxyl or a 5- to 10-membered heterocyclic group containing at least one (preferably 1 to 4, further preferably 1 to 2) of 1 to 3 kinds (preferably 1 to 2 kinds) of heteroatoms selected from an oxygen atom, a sulfur atom and a nitrogen atom is preferably used.

As the "optionally substituted C₆₋₁₄ aryl group" represented by R⁶, for example, a C₆₋₁₄ aryl group (e.g. phenyl) optionally substituted with a halogen atom (e.g. fluorine etc.) or C₁₋₆ alkoxy (e.g. methoxy, ethoxy etc.) is used.

As the "optionally substituted C₇₋₁₆ aralkyl group" represented by R⁶, a C₇₋₁₆ aralkyl group (e.g. benzyl, phenylethyl etc.) optionally substituted with a substituent selected from a halogen atom (e.g. fluorine etc.), a C₁₋₆ alkyl group (e.g. methyl, ethyl, propyl, isopropyl, butyl, isobutyl, sec-butyl, tert-butyl etc.) optionally substituted with a halogen atom (e.g. fluorine etc.), and C₁₋₆ alkoxy (e.g. methoxy, ethoxy, propoxy etc.) is used.

As the "optionally substituted benzyl group" represented by R⁶, a benzyl group optionally substituted with a substituent selected from a halogen atom (e.g. fluorine etc.), a C₁₋₆ alkyl group (e.g. methyl, ethyl, propyl, isopropyl, butyl, isobutyl, sec-butyl, tert-butyl etc.) optionally substituted with a halogen atom (e.g. fluorine etc.), and C₁₋₆ alkoxy (e.g. methoxy, ethoxy, propoxy etc.) is preferably used.

In addition, as R⁶, an optionally substituted benzyl group or an optionally substituted phenyl group is also preferable. As the substituent which may be possessed by the benzyl group or the phenyl group, the same group as the substituent (substituent A group) which may be possessed by the ring A is used and, inter alia, a halogen atom (e.g. fluorine etc.), a C₁₋₆ alkyl group (e.g. methyl, ethyl, propyl, isopropyl, butyl, isobutyl, sec-butyl, tert-butyl etc.), and C₁₋₆ alkoxy (e.g. methoxy, ethoxy, propoxy etc.) is preferably used.

In particular, as R⁶,
(i) a C₁₋₆ alkyl group (e.g. methyl, ethyl, propyl, isopropyl, butyl, isobutyl, sec-butyl, tert-butyl, etc., particularly a C₁₋₃ alkyl group such as methyl, ethyl, and propyl) optionally substituted with amino, carboxyl, or a 5- to 10-membered heterocyclic group containing at least one (preferably 1 to 4, further preferably 1 to 2) of 1 to 3 kinds (preferably 1 to 2 kinds) of heteroatoms selected from an oxygen atom, a sulfur atom and a nitrogen atom (e.g. 2-pyridyl, 4-piperidinyl etc.),
(ii) a C₆₋₁₄ aryl group (e.g. phenyl etc.), and
(iii) a C₇₋₁₆ aralkyl group (e.g. benzyl, phenylethyl etc.) optionally substituted with a substituent selected from a halogen atom (e.g. fluorine etc.), and C₁₋₆ alkoxy (e.g. methoxy, ethoxy, propoxy etc.) are preferable.

As a ring A, X, R¹ and R², the same groups as those described above are preferably used.

Among the compound represented by the formula (I),
(1) a compound represented by the formula; 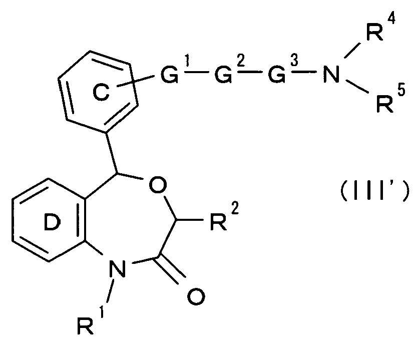
   wherein respective symbols are as defined above, provided that 3,5-trans-N-(2-fluorobenzyl)-5-[3-(3-tert-butoxycarbonylaminopropyl)aminomethylphenyl]-7-chloro-1-neopentyl-2-oxo-1, 2, 3, 5-tetrahydro-4, 1-benzoxazepine-3-acetamide, 3,5-trans-N-(2-fluorobenzyl)-5-[3-(3-aminopropyl)aminomethylphenyl]-7-chloro-1-neopentyl-2-oxo-1,2,3,5-tetrahydro-4,1-benzoxazepine -3-acetamide, 3,5-trans-N-(2-fluorobenzyl)-5-(3-aminoacetylaminomethylphenyl)-1-benzyl-7-chloro-2-oxo-1,2,3,5-tetrahydro-4,1-benzoxazepine-3-acetamide, 3,5-trans-N-(2-fluorobenzyl)-1-(4-biphenylmethyl)-7-chloro-2-oxo-5-[3-[(piperidin-4-yl)carbonylaminomethyl]phenyl]-1,2,3,5-tetrahydro-4,1-benzoxazepine-3-acetamide, 3,5-trans-N-(2-fluorobenzyl)-5-[2-(3-aminopropyloxy)phenyl]-7-chloro-1-isobutyl-2-oxo-1,2,3,5-tetrahydro-4,1-benzoxazepine-3-acetamide, 3-5-trans-N-(2-fluorobenzyl)-5-[4-(3-aminopropyloxy)-2-methoxyphenyl]-7-chloro-1-neopenpyl-2-oxo-1,2,3,5-tetrahydro-4,1-benzoxazepine-3-acetamide, 7-chloro-5-[2-[3-[[(1,1-dimethylethoxy)carbonyl]amino]propoxy]phenyl]-1,2,3,5-tetrahydro-1-(2-methylpropyl)-2-oxo-4,1-benzoxazepine-3-ylacetic acid ethyl ester, 7-chloro-5-[4-[3-[[(1,1-dimethylethoxy)carbonyl]amino]propoxy]-2-methoxyphenyl]-1-(2,2-dimethylpropyl)-1,2,3,5-tetrahydro-2-oxo-4,1-benzoxazepin-3-ylacetic acid ethyl ester are excluded,
(2) a compound represented by the formula; 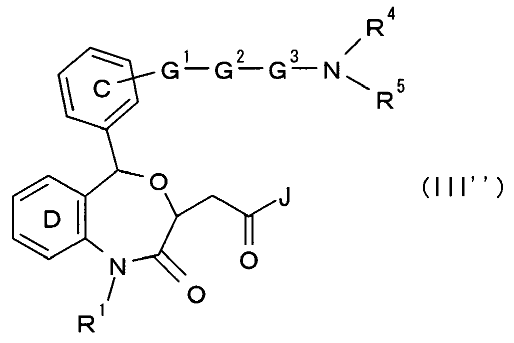
   wherein respective symbols are as defined above, provided that when R⁴ is a hydrogen atom, R⁵ is not a hydrogen atom or a tert-butoxycarbonyl group,
(3) a compound represented by the formula; 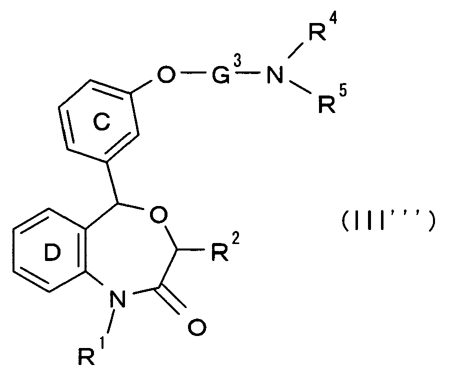
   wherein respective symbols are as defined above, and
(4) a compound represented by the formula;

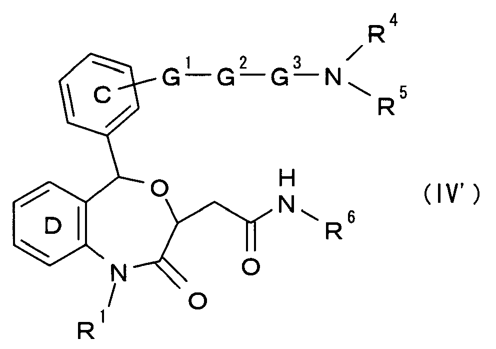

wherein respective symbols are as defined above, provided that when R⁴ is a hydrogen atom, R⁵ is not a hydrogen atom or a tert-butoxycarbonyl group, are novel compounds.

As R¹, an optionally substituted hydrocarbon group is preferable and, inter alia, an optionally substituted C₁₋₈ alkyl group, and an optionally substituted C₇₋₁₆ aralkyl group are preferable.

As the "optionally substituted C₁₋₈ alkyl group", for example, a C₁₋₈ alkyl group (e.g. methyl, ethyl, propyl, isopropyl, butyl, isobutyl, sec-butyl, tert-butyl, neopentyl etc.) optionally substituted with a halogen atom (e.g. fluorine etc.), hydroxyl, C₁₋₆ alkanoyloyloxy (e.g. acetoxy), carboxyl, or C₁₋₆ alkoxy-carbonyl (e.g. tert-butoxycarbonyl) is used, inter alia, a branched C₃₋₆ alkyl group such as isobutyl, sec-butyl, tert-butyl, and neopentyl is preferable, and neopentyl is particularly preferable.

As the "optionally substituted C₇₋₁₆ aralkyl group", for example, a C₇₋₁₆ aralkyl group optionally substituted with C₁₋₆ alkoxy (e.g. methoxy), or an optionally halogenated phenyl (e.g. phenyl-C₁₋₃ alkyl) is used, and 4-biphenylmethyl, and 2,4-dimethoxy-benzyl are preferable.

As R¹, a branched C₃₋₆ alkyl group such as isobutyl, sec-butyl, tert-butyl, and neopentyl is preferable, and neopentyl is particularly preferable.

As R², the same groups as those as described above are preferable.

As G¹, a bond and the aforementioned optionally substituted C₁₋₃ alkylene group are preferable.

As G², -O- is preferable. Inter alia, the case where G¹ is a bond and G² is -O- is preferable.

As G³, the aforementioned optionally substituted C₂₋₆ alkylene group is preferable and, inter alia, ethylene and propylene are preferable.

As R⁴, a hydrogen atom, a C₁₋₆ alkyl group (e.g. methyl), a C₇₋₁₆ aralkyl group (e.g. phenyl-C₁₋₃ alkyl group etc.), and a C₁₋₆ alkoxycarbonyl group (e.g. tert-butoxycarbonyl) are preferable, inter alia, a hydrogen atom, and a C₁₋₆ alkyl group are preferable, and a hydrogen atom is particularly preferable.

As R⁵, a hydrogen atom, an optionally substituted C₁₋₆ alkyl group, an optionally substituted C₂₋₆ alkenyl group, an optionally substituted C₇₋₁₆ aralkyl group, and an optionally esterified carboxyl group (except for free carboxyl group) are preferable and, inter alia an optionally substituted C₁₋₆ alkyl group, a phenyl-C₂₋₆ alkenyl group, and an optionally substituted C₇₋₁₆ aralkyl group are preferable.

As the "optionally substituted C₁₋₆ alkyl group", for example, a C₁₋₆ alkyl group optionally substituted with a substituent selected from the substituent B group and, inter alia, a C₁₋₆ alkyl group (a C₁₋₃ alkyl group such as methyl, and ethyl) optionally substituted with a substituent selected from phenoxy, and a 5- to 10-membered heterocyclic group (e.g. furyl, thienyl, pyridyl, indolyl etc.) containing at least one (preferably 1 to 4, more preferably 1 or 2) of 1 to 3 kinds of heteroatoms selected from an oxygen atom, a sulfur atom and a nitrogen atom is preferable.

As the "optionally substituted C₂₋₆ alkenyl group", for example, a C₂₋₆ alkenyl group optionally substituted with a substituent selected from the substituent B group is used and, inter alia, a C₂₋₆ alkenyl group optionally substituted with C₆₋₁₄ aryl (e.g. phenyl) is preferable.

As the "optionally substituted C₇₋₁₆ aralkyl group", for example, a C₇₋₁₆ aralkyl group optionally substituted with a substituent selected from the substituent B group (e.g. phenyl-C₁₋₆ alkyl, naphthyl-C₁₋₆ alkyl etc.) is used and, inter alia, a C₇₋₁₆ aralkyl group optionally substituted with a substituent selected from a halogen atom (e.g. chlorine atom) and C₁₋₆ alkoxy (e.g. methoxy) is preferable.

As the "optionally esterified carboxyl group (expect for free carboxyl group)", the same group as the "optionally esterified carboxyl group" of the substituent A group (except for free carboxyl group) is used and, inter alia, a C₁₋₆ alkoxylcarbonyl group such as tert-butoxylcarbonyl is preferable.

Inter alia, as R⁵, a hydrogen atom an optionally substituted C₁₋₆ alkyl group, an optionally substituted C₂₋₆ alkenyl group, and an optionally substituted C₇₋₁₆ aralkyl group are preferable, and a C₇₋₁₆ aralkyl group (e.g. phenyl-C₁₋₃ alkyl group) optionally substituted with a halogen atom (e.g. chlorine atom), a C₁₋₆ alkyl group, and a phenyl-C₂₋₆ alkenyl group are preferable.

When G³ is an optionally substituted divalent hydrocarbon group other than a carbonyl group (e.g. the aforementioned optionally substituted C₂₋₆ alkylene group), and R⁴ is a hydrogen atom, the case where R⁵ is not a hydrogen atom or a tert-butoxycarbonyl group is preferable.

As R⁶, for example, an "optionally substituted C₁₋₆ alkyl group", an "optionally substituted C₆₋₁₄ aryl group", and an "optionally substituted C₇₋₁₆ aralkyl group" are preferable, inter alia, an "optionally substituted C₁₋₆ alkyl group", and an "optionally substituted C₇₋₁₆ aralkyl group" are preferable, and an "optionally substituted C₁₋₆ alkyl group", and an "optionally substituted benzyl group" are particularly preferable.

As the "optionally substituted C₁₋₆ alkyl group" represented by R⁶, a C₁₋₆ alkyl group (e.g. methyl, ethyl, propyl, isopropyl, butyl, isobutyl, sec-butyl, tert-butyl, etc., particularly a C₁₋₃ alkyl group such as methyl, ethyl and propyl) optionally substituted with amino, carboxyl, mono- or di-C₁₋₆ alkylamino (e.g. methylamino, ethylamino, propylamino, butylamino, dimethylamino, diethylamino, etc.), mono- or di-C₇₋₁₆ aralkylamino (e.g. benzylamino etc.) cyano, halogen atom (e.g. fluorine etc.), C₁₋₆ alkoxy (e.g. methoxy, ethoxy, etc.), C₃₋₈ cycloalkyl (e.g. cyclopropyl, cyclobutyl, cyclopentyl, cyclohexyl etc.), or a 5- to 10-membered heterocyclic group (e.g. 2-thienyl, 2-thiazolyl, 2-pyridyl, 3-pyridyl, 4-pyridyl, 2-furanyl, 2-tetrahydrofuranyl, 2-tetrahydropyranyl, 4-piperidinyl etc.) containing at least one (preferably 1 to 4, further preferably 1 to 2) of 1 to 3 kinds (preferably 1 to 2 kinds) of heteroatoms selected from an oxygen atom, a sulfur atom and a nitrogen atom is used, inter alia, a C₁₋₆ alkyl group (e.g. methyl, ethyl, propyl, isopropyl, butyl, isobutyl, sec-butyl, tert-butyl etc., particularly a C₁₋₃ alkyl group such as methyl, ethyl, and propyl) optionally substituted with amino, carboxyl, or 5- to 10-membered heterocyclic group (e.g. 2-pyridyl, 4-piperidinyl etc.) containing at least one (preferably 1 to 4, further preferably 1 to 2) of 1 to 3 kinds (preferably 1 or 2 kinds) selected from an oxygen atom, a sulfur atom and a nitrogen atom is preferably used.

As the "optionally substituted C₆₋₁₄ aryl group" represented by R⁶, for example, a C₆₋₁₄ aryl group (e.g. phenyl) optionally substituted with a halogen atom (e.g. fluorine etc.), or C₁₋₆ alkoxy (e.g. methoxy, ethoxy etc.) is used.

As the "optionally substituted C₇₋₁₆ aralkyl group" represented by R⁶, a C₇₋₁₆ aralkyl group (e.g. benzyl, phenylethyl etc.) optionally substituted with a substituent selected from a halogen atom (e.g. fluorine etc.), a C₁₋₆ alkyl group (e.g. methyl, ethyl, propyl, isopropyl, butyl, isobutyl, sec-butyl, tert-butyl etc.) optionally substituted with a halogen atom (e.g. fluorine etc.), and C₁₋₆ alkoxy (e.g. methoxy, ethoxy, propoxy etc.) is used.

As the "optionally substituted benzyl group" represented by R⁶, a benzyl group optionally substituted with a substituent selected from a halogen atom (e.g. fluorine etc.), a C₁₋₆ alkyl group (e.g. methyl, ethyl, propyl, isopropyl, butyl, isobutyl, sec-butyl, tert-butyl, etc.) optionally substituted with a halogen atom (e.g. fluorine etc.), and C₁₋₆ alkoxy (e.g. methoxy, ethoxy, propoxy etc.) is preferably used.

In addition, as R⁶, an optionally substituted benzyl group or an optionally substituted phenyl group is also preferable, and a benzyl group or phenyl group optionally substituted with a substituent such as a halogen atom (e.g. fluorine etc.), a C₁₋₆ alkyl group (e.g. methyl, ethyl, propyl, isopropyl, butyl, isobutyl, sec-butyl, tert-butyl, etc.) optionally substituted with a halogen atom (e.g. fluorine etc.), and C₁₋₆ alkoxy (e.g. methoxy, ethoxy, propoxy etc.) is particularly preferable.

Particularly, as R⁶,
(i) a C₁₋₆ alkyl group (e.g. methyl, ethyl, propyl, isopropyl, butyl, isobutyl, sec-butyl, tert-butyl etc., particularly a C₁₋₃ alkyl group such as methyl, ethyl, and propyl) optionally substituted with amino, carboxyl, or a 5- to 10-membered heterocyclic group (e.g. 2-pyridyl, 4-piperidinyl etc.) containing at least one (preferably 1 to 4, further preferably 1 to 2) of 1 to 3 kinds (preferably 1 to 2 kinds) of heteroatoms selected from an oxygen atom, a sulfur atom and a nitrogen atom,
(ii) a C₆₋₁₄ aryl group (e.g. phenyl etc.), and
(iii) a C₇₋₁₆ aralkyl group (e.g. benzyl, phenylethyl etc.) optionally substituted with a substituent selected from a halogen atom (e.g. fluorine etc.), and C₁₋₆ alkoxy (e.g. methoxy, ethoxy, propoxy etc.); are preferable.

As the "optionally substituted hydroxy group" represented by J, the aforementioned group represented by the formula -COO-R¹⁴ is preferable.

As the "optionally substituted amino group" represented by J, (i) a group represented by the aforementioned formula -CONR¹²(R¹³), and (ii) a group represented by the aforementioned -CO-Q' (Q' represents an optionally substituted 5- to 8-membered cyclic amino group) are preferable.

In addition, as J, a hydroxy group, an optionally substituted lower (C₁₋₆)alkoxy group, an amino group optionally substituted with an optionally substituted alkyl group (e.g. C₁₋₆ alkyl group), and an optionally substituted cyclic amino group are preferable.

As the "optionally substituted lower (C₁₋₆)alkoxy group", a C₁₋₆ alkoxy group (e.g. methoxy, ethoxy) optionally substituted with a substituent selected from the substituent B group is used and, inter alia, an unsubstituted C₁₋₆ alkoxy group is preferable.

As the "amino group optionally substituted with an optionally substituted alkyl group", an amino group optionally substituted with a C₁₋₆ alkyl group optionally substituted with a substituent selected from the substituent B is used, inter alia, an amino group optionally substituted with a C₁₋₆ alkyl group (e.g. methyl, ethyl, propyl, isopropyl, butyl, isobutyl, sec-butyl, tert-butyl etc., particularly a C₁₋₃ alkyl group such as methyl, ethyl, and propyl) optionally substituted with amino, carboxyl, mono- or di-C₁₋₆ alkylamino (e.g. methylamino, ethylamino, propylamino, butylamino, dimethylamino, diethylamino etc.), mono- or di-C₇₋₁₆ aralkylamino (e.g. benzylamino etc.), cyano, halogen atom (e.g. fluorine etc.), C₁₋₆ alkoxy (e.g. methoxy, ethoxy etc.), C₃₋₈ cycloalkyl (e.g. cyclopropyl, cyclobutyl, cyclopentyl, cyclohexyl etc.), or a 5- to 10-membered heterocyclic group containing at least one (preferably 1 to 4, further preferably 1 to 2) of 1 to 3 kinds (preferably 1 to 2 kinds) of heteroatoms selected from an oxygen, a sulfur atom and a nitrogen atom (e.g. 2-thienyl, 2-thiazolyl, 2-pyridyl, 3-pyridyl, 4-pyridyl, 2-furanyl, 2-tetrahydrofuranyl, 2-tetrahydropyranyl, 4-piperidinyl etc.) is used and, inter alia, a C₁₋₆ alkyl group (e.g. methyl, ethyl, propyl, isopropyl, butyl, isobutyl, sec-butyl, tert-butyl etc.), particularly a C₁₋₃ alkyl group such as methyl, ethyl, and propyl) optionally substituted with amino, carboxyl, or a 5- to 10-membered heterocyclic group (e.g. 2-pyridyl, 4-piperidinyl etc.) is preferably used.

As the "optionally substituted cyclic amino group", for example a 5- to 8-membered cyclic amino group (e.g. 1-piperidinyl, piperazino, morpholino, 1,4-diazepanyl etc.) optionally substituted with a substituent selected from the substituent B group and, inter alia, a 5- to 8-membered cyclic amino group optionally substituted with a substituent selected from C₁₋₆ alkyl (e.g. methyl), amino, C₁₋₆ alkoxycarbonylamino (e.g. tert-butoxycarbonylamino), hydroxyl, carboxyl, C₁₋₆ alkoxycarbonyl (e.g. methoxcarbonyl, ethoxycarbonyl, tert-butoxycarbonyl etc.), C₁₋₆ alkylcarbonyl-oxy (e.g. acetoxy) and a 5- to 8-membered cyclic amino group (e.g. 1-piperidinyl) is preferably used.

In particular, as J, 5- to 8-membered cyclic amino group (e.g. 1- piperidinyl, 4- piperidinyl, morpholino, 1,4-diazepanyl etc.) optionally substituted with C₁₋₆ alkyl (e.g. methyl), C₆₋₁₄ aryl (e.g. phenyl), hydroxy, carboxyl, amino, C₁₋₆ alkoxycarbonyl (e.g. methoxycarbonyl, ethoxycarbonyl, tert-butoxycarbonyl etc.), C₁₋₆ alkylcarbonyl-oxy (e.g. acetoxy), or a 5- to 8-membered cyclic amino group (e.g. 1-piperidinyl) is preferable.

As the ring C, a benzene ring optionally further substituted with a halogen atom or a C₁₋₆ alkoxy group in addition to with a group represented by the formula; 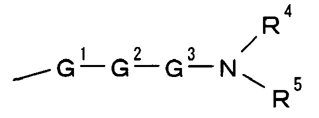 is preferable.

As the ring D, a benzene ring optionally substituted with a halogen atom (e.g. chlorine atom), a C₁₋₆ alkoxy group, or a C₆₋₁₄ aryl group (e.g. phenyl) is preferable, and a benzene ring optionally substituted with a halogen atom (e.g. chlorine atom) is preferable.

As the compound represented by the formula (I), specifically, compounds prepared in Reference Examples 7 to 10, 12, 14 to 24, and 27 to 32, Examples 1 to 98 described hereinafter are preferable.

As the compound represented by the formula (III'), specifically, compounds prepared in Examples 1 to 98 are preferable.

A prodrug of the compound represented by the formula (I) or a salt thereof [hereinafter, referred to as compound (I) in some cases] refers to a compound which is converted into a compound (I) by a reaction with an enzyme or gastric acid under the physiological condition in a living body, that is, a compound which causes oxidation, reduction or hydrolysis enzymatically, and is changed into a compound (I), and a compound which causes hydrolysis with gastric acid, and is changed into a compound (I). Examples of a prodrug of a compound (I) include a compound in which an amino group of a compound (I) is acylated, alkylated, or phosphorylated (e.g. a compound in which an amino group of a compound (I) is eicosanoylated, alanylated, pentylaminocarbonylated, (5-methyl-2-oxo-1, 3-dioxolan-4-yl) methoxycarbonylated, tetrahydrofuranylated, pyrrolidylmethylated, pivaloyloxymethylated, or tert-butylated), a compound in which a hydroxy group of a compound (I) is acylated, alkylated, phosphorylated, or converted into borate (e.g. a compound in which a hydroxy group of a compound (I) is acetylated, palmitoylated, propanoylated, pivaloylated, succinylated, fumarylated, alanylated, or dimethylaminomethylcarbonylated), and a compound in which a carboxyl group of a compound (I) is esterified, or amidated (e.g. a compound in which a carboxy group of a compound (I) is ethylesterified, phenylesterified, carboxymethylesterified, dimethylaminomethylesterified, pivaloyloxymethylesterified, ethoxycarbonyloxyethylesterified, phthalidylesterified, (5-methyl-2-oxo-1,3-dioxolan-4-yl)methylesterified, cyclohexyloxycarbonylethylesterified, or methylamidated). These compounds can be prepared from the compound (I) by the known per se method.

Alternatively, a prodrug of the compound (I) may be a compound which is changed into a compound (I) under the physiological condition, described in "Development of Medicament", vol. 7, Molecular Design, p163-198 published by Hirokawashoten in 1990.

Alternatively, the compound (I) may be labeled with an isotope (e.g. ³H, ¹⁴C, ³⁵S, ¹²⁵I etc.).

Further, a compound (I) may be an anhydride or a hydrate.

Examples of the salt of the compound (I) include a metal salt, an ammonium salt a salt with an organic base, a salt with an inorganic acid, a salt with an organic acid, and a salt with basic or acidic amino acid. Preferable examples of the metal salt include an alkali metal salt such as a sodium salt, and a potassium salt; an alkaline earth metal salt such as a calcium salt, a magnesium salt, and a barium salt; an aluminum salt. Preferable example of the salt with an organic base include a base with trimethylamine, triethylamine, pyridine, picoline, 2,6-lutidine, ethanolamine, diethanolamine, triethanolamine, cyclohexylamine, dicyclohexylamine, or N, N'-dibenzylethylenediamine. Preferable examples of the salt with an inorganic acid include a salt with hydrochloric acid, hydrobromic acid, nitric acid, sulfuric acid, or phosphoric acid. Preferable examples of the salt with an organic acid include a salt with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid or p-toluenesulfonic acid. Preferable examples of the salt with basic amino acid include a salt with arginine, lysine, or ornithine, and preferable examples of the salt with acidic amino acid include a salt with aspartic acid, or glutamic acid.

Among them, a pharmaceutically acceptable salt is preferable. For example, when a compound has an acidic functional group, examples include an inorganic salt such as an alkali metal salt (e.g. sodium salt, potassium salt etc.) an alkaline earth metal salt (e.g. calcium salt, magnesium salt, barium salt etc.), and an ammonium salt and, when the compound has a basic functional group, examples include a salt with an inorganic acid such as hydrochloric acid, hydrobromic acid, nitric acid, sulfuric acid, and phosphoric acid, and a salt with an organic acid such as acetic acid, phthalic acid, fumaric acid, oxalic acid, tartaric acid, maleic acid, citric acid, succinic acid, methanesulfonic acid, and p-toluenesulfonic acid.

When the present compound contains an optical isomer, a steric isomer, a positional isomer, or a rotational isomer, these isomers are included in the compounds of the present invention, and each of them can be obtained as an isolated product by a per se known synthesizing or separating method. For example, when there are optical isomers in the compound of the present invention, an optical isomer resolved from the compound is also included in the compound of the present invention.

An optical isomer can be prepared by a per se known method. Specifically, an optical isomer is obtained by performing optical resolution using an optically active synthetic intermediate, or performing optical resolution of a final racemic compound according to a conventional method.

As an optical resolution method, a per se known method such as a fractionation recrystallizing method, a chiral column method, and a diastereomer method is used.

### 1) Fractionation recrystallizing method

A method of forming a salt of a racemic compound with an optically active compound (e.g. (+)-mandelic acid, (-)-mandelic acid, (+)-tartaric acid, (-)-tartaric acid, (+)-1-phenethylamine, (-)-1-phenethylamine, cinchonine, (-)-chichonidine, brucine etc.), separating this by a fractionation recrystallizing method and, optionally, performing a neutralizing step to obtain a free optical isomer.

### 2) Chiral column method

A method of subjecting a racemic compound or a salt thereof to an optical isomer separating column (chiral column) to separate an optical isomer. For example, in case of liquid chromatography, a mixture of optical isomers is added to a chiral column such as ENANTIO-OVM (manufactured by Tosoh Corporation) and CHIRAL series manufactured by Daicel Chemical Industries, Ltd., and the column is developed with water, various buffers (e.g. phosphate buffer), or an organic solvent (e.g. ethanol, methanol, isopropanol, acetonitrile, trifluoroacetic acid, diethylamine etc.) alone or a mixed solution, thereby, an optical isomer is separated. In addition, for example, in the case of gas chromatography, an optical isomer is separated using a chiral column such as CP-Chirasil-DeX CB (manufactured by GL Science).

### 3) Diastereomer method

A method of obtaining an optical isomer by converting a mixture of a racemic modification into a mixture of a diastereomer by a chemical reaction with an optically active reagent, converting this into a single substance via a conventional separating procedure (e.g. fractionation recrystallization, chromatography etc.) and, thereafter, severing an optically active reagent site by chemical treatment such as a hydrolysis reaction. For example, when the present compound has hydroxy or primary or secondary amino in a molecule, by subjecting the compound and an optically active organic acid (e.g. MTPA [α-methoxy-α-(trifluoromethyl)phenylacetic acid], (-)-menthoxyacetic acid etc.) to a condensing reaction, a diastereomer as an ester form or an amide form is obtained. On the other hand, when the present compound has a carboxylic acid group, by subjecting the compound and an optical active amine or an alcohol reagent to a condensing reaction, a diastereomer as an amide form or an ester form is obtained. A separated diastereomer is converted into an optical isomer of an original compound by subjecting to an acid hydrolyzing or basic hydrolyzing reaction.

The compound (I) or a salt thereof can be prepared by the methods described, for example, in WO98/47882, EP-567029, WO97/10224, and WO93/17129, or methods similar thereto.

The novel compound represented by the general formula (III') or a salt thereof can be prepared by the following methods or a similar method.

In the following each preparing method, when an alkylation reaction, a reduction reaction, an oxidation reaction, a protecting or deprotecting reaction, or a reductive alkylation reaction is performed, these reactions are performed according to per se known methods. Examples of such methods include the methods described, for example, in ORGANIC FUNCTIONAL GROUP PREPARATIONS 2^{nd} edition, ACADEMIC PRESS, INC. published in 1989; Comprehensive Organic Transformations, VCH Publishers Inc., 1989.

For example, the compound (III') can be prepared from a compound represented by the general formula (A-1); 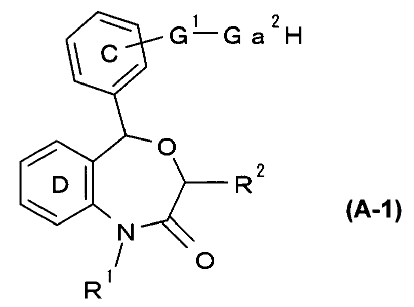
wherein Ga² represents -O-, -NR⁸- (R⁸ represents a hydrogen atom, an optically substituted divalent hydrocarbon group or an optionally substituted heterocyclic group) or -S-, and the other symbols are defined as above, which can be prepared by the methods described, for example, in WO98/47882, EP-567029, WO97/10224, and WO93/17129, or methods similar thereof, or a salt thereof.

### (Method A)

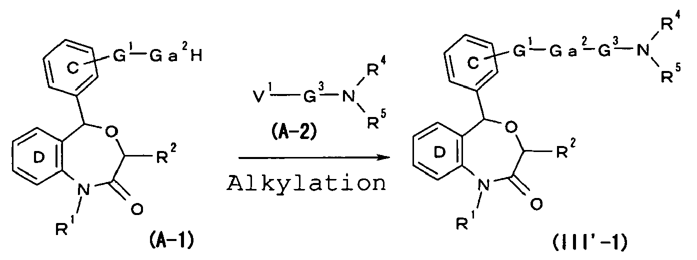

In the formula, V¹ represents a leaving group (e.g. a halogen atom (e.g. a chlorine atom, a bromine atom, an iodine atom) or R²¹SO₂-O- (R²¹ represents an optionally halogenated lower alkyl group or an optionally substituted phenyl group)), and the other symbols are as defined above.

The reaction from the compound represented by the general formula (A-1) or a salt thereof into the compound represented by the general formula (III'-1) or a salt thereof in the above (Method A) can be performed in a solvent such as an ether solvent (e.g. diethyl ether, tetrahydrofuran, dioxane etc.), a hydrocarbon solvent (e.g. benzene, toluene, hexane, heptane etc.), an alcohol solvent (e.g. methanol, ethanol, propanol etc.), acetone, and dimethylformamide and, if necessary, in the presence of a base (e.g. sodium bicarbonate, potassium bicarbonate, sodium carbonate, potassium carbonate, sodium hydride, potassium hydride etc.). In this reaction, the compound represented by the general formula (A-2) or a salt thereof is used at about 1 to 10 mole equivalents, preferably about 1 to 2 mole equivalent relative to 1 mole of the compound represented by the general formula (A-1) or a salt thereof. At this time, the reaction temperature is about 0 to 100°C, preferably about 20 to 50°C, and the reaction time is about 1 to 24 hours, preferably about 3 to 10 hours.

The thus obtained compound (III'-1) can be isolated and purified by known separation and purification means such as concentration, concentration under reduced pressure, solvent extraction, crystallization, recrystallization, reextraction, chromatography, and preparative high performance liquid chromatography (HPLC).

### (Method B)

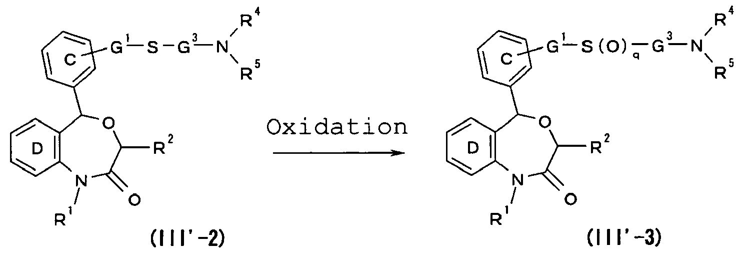

In the formula, q represents an integer of 1 or 2, and the other symbols are as defined above, and the compound represented by the general formula (III'-2) represents a compound in which Ga is -S- in the compound represented by the general formula (III'-1).

The reaction from the compound represented by the general formula (III'-2) or a salt thereof into the compound represented by the general formula (III'-3) or a salt thereof in the above (Method B) is an oxidation reaction of a sulfur atom, and can be performed in a single solvent such as an ether solvent (e.g. diethyl ether, tetrahydrofuran, dioxane etc.), a hydrocarbon solvent (e.g. benzene, toluene, hexane, heptane etc.) a halogenated hydrocarbon solvent (e.g. dichloromethane, chloroform, dichloroethane, chlorobenzene etc.), and an alcohol solvent (e.g. methanol, ethanol, propanol etc.), acetone, acetonitrile, dimethylformamide, or water or in a mixed solvent thereof, using an oxidizing agent (e.g. peracetic acid, permetachlorobenzoic acid, oxone, sodium metaperiodate). In this reaction, the oxidizing agent is used at about 1 to 10 mole equivalent, preferably about 1 to 2 mole equivalent relative to 1 mole of the compound represented by the general formula (III'-2) or a salt thereof. At this time, the reaction temperature is about 0 to 100°C, preferably about 0 to 50°C, and the reaction time is about 1 to 24 hours, preferably about 1 to 10 hours.

The thus obtained compound (III'-3) can be isolated and purified by the known separation and purification means such as concentration, concentration under reduced pressure, solvent extraction, crystallization, recrystallization, reextraction, chromatography, and preparative HPLC.

### (Method C)

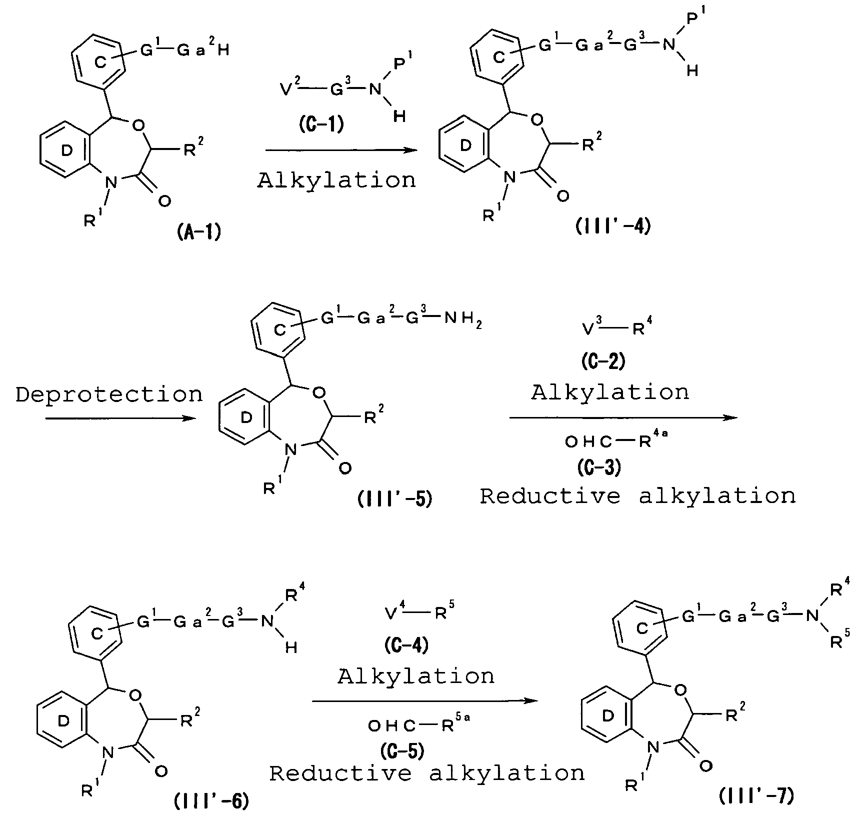

In the formula, V², V³ and V⁴ each represents a leaving group (e.g. a halogen atom (e.g. a chlorine atom, a bromine atom, a iodine atom) or R²² SO₂-O- (R²² represents an optionally halogenated lower alkyl group or an optionally substituted phenyl group)), R^{4a} and R^{5a} each represents a group obtained by removing a methylene group from R⁴ or R⁵, P¹ represents a protecting group for an amino group and the other symbols are as defined above.

The reaction from the compound represented by the general formula (A-1) or a salt thereof into the compound represented by the general formula (III'-4) or a salt thereof in the above (Method C) is an alkylation reaction, and can be performed in a solvent such as an ether solvent (e.g. diethyl ether, tetrahydrofuran, dioxane etc.), a hydrocarbon solvent (e.g. benzene, toluene, hexane, heptane etc.), an alcohol solvent (e.g. methanol, ethanol, propanol etc.), acetone and dimethylformamide and, if necessary, in the presence of a base (e.g. sodium bicarbonate, potassium bicarbonate, sodium carbonate, potassium carbonate, sodium hydride, potassium hydride etc.). In this reaction, the compound represented by the general formula (C-1) or a salt thereof is used at about 1 to 10 mole equivalent, preferably about 1 to 2 mole equivalent relative to 1 to mole of the compound represented by the general formula (A-1) or a salt thereof. At this time, the reaction temperature is about 0 to 100°C, preferably about 20 to 50°C, and a reaction time is about 1 to 24 hours, preferably about 3 to 10 hours.

The thus obtained compound (III'-4) can be isolated and purified by a known separation and purification means such as concentration, concentration under reduced pressure, solvent extraction, crystallization, recrystallization, reextraction, chromatography, and preparative HPLC.

The preparation step from the compound represented by the general formula (III'-4) or a salt thereof into the compound represented by the general formula (III'-5) or salt thereof in the above (Method C) is a deprotection reaction of the amino group, and a per se known method is used in a solvent such as an ether solvent (e.g. diethyl ether, tetrahydrofuran, dioxane etc.), a hydrocarbon solvent (e.g. benzene, toluene, hexane, heptane etc.), an alcohol solvent (e.g. methanol, ethanol, propanol, butanol etc.), a halogenated solvent (e.g. dichloromethane, dichloroethane, chloroform etc.), acetone, acetonitrile, ethyl acetate, and dimethylformamide. P¹ can be removed by catalytic reduction using palladium, or platinum as a catalyst when P¹ is a carbobenzyloxy group, or by dissolving or suspending in an acid (e.g. hydrochloric acid, hydrobromic acid, trifluoroacetic acid, a solution of hydrogen chloride in ether acetate etc.) when P¹ is a tert-butoxycarbonyl group. At this time, the reaction temperature is about 0 to 100 °C, preferably about 0 to 50°C, and the reaction time is about 0.1 to 24 hours, preferably about 1 to 10 hours.

The thus obtained compound (III'-5) can be isolated and purified by a known separation and purification means such as concentration, concentration under reduced pressure, solvent extraction, crystallization, recrystallization, reextraction, chromatography, and preparative HPLC.

The reaction from the compound represented by the general formula (III'-5) or a salt thereof into the compound represented by the general formula (III'-6) or a salt thereof in the above (Method C) can be performed in a solvent such as an ether solvent (e.g. diethyl ether, tetrahydrofuran, dioxane etc.), a hydrocarbon solvent (e.g. benzene, toluene, hexane, heptane etc.), an alcohol solvent (e.g. methanol, ethanol, propanol etc.), acetone, and dimethylformamide and, if necessary, in the presence of a base (e.g. sodium bicarbonate, potassium bicarbonate, sodium carbonate, potassium carbonate, sodium hydride, potassium hydride etc.). In this reaction, the compound represented by the general formula (C-2) or a salt thereof is used at about 1 to 10 mole equivalent, preferably about 1 to 2 mole equivalent relative to 1 mole of the compound represented by the general formula (III'-5) or a salt thereof. At this time, the reaction temperature is about 0 to 100°C, preferably about 20 to 50°C, and the reaction time is about 1 to 24 hours, preferably about 3 to 10 hours.

In addition, the compound represented by the general formula (III'-6) or a salt thereof can be also prepared from the compound represented by the general formula (III'-5) or a salt thereof and the compound represented by the general formula (C-3) or a salt thereof by applying a catalytic reduction reaction, or a reductive amination reaction using sodium borohydride, sodium triacetoxyborohydride or sodium cyanoborohydride, in a solvent such as an ether solvent (e.g. diethyl ether, tetrahydrofuran, dioxane etc.), a hydrocarbon solvent (e.g. benzene, toluene, hexane, hepane etc.), and an alcohol solvent (e.g. methanol, ethanol, propanol, butanol etc.). It is preferable that this reaction is performed in the presence of an acid such as acetic acid and trifluoroacetic acid. At this time, the compound represented by the general formula (C-3) or a salt thereof is used at about 1 to 10 mole equivalent, preferably about 1 to 2 mole equivalent, the acid is used at 0 to 5 mole equivalent, preferably 0 to 1.5 mole equivalent, and the reducing agent is used at about 0.3 to 5 mole equivalent, preferably about 0.5 to 1 mole equivalent relative to 1 mole of the compound represented by the general formula (III'-5) or a salt thereof. At this time, the reaction temperature is about 0 to 100°C, preferably about 10 to 70°C, and the reaction time is about 1 to 24 hours, preferably about 3 to 10 hours.

The thus obtained compound (III'-6) can be isolated and purified by a known separation and purification means such as concentration, concentration under reduced pressure, solvent extraction, crystallization, recrystallization, reextraction, chromatography, and preparative HPLC.

The reaction from the compound represented by the general formula (III'-6) or a salt thereof into the compound represented by the general formula (III'-7) or a salt thereof in the above (Method C) can be performed using the compound represented by the general formula (C-4) or the general formula (C-5) or a salt thereof by using the same reaction as that of the preparation step of the compound represented by the general formula (III'-6) from the compound represented by the general formula (III'-5). The thus obtained compound (III'-7) can be isolated and purified by a known separation and purification means such as concentration, concentration under reduced pressure, solvent extraction, crystallization, recrystallization, reextraction, chromatography, and preparative HPLC.

In each reaction in the aforementioned process for preparing the compound (III') or each reaction of synthesizing the starting compound, when the starting compound has an amino group, a carboxyl group, or a hydroxy group as a substituent, a protecting group which is generally used in peptide chemistry may be introduced into these groups and, if necessary, a protecting group is removed after the reaction, thereby, the objective compound can be obtained.

As the protecting group for an amino group, for example, formyl, optionally substituted, C₁₋₆ alkyl-carbonyl (e.g. acetyl, ethylcarbonyl etc.), phenylcarbonyl, C₁₋₆ alkyl-oxycarbonyl (e.g. methoxycarbonyl, ethoxycarbonyl, tert-butoxycarbonyl (Boc) etc.), allyloxycarbonyl (Aloc), phenyloxycarbonyl, fluorenylmethyloxycarbonyl (Fmoc), C₇₋₁₀ aralkyl-carbonyl (e.g. benzylcarbonyl etc.), C₇₋₁₀ aralkyloxycarbonyl (e.g. benzyloxycarbonyl (Z) etc.), C₇₋₁₀ aralkyl (e.g. benzyl, etc.), trityl, phthaloyl or N, N-dimethylaminomethylene is used. As the substituent for them, a phenyl group, a halogen atom (e.g. fluorine, chlorine, bromine, iodide), C₁₋₆ alkyl-carbonyl (e.g. methylcarbonyl, ethylcarbonyl, butylcarbonyl etc.), and a nitro group are used, and the number of substituents is around 1 to 3.

As the protecting group for a carboxyl group, for example, optionally substituted, C₁₋₆ alkyl (e.g. methyl, ethyl, n-propyl, i-propyl, n-butyl, tert-butyl etc.), allyl, benzyl, phenyl, trityl or trialkylsilyl is used. As the substituent for them, a halogen atom (e.g. fluorine, chlorine, bromine, iodine), formyl, C₁₋₆ alkyl-carbonyl (e.g. acetyl, ethylcarbonyl, butylcarbonyl etc.), and a nitro group are used, and the number of substituents is about 1 to 3.

As the protecting group for a hydroxyl group, for example, optionally substituted, C₁₋₆ alkyl (e.g. methyl, ethyl, n-propyl, i-propyl, n-butyl, tert-butyl etc.), C₇₋₁₀ aralkyl (e.g. benzyl etc.), formyl, C₁₋₆ alkyl-carbonyl (e.g. acetyl, ethylcarbonyl etc.), benzoyl, C₇₋₁₀ aralkyl-carbonyl (e.g. benzylcarbonyl etc.), tetrahydropyranyl, furanyl or silyl is used. As a substituent for them, a halogen atom (e.g. fluorine, chlorine, bromine, iodine), C₁₋₆ alkyl (e.g. methyl, ethyl, n-propyl etc.), phenyl, C₇₋₁₀ aralkyl (e.g. benzyl), C₁₋₆ alkoxy (e.g. methoxy, ethoxy, n-propoxy etc.), and a nitro group are used, and the number of substituents is around 1 to 4.

In addition, as a method of removing a protecting group, a known per se method or a similar method is used, for example, a method of treating with an acid, a base, reduction, ultraviolet light, hydrazine, phenylhydrazine, sodium N-methyldithiocarbamate, tetrabutylammonium fluoride, or palladium acetate is used.

Since the RFRP receptor function modulating agent containing the compound (I) or a salt thereof or a prodrug thereof (hereinafter, abbreviated as compound (I) of the present invention) has low toxicity (e.g. acute toxicity, chronic toxicity, hereditary toxicity, reproduction toxicity, cardiac toxicity, drug interaction, carcinogenecity) and little side effect, it is useful as a safe medicament.

AN RFRP receptor is a receptor to which a peptide having an RF amide structure can bind, and examples include a G protein conjugated-type receptor protein OT7T022 (e.g. a human RFRP receptor having an amino acid sequence represented by SEQ ID No.: 1, a rat RFRP receptor having an amino acid sequence represented by SEQ ID No.:2) described in WO 00/29441, and a mouse RFRP receptor having an amino acid sequence represented by SEQ ID No.:3.

Function modulating refers to both of activity of inhibiting the function of an RFRP receptor (e.g. RFRP receptor antagonizing activity, RFRP receptor antagonist activity), and activity of promoting the function of an RFRP receptor (e.g. RFRP receptor agonistic activity, RFRP receptor agonist activity). In the present invention, activity of inhibiting the function of an RFRP receptor, inter alia, RFRP receptor antagonist activity is more preferable.

RFRP receptor function modulating activity, RFRP receptor agonist activity, and RFRP receptor antagonist activity can be measured using a method of screening a compound by changing binding property between RFRP and OT7T022 described in WO 00/29441.

Since the agent for modulating the function of an RFRP receptor of the present invention exhibits excellent RFRP receptor function modulating activity, particularly, RFRP receptor antagonism (RFRP receptor antagonist activity) to a mammal (e.g. mouse, rat, hamster, rabbit, cat, dog, cow, sheep, monkey, human etc.), and is excellent in (oral) absorbing property, and (metabolism) stability, it is useful as an agent for preventing or treating RFRP-associated morbid state or a disease involved in RFRP, an analgesic, an agent for promoting analgesic activity of another analgesic (e.g. morphine), or an agent for avoiding resistance due to another analgesic (e.g. morphine).

Further, the agent for modulating the function of an RFRP receptor of the present invention is useful as a prolactin secretion modulating agent, preferably also as an agent for suppressing the prolactin secretion, and is useful as an agent for preventing or treating, for example, hyperprolactinenia, pituitary gland tumor, diencephalons, emmeniopathy, stress, autoimmune disease, prolactinoma, infertility, impotence, amenorrhea, lactic leakage, acromegaly, Chiari-Frommel syndrome, Argonz-del Castilo syndrome, Forbes-Albright syndrome, breast cancer lymphoma, Sheehan's syndrome, or spermatogenesis abnormality.

Further, the agent for modulating the function of an RFRP receptor of the present invention is useful as an agent for preventing, treating or improving a muscular disease, adrenal gland function disorder, spasm, aggressive behavior, walking abnormality, body temperature elevation, decrease in the number of leukocyte, decrease in the number of platelet, increase in spontaneous behavior amount or decrease in a muscular force.

Further, the agent for modulating the function of an RFRP receptor of the present invention is useful as an agnet for modulating the male hormone secretion, preferably as an agent for inhibiting the male hormone secretion (an agent for suppressing the male hormone secretion). Specifically, the agent for modulating the function of an RFRP receptor of the present invention is useful as an agent for preventing or treating, for example, male gonad function failure, male infertility accompanied with spermatogenesis function disorder, aplastic anemia, marrow fibrosis, renal anemia, pain alleviation of terminal female sexual organ cancer, breast cancer (e.g. unoperational breast cancer), mastopathy, mammary gland tumor, or gynecomastia.

Further, the agent for modulating the function of an RFRP receptor of the present invention is useful as an agent for suppressing the pancreatic glucagon secretion, a blood glucose lowering agent, a uropoiesis suppressing agent, or an agent for suppressing the deterioration of memory and learning abilities (an agent for suppressing the memory decrease), and is useful as an agent for preventing or treating diabetes, glucose tolerance disorder, ketosis, acidosis, diabetic neuropathy, nephropathy, diabetic retinopathy, pollakiuria, nocturnal enuresis, hyperlipemia, sexual function disorder, skin disease, arthritis, osteopenia, arteriosclerosis, thrombotic diseases, maldigestion, or memory and learning disabilities.

Further, the agent for modulating the function of an RFRP receptor of the present invention is useful, for example, as a bladder constriction inhibiting agent, and is useful as an agent for preventing or treating urine incontinence, lower urinary tract disease, urge micturition due to excessively active bladder, pollakiuria, or hypotonic bladder accompanied with excessively active bladder.

In particular, then agent for modulating the function of an RFRP receptor of the present invention is useful as an analgesic, or as an agent for preventing or treating memory and learning disabilities.

When the compound (I) of the present invention is applied to each of the aforementioned diseases, it is possible to conveniently use a drug or a treating method which is conventionally used in those diseases jointly.

Further, when the compound (I) of the present invention is applied to each of the aforementioned diseases, it is also possible to use a biological preparation (e.g. antibody, vaccine preparation), or it is also possible to apply as a joint treating method by combining with genetic therapy.

The compound (I) of the present invention can be orally or parenterally administered as it is, or by blending a pharmacologically acceptable carrier.

Examples of a dosage form of the agent for modulating the function of an RFRP receptor of the present invention when orally administered, include tablets (including sugar-coated tablets, film-coated tablets), pills, granules, powders, capsules (including soft capsules, microcapsules), syrups, emulsions, and suspensions, and examples of a dosage form when administered parenterally include injectables, infusions, drops, and suppositories. In addition, it is effective to formulate into sustained-release preparations by combining with a suitable base (e.g. a copolymer of butyric acid, a polymer of glycolic acid, a copolymer of butyric acid-glycolic acid, a mixture of a polymer of butyric acid and a polymer of glycolic acid, polyglycerol fatty acid ester etc.).

A content of the compound (I) of the present invention in the preparation of the present invention varies depending on a form of a preparation, and is usually about 0.01 to 100% by weight, preferably about 2 to 85% by weight, further preferably about 5 to 70% by weight relative to the whole preparation.

As the method of preparing the compound (I) of the present invention into the aforementioned dosage form, a known preparing method which is generally used in the art can be applied. In addition, when prepared into the aforementioned dosage form, the dosage form can be prepared, if necessary, by conveniently blending an appropriate amount of excipients, binders, disintegrating agents, lubricants, sweeteners, surfactants, suspending agents or emulsifying agents which are conventionally used in the pharmaceutical art when prepared into the dosage form.

For example, when the compound (I) of the present invention is prepared into tablets, they can be prepared so that they contain excipients, binders, disintegrating agents, lubricants or the like and, when prepared into pills or granules, they can be prepared so that they contain excipients, binders, disintegrating agents or the like. In addition, when prepared into powders or capsules, they can be prepared so that they contain excipients and, when prepared into syrups, they can be prepared so that they contain sweeteners and, when prepared into emulsions or suspensions, they can be prepared so that they contain suspending agents, surfactants, emulsifying agents or the like.

Examples of excipients include lactose, white sugar, glucose, starch, sucrose, microcrystalline cellulose, powdered glycyrrhiza, mannitol, sodium bicarbonate, calcium phosphate, and calcium sulfate.

Examples of binders include 5 to 10 weight% starch paste, 10 to 20 weight% gum arabic solution or gelatin solution, 1 to 5 weight% tragacanth solution, carboxymethylcellulose solution, sodium alginate solution, and glycerin.

Examples of disintegrating agents include starch, and calcium carbonate.

Examples of lubricants include magnesium stearate, stearic acid, calcium stearate, and purified talc.

Examples of sweeteners include glucose, fructose, inverted sugar, sorbitol, xylytol, glycerin, and simple syrup.

Examples of surfactants include sodium laurylsulfate Polysorbate 80, sorbitan monofatty acid ester, and polyoxyl stearate 40.

Examples of suspending agents include gum arabic, sodium alginate, sodium carboxymethylcellulose, methylcellulose, and bentonite.

Examples of emulsifying agents include gum arabic, tragacanth, gelatin, and Polysorbate 80.

Further, when the compound (I) of the present invention is prepared into the aforementioned dosage form, optionally, an appropriate amount of colorants, preservatives, flavors, corrigents, stabilizers, and thickeners which are conventionally used in the purification art can be added.

The agent for modulating the function of an RFRP receptor of the present invention is safe, and has low toxicity, therefore, it can be safely used. A daily dose varies depending on the state and a weight of a patient, a kind of a compound, and an administration route. For example, when orally administered to a patient for the purpose of analgesic effect, a dose per adult (weight about 60 kg a day) is, as expressed as an effective ingredient (present compound (I)), about 1 to 1000 mg, preferably about 3 to 300 mg, further preferably about 10 to 200 mg, and the dose can be administered once, or by dividing into two to three portions.

When the compound (I) of the present invention is parenterally administered, usually, it is administered in a form of liquids (e.g. injectables). A unit dose thereof varies depending on an administration subject, a subject organ, symptom, and an administration method. For example, in the form of injectables, it is advantageous to administer, by intravenous injection, usually about 0.01 to about 100 mg, preferably about 0.01 to about 50 mg, more preferably about 0.01 to about 20 mg per weight 1 kg. Examples of injectables include, in addition to intravenous injectables, subcutaneous injectables, intradermal injectables, intramuscular injectables, and drop injectables, and examples of long acting preparations include iontophoresis transdermal agents. Such the injectables are prepared by the known per se method, that is, by dissolving, suspending or emulsifying the compound (I) of the present invention in an aqueous solution or an oily solution. Examples of the aqueous solution for injection include isotonics (e.g. D-sorbitol, D-mannitol, sodium chloride etc.) containing a physiological saline, glucose and other additives, and the aqueous solution may be used together with appropriate dissolution aides such as alcohol (e.g. ethanol), polyalcohol (e.g. propylene glycol, polyethylene glycol), or nonionic surfactant (e.g. Polysorbate 80, HCO-50). Examples of the oily solution include a sesame oil, and a soybean oil, and it may be used together with benzyl benzoate, or benzyl alcohol as a dissolution aide. In addition, buffers (e.g. phosphate buffer, sodium acetate buffer), soothing agents (e.g. benzalkonium chloride, procaine hydrochloride etc.), stabilizers (e.g. human serum albumin, polyethylene glycol etc.), and preservatives (e.g. benzyl alcohol, phenol etc.) may be blended. Prepared injectables are usually filled into an ampoule.

Examples of a drug which can be used together with the compound (I) of the present invention (hereinafter, abbreviated as joint use drug in some cases) include another diabetes treating agent, a diabetic complication treating agent, a hyperlipemia treating agent, a hypotensive agent, an anti-obesity agent, a diuretic, a chemical therapy agent, an immunological therapy agent, an immunological regulating drug, an anti-inflammatory, an anti-thrombus agent, an osteoporosis treating agent, an antibacterial agent, an anti-fungus agent, an anti-protozoan agent, an antibiotic, an antitussive or expectorant, a sedative, an anesthetic, an anti-ulcer drug, a tranquilizer, an anti-psychosis drug, an anti-tumor drug, a muscular relaxant, an anti-epilepsy drug, an antidepressant, an anti-allergy drug, a cardiotonic, an anti-arrhythmia drug, a vasodilator, a vasoconstrictor, a narcotic antagonist, a vitamin drug, a vitamin derivative, an anti-asthma drug, an anti-dementia drug, pollakiuria or urine incontinence treating drug, a dysuria treating drug, an atopic dermatitis treating drug, an allergic rhinitis treating drug, a vasopressor, an endotoxin antagonist or antibiotic, a signal transmission inhibiting drug, an inflammatory mediator action inhibiting drug, an inflammatory mediator action inhibiting antibody, an anti-inflammatory mediator action inhibiting drug, and an anti-inflammatory mediator action inhibiting antibody. Specific examples are as follows:

Examples of the other diabetes treating agent include an insulin preparation (e.g. an animal insulin preparation extracted from cow or pig pancreas; a human insulin preparation synthesized by genetic engineering using Escherichia coli or yeast; zinc insulin, zinc protamineinsulin; a fragment or a derivative of insulin (e.g. INS-1 etc., an oral insulin preparation etc.), an insulin sensitivity potentiating agent (e.g. pioglitazone or a salt thereof (preferably hydrochloride), troglitazone, rosiglitazone or a salt thereof (preferably maleate), Reglixane (JTT-501), Netoglitazone (MCC-555), YM-440, GI-262570, KRP-297, FK-614, CS-011, (γE)-γ-[[[4-[(5-methyl-2-phenyl-4-oxazolyl)methoxy]phenyl]methoxy]imino]benzenebutanoic acid, a compound described in WO 99/58510 (e.g. (E)-4-[4-(5-methyl-2-phenyl-4-oxazolylmethoxy)benzyloxyimino]-4-phenylbutyric acid, a compound described in WO 01/38325, Tesaglitazar (AZ-242), Ragaglitazar (NN-622), BMS-298585, ONO-5816, BM-13-1258, LM-4156, MBX-102, LY-519818, MX-6054, LY-510929, Balaglitazone (NN-2344), T-131 or a salt thereof, THR-0921), an α-glucosidase inhibitor (e.g. voglibose, acarbose, miglitol, emiglitate etc.), a biguanide agent (e.g. phenformin, metformin, buformin etc.), an insulin secretion promoting agent [sulfonylurea agent (e.g. tolbutamide, glibenclamide, gliclazid, chlorpropamide, tolazamide, acetohexamide, glyclopyramide, glimepiride etc.), repaglinide, senaglinide, mitiglinide or a calcium salt hydrate thereof, nateglinide etc.], a GLP-1 receptor agonist [e.g. GLP-1, GLP-1MR agent, NN-2211, AC-2993 (exendin-4), BIM-51077, Aib (8,35)hGLP-1(7,37)NH₂, CJC-1131 etc.], dipeptidylpeptidase IV inhibitor (e.g. NVP-DPP-278, PT-100, P32/98, P93/01, NVP-DPP-728, LAF237, TS-021 etc.), a β3 agonist (e.g. CL-316243, SR-58611-A, UL-TG-307, AJ-9677, AZ40140 etc.), an aniline agonist (e.g. pramlintide etc.), a phosphotyrosine phosphatase inhibitor (e.g. vanadic acid etc.), a glyconeogenesis inhibitor (e.g. glycogen phosphorylase inhibitor, glucose-6-phosphatase inhibitor, glucagons inhibitor etc.), a SGLT (sodium-glucose cotransporter) inhibitor (e.g. T-1095 etc.), a 11β-hydroxysteroid dehydrogenase inhibitor (e.g. BVT-3498 etc.), adiponectin or an agonist thereof, an IKK inhibitor (e.g. AS-2868 etc.), a leptin resistance improving drug, a somatostatin receptor agonist (a compound described in WO 01/25228, WO 03/42204, a compound described in WO 98/44921, WO 98/45285, WO 99/22735 etc.), and a glucokinase activating drug (e.g. Ro-28-1675).

Examples of the diabetic complication treating agent include an aldose reductase inhibitor (e.g. Torestat, epalrestat, zenarestat, zopolrestat, fidarestat (SNK-860), minalrestat (ARI-509), CT-112 etc.), a neurotrophic factor and a drug for increasing it (e.g. NGF, NT-3, BDNF, a neurotrophin production-secretion promoting agent described in WO 01/14372 (e.g. 4-(4-chlorophenyl)-2-(2-methyl-1-imidazolyl)-5-[3-(2-methylphenoxy)propyl]oxazole etc.) etc.), a protein kinase C (PKC) inhibitor (e.g. LY-333531 etc.), an AGE inhibitor (e.g. ALT-945, pimagedine, piratoxathine, N-phenasylthiazolium bromide (ALT-766), EXO-226, ALT-711, Pyridorin, pyridoxamine etc.), an active oxygen scavenger (e.g. thioctic acid etc.), a brain vasodilator (e.g. tiapride etc.), a somatostatin receptor agonist (BIM23190), a apoptosis signal regulating kinase-1 (ASK-1) inhibitor.

Examples of the hyperlipemia treating agent include a statin compound which is a cholesterol synthesis inhibiting agent (e.g. pravastatin, simvastatin, lovastatin, atorvastatin, fluvastatin, cerivastatin or a salt thereof (e.g. sodium salt etc.) etc.), a squalene synthesizing enzyme inhibitor (e.g. a compound described in WO 97/10224, for example, N-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]piperidine-4-acetic acid etc.), a fibrate compound (e.g. bezafibrate, clofibrate, simfibrate, clinofibrate etc.), and an antioxidant (e.g. lipoic acid, producol).

Examples of the hypotensive agent include an angiotensin converting enzyme inhibitor (e.g. captopril, enalapril, derapril etc.), an angiotensin II antagonist (e.g. losartan, candesartan cilexetil, eprosartan, valsartan, telmisartan, irbesartan, tasosartan, 1-[[2'-(2,5-dihydro-5-oxo-4H-1,2,4-oxadiazol-3-yl)biphenyl-4-yl]methyl]-2-ethoxy-1H-benzimidazole-7-carboxylic acid etc.), a calcium antagonist (e.g. manidipine, nifedipine, amlodipine, efonidipine, nicardipine etc.), and clonidine.

Examples of the anti-obesity agent include a neutral anti-obesity drug (e.g. dexfenfluramine, fenfluramine, phentermine, sibutramine, amfepramone, dexamphetamine, mazindol, phenylpropanolamine, clobenzorex; MCH receptor antagonist (e.g. SB-568849; SNAP-7941; a compound included in WO 01/82925 and WO 01/87834 etc.); a neuropeptide Y antagonist (e.g. CP-422935 etc.); a cannabinoid receptor antagonist (e.g. SR-141716, SR-147778 etc.); a ghrelin antagonist; a 11β-hydroxysteroid dehydrogenase inhibitor (e.g. BVT-3498 etc.) etc.), a pancreatic lipase inhibitor (e.g. orlistat, ATL-962 etc.), a β3 agonist (e.g. CL-316243, SR-58611-A, UL-TG-307, AJ-9677, AZ40140 etc.), a peptidic appetite inhibitor (e.g. leptin, CNTF (CORPUS ciliare neurotrophic factor) etc.), a cholecystokinin agonist (e.g. rinchitript, FPL-15849 etc.), and an eating inhibitor (e.g. P-57 etc.).

Examples of the diuretic include a xanthine derivative (e.g. sodium salicylate theobromine, calcium salicylate theobromine etc.), a thiazide preparation (e.g. ethiazide, cyclopentiazide, trichlormethiazide, hydrochlorothiazide, hydroflumethiazide, benzylhydrochlorothiazide, penflutizide, polythiazide, methyclothiazide etc.), an anti-aldosterone preparation (e.g. spironolactone, triamteren etc.), a decarboxylase inhibitor (e.g. acetazolamide etc.), a chlorobenzenesulfonamide preparation (e.g. chlortalidone, mefruside, indapamide etc.), azosemide, isosorbide, ethacrynic acid, piretanide, bumetanide, and furosemide.

Examples of the chemical therapy agent include an alkylating agent (e.g. cyclophosphamide, ifosfamide etc.), a metabolism antagonist (e.g. methotrexate, 5-fluorouracil etc.), an anti-cancer antibiotic (e.g. mitomycin, adriamycin etc.), a plant-derived anti-cancer agent (e.g. vincristine, vindesin, taxol etc.), cisplatin, carboplatin, and etoposide. Inter alia, Furtulon or NeoFurtulon which is a 5-fluorouracil derivative is preferable.

Examples of the immunological therapy agent include a microorganism or bacterium component (e.g. muramyl dipeptide derivative, Picibanil etc.), a polysaccharide having immunity potentiating activity (e.g. lentinan, sizofiran, Krestin etc.), cytokine obtained by a genetic engineering method (e.g. interferon, interleukin (IL) etc.), and a colony stimulating factor (e.g. granulocyte colony stimulating factor, erythropoietin etc.) and, inter alia, interleukins such as IL-1, IL-2, and IL-12 are preferable.

Examples of the anti-inflammatory include non-steroidal anti-inflammatory such as aspirin, acetoaminophen, and indometacin.

Examples of the anti-thrombus agent include heparin (e.g. heparin sodium, heparin calcium, dalteparin sodium etc.), warfarin (warfarin potassium etc.), an anti-thrombin drug (e.g. aragatroban etc.), a thrombolytic drug (e.g. urokinase, tisokinase, alteplase, nateplase, monteplase, pamiteplase etc.), a platelet aggregation inhibitor (ticlopidine hydrochloride, cilostazol, ethyl eicosapentoate, beraprost sodium, sarpogrelate hydrochloride etc.).

Examples of the osteoporosis treating agent include alfacalcidol, calcitriol, elcatonin, calcitonin salmon, estriol, ipriflavone, pamidronate disodium, alendronate sodium hydrate, and incadronate disodium.

Examples of the vitamin drug include vitamin B₁, and vitamin B₁₂.

Examples of the anti-dementia agent include tacrine, donepezil, rivastigmine, and galantamine.

Examples of the pollakiuria•urine incontinence treating drug include flavoxate hydrochloride, oxybutynin hydrochloride, and propiverine hydrochloride.

Examples of the dysuria treating drug include an acetylcholine esterase inhibitor (e.g. distigmine).

Further, drugs which are recognized to have cachexia improving activity in an animal model or a clinical test, that is, a cyclooxygenase inhibitor (e.g. indometacin etc.) [Cancer Research, vol. 49, p5935-5939, 1989] a progesterone derivative (e.g. megestrol acetate) [Journal of Clinical Oncology, vol. 12, p213-225, 1994], a glycosteroid (e.g. dexamethasone etc.), a metochropramide drug, tetrahydrocannabinol drug (the references are the same as those described above), a lipid metabolism improving agent (e.g. eicosapentaenoic acid etc.) [British Journal of Cancer, vol. 68, p314-318, 1993], a growth hormone, IGF-1, or TNF-α which is a factor inducing cachexia, LIF, IL-6, and an antibody to oncostatin M can be used together with the compound (I) of the present invention.

Further, a glycosylation inhibitor (e.g. ALT-711 etc.), a nerve regeneration stimulator (e.g. Y-128, VX853, prosaptide etc.), an antidepressant (e.g. desipramine, amitriptyline, imipramin), an anti-epilepsy drug (e.g. lamotrigine, Trileptal, Keppra, Zonegran, Pregabalin, Harkoseride, carbamazepine), an anti-arrhythmia drug (e.g. mexiletine), an acetylcholine receptor ligand (e.g. ABT-594), an endothelin receptor antagonist (e.g. ABT-627), a monoamine uptake inhibitor (e.g. tramadol), a morphine analgesic (e.g. morphine), a GABA receptor agonist (e.g. gabapentin, gabapentin MR agent), an α2 receptor agonist (e.g. clonidine), a local analgesic (e.g. capsaicin), an anti-anxiety drug (e.g. benzothiazepine), a phosphodiesterase inhibitor (e.g. sindenafil), and a dopamine receptor agonist (e.g. apomorphine) can be also used together with the compound (I) of the present invention.

By combining the compound (I) of the present invention and the joint use drug, the following excellent effects can be obtained:
(1) A dose thereof can be decreased as compared with the case where the compound (I) of the present invention or the joint use drug is administered alone.
(2) A drug to be used with the compound (I) of the present invention jointly can be selected depending on symptom of a patient (slight, severe).
(3) By selecting the joint use drug having different mechanism of action from that of the compound (I) of the present invention, a treating period can be set long.
(4) By selecting the joint use drug having different mechanism of action from that of the compound (I) of the present invention, durability of the treating effect can be realized.
(5) By using the compound (I) of the present invention and the joint use drug jointly, the synergistic effect is obtained

Hereinafter, joint use of the compound (I) of the present invention and the joint use drug will be referred to as "joint use agent of the present invention".

Upon use of the joint use agent of the present invention, an administration time of the compound (I) of the present invention and the joint use drug is not limited, but the compound (I) of the present invention and the joint use drug may be administered to an administration subject simultaneously, or at different times. A dose of the joint use drug may be according to a dose which is clinically used, and can be appropriately selected depending on an administration subject, an administration route, a disease, and a combination.

An administration form of the joint use agent of the present invention is not particularly limited, but the form is enough as long as the compound (I) of the present invention and the joint use drug are combined at administration. Examples of such the administration form include (1) simultaneous formulation of the compound (I) of the present invention and the joint use drug into a preparation, and administration of the resulting single preparation, (2) simultaneous administration of two kinds of preparations obtained by separately preparing the compound (I) of the present invention and the joint use drug into a preparation through the same administration route, (3) administration of two kinds of preparations obtained by separately formulating the compound (I) of the present invention and the joint use drug into a preparation through the same administration route at different times, (4) simultaneous administration of two kinds of preparations obtained by separately formulating the compound (I) of the present invention and the joint use drug through different administration routes, and (5) administration of two kinds of preparations obtained by separately formulating the compound (I) of the present invention and the joint use drug through different administration routes at different times (e.g. administration in an order of the compound (I) of the present invention; the joint use drug, or administration in a reverse order).

The joint use agent of the present invention has low toxicity and, for example, the compound (I) of the present invention or (and) the joint use drug are mixed with a pharmacologically acceptable carrier according to the known per se method to prepare a pharmaceutical composition such as tablets (including sugar-coated tablets, film-coated tablets), powders, granules, capsules (including soft capsules), liquids, injectables, suppositories and sustained-release preparations, which can be safely administered orally or parenterally (e.g. local, rectal, intravenous administration etc.). An injectable can be administered intravenously, intramuscularly, or subcutaneously, or can be administered into organs, or can be directly administered to a lesion.

Examples of a pharmacologically acceptable carrier which may be used in preparing the joint use agent of the present invention include the same carriers as those for the aforementioned pharmacologically acceptable carrier which may be used in preparing a drug of the present invention. In addition, if necessary, additives such as antiseptics, antioxidants, colorants, sweeteners, adsorbing agents, and wetting agents which may be used in preparing a drug of the present invention can be appropriately used at an appropriate amount.

A ratio of blending the compound (I) of the present invention and the joint use drug in the joint use agent of the present invention can be appropriately selected depending on an administration subject, an administration route, and a disease.

For example, a content of the compound (I) of the present invention in the joint use agent of the present invention is different depending on a form of a preparation, and is usually about 0.01 to 100% by weight, preferably about 0.1 to 50% by weight, further preferably about 0.5 to 20% by weight relative to a whole preparation.

A content of the joint use drug in the joint use agent of the present invention is different depending on a form of a preparation and is usually about 0.01 to 90% by weight, preferably about 0.1 to 50% by weight, further preferably about 0.5 to 20% by weight relative to a whole preparation.

A content of an additive such as a carrier in the joint use agent of the present invention is different depending on a form of a preparation, and is usually about 1 to 99.99% by weight, preferably about 10 to 90% by weight relative to a whole preparation.

In addition, when the compound (I) of the present invention and the joint use drug are separately formulated into a preparation, the same content may be used.

These preparations can be usually prepared by the known per se method which is generally used in a preparation step.

For example, the compound (I) of the present invention or the joint use drug together with dispersants (e.g. Tween 80 (manufactured by Atlas Powder, USA), HCO 60 (manufactured by Nikko Chemicals Co., Ltd.), polyethylene glycol, caboxymethylcellulose, sodium alginate, hydroxypropylmethylcellulose, dextrin etc.), stabilizers (e.g. ascorbic acid, sodium pyrosulfite etc.), surfactants (e.g. Polysorbate 80, macrogol etc.), solubilizers (e.g. glycerin, ethanol etc.), buffers (e.g. phosphoric acid and an alkali metal salt thereof, citric acid and an alkali metal salt thereof etc.), isotonics (e.g. sodium chloride, potassium chloride, mannitol, sorbitol, glucose etc.), pH adjusting agents (e.g. hydrochloric acid, sodium hydroxide etc.), preservatives (e.g. ethyl paraoxybenzoate, benzoic acid, methyl paraben, propyl paraben, benzyl alcohol etc.), dissolving agents (e.g. concentrated glycerin, meglumine etc.), dissolution aides (e.g. propylene glycol, white sugar etc.), and soothing agents (e.g. glucose, benzyl alcohol etc.) is prepared into an aqueous injectable, or is dissolved, suspended or emulsified in a vegetable oil such as an olive oil, a sesame oil, a cottonseed oil, and a corn oil, or in a dissolution aide such as propylene glycol to prepare an oily injectable, which may be used as an injectable.

Alternatively, according to a per se known method, by adding, for example, an excipient (e.g. lactose, white sugar, starch etc.), a disintegrating agent (e.g. starch, calcium carbonate etc.), a binder (e.g. starch, gum arabic, carboxymethylcellulose, polyvinylpyrrolidone, hydroxypropylcellulose etc.) or a lubricant (e.g. talc, magnesium stearate, polyethylene glycol 6000) to the compound (I) of the present invention or the joint use drug to compression-mold them and, if necessary, performing coating by the known per se method for the purpose of taste masking, enteric solubility or durability, or an oral preparation can be obtained. As a coating agent used in coating, for example, hydroxypropylmethylcellulose, ethylcellulose, hydroxymethylcellulose, hydroxypropylcellulose, polyoxyethylene glycol, Tween 80, Pluronic F68, cellulose acetate phthalate, hydroxypropylmethylcellulose phthalate, hydroxymethylcellulose acetate succinate, Eudragit (manufactured by Rohm, Germany, a methacrylic acid•acrylic acid copolymer) and a pigment (e.g. bengal, titanium dioxide etc.) are used. An oral preparation may be any of a rapid-releasing preparation and a sustained-release preparation.

Further, according to a per se known method, by mixing the compound (I) of the present invention or the joint use drug with an oily base, an aqueous base or an aqueous gel base, an oily or aqueous solid, semisolid or liquid suppository can be obtained. Examples of the oily base include glyceride of higher fatty acid [e.g. cacao butter, witepsol (manufactured by Dynamite Novel, Germany)], middle fatty acid [e.g. miglyol (manufactured by Dynamite Novel, Germany) etc.], and a vegetable oil (e.g. sesame oil, soybean oil, cottonseed oil etc.). Examples of the aqueous base include polyethylene glycol, and propylene glycol. Examples of the aqueous gel base include natural gums, a cellulose derivative, a vinyl polymer, and an acrylic acid polymer.

Examples of the sustained-release preparation include sustained-release microcapsules. The sustained-release microcapsule is prepared by the known per se method such as the method shown in the following [2].

It is preferable that the compound (I) of the present invention is molded into an oral preparation such as a solid preparation (e.g. powders, granules, tablets, capsules), or is molded into a rectal preparation such as suppositories. An oral preparation is particularly preferable.

The joint use drug can be formulated into the aforementioned dosage form depending on a kind of a drug.
[1] Injectables of the compound (I) of the present invention or the joint use drug and preparation thereof,
[2] sustained-release preparations or rapid-releasing preparations of the compound (I) of the present invention or the joint use drug, and preparation thereof, and [3] sublingual tablets, buccal or oral rapid disintegrating agents of the compound (I) of the present invention or the joint use drug, and preparation thereof will be specifically shown below.

### [1] Injectables and preparation thereof

An injectable in which the compound (I) of the present invention or the joint use drug is dissolved in water is preferable. The injectable may contain benzoate or/and salicylate.

The injectable is obtained by dissolving both of the compound (I) of the present invention or the joint drug and, optionally, benzoate or/and salicylate in water.

Examples of a salt of benzoic acid and salicylic acid include an alkali metal salt such as sodium, and potassium, an alkaline earth metal salt such as calcium, and magnesium, an ammonium salt, a meglumine salt, and an organic acid salt such as trometamol.

A concentration of the compound (I) of the present invention or the joint use drug in an injectable is about 0.5 to 50 w/v%, preferably about 3 to 20 w/v%. A concentration of benzoate or/and salicylate is about 0.5 to 50 w/v%, preferably about 3 to 20 w/v%.

In addition, an additive which is generally used in injectables, such as a stabilizing agent (e.g. ascorbic acid, sodium pyrosulfite etc.), a surfactant (e.g. Polysorbate 80, macrogol etc.), a solubilizer (e.g. glycerin, ethanol etc.), a buffer (e.g. phosphoric acid and an alkali metal salt thereof, citric acid and an alkali metal salt thereof), tonicity agent (e.g. sodium chloride, potassium chloride etc.), a dispersant (e.g. hydroxypropylmethylcellulose, dextrin), a pH adjusting agent (e.g. hydrochloric acid, sodium hydroxide etc.), a preservative (e.g. ethyl paraoxybenzoate, benzoic acid etc.), a dissolving agent (e.g. concentrated glycerin, meglumine etc.), a dissolution aide (e.g. propylene glycol, white sugar etc.), and a soothing agent (e.g. glucose, benzyl alcohol etc.) can be appropriately blended into the present injectable. These additives are generally blended at a ratio which is usually used in injectables.

Suitably, injectables are adjusted to a pH of 2 to 12, preferably a pH of 2.5 to 8.0 by adding a pH adjusting agent.

Injectables are obtained by dissolving both of the compound (I) of the present invention or the joint use drug and, optionally, benzoate or/and salicylate and, if necessary, the aforementioned additive in water. Dissolution of them may be performed in any order, and can be appropriately performed as in the previous process for preparing injectables.

Suitably, an aqueous solution for injection is warmed, and can be supplied as an injectable by filtration sterilization or high pressure heating sterilization as in the conventional injectable.

Suitably, an aqueous solution for injectable is subjected to high pressure heating sterilization for 5 to 30 minutes, for example, under conditions of 100 to 121°C.

Further, an injectable may be formulated into a preparation to which antibacterial property of a solution is imparted so that it can be used as a multiple divided administration preparation.

### [2] Sustained-release preparations or rapid-releasing preparations and preparation thereof

Sustained-release preparations in which a core containing the compound (I) of the present invention or the joint use drug is optionally covered with a covering agent such as a water-insoluble substance or a wetting polymer are preferable. For example, once a day administration-type oral sustained-release preparations are preferable.

Examples of the water-insoluble substance used in a covering agent include cellulose ethers such as ethylcellulose, and butylcellulose, cellulose esters such as cellulose acetate, and cellulose propionate, polyvinyl esters such as polyvinyl acetate, and polyvinyl butyrate, acrylic acid-based polymer such as an acrylic acid/methacrylic acid copolymer, a methyl methacrylate copolymer, an ethoxyethyl methacrylate/cinnamoethyl methacrylate/aminoalkyl methacrylate copolymer, polyacrylic acid, polymethacrylic acid, a methacrylic acid alkylamide copolymer, poly(methyl methacrylate), polymethacrylate, polymethacrylamide, an aminoalkyl methacrylate copolymer, poly(methacrylic acid anhydride), a glycidyl methacrylate copolymer, inter alia, Eudragit (Rohm·Firma) such as Eudragit RS-100, RL-100, RS-30D, RL-30D, RL-PO, RS-PO (an ethyl acrylate•methyl methacrylate-trimethyl methacrylate chloride•ethyl ammonium copolymer), and Eudragit NE-30D (a methyl methacrylate·ethyl acrylate copolymer), a hydrogenated oil such as hydrogenated castor oil (e.g. Lovely wax (Freund Industry) etc.), waxes such as carnauba wax, fatty acid glycerin ester, and paraffin, and polyglycerin fatty acid ester.

As a wettable polymer, a polymer having an acidic dissociating group and exhibiting pH dependent swelling is preferable, and a polymer having an acidic dissociating group, which is slightly swollen in an acidic region such as in stomach, and in which swelling becomes great in a neutral region such as in small intestine and large intestine is preferable.

Examples of such the polymer having an acidic dissociating group and exhibiting pH dependent swelling include crosslinking-type polyacrylic acid polymer such as Carbomer 934P, 940, 941, 974P, 980, 1342 etc., polycarbophil, and calcium polycarbophil (all manufactured by BF Goodrich), and Hiviswako 103, 104, 105, and 304 (all manufactured by Wako Pure Chemical Industries, Ltd.).

A covering agent used in sustained-release preparations may further contain a hydrophilic substance.

Examples of the hydrophilic substance include polysaccharides optionally having a sulfate group such as pullulan, dextrin, alginic acid alkali metal salt, polysaccharides having a hydroxyalkyl group or a carboxyalkyl group such as hydroxypropylcellulose, hydroxypropylmethylcellulose, and sodium carboxymethylcellulose, methylcellulose, polyvinylpyrrolidone, polyvinyl alcohol, and polyethylene glycol.

A content of the water-insoluble substance in a covering agent of sustained-release preparations is about 30 to about 90% (w/w), preferably about 35 to about 80% (w/w), further preferably about 40 to 75% (w/w), and a content of the wettable polymer is about 3 to about 30% (w/w), preferably about 3 to about 15% (w/w). A covering agent may further contain a hydrophilic substance and, in that case, a content of the hydrophilic substance in a covering agent is about 50% (w/w) or lower, preferably about 5 to about 40% (w/w), further preferably about 5 to about 35% (w/w). Herein, the % (w/w) indicates weight% relative to a covering agent composition obtained by removing a solvent (e.g. water, lower alcohol such as methanol, ethanol etc.) from a covering agent solution.

A sustained-release preparation is manufactured by preparing a core containing a drug and, then, covering a core with a covering agent solution obtained by heating-dissolving a water-insoluble substance or a wettable polymer, or dissolving or dispersing in a solvent, as exemplified below.

### I. Preparation of core containing drug

A form of a core containing a drug to be covered with a covering agent (hereinafter, simply referred to as core in some cases) is not particularly limited, but a core is formed into a particulate shape such as a granule or a fine particle.

When a core is a granule or a fine particle, an average particle diameter thereof is preferably about 150 to 2,000 µm, further preferably about 500 to about 1,400 µm.

Preparation of a core can be performed by a conventional manufacturing method. For example, a core is prepared by mixing a drug with an appropriate excipient, binder, disintegrating agent, lubricant, and stabilizing agent, and forming a particle by a wet extrusion granulating method or a fluidized layer granulating method.

A drug content of a core is about 0.5 to about 95% (w/w), preferably about 5.0 to about 80% (w/w), further preferably about 30 to about 70% (w/w).

As an excipient contained in a core, sugars such as white sugar, lactose, mannitol, and glucose, starch, crystalline cellulose, calcium phosphate, and corn starch are used. Inter alia, crystalline cellulose, and corn starch are preferable.

As the binder, for example, polyvinyl alcohol, hydroxypropylcellulose, polyethylene glycol, polyvinylpyrrolidone, Pluronic F68, gum arabic, gelatin, and starch are used. As the disintegrating agent, for example, calcium carboxymethylcellulose (ECG505), sodium croscarmelose (Ac-Di-Sol), crosslinking-type polyvinylpyrrolidone (crospovidone), and low-substituted hydroxypropylcellulose (L-HPC) are used. Inter alia, hydroxypropylcellulose, polyvinylpyrrolidone, and low-substituted hydroxypropylcellulose are preferable. As the lubricant, or the aggregation preventing agent, for example, talc, and magnesium stearate and an inorganic salt thereof are used and, as the lubricating agent, polyethylene glycol is used. As the stabilizing agent, an acid such as citric acid, succinic acid, fumaric acid, and maleic acid is used.

A core may be prepared by a rolling granulating method, a pan coating method, a fluidized layer coating method or a melt granulating method in which a small amount of a drug or a mixture of this and an excipient or a lubricant is added while a binder dissolved in a suitable solvent such as water, and a lower alcohol (e.g. methanol, ethanol etc.) is sprayed on an inert carrier particle as a center of a core, in addition to the aforementioned manufacturing method. As the inert carrier particle, a carrier prepared with white sugar, lactose, starch, crystalline cellulose, or waxes can be used, and a particle having an average particle diameter of about 100 µm to about 1,500 µm is preferable.

In order to separate a drug contained in a core, and a covering agent, a surface of a core may be covered with a protecting agent. As the protecting agent, for example, the aforementioned hydrophilic substance or water-insoluble substance is used. Preferably, as the protecting agent, polyethylene glycol, a polysaccharide having a hydroxyalkyl group or a carboxyalkyl group is used. More preferably, hydroxypropylmethylcellulose, and hydroxypropylcellulose are used. The protecting agent may contain an acid such as tartaric acid, citric acid, succinic acid, fumaric acid, and maleic acid as a stabilizing agent, or a lubricant such as talc. When the protecting agent is used, a covering amount thereof is about 1 to about 15% (w/w), preferably about 1 to about 10% (w/w), further preferably about 2 to about 8% (w/w) relative to a core.

The protecting agent can be covered by a conventional coating method and, specifically, can be covered by spray-coating the protecting agent on a core, for example, by a fluidized layer coating method or a pan coating method.

### II. Covering of core with covering agent

Sustained-release preparations are prepared by covering a core obtained in the aforementioned I with a covering solution obtained by heating-dissolving the aforementioned water-insoluble substance and pH dependent wettable polymer, and hydrophilic substance, or dissolving or dispersing them in a solvent.

Examples of a method of covering a core with a covering solution include a spray coating method.

A compositional ratio of a water-insoluble substance, a wettable polymer or a hydrophilic substance in a covering agent solution is appropriately selected so that contents of respective components in a cover become the aforementioned contents, respectively.

A covering amount of a covering agent is about 1 to about 90% (w/w), preferably about 5 to about 50% (w/w), further preferably about 5 to 35% (w/w) relative to a core (not containing a covering amount of a protecting agent).

As a solvent for a covering agent solution, water or an organic solvent alone, or a mixed solution of both of them may be used. Upon use of a mixed solution, a ratio of mixing water and an organic solvent (water/organic solvent: weight ratio) can be changed in a range of 1 to 100%, preferably 1 to about 30%. The organic solvent is not particularly limited as far as it dissolves a water-insoluble substance, but for example, lower alcohol such as methyl alcohol, ethyl alcohol, isopropyl alcohol, and n-butyl alcohol, lower alkanone such as acetone, acetonitrile, chloroform, and methylene chloride are used. Among them, a lower alcohol is preferable, and ethyl alcohol, and isopropyl alcohol are particularly preferable. Water, and a mixed solution of water and an organic solvent are preferably used as a solvent for a covering agent. Thereupon, if necessary, in order to stabilize a covering agent solution, an acid such as tartaric acid, citric acid, succinic acid, fumaric acid, and maleic acid may be added to the covering agent solution.

Operation when covered by spray coating can be performed by a conventional coating method and, specifically, can be performed by spray coating a covering solution on a core, for example, by a fluidized layer coating method, or a pan coating method. Thereupon, if necessary, talc, titanium oxide, magnesium stearate, calcium stearate, and light silicic acid anhydride as a lubricant, and glycerin fatty acid ester, hydrogenated castor oil, triethyl citrate, cetyl alcohol, and stearyl alcohol as a plasticizer may be added.

After covering with a covering agent, if necessary, an antistatic agent such as talc may be mixed therein.

A rapid-releasing preparation may be liquid (solutions, suspensions, emulsions etc.) or solid (particles, pills, tablets etc.). As a rapid-releasing preparation, an oral administration agent, and a parenteral administration agent such as injectable are used, and an oral preparation agent is preferable.

A rapid-releasing preparation may usually contain a carrier, an additive and an excipient (hereinafter, abbreviated as excipient in some cases) which are conventional in the pharmacy field, in addition to a drug which is an active ingredient. An excipient agent used is not particularly limited as far as it is an excipient which is conventionally used as a preparation excipient. Examples of an excipient for an oral solid preparation include lactose, starch, corn starch, crystalline cellulose (Avicel PH101 manufactured by Asahi Chemical Industry Co., Ltd.), powder sugar, granulated sugar, mannitol, light silicic anhydride, magnesium carbonate, calcium carbonate, and L-cysteine, preferably corn starch and mannitol. These excipients can be used alone, or by combining two or more kinds of them. A content of an excipient is, for example, about 4.5 to about 99.4 w/w%, preferably about 20 to about 98.5 w/w%, further preferably about 30 to about 97 w/w% relative to a total amount of a rapid-releasing preparation.

A content of a drug in a rapid-releasing preparation can be appropriately selected from a range of about 0.5 to about 95%, preferably about 1 to about 60% relative to a total amount of a rapid-releasing preparation.

When a rapid-releasing preparation is an oral solid preparation, it usually contains a disintegrating agent in addition to the aforementioned components. As such the disintegrating agent, for example, calcium carboxymethylcellulose (ECG-505 manufactured by Gotokuyakuhin), sodium croscarmelose (e.g. Acsisol manufactured by Asahi Chemical Industry Co., Ltd.), crospovidone (e.g. Colidone CL manufactured by BASF), low-substituted hydroxypropylcellulose (manufactured by Shin-Etsu Chemical Co., Ltd.), carboxymethylstarch (manufactured by Matsutani Chemical Industry Co., Ltd.), sodium carboxymethylstarch (Extract Protub manufactured by Kimura Sangyo), and partially gelatinized starch (PCS manufactured by Asahi Chemical Industry Co., Ltd.) are used. For example, a disintegrating agent which disintegrates a granule by contacting with water to absorb water or be swollen, or making a channel between an active ingredient and an excipient constituting a core, can be used. These disintegrating agents can be used alone, or by combining two or more kinds of them. An amount of a disintegrating agent to be blended is appropriately selected depending on a kind and a blending amount of a drug to be used, and preparation design of releasability, and is, for example, about 0.05 to about 30 w/w%, preferably about 0.5 to about 15 w/w% relative to a total amount of a rapid-releasing preparation.

When a rapid-releasing preparation is an oral solid preparation, the preparation may further contain, optionally, an additive which is conventional in a solid preparation, in addition to the aforementioned composition. As such the additive, for example, a binder (e.g. sucrose, gelatin, gum arabic powder, methyl cellulose, hydroxypropylcellulose, hydroxypropylmethylcellulose, carboxymethylcellulose, polyvinylpyrrolidone, pullulan, dextrin etc.), a lubricant (e.g. polyethylene glycol, magnesium stearate, talc, light silicic anhydride (e.g. Aerosil (Nippon Aerosil)), a surfactant (e.g. anionic surfactant such as sodium alkylsulfate, nonionic surfactant such as polyoxyethylene fatty acid ester and polyoxyethylene sorbitan fatty acid ester, polyoxyethylene castor oil derivative etc.), a colorant (e.g. tar-based pigment, caramel, bengal, titanium oxide, riboflavins) and, if necessary, a corrigent (e.g. sweetener, flavor etc.), an adsorbing agent, an antiseptic, a wetting agent, and an antistatic agent are used. In addition, an organic acid such as tartaric acid, citric acid, succinic acid, and fumaric acid as a stabilizing agent may be added.

As the binder, hydroxypropylcellulose, polyethylene glycol and polyvinylpyrrolidone are preferably used.

A rapid-releasing preparation can be prepared by mixing the aforementioned respective components and, if necessary, further kneading the mixture, and molding this, based on the conventional technique for manufacturing a preparation. The mixing is performed by a generally used method, for example, by mixing and kneading. Specifically, for example, when a rapid-releasing preparation is formed into a particle shape, by the same procedure as the aforementioned process for preparing a core of the sustained-release preparation, the rapid-releasing preparation can be prepared by mixing components using a vertical granulator, a universal kneader (manufactured by Hatatekkosho), or a fluidized layer granulator FD-5S (manufactured by Powlex) and, thereafter, performing granulation by a wet extrusion granulating method or a fluidized layer granulating method.

The thus obtained rapid-releasing preparation and sustained-release preparation as they are, or after convenient separate formulation into a preparation with a pharmacy excipient by a conventional method, may be formulated into preparations which are administered simultaneously, or which are administered at an arbitrary interval by combination, or both may be used as they are, or both may be conveniently formulated into one oral preparation (e.g. granules, fine particles, tablets, capsules etc.) with a pharmacy excipient. Both preparations may be made into granules or fine particles, and they may be filled into the same capsule to obtain an oral preparation.

### [3] Sublingual tablets, buccals or oral rapid disintegrating preparations and preparation thereof

Sublingual tablets, buccal preparations, and oral rapid disintegrating preparations may be a solid preparation such as tablets, or may be oral mucosal applying tablets (films).

As the sublingual tablet, the buccal or the oral rapid disintegrating preparation, preparations containing the compound (I) of the present invention or the joint use drug, and an excipient are preferable. Alternatively, an auxiliary agent such as a lubricant, tonicity agent, a hydrophilic carrier, a water-dispersible polymer, and a stabilizing agent may be contained. Alternatively, in order to facilitate absorption and enhance bioavailability, β-cyclodextrin or a β-cyclodextrin derivative (e.g. hydroxypropyl-β-cyclodextrin etc.) may be contained. Examples of the excipient include lactose, white sugar, D-mannitol, starch, crystalline cellulose, and light silicic anhydride. Examples of the lubricant include magnesium stearate, calcium stearate, talc, and colloidal silica and, particularly, magnesium stearate and colloidal silica are preferable. Examples of the isotonic include sodium chloride, glucose, fructose, mannitol, sorbitol, lactose, saccharose, glycerin, and urea and, particularly, mannitol is preferable. Examples of the hydrophilic carrier include crystalline cellulose, ethylcellulose, crosslinking polyvinylpyrrolidone, light silicic anhydride, silicic acid, dicalcium phosphate, and a swelling hydrophilic carrier such as calcium carbonate and, particularly, crystalline cellulose (e.g. microcrystalline cellulose etc.) is preferable. Examples of the water-dispersible polymer include a gum (e.g. tragacanth gum, acacia gum, guar gum), alginate (e.g. sodium alginate), cellulose derivative (e.g. methylcellulose, carboxymethylcellulose, hydroxymethylcellulose, hydroxypropylcellulose, hydroxypropylmethylcellulose), gelatin, water-soluble starch, polyacrylic acid (e.g. carbomer), polymethacrylic acid, polyvinyl alcohol, polyethylene glycol, polyvinylpyrrolidone, polycarbophil, ascorbic acid, and palmitate, and hydroxypropylmethylcellulose, polyacrylic acid, alginate, gelatin, carboxymethylcellulose, polyvinylpyrrolidone, and polyethylene glycol are preferable. Particularly, hydroxypropylmethylcelullose is preferable. Examples of the stabilizing agent include cysteine, thiosorbitol, tartaric acid, citric acid, sodium carbonate, ascorbic acid, glycine, and sodium sulfite and, particularly, citric acid and ascorbic acid are preferable.

A sublingual tablet, a buccal or an oral rapid disintegrating preparation can be prepared by mixing the compound (I) of the present invention or the joint use drug and an excipient by the known per se method. Further, if desired, the aforementioned auxiliary agent such as a lubricant, tonicity agent, a hydrophilic carrier, a water-dispersible polymer, a stabilizing agent, a colorant, a sweetener, and an antiseptic may be mixed therein. After the aforementioned components are mixed simultaneously or at different times, a sublingual tablet, a buccal tablet or an oral rapid disintegrating tablet is obtained by molding by compression under pressure. In order to obtain a suitable hardness, the tablet may be prepared by wetting or wet-swelling the material, if necessary, using a solvent such as water and an alcohol before or after a stage of compression molding and, after molding, drying this.

When molded into a mucosal applying tablet (film), the compound (I) of the present invention or the joint use drug and the aforementioned water-dispersible polymer (preferably hydroxypropylcellulose, hydroxypropylmethylcellulose) and excipient are dissolved in a solvent such as water, and the resulting solution is cast to obtain a film. Further, an additive such as a plasticizer, a stabilizer, an antioxidant, a preservative, a colorant, a buffer and a sweetener may be added. In order to impart suitable elasticity to a film, glycols such as polyethylene glycol and propylene glycol may be contained, or in order to enhance adherability of a film to a mucosal lining in an oral cavity, a biological adhesive polymer (e.g. polycarbophil, carbopol) may be contained. Casting is performed by pouring a solution on a non-adhesive surface, spreading this to a uniform thickness (preferably around 10 to 1000 micron) with a coating equipment such as a doctor blade, and drying the solution to form a film. The thus formed film may be dried at room temperature or under warming, and cut into a desired surface area.

Examples of a preferable oral rapid disintegrating preparation include a solid rapid diffusing agent composed of a net-like material of the compound (I) of the present invention or the joint use drug, and a water-soluble or water-diffusing carrier which is inert to the compound (I) of the present invention or the joint use drug. The net-like material is obtained by sublimating a solvent from a solid composition composed of a solution in which the compound (I) of the present invention or the joint use drug is dissolved in a suitable solvent.

It is preferable that a composition of the oral rapid disintegrating preparation contains a matrix forming agent and a secondary component in addition to the compound (I) of the present invention or the joint use drug.

The matrix forming agent includes gelatins, dextrins, as well as animal proteins or plant proteins such as soybean, wheat and psyllium seed proteins; gummy substances such as gum arabic, guar gum, agar and xanthane; polysaccharides; alginates; carboxymethylcelluloses; carrageenans; dextrans; pectins; synthetic polymers such as polyvinylpyrrolidone; substances derived from gelatin-gum arabic complex. Further, the matrix forming agent includes sugars such as mannitol, dextrose, lactose, galactose and trehalose; cyclic sugars such as cyclodextrin; inorganic salts such as sodium phosphate, sodium chloride and aluminum silicate; amino acids of a carbon number of 2 to 12 such as glycine, L-alanine, L-aspartic acid, L-glutamic acid, L-hydroxyproline, L-isoleucine, L-leucine, and L-phenylalanine.

One or more kinds of the matrix forming agents can be introduced into a solution or a suspension before solidification thereof. Such the matrix forming agent may be present in addition to a surfactant, or may be present without a surfactant. The matrix forming agent can assist to maintain the diffused state of the compound (I) of the present invention or the joint use drug as it is in a solution or a suspension, in addition to formation of a matrix.

A composition may contain a secondary component such as a preservative, an antioxidant, a surfactant, a viscosity increasing agent, a colorant, a pH adjusting agent, a flavor, a sweetener and a taste masking agent. Examples of a suitable colorant include red, black and yellow iron oxides, as well as FD & C dyes such as FD & C Blue No. 2 and FD & C Red No. 40 of Ellis And Ebelar. Examples of a suitable flavor include mint, raspberry glycyrrhiza, orange, lemon, grapefruit, caramel, vanilla, cherry and grape flavor, and a combination thereof. Examples of a suitable pH adjusting agent include citric acid, tartaric acid, phosphoric acid, hydrochloric acid and maleic acid. Examples of a suitable sweetener include aspartame, acesulphame K and taumatin. Examples of a suitable taste masking agent include sodium bicarbonate, ion exchange resin, cyclodextrin inclusion compound, adsorbing substance and microcapsulated apomorphine.

A preparation contains the compound (I) of the present invention or the joint use drug usually at about 0.1 to about 50% by weight, preferably at about 0.1 to about 30% by weight, and a preparation (the aforementioned sublingual tablet, buccal) which can dissolve 90% or more of the compound (I) of the present invention or the joint use drug (in water) for about 1 minute to about 60 minutes, preferably about 1 minute to about 15 minutes, more preferably about 2 minutes to about 5 minutes, and an oral rapid disintegrating preparation which is disintegrated in 1 to 60 seconds, preferably 1 to 30 seconds, further preferably 1 to 10 seconds after administered into an oral cavity, are preferable.

A content of the excipient relative to a whole preparation is about 10 to about 99% by weight, preferably about 30 to about 90% by weight. A content of β-cyclodextrin or a β-cyclodextrin derivative relative to a whole preparation is 0 to about 30% by weight. A content of a lubricant to a whole preparation is about 0.01 to about 10% by weight, preferably about 1 to about 5% by weight. A content of tonicity agent to a whole preparation is about 0.1 to about 90% by weight, preferably about 10 to about 70% by weight. A content of a hydrophilic carrier to a whole preparation is about 0.1 to about 50% by weight, preferably about 10 to about 30% by weight. A content of a water-dispersible polymer to a whole preparation is about 0.1 to about 30% by weight, preferably about 10 to about 25% by weight. A content of a stabilizing agent relative to a whole preparation is about 0.1 to about 10% by weight, preferably about 1 to about 5% by weight. The aforementioned preparation may further contain an additive such as a colorant, a sweetener, and an antiseptic, if necessary.

A dose of the joint use of the present invention is different depending on a kind of the compound (I) of the present invention, an age, a weight, symptom, a dosage form, an administration method, and an administration term and, for example, about 0.01 to about 1000 mg/kg, preferably about 0.01 to about 100 mg/kg, more preferably about 0.1 to about 100 mg/kg, particularly about 0.1 to about 50 mg/kg, inter alia, about 1.5 to about 30 mg/kg per day in terms of the compound of the present invention and the joint use drug is intravenously administered per a diabetic patient (adult, weight about 60 kg) once to a few times a day. Of course, since a dose varies under the various conditions as described above, an amount smaller than the aforementioned dose is sufficient in some cases, and an amount exceeding the aforementioned range must be administered in some cases.

Any amount of the joint use drug may be set in such as range that side effect does not become problematic. A one day dose as the joint use drug is different depending on an extent of symptom, an age, a sex, a weight, and a difference in sensitivity of an administration subject, a term, and an interval of administration, nature, compounding, a kind, and a kind of an active ingredient of a drug preparation, and is not particularly limited. An amount of a drug is usually about 0.001 to 2000 mg, preferably about 0.01 to 500 mg, further preferably about 0.1 to 100 mg per 1 kg of a weight of a mammal by oral administration, and this is usually administered by dividing into portions administered once to four times a day.

Upon administration of the joint use agent of the present invention, the compound (I) of the present invention and the joint use drug may be administered at the same time, or after administration of the joint use drug, the compound (I) of the present invention may be administered, or after administration of the compound (I) of the present invention, the joint use drug may be administered. When they are administered at an interval, the interval is different depending on an active ingredient to be administered, a dosage form, and an administration method, and for example, when the joint use drug is administered first, there is a method of administering the compound (I) of the present invention in 1 minute to 3 days, preferably 10 minutes to 1 day, more preferably 15 minutes to 1 hour after administration of the joint use drug. When the compound (I) of the present invention is administered first, there is a method of administering the joint use drug in 1 minute to 1 day, preferably 10 minutes to 6 hours, more preferably 15 minutes to 1 hour after administration of the compound (I) of the present invention.

As a preferable administering method, for example, about 0.001 to 200 mg/kg of the joint use drug which has been formulated into an oral administration preparation is orally administered and, after about 15 minutes, about 0.005 to 100 mg/kg of the compound (I) of the present invention which has been formulated into an oral administration preparation is orally administered as a single dose.

### Examples

The present invention will be explained in detail by way of the following Reference Examples, Examples, Preparation Examples and Test Examples, but these examples are merely actual examples, and do not limit the present invention, and a variation is possible in such a range that it is not apart from the scope of the present invention.

Elution in column chromatography of Reference Examples, and Examples was performed under observation with TLC (Thin Layer Chromatography). In TLC observation, 60F₂₅₄ manufactured by Merck, or NH manufactured by Fuji Silicia Chemical was adopted as a TLC plate, a solvent used as an elution solvent in column chromatography was adopted as a developing solvent, and a UV detector was adopted as a detecting method. As the column silica gel, Kiesel Gel 60 (70 to 230 mesh) or Kiesel Gel 60 (230 to 400 mesh) manufactured by Merck was used. NMR spectra were measured with Varian Gemini 200-type, Varian Mercury 300-type or Bruker DPX-300-type spectrometer using tetramethylsilane as an internal or external standard, and a chemical shift is shown in a δ value, and a coupling constant is shown in Hz. IR spectra were measured with Shimadzu FTIR-8200-type spectrometer.

In a Reference Examples, and Examples, HPLC was measured under the following conditions, and purity and the like were determined.

Measuring equipment: Shimadzu Corporation LC-10Avp system (when otherwise is indicated) or Ajirent 1100 system
Column: CAPSEL PAK C18UG120 S-3 µm, 2.0×50 mm
Solvent: A solution; 0.1% trifluoroacetic acid containing water,
B solution; 0.1% trifluoroacetic acid containing acetonitrile
Gradient cycle: (A method): 0.00 minute (A solution/B solution = 90/10), 2.00 minutes (A solution/B solution = 5/95), 2.75 minutes (A solution/B solution = 5/95), 2.76 minutes (A solution/B solution = 90/10), 3.45 minutes (A solution/B solution = 90/10), or (B method): 0.00 minute (A solution/B solution = 90/10), 4.00 minutes (A solution/B solution = 5/95), 5.50 minutes (A solution/B solution = 5/95), 5.51 minutes (A solution/B solution = 90/10), 8.00 minutes (A solution/B solution = 90/10)
Injection amount: 10 µl, flow rate: 0.5 ml/min, detecting method: UV 220 nm

In Reference Examples, and Examples, mass spectra (MS) were measured under the following conditions.

Measurement equipment: Micromass Platform II, Waters ZQ, Waters ZMD, or JEOL. Ltd. JMS-AX505W
Ionization method: Atmospheric Pressure Chemical Ionization (APCI), Electron Spray Ionization (ESI), or Fast Atom Bombardment (FAB)

In purification of compounds in Reference Examples, and Examples, in addition to column chromatography, the following preparative HPLC equipment or intermediate pressure preparative LC equipment was used.
1) Preparative HPLC equipment: Gilson High Throughput Purification System
   Column: YMC Combiprep ODS-A S-5 µm, 50x20 mm
   Solvent: A solution; 0.1% trifluoroacetic acid containing water
   B solution; 0.1% trifluoroacetic acid containing acetonitrile
   Gradient cycle: 0.00 minute (A solution/B solution = 90/10), 1.20 minutes (A solution/B solution = 90/10), 4.75 minutes (A solution/B solution = 0/100), 7.30 minutes (A solution/B solution = 0/100), 7.40 minutes (A solution/B solution = 90/10), 7.50 minutes (A solution/B solution = 90/10)
   Flow rate: 25 ml/min, detecting method: UV 220 nm
2) Intermediate pressure preparative LC equipment: Moritex parallel purification system (PURIF 8)
   column: Yamazen HI-FLASH™ COLUMN (silica gel: 40 µm, 60 Å), 26x100 mm or 20x65 mm
   Flow rate: 20 ml/min
   Detecting method: UV 254 nm

In a mixed solvent, a numerical indicated in ( ) is a volume mixing ratio of each solvent. And, % in a solution represents a g number in 100 ml of a solution.

In addition, symbols in Reference Examples, and Examples have the following meanings.
S: singlet
d: doublet
t: triplet
q: quartet
quint: quintet
dd: double doublet
m: multiplet
br: broad
brs: broad singlet
J: coupling constant
CDCl₃: chloroform-d
DMSO-d₆: dimethyl sulfoxide-d₆
¹H-NMR: proton nuclear magnetic resonance
WSC: water-soluble carbodiimide
THF: tetrahydrofuran
DMF: dimethylformamide
DMSO: dimethyl sulfoxide
DNA: deoxyribonucleic acid
cDNA: complementary deoxyribonucleic acid
A: adenine
T: thymine
G: guanine
C: cytosine
Gly: glycine
Ala: alanine
Val: valine
Leu: leucine
Ile: isoleucine
Ser: serine
Thr: threonine
Cys: cysteine
Met: methionine
Glu: glutamic acid
Asp: aspartic acid
Lys: lysine
Arg: arginine
His: histidine
Phe: phenylalanine
Tyr: tyrosine
Trp: tryptophan
Pro: proline
Asn: asparagine
Gln: glutamine

SEQ ID Nos. in Sequence Listing in the present specification represent the following sequences.
[SEQ ID No.: 1]
   An amino acid sequence of a human-type RFRP receptor (0T7T022) is shown.
[SEQ ID No.: 2]
   An amino acid sequence of a rat-type RFRP receptor is shown.
[SEQ ID No.: 3]
   An amino acid sequence of a mouse-type RFRP receptor is shown.
[SEQ ID No.: 4]
   An amino acid sequence of human RFRP is shown.

### Reference Example 1

### 3-Hydroxy-2-methoxybenzaldehyde

Sodium hydride (5.79 g) was added to a dimethyl sulfoxide (80 ml) solution of 2,3-dihydroxybenzaldehyde (20.0 g), and the mixture was stirred at room temperature for 1 hour. To the reaction mixture was added methyl iodide (9.0 ml), and the mixture was further stirred at room temperature for 20 hours. The resulting reaction mixture was partitioned between ethyl acetate (1000 ml) and water (500 ml). The aqueous layer was further extracted with ethyl acetate (1000 ml), and the organic layers were combined, washed with an aqueous saturated sodium chloride solution, dried over anhydrous magnesium sulfate, and concentrated under reduced pressure. The residue was purified by silica gel chromatography, and crystallized using diisopropyl ether and hexane to obtain the title compound (11.5 g) as a pale brown crystal.
¹H-NMR (CDCl₃) δ: 3.98 (3H, s), 5.86 (1H, s), 7.12-7.40 (3H, m), 10.27 (1H, s).

### Reference Example 2

### 3-Benzyloxy-2-methoxybenzaldehyde

To a solution of the compound obtained in Reference Example 1 (11.4 g), potassium carbonate (22.8 g) and potassium iodide (2.49 g) in DMF (130 ml) was added benzyl bromide (9.8 ml), and the mixture was stirred at room temperature for 17 hours. The resulting reaction mixture was concentrated under reduced pressure, and the residue was partitioned between ethyl acetate (1500 ml) and water (1500 ml). The organic layer was washed successively with water and an aqueous saturated sodium chloride solution, dried over anhydrous magnesium sulfate, and concentrated under reduced pressure. The residue was purified by silica gel chromatography, and crystallized using diisopropyl ether and hexane to obtain the title compound (13.7 g) as a pale yellow crystal.
¹H-NMR (CDCl₃) δ: 4.03 (3H, s), 5.16 (2H, s), 7.09-7.47 (8H, m), 10.45 (1H, d, J = 0.66 Hz).

### Reference Example 3

### N-(4-Chlorophenyl)-2,2-dimethylpropionamide

To an acetonitrile (80 ml) solution of 4-chloroaniline (10.0 g) and triethylamine (16.4 ml) was added dropwise pivaloyl chloride (14.5 ml) under ice-cooling. A temperature of the mixture was raised to room temperature, followed by mixing for 5 hours. The resulting reaction mixture was concentrated under reduced pressure, and the residue was partitioned between ethyl acetate (1500 ml) and water (1500 ml). The organic layer was washed successively with water, an aqueous saturated sodium bicarbonate solution, and an aqueous saturated sodium chloride solution, dried over anhydrous magnesium sulfate, and concentrated under reduced pressure. The residue was crystallized using ethyl ether and hexane to obtain the title compound (15.0 g) as colorless crystals.
¹H-NMR (CDCl₃) δ: 1.31 (9H, s), 7.26-7.50 (4H, m).

### Reference Example 4

### N-{2-[(3-Benzyloxy-2-methoxyphenyl)hydroxymethyl]-4-chlorophenyl}-2,2-dimethylpropionamide

A tetrahydrofuran (120 ml) solution of the compound obtained in Reference Example 3 (11.5 g) was subjected to nitrogen replacement, and a 1.6M hexane solution (72 ml) of n-butyllithium was slowly added dropwise at -50°C with stirring. A temperature of the reaction mixture was raised to room temperature, followed by stirring for 2.5 hours. The reaction mixture was cooled again to -50°C, and a tetrahydrofuran (50 ml) solution of the compound obtained in Reference Example 2 (14.5 g) was added dropwise. A temperature of the reaction mixture was raised to room temperature, followed by stirring for 1 hour. The resulting reaction mixture was diluted with water, and extracted with ethyl acetate (1000 ml) two times. The organic layers were combined, washed with an aqueous saturated sodium chloride solution, dried over anhydrous magnesium sulfate, and concentrated under reduced pressure. The residue was purified by silica gel chromatography, and crystallized using diethyl ether and hexane to obtain the title compound (19.3 g) as colorless crystals.
¹H-NMR (CDCl₃) δ: 1.12 (9H, s), 3.93 (3H, s), 4.29 (1H, d, J = 4.5 Hz), 5.14 (2H, d, J = 1.4 Hz), 5.99 (1H, d, J = 4.5 Hz), 6.52 (1H, dd, J = 7.1, 1.9 Hz), 6.94-7.02 (3H, m), 7.28-7.47 (6H, m), 8.17 (1H, d, J = 8.7 Hz), 9.20 (1H, bs).

### Reference Example 5

### (2-Amino-5-chlorophenyl)-(3-benzyloxy-2-methoxyphenyl)methanol

To a tetrahydrofuran (50 ml) solution of the compound obtained in Reference Example 4 (20.0 g) was added 9N sulfuric acid (33.2 ml), and the mixture was heated to reflux for 5 hours. The reaction mixture was cooled with an ice, and a 4N aqueous sodium hydroxide solution (90 ml) was added slowly to basify the solution. The resulting reaction mixture was diluted with water, and extracted with ethyl acetate (1000 ml) two times. The organic layers were combined, washed successively with water and an aqueous saturated sodium chloride solution, dried over anhydrous magnesium sulfate, and concentrated under reduced pressure. The residue was purified by silica gel chromatography to obtain the title compound (11.5 g) as colorless crystals.
¹H-NMR (CDCl₃) δ: 3.12 (1H, d, J = 5.3 Hz), 3.87 (3H, s), 4.21 (2H, bs), 5.13 (2H, s), 6.03 (1H, d, J = 5.2 Hz), 6.59 (1H, d, J = 8.3 Hz), 6.86-7.10 (5H, m), 7.32-7.47 (5H, m).

### Reference Example 6

### (3-Benzyloxy-2-methoxyphenyl)-[5-chloro-2-(2,2-dimethylpropylamino)phenyl]methanol

To a methanol (80 ml) solution of the compound obtained in Reference Example 5 (9.5 g), pivalaldehyde (2.35 g), and acetic acid (4.1 ml) was added sodium cyanotrihydroborate (2.26 g), and the mixture was stirred at room temperature for 2 hours. The resulting reaction mixture was diluted with a 5% aqueous potassium bicarbonate solution, and concentrated under reduced pressure. The residue was partitioned between ethyl acetate (1000 ml) and water (1000 ml), and the organic layer was washed successively with an aqueous saturated sodium carbonate solution and an aqueous saturated sodium chloride solution, dried over anhydrous magnesium sulfate, and concentrated under reduced pressure. The residue was crystallized using diethyl ether and hexane to obtain the title compound (10.2 g) as colorless crystals.
¹H-NMR (CDCl₃) δ: 0.93 (9H, s), 2.83 (2H, d, J = 4.5 Hz), 3.21 (1H, d, J = 5.5 Hz), 3.87 (3H, s), 4.77-4.92 (1H, br), 5.13 (2H, s), 5.99(1H, d, J = 5.1 Hz), 6.57 (1H, d, J = 8.7Hz), 6.78-7.16 (5H, m), 7.30-7.49 (5H, m).

### Reference Example 7

### [3,5-trans-5-(3-Benzyloxy-2-methoxyphenyl)-7-chloro-1-(2,2-dimethylpropyl)-2-oxo-1,2,3,5-tetrahydro-4,1-benzoxazepin-3-yl]acetic acid ethyl ester

(1) 3-[{2-[(3-Benzyloxy-2-methoxyphenyl)hydroxymethyl]-4-chlorophenyl}-(2,2-dimethylpropyl)-carbamoyl]acrylic acid ethyl ester
   To a dichloromethane (200 ml) suspension of the compound obtained in Reference Example 6 (10.0 g) and sodium bicarbonate (5.35 g) was added dropwise a dichloromethane (30 ml) solution of ethyl (E)-4-chloro-4-oxo-2-butenoate, and the mixture was stirred at room temperature for 3 hours. The resulting reaction mixture was filtered, and the filtrate was concentrated under reduced pressure. The residue was partitioned between ethyl acetate (1000 ml) and water (1000 ml), and the organic layer was washed with an aqueous saturated sodium chloride solution, dried over anhydrous magnesium sulfate, and concentrated under reduced pressured. The residue was purified by silica gel chromatography to obtain the title compound (12.2 g) as a colorless oil.
(2) [3,5-trans-5-(3-Benzyloxy-2-methoxyphenyl)-7-chloro-1-(2,2-dimethylpropyl)-2-oxo-1,2,3,5-tetrahydro-4,1-benzoxazepin-3-yl]acetic acid ethyl ester
   An ethanol (140 ml) suspension of the compound obtained in Reference Example 7 (1) (12.1 g) and potassium carbonate (3.54 g) was stirred at room temperature for 19 hours, and the resulting reaction mixture was concentrated under reduced pressure. The residue was partitioned between ethyl acetate (1000 ml) and water (1000 ml), and the organic layer was washed with an aqueous saturated sodium chloride solution, dried over anhydrous magnesium sulfate, and concentrated under reduced pressure. The residue was crystallized with diethyl ether and hexane to obtain the title compound (10.7 g) as colorless crystals.
   ¹H-NMR (CDCl₃) δ: 0.94 (9H, s), 1.24 (3H, t, J = 7.1 Hz), 2.77 (1H, dd, J = 16.5, 6.0 Hz), 3.03 (1H, dd, J = 16.4, 7.7 Hz), 3.37 (1H, d, J = 13.9 Hz), 3.66 (3H, s), 4.04-4.22 (2H, m), 4 . 39 (1H, dd, J = 7.6, 6.0 Hz), 4 . 51 (1H, d, J = 13.9 Hz), 5.13 (2H, s), 6.28 (1H, s), 6.63 (1H, d, J = 1.7 Hz), 6.99-7.49 (10H, m).

### Reference Example 8

### [3,5-trans-5-(3-Benzyloxy-2-methoxyphenyl)-7-chloro-1-(2,2-dimethylpropyl)-2-oxo-1,2,3,5-tetrahydro-4,1-benzoxazepin-3-yl]acetic acid

To an ethanol/tetrahydrofuran (125 ml/150 ml) solution of the compound obtained in Reference Example 7 (10.6 g) was added a 1N aqueous sodium hydroxide solution (45 ml), and the mixture was heated at 60°C for 45 minutes. The reaction mixture was cooled, and 1N hydrochloric acid (80 ml) was added to neutralize the solution, and the resulting mixture was concentrated under reduced pressure. The residue was partitioned between ethyl acetate (1000 ml) and water (1000 ml), and the organic layer was washed with an aqueous saturated sodium chloride solution, dried over anhydrous magnesium sulfate, and concentrated under reduced pressure. The residue was crystallized using diethyl ether and hexane to obtain the title compound (9.4 g) as colorless crystals.
¹H-NMR (CDCl₃) δ: 0.95 (9H, s), 2.84 (1H, dd, J = 16.4, 5.4 Hz), 3.07 (1H, dd, J = 16.4, 7.5 Hz), 3.38 (1H, d, J = 13.8 Hz), 3.66 (3H, s), 4.34 (1H, dd, J = 7.4, 5.4 Hz), 4.52 (1H, d, J = 13.9 Hz), 5.13 (2H, s), 6.27 (1H, s), 6.65 (1H, d, J = 2.0 Hz), 7.00-7.49 (10H, m).

### Reference Example 9

### 2-[3,5-trans-5-(3-Benzyloxy-2-methoxyphenyl)-7-chloro-1-(2,2-dimethylpropyl)-2-oxo-1,2,3,5-tetrahydro-4,1-benzoxazepin-3-yl]-N-(2-fluorobenzyl)acetamide

To a DMF (150 ml) solution of the compound obtained in Reference Example 8 (9.3 g), HOBt (2.80 g), and WSC (3.98 g) was added a DMF (30 ml) solution of 2-fluorobenzylamine (2.60 g), the mixture was stirred at room temperature for 2.5 hours, and the reaction mixture was concentrated under reduced pressure. The residue was partitioned between ethyl acetate (1000 ml) and water (1000 ml), and the organic layer was washed successively with an aqueous saturated sodium bicarbonate solution and an aqueous saturated sodium chloride solution, washed with anhydrous magnesium sulfate, and concentrated under reduced pressure. The residue was crystallized using diethyl ether and hexane to obtain the title compound (10.7 g) as colorless crystals. ¹H-NMR (CDCl₃) δ: 0.93 (9H, s), 2.69 (1H, dd, J = 14.3, 6.1 Hz), 2.87 (1H, dd, J = 14.3, 6.9 Hz), 3.35 (1H, d, J = 13.8 Hz), 3.65 (3H, s), 4.10-4.52 (4H, m), 5.13 (2H, s), 6.26 (1H, s), 6.30 (1H, brs), 6.62 (1H, d, J = 2.2 Hz), 6.99-7.49 (14H, m).

### Reference Example 10

### 2-[3,5-trans-7-Chloro-5-(3-hydroxy-2-methoxyphenyl)-1-(2,2-dimethylpropyl)-2-oxo-1,2,3,5-tetrahydro-4,1-benzoxazepin-3-yl]-N-(2-fluorobenzyl)acetamide

To an ethyl acetate (200 ml) suspension of the compound obtained in Reference Example 9 (9.3 g) and 10% palladium carbon (1.0 g) was added 5N hydrochloric acid (10 ml), and the mixture was stirred at room temperature for 1.5 hours under the hydrogen gas atmosphere. The reaction mixture was filtered with Celite, and the filtrate was concentrated under reduced pressure. The residue was partitioned between ethyl acetate (1000 ml) and water (1000 ml), and the organic layer was washed with an aqueous saturated sodium chloride solution, dried over anhydrous magnesium sulfate, and concentrated under reduced pressure. The residue was crystallized using diethyl ether and hexane to obtain the title compound (8.8 g) as a white powder.
¹H-NMR (CDCl₃) δ: 0.94 (9H, s), 2.69 (1H, dd, J = 14.4, 6.1 Hz), 2.87 (1H, dd, J = 14.4, 6.8 Hz), 3.37 (1H, d, J = 13.9 Hz), 3.58 (3H, s), 4.39-4.52 (4H, m), 5.51 (1H, s), 6.23 (1H, s), 6.29 (1H, brs), 6.62 (1H, d, J = 2.2 Hz), 6.96-7.41 (9H, m).

### Reference Example 11

### 3-Bromopropylcarbamic acid tert-butyl ester

To a water/tetrahydrofuran (15 ml/15 ml) suspension of 3-bromopropylamine hydrobromide (3.0 g) and sodium carbonate (2.18 g) was added a tetrahydrofuran (10 ml) solution of di(tert-butyl) dicarbonate (3.14 g), and the mixture was stirred at room temperature for 20 hours. The resulting reaction mixture was concentrated under reduced pressure, and the residue was partitioned between ethyl acetate (500 ml) and water (500 ml). The organic layer was washed successively with 1N hydrochloric acid, an aqueous saturated sodium bicarbonate solution, and an aqueous saturated sodium chloride solution, dried over anhydrous magnesium sulfate, and concentrated under reduced pressure to obtain the title compound (3.3 g) as a colorless oil.
¹H-NMR (CDCl₃) δ: 1.44 (9H, s), 2.05 (2H, quint, J = 6.5 Hz), 3.28 (2H, q, J = 6.5 Hz), 3.44 (2H, t, J = 6.5Hz), 4.66 (1H, brs).

### Reference Example 12

According to the method described in WO 98/47882, the following Compounds A to X were obtained.
A: The compound described in Example 41 of WO 98/47882 3,5-trans-N-(2-Fluorobenzyl)-5-(3-benzylaminomethylphenyl)-7-chloro-1-neopentyl-2-oxo-1,2,3,5-tetrahydro-4,1-benzoxazepine-3-acetamide hydrochloride
B: The compound described in Example 42 of WO 98/47882 3,5-trans-N-(2-Fluorobenzyl)-7-chloro-1-neopentyl-2-oxo-5-[3-(piperidin-1-yl)methylphenyl]-1,2,3,5-tetrahydro-4,1-benzoxazepine-3-acetamide hydrochloride
C: The compound described in Example 43 of WO 98/47882 3,5-trans-N-(2-Fluorobenzyl)-7-chloro-5-(3-methylaminomethylphenyl)-1-neopentyl-2-oxo-1,2,3,5-tetrahydro-4,1-benzoxazepine-3-acetamide hydrochloride
D: The compound described in Example 44 of WO 98/47882 3,5-trans-N-(2-Fluorobenzyl)-7-chloro-5-(3-dimethylaminomethylphenyl)-1-neopentyl-2-oxo-1,2,3,5-tetrahydro-4,1-benzoxazepine-3-acetamide hydrochloride
E: The compound described in Example 80 of WO 98/47882 3,5-trans-N-(2-Fluorobenzyl)-5-(3-aminophenyl)-7-chloro-1-neopentyl-2-oxo-1,2,3,5-tetrahydro-4,1-benzoxazepine-3-acetamide hydrochloride
F: The compound described in Example 83 of WO 98/47882 3,5-trans-N-(2-Fluorobenzyl)-5-[3-(2-aminoethyl)phenyl]-7-chloro-1-neopentyl-2-oxo-1,2,3,5-tetrahydro-4,1-benzoxazepine-3-acetamide hydrochloride
G: The compound described in Example 85 of WO 98/47882 3,5-trans-N-(2-Fluorobenzyl)-5-[4-(2-aminoethyl)phenyl]-1-(4-biphenylmethyl)-7-chloro-2-oxo-1,2,3,5-tetrahydro-4,1-benzoxazepine-3-acetamide hydrochloride
H: The compound described in Example 93 of WO 98/47882 3,5-trans-N-(2-Fluorobenzyl)-5-[3-[(1-amino-1-methyl)ethyl]phenyl]-1-(4-biphenylmethyl)-7-chloro-2-oxo-1,2,3,5-tetrahydro-4,1-benzoxazepine-3-acetamide hydrochloride
I: The compound described in Example 123 of WO 98/47882 3,5-trans-N-(2-Fluorobenzyl)-5-[3-(3-tert-butoxycarbonylaminopropyl)aminomethylphenyl]-7-chloro-1-neopentyl-2-oxo-1,2,3,5-tetrahydro-4,1-benzoxazepine-3-acetamide
J: The compound described in Example 124 of WO 98/47882 3,5-trans-N-(2-Fluorobenzyl)-5-[3-(3-aminopropyl)aminomethylphenyl]-7-chloro-1-neopentyl-2-oxo-1,2,3,5-tetrahydro-4,1-benzoxazepine-3-acetamide dihydrochloride
K: The compound described in Example 125 of WO 98/47882 3,5-trans-N-(2-Fluorobenzyl)-5-[3-aminoacetylaminomethylphenyl]-1-benzyl-7-chloro-2-oxo-1,2,3,5-tetrahydro-4,1-benzoxazepine-3-acetamide hydrochloride
L: The compound described in Example 126 of WO 98/47882 3,5-trans-N-(2-Fluorobenzyl)-1-(4-biphenylmethyl)-7-chloro-2-oxo-5-[3-[(piperidin-4-yl)carbonylaminomethyl]phenyl]-1,2,3,5-tetrahydro-4,1-benzoxazepine-3-acetamide hydrochloride
M: The compound described in Example 127 of WO 98/47882 3,5-trans-N-(2-Fluorobenzyl)-5-[2-(3-aminopropyloxy)phenyl]-7-chloro-1-isobutyl-2-oxo-1,2,3,5-tetrahydro-4,1-benzoxazepine-3-acetamide hydrochloride
N: The compound described in Example 128 of WO 98/47882 3,5-trans-N-(2-Fluorobenzyl)-5-[4-(3-aminopropyloxy)-2-methoxyphenyl]-7-chloro-1-neopentyl-2-oxo-1,2,3,5-tetrahydro-4,1-benzoxazepine-3-acetamide hydrochloride
O: The compound described in Example 133 of WO 98/47882 3,5-trans-N-(2-Fluorobenzyl)-7-chloro-1-(3,3-dimethylbutyl)-2-oxo-5-(3-tritylaminomethylphenyl)-1,2,3,5-tetrahydro-4,1-benzoxazepine-3-acetamide
P: The compound described in Example 146 of WO 98/47882 3,5-trans-N-(2-Fluorobenzyl)-5-[4-[(1-amino-1-methyl)ethyl]phenyl]-1-(4-biphenylmethyl)-7-chloro-2-oxo-1,2,3,5-tetrahydro-4,1-benzoxazepine-3-acetamide hydrochloride
Q: The compound described in Example 152 of WO 98/47882 3,5-trans-N-(2-Fluorobenzyl)-5-[2-(2-aminoethyl)phenyl]-1-(4-biphenylmethyl)-7-chloro-2-oxo-1,2,3,5-tetrahydro-4,1-benzoxazepine-3-acetamide
R: The compound described in Example 214 of WO 98/47882 (3,5-trans)-N-(2-Fluorobenzyl)-5-(3-aminomethylphenyl)-7-chloro-2,3,4,5-tetrahydro-2-oxo-1H-1,4-benzodiazepine-3-acetamide dihydrochloride
S: The compound described in Example 216 of WO 98/47882 (3,5-trans)-N-(2-Fluorobenzyl)-5-(3-aminomethylphenyl)-1-(4-biphenylmethyl)-7-chloro-2,3,4,5-tetrahydro-2-oxo-1H-1,4-benzodiazepine-3-acetamide dihydrochloride
T: The compound described in Example 218 of WO 98/47882 (3,5-trans)-N-(2-Fluorobenzyl)-5-(3-aminomethylphenyl)-1-(4-biphenylmethyl)-7-chloro-4-methyl-2,3,4,5-tetrahydro-2-oxo-1H-1,4-benzodiazepine-3-acetamide dihydrochloride
U: The compound described in Example 220 of WO 98/47882 3,5-trans-N-(2-Fluorobenzyl)-5-(3-aminomethylphenyl)-1-(4-biphenylmethyl)-7-chloro-2-oxo-1,2,3,5-tetrahydro-4,1-benzothiazepine-3-acetamide hydrochloride
V: The compound described in Example 222 of WO 98/47882 3,5-trans-N-(2-Fluorobenzyl)-5-(3-aminomethylphenyl)-1-(4-biphenylmethyl)-7-chloro-1,2,3,5-tetrahydro-2-oxo-4,1-benzothiazepine-3-acetamide S-oxide hydrochloride
W: The compound described in Example 224 of WO 98/47882 3,5-trans-N-(2-Fluorobenzyl)-5-(3-aminomethylphenyl)-1-(4-biphenylmethyl)-7-chloro-1,2,3,5-tetrahydro-2-oxo-4,1-benzothiazepine-3-acetamide S-dioxide hydrochloride
X: The compound described in Example 234 of WO 98/47882 3,5-trans-N-(2-Fluorobenzyl)-5-(5-aminomethyl-2-methoxyphenyl)-1-(4-biphenylmethyl)-7-chloro-2-oxo-1,2,3,5-tetrahydro-4,1-benzoxazepine-3-acetamide hydrochloride

### Example 1

### [3-(3-{3,5-trans-7-Chloro-1-(2,2-dimethylpropyl)-3-[(2-fluorobenzylcarbamoyl)methyl]-2-oxo-1,2,3,5-tetrahydro-4,1-benzoxazepin-5-yl}-2-methoxyphenoxy)propyl]carbamic acid tert-butyl ester

To a DMF (20 ml) suspension of the compound obtained in Reference Example 10 (0.91 g) and potassium carbonate (0.34 g) was added a DMF (5 ml) solution of the compound obtained in Reference Example 11 (0.47 g), and the mixture was stirred at 90°C for 2 hours. The resulting reaction mixture was concentrated under reduced pressure, and the residue was partitioned between ethyl acetate (1000 ml) and water (1000 ml). The organic layer was washed with an aqueous saturated sodium chloride solution, dried over anhydrous magnesium sulfate, and concentrated under reduced pressure. The residue was purified by silica gel chromatography to obtain the title compound (0.94 g) as a colorless oil.
¹H-NMR (CDCl₃) δ: 0.94 (9H, s), 1.42 (9H, s), 2.02 (2H, m), 2.69 (1H, dd, J = 14.3, 6.1 Hz), 2.86 (1H, dd, J = 14.3, 6.9 Hz), 3.28-3.41 (2H, m), 3.35 (1H, d, J = 13.7 Hz), 3.62 (3H, s), 4.08 (2H, t, J = 6.0 Hz), 4.35-4.60 (4H, m), 4.85 (1H, br), 6.25 (1H, s), 6.29 (1H, br), 6.60 (1H, d, J = 2.2 Hz), 6.94-7.38 (9H, m).

### Example 2

### 2-[3,5-trans-5-[3-(3-Aminopropoxy)-2-methoxyphenyl]-7-chloro-1-(2,2-dimethylpropyl)-2-oxo-1,2,3,5-tetrahydro-4,1-benzoxazepin-3-yl]-N-(2-fluorobenzyl)acetamide hydrochloride

To an ethyl acetate (4 ml) solution of the compound obtained in Example 1 (0.94 g) was added a 4N hydrochloric acid/ethyl acetate solution (4 ml), and the mixture was stirred at room temperature for 1.5 hours. The resulting reaction mixture was concentrated under reduced pressure, and the residue was dissolved in ethanol, ethyl acetate and ether were then added. The resulting precipitate was filtered to obtain the title compound (0.77 g) as a white powder.
¹H-NMR (CD₃OD) δ 0.95 (9H, s), 2.18 (2H, m), 2.75 (2H, d, J = 6.7 Hz), 3.17 (2H, m) 3.52-3.64 (1H, m), 3.61 (3H, s), 4.20 (2H, t, J = 5.8 Hz), 4.35-4.50 (4H, m), 6.23 (1H, s), 6.49 (1H, d, J = 2.4 Hz), 7.02-7.63 (9H, m).

### Example 3

### 2-(3,5-trans-7-Chloro-1-(2,2-dimethylpropyl)-5-{2-methoxy-3-[3-(3-phenylpropylamino)propoxy]phenyl}-2-oxo-1,2,3,5-tetrahydro-4,1-benzoxazepin-3-yl)-N-(2-fluorobenzyl)acetamide hydrochloride

To a methanol (5 ml) solution of the compound obtained in Example 2 (300 mg) and 3-phenylpropanealdehyde (66 mg) was added sodium cyanotrihydroborate (35 mg), and the mixture was stirred at room temperature for 3 hours. The resulting reaction mixture was diluted with water, and extracted with ethyl acetate (100 ml). The organic layer was washed with an aqueous saturated sodium chloride solution, dried over anhydrous magnesium sulfate, and concentrated under reduced pressure. The residue was purified by silica gel chromatography to obtain a colorless oil (119 mg). The resulting oil (119 mg) was dissolved in ethyl acetate (2 ml), and to the solution was added a 4N hydrochloric acid/ethyl acetate solution (0.1 ml), followed by stirring at room temperature for 2 hours. The resulting reaction mixture was concentrated under reduced pressure, and the residue was crystallized using ethyl acetate and ether to obtain the title compound (113 mg) as colorless crystals.
¹H-NMR (CD₃OD) δ: 0.93 (9H, s), 1.98 (2H, m), 2.20 (2H, m), 2.70 (2H, t, J = 7.7 Hz), 2.75 (2H, d, J = 6.7 Hz), 3.02-3.06 (2H, m), 3.23 (2H, m), 3.54 (1H, m), 3.59 (3H, s), 4.20 (2H, t, J = 5.7 Hz), 4.34-4.50 (4H, m), 6.23 (1H, s), 6.49 (1H, d, J = 2.5 Hz), 7.02-7.61 (14H, m).

### Example 4

### 2-{3,5-trans-7-Chloro-1-(2,2-dimethylpropyl)-5-[2-methoxy-3-(3-(pentylamino)propoxy)phenyl]-2-oxo-1,2,3,5-tetrahydro-4,1-benzoxazepin-3-yl}-N-(2-fluorobenzyl)acetamide hydrochloride

Using the compound obtained by Example 2 and valeraldehyde and according to the same manner as that of Example 3, the title compound (25 mg) was obtained as colorless crystals.
¹H-NMR (CD₃OD) δ: 0.92 (3H, t, J = 7.0Hz), 0.95 (9H, s), 1.30-1.41 (4H, m), 1.67 (2H, m), 2.21 (2H, m), 2.75 (2H, d, J = 6.7 Hz), 2.98-3.04 (2H, m), 3.24 (2H, m), 3.55-3.62 (1H, m), 3.61 (3H, s), 4.21 (2H, t, J = 5.7 Hz), 4.38-4.50 (4H, m), 6.23 (1H, s), 6.50 (1H, d, J = 2.4Hz), 7.03-7.65 (9H, m) .

### Example 5

### trans-2-(5-{3-[3-(Benzylamino)propoxy]-2-methoxyphenyl}-7-chloro-1-(2,2-dimethylpropyl)-2-oxo-1,2,3,5-tetrahydro-4,1-benzoxazepin-3-yl)-N-(2-fluorobenzyl)acetamide hydrochloride

An ethanol (5 ml) solution of the compound obtained in Example 2 (100 mg) and benzaldehyde (17 mg) was stirred at room temperature for 15 hours, and concentrated under reduced pressure. The residue was dissolved in ethanol (5 ml), sodium borohydride (12 mg) was added, and the mixture was stirred at room temperature for 4 hours. The reaction mixture was concentrated under reduced pressure, and the residue was partitioned between ethyl acetate and water. The organic layer was washed with an aqueous saturated sodium chloride solution, dried over anhydrous magnesium sulfate, and concentrated under reduced pressure. The residue was purified by silica gel chromatography to obtain colorless oil. To a methylene chloride (3 ml) solution of this oil was added 1N etheric hydrochloric acid (0.5 ml), and the mixture was stirred at room temperature for 5 hours. The reaction mixture was concentrated under reduced pressure, and the resulting residue was crystallized from methylene chloride-diethyl ether to obtain the title compound (60 mg) as colorless crystals.
¹H-NMR (DMSO-d₆) δ: 0.88 (9H, s), 2.07-2.20 (2H, m), 2.62-2.66 (2H, m), 3.04-3.16 (2H, m), 3.44 (3H, m), 3.60 (1H, d, J = 13.8 Hz), 4.08-4.39 (8H, m), 6.08 (1H, s), 6.38 (1H, d, J = 2.5 Hz), 7.38-7.59 (6H, m), 7.75 (1H, d, J = 8.8 Hz), 8.47 (1H, d, J = 5.9 Hz), 8.95 (2H, brs).

### Example 6

### trans-2-[7-Chloro-1-(2,2-dimethylpropyl)-5-(3-{3-[(2-furylmethyl)amino]propoxy}-2-methoxyphenyl)-2-oxo-1,2,3,5-tetrahydro-4,1-benzoxazepin-3-yl]-N-(2-fluorobenzyl)acetamide hydrochloride

From the compound obtained in Example 2 (160 mg) and furfural (25 mg) and according to the same manner as that of Example 3, the title compound (20 mg) was obtained as colorless crystals.
¹H-NMR (DMSO-d₆) δ: 0.88 (9H, s), 2.02-2.13 (2H, m), 2.58-2.68 (2H, m), 3.03-3.13 (2H, m), 3.48 (3H, m), 3.60 (1H, d, J = 14.1 Hz), 4.04-4.17 (2H, m), 4.18-4.40 (6H, m), 6.09 (1H, s), 6.38 (1H, d, J = 2.5 Hz), 6.51 (1H, dd, J = 1.9, 3.3 Hz), 6.60 (1H, d, J = 3.3 Hz), 7.03 (1H, dd, J = 2.5, 8.7 Hz), 7.09-7.21 (4H, m), 7.26-7.37 (2H, m), 7.54 (1H, dd, J = 2.5, 8.7 Hz), 7.72-7.79 (2H, m), 8.46 (1H, d, J = 6.0 Hz), 8.97 (2H, brs).

### Example 7

### trans-2-[7-Chloro-1-(2,2-dimethylpropyl)-5-(2-methoxy-3-{3-[(2-thienylmethyl)amino]propoxy}phenyl)-2-oxo-1,2,3,5-tetrahydro-4,1-benzoxazepin-3-yl]-N-(2-fluorobenzyl)acetamide hydrochloride

From the compound obtained in Example 2 (160 mg) and 2-thiophenecarboxyaldehyde (29 mg) and according to the same manner as that of Example 3, the title compound (70 mg) was obtained as colorless crystals.
¹H-NMR (DMSO-d₆) δ: 0.88 (9H, s), 2.05-2.20 (2H, m), 2.58-2.68 (2H, m), 3.05-3.17 (2H, m), 3.47 (3H, m), 3.60 (1H, d, J = 13.9 Hz), 4.07-4.38 (6H, m), 4.39-4.47 (2H, m), 6.08 (1H, s), 6.38 (1H, d, J = 2.5 Hz), 7.03-7.21 (6H, m), 7.26-7.38 (3H, m), 7.54 (1H, dd, J = 2.5, 8.7 Hz), 7.63 (1H, dd, J = 1.2, 5.1 Hz), 7.75 (1H, d, J = 8.8 Hz), 8.46 (1H, d, J = 5.9 Hz), 8.96 (2H, brs).

### Example 8

### trans-2-[7-Chloro-1-(2,2-dimethylpropyl)-5-(2-methoxy-3-{3-[(2-pyridinylmethyl)amino]propoxy}phenyl)-2-oxo-1,2,3,5-tetrahydro-4,1-benzoxazepin-3-yl]-N-(2-fluorobenzyl)acetamide hydrochloride

From the compound obtained in Example 2 (300 mg) and 2-pyridinecarboxyaldehyde (50 mg) and according to the same manner as that of Example 3, the title compound (90 mg) was obtained as colorless crystals.
¹H-NMR(DMSO-d₆) δ: 0.88 (9H, s), 2.13-2.33 (2H, m), 2.60-2.70 (2H, m), 3.12-3.26 (2H, m), 3.48 (3H, m), 3.60 (1H, d, J = 14.0 Hz), 4.10-4.40 (8H, m), 6.08 (1H, s), 6.38 (1H, d, J = 2.5 Hz), 7.04 (1H, dd, J = 1.7, 6.6 Hz), 7.09-7.20 (4H, m), 7.27-7.36 (2H, m), 7.39-7.45 (1H, m), 7.47-7.57 (2H, m), 7.75 (1H, d, J = 8.9 Hz), 7.85-7.95 (1H, m), 8.46 (1H, d, J = 5.9 Hz), 8.60-8.62 (1H, m), 9.17 (2H, brs).

### Example 9

### trans-2-[7-Chloro-1-(2,2-dimethylpropyl)-5-(2-methoxy-3-{3-[(2-naphthylmethyl)amino]propoxy}phenyl)-2-oxo-1,2,3,5-tetrahydro-4,1-benzoxazepin-3-yl]-N-(2-fluorobenzyl)acetamide hydrochloride

From the compound obtained in Example 2 (200 mg) and 2-naphthoaldehyde (48 mg) and according to the same manner as that of Example 3, the title compound (20 mg) was obtained as colorless crystals.
¹H-NMR (DMSO-d₆) δ: 0.84 (9H, s), 2.10-2.21 (2H, m), 2.56-2.67 (2H, m), 3.05-3.16 (2H, m), 3.23 (3H, m), 3.59 (1H, d, J = 13.9 Hz), 4.08-4.40 (8H, m), 6.02 (1H, s), 6.34 (1H, d, J = 2.5 Hz), 6.98-7.04 (1H, m), 7.08-7.20 (4H, m), 7.25-7.34 (2H, m), 7.50-7.58 (3H, m), 7.61-7.67 (1H, m), 7.73 (1H, d, J = 8.9 Hz), 7.86-8.05 (4H, m), 8.46 (1H, d, J = 5.8 Hz), 9.12 (2H, brs).

### Example 10

### trans-2-[7-Chloro-1-(2,2-dimethylpropyl)-5-[2-methoxy-3-{3-[(1-naphathylmethyl)amino]propoxy}phenyl]-2-oxo-1,2,3,5-tetrahydro-4,1-benzoxazepin-3-yl]-N-(2-fluorobenzyl)acetamide hydrochloride

From the compound obtained in Example 2 (200 mg) and 1-naphthoaldehyde (29 mg) and according to the same manner as that of Example 3, the title compound (10 mg) was obtained as colorless crystals.
¹H-NMR (DMSO-d₆) δ: 0.88 (9H, s), 2.10-2.23 (2H, m), 2.61-2.68 (2H, m), 3.18-3.26 (2H, m), 3.41 (3H, m), 3.61 (1H, d, J = 13.8 Hz), 4.09-4.39 (6H, m), 4.63-4.70 (2H, m), 6.08 (1H, s), 6.37 (1H, d, J = 2.5 Hz), 7.04 (1H, dd, J = 2.8, 6.2 Hz), 7.10-7.21 (4H, m), 7.27-7.36 (2H, m), 7.51-7.78 (6H, m), 7.96-8.04 (2H, m), 8.24 (1H, d, J = 8.4 Hz), 8.46 (1H, d, J = 5.8 Hz), 8.95 (2H, brs).

### Example 11

### trans-2-[7-Chloro-1-(2,2-dimethylpropyl)-5-[2-methoxy-3-{3-[(4-phenylbutyl)amino]propoxy}phenyl]-2-oxo-1,2,3,5-tetrahydro-4,1-benzoxazepin-3-yl]-N-(2-fluorobenzyl)acetamide hydrochloride

From the compound obtained in Example 2 (150 mg) and 4-phenylbutanal (34 mg) and according to the same manner as that of Example 3, the title compound (10 mg) was obtained as colorless crystals.
¹H-NMR (DMSO-d₆) δ: 0.87 (9H, s), 1.50-1.70 (6H, m), 2.05-2.20 (2H, m), 2.55-2.68 (4H, m), 3.04-3.20 (2H, m), 3.50 (3H, m), 3.60 (1H, d, J = 14.0 Hz), 4.02-4.39 (6H, m), 6.09 (1H, s), 6.38 (1H, d, J = 2.4 Hz), 7.00-7.36 (11H, m), 7.49-7.58 (1H, m), 7.65-7.78 (2H, m), 8.45 (1H, d, J = 5.8 Hz), 9.25 (2H, brs).

### Example 12

### trans-2-{7-Chloro-5-[3-(3-{[3-(4-chlorophenyl)propyl]amino}propoxy)-2-methoxyphenyl]-1-(2,2-dimethylpropyl)-2-oxo-1,2,3,5-tetrahydro-4,1-benzoxazepin-3-yl}-N-(2-fluorobenzyl)acetamide hydrochloride

From the compound obtained in Example 2 (150 mg) and 3-(4-chlorophenyl)propanal (39 mg) and according to the same manner as that of Example 3, the title compound (105 mg) was obtained as colorless crystals.
¹H-NMR (DMSO-d₆) δ: 0.87 (9H, s), 1.82-1.95 (2H, m), 2.04-2.16 (2H, m), 2.60-2.68 (4H, m), 2.87-2.95 (2H, m), 3.02-3.12 (2H, m), 3.51 (3H, m), 3.60 (1H, d, J = 13.9 Hz), 4.09-4.39 (6H, m), 6.09 (1H, s), 6.38 (1H, d, J = 2.5 Hz), 7.04 (1H, dd, J = 2.6, 6.6 Hz), 7.10-7.38 (10H, m), 7.54 (1H, dd, J = 2.5, 8.8 Hz), 7.74 (1H, d, J = 8.8 Hz), 8.46 (1H, t, J = 5.9 Hz), 8.54 (2H, brs).

### Example 13

### trans-2-{7-Chloro-5-[3-(3-{[3-(3-chlorophenyl)propyl]amino}propoxy)-2-methoxyphenyl]-1-(2,2-dimethylpropyl)-2-oxo-1,2,3,5-tetrahydro-4,1-benzoxazepin-3-yl}-N-(2-fluorobenzyl)acetamide hydrochloride

From the compound obtained in Example 2 (200 mg) and 3-(3-chlorophenyl)propanal (52 mg) and according to the same manner as that of Example 3, the title compound (120 mg) was obtained as colorless crystals.
¹H-NMR (DMSO-d₆) δ: 0.87 (9H, s), 1.80-1.94 (2H, m), 2.02-2.15 (2H, m), 2.60-2.70 (4H, m), 2.84-2.96 (2H, m), 3.02-3.13 (2H, m), 3.51 (3H, m), 3.60 (1H, d, J = 13.9 Hz), 4.07-4.39 (6H, m), 6.09 (1H, s), 6.38 (1H, d, J = 2.5 Hz), 7.04 (1H, dd, J = 2.5, 6.7 Hz), 7.10-7.21 (5H, m), 7.24-7.37 (5H, m), 7.55 (1H, dd, J = 2.5, 8.8 Hz), 7.74 (1H, d, J = 8.8 Hz), 8.40 (2H, brs), 8.46 (1H, t, J = 5.8 Hz).

### Example 14

### trans-2-{7-Chloro-5-[3-(3-{[3-(2-chlorophenyl)propyl]amino}propoxy)-2-methoxyphenyl]-1-(2,2-dimethylpropyl)-2-oxo-1,2,3,5-tetrahydro-4,1-benzoxazepin-3-yl}-N-(2-fluorobenzyl)acetamide hydrochloride

From the compound obtained in Example 2 (150 mg) and 3-(2-chlorophenyl)propanal (39 mg) and according to the same manner as that of Example 3, the title compound (90 mg) was obtained as colorless crystals.
¹H-NMR (DMSO-d₆) δ: 0.87 (9H, s), 1.82-1.95 (2H, m), 2.03-2.14 (2H, m), 2.60-2.68 (2H, m), 2.71-2.80 (2H, m), 2.92-3.00 (2H, m), 3.02-3.12 (2H, m), 3.52 (3H, m), 3.60 (1H, d, J = 14.0 Hz), 4.06-4.39 (6H, m), 6.09 (1H, s), 6.38 (1H, d, J = 2.5 Hz), 7.04 (1H, dd, J = 2.7, 6.5 Hz), 7.10-7.46 (10H, m), 7.54 (1H, dd, J = 2.5, 8.7 Hz), 7.74 (1H, d, J = 8.9 Hz), 8.46 (1H, t, J = 5.8 Hz), 8.50 (2H, brs).

### Example 15

### trans-2-[7-Chloro-1-(2,2-dimethylpropyl)-5-(2-methoxy-3-{3-[methyl(3-phenylpropyl)amino]propoxy}phenyl)-2-oxo-1,2,3,5-tetrahydro-4,1-benzoxazepin-3-yl]-N-(2-fluorobenzyl)acetamide hydrochloride

To an acetonitrile/tetrahydrofuran (5 ml/1 ml) solution of the compound obtained in Example 3 (47 mg) and a 37% aqueous formalin solution (0.5 ml) was added sodium cyanotrihydroborate (8 mg), the mixture was stirred at room temperature for 10 minutes, and acetic acid (31 mg) was added, followed by further stirring for 3 hours. The reaction mixture was concentrated under reduced pressure, the residue was partitioned between ethyl acetate (50 ml) and an aqueous saturated sodium bicarbonate solution (50 ml), and the organic layer was washed successively with an aqueous saturated sodium bicarbonate solution and an aqueous saturated sodium chloride solution. This solution was dried over anhydrous magnesium sulfate, and concentrated under reduced pressure. The residue was purified by alumina chromatography to obtain colorless oil (22 mg). To a methylene chloride (3 ml) solution of this oil (22 mg) was added 1N etheric hydrochloric acid (0.5 ml), and the mixture was stirred at room temperature for 5 minutes. The reaction mixture was concentrated under reduced pressure, and the resulting residue was crystallized from methylene chloride-diethyl ether to obtain the title compound (20 mg) as colorless crystals.
¹H-NMR (DMSO-d₆) δ: 0.87 (9H, s), 1.85-2.00 (2H, m), 2.05-2.20 (2H, m), 2.55-2.70 (4H, m), 2.79 (3H, brs), 3.00-3.22 (2H, m), 3.40-3.65 (6H, m), 4.05-4.40 (6H, m), 6.08 (1H, s), 6.37 (1H, d, J = 2.5 Hz), 7.04 (1H, dd, J = 2.9, 6.3 Hz), 7.10-7.35 (11H, m), 7.54 (1H, dd, J = 2.5, 8.8 Hz), 7.74 (1H, d, J = 8.8 Hz), 8.47 (1H, t, J = 5.8 Hz), 9.48 (1H, brs).

### Example 16

### trans-2-[7-Chloro-1-(2,2-dimethylpropyl)-5-(2-methoxy-3-{3-[(2-phenoxyethyl)amino]propoxy}phenyl)-2-oxo-1,2,3,5-tetrahydro-4,1-benzoxazepin-3-yl]-N-(2-fluorobenzyl)acetamide hydrochloride

To a methylene chloride (3 ml) solution of the compound obtained in Example 2 (150 mg) and pyridine (55 mg) was added o-nitrobenzenesulfonyl chloride (61 mg) under ice-cooling, and the mixture was stirred at room temperature for 3 hours. The reaction mixture was poured into an aqueous saturated sodium bicarbonate solution, and extracted with ethyl acetate. The extract was washed successively with 1N hydrochloric acid, an aqueous saturated sodium bicarbonate solution and an aqueous saturated sodium chloride solution, dried over anhydrous magnesium sulfate, and concentrated under reduced pressure. The residue was dissolved in dimethylformamide (5 ml), to this solution were added 2-phenoxyethyl bromide (92 mg), potassium iodide (76 mg) and cesium carbonate (225 mg), and the mixture was stirred at 80°C for 20 hours. The reaction mixture was poured into water, and extracted with ethyl acetate. The extract was washed successively with water and an aqueous saturated sodium chloride solution, dried over anhydrous magnesium sulfate, and concentrated under reduced pressure. The residue was purified by silica gel chromatography to obtain a colorless oil (105 mg). To a dimethylformamide (4 ml) solution of this colorless oil (90 mg) were added thiophenol (32 mg) and cesium carbonate (160 mg), and the mixture was stirred at room temperature for 15 hours. The reaction mixture was poured into water, and extracted with ethyl acetate. The extract was washed successively with water and an aqueous saturated sodium chloride solution, dried over anhydrous magnesium sulfate, and concentrated under reduced pressure. The residue was purified by silica gel chromatography to obtain a colorless oil (70 mg). To a methylene chloride (3 ml) solution of this oil (70 mg) was added 1N etheric hydrochloric acid (0.5 ml), and the mixture was stirred at room temperature for 5 minutes. The reaction mixture was concentrated under reduced pressure, and the resolution residue was crystallized from methylene chloride-diethyl ether to obtain the title of the compound (50 mg) as colorless crystals.
¹H-NMR (DMSO-d₆) δ: 0.87 (9H, s), 2.09-2.21 (2H, m), 2.56-2.72 (2H, m), 3.12-3.25 (2H, m), 3.33-3.41 (2H, m), 3.48 (3H, s), 3.60 (1H, d, J = 14.0 Hz), 4.08-4.40 (8H, m), 6.08 (1H, s), 6.38 (1H, d, J = 2.4 Hz), 6.92-7.08 (4H, m), 7.10-7.20 (4H, m), 7.26-7.37 (4H, m), 7.54 (1H, dd, J = 2.4, 8.8 Hz), 7.74 (1H, d, J = 8.8 Hz), 8.46 (1H, t, J = 5.9 Hz), 8.78 (2H, brs).

### Examples 17

### trans-2-[7-Chloro-1-(2,2-dimethylpropyl)-5-(2-methoxy-3-{3-[(2-phenylethyl)amino]propoxy}phenyl)-2-oxo-1,2,3,5-tetrahydro-4,1-benzoxazepin-3-yl]-N-(2-fluorobenzyl)acetamide hydrochloride

From the compound obtained in Example 2 (150 mg) and according to the same manner as that of Example 16, the title compound (20 mg) was obtained as colorless crystals.
¹H-NMR (DMSO-d₆) δ: 0.88 (9H, s), 2.07-2.19 (2H, m), 2.62-2.66 (2H, m), 2.83-2.96 (2H, m), 3.17-3.27 (4H, m), 3.52 (3H, s), 3.60 (1H, d, J = 13.7 Hz), 4.09-4.39 (6H, m), 6.09 (1H, s), 6.38 (1H, d, J = 2.5 Hz), 7.02-7.10 (1H, m), 7.11-7.38 (11H, m), 7.54 (1H, dd, J = 2.5, 8.8 Hz), 7.75 (1H, d, J = 8.8 Hz), 8.46 (1H, t, J = 5.8 Hz), 8.59 (2H, brs).

### Example 18

### trans-2-[7-Chloro-1-(2,2-dimethylpropyl)-5-[2-methoxy-3-(3-{[(2E)-3-phenyl-2-propenyl]amino}propoxy)phenyl]-2-oxo-1,2,3,5-tetrahydro-4,1-benzoxazepin-3-yl]-N-(2-fluorobenzyl)acetamide hydrochloride

From the compound obtained in Example 2 (150 mg) and the according to the same manner as that of Example 16, the title compound (50 mg) was obtained as colorless crystals.
¹H-NMR (DMSO-d₆) δ: 0.85 (9H, s), 2.06-2.20 (2H, m), 2.61-2.65 (2H, m), 3.05-3.20 (2H, m), 3.42 (3H, s), 3.59 (1H, d, J = 14.0 Hz), 3.70-3.83 (2H, m), 4.05-4.40 (6H, m), 6.06 (1H, s), 6.20-6.32 (1H, m), 6.36 (1H, d, J = 2.5 Hz), 6.81 (1H, d, J = 15.9 Hz), 7.00-7.08 (1H, m), 7.10-7.22 (4H, m), 7.27-7.48 (7H, m), 7.54 (1H, dd, J = 2.5, 8.9 Hz), 7.74 (1H, d, J = 8.9 Hz), 8.46 (1H, t, J = 5.7 Hz), 8.80 (2H, brs).

### Example 19

### trans-2-[7-Chloro-1-(2,2-dimethylpropyl)-5-[2-methoxy-3-(3-{[3-(4-methoxyphenyl)propyl]amino}propoxy)phenyl]-2-oxo-1,2,3,5-tetrahydro-4,1-benzoxazepin-3-yl]-N-(2-fluorobenzyl)acetamide hydrochloride

From the compound obtained in Example 2 (150 mg) and according to the same manner as that of Example 16, the title compound (70 mg) was obtained as colorless crystals.
¹H-NMR (DMSO-d₆) δ: 0.87 (9H, s), 1.77-1.90 (2H, m), 2.03-2.14 (2H, m), 2.53-2.68 (4H, m), 2.84-2.93 (2H, m), 3.02-3.10 (2H, m), 3.51 (3H, s), 3.60 (1H, d, J = 14.2 Hz), 3.71 (3H, s), 4.08-4.39 (6H, m), 6.09 (1H, s), 6.38 (1H, d, J = 2.5 Hz), 6.83-6.88 (2H, m), 7.04 (1H, dd, J = 2.7, 6.7 Hz), 7.09-7.21 (6H, m), 7.27-7.36 (2H, m), 7.54 (1H, dd, J = 2.5, 8.8 Hz), 7.74 (1H, d, J = 8.8 Hz), 8.46 (1H, t, J = 5.9 Hz), 8.50 (2H, brs).

### Example 20

### trans-2-[7-Chloro-1-(2,2-dimethylpropyl)-5-[2-methoxy-3-(3-{[3-(3-methoxyphenyl)propyl]amino}propoxy)phenyl]-2-oxo-1,2,3,5-tetrahydro-4,1-benzoxazepin-3-yl]-N-(2-fluorobenzyl)acetamide hydrochloride.

From the compound obtained in Example 2 (150 mg) and according to the same manner as that of Example 16, the title compound (20 mg) was obtained as colorless crystals.
¹H-NMR (DMSO-d₆) δ: 0.87 (9H, s), 1.80-1.95 (2H, m), 2.02-2.14 (2H, m), 2.55-2.68 (4H, m), 2.85-2.95 (2H, m), 3.01-3.11 (2H, m), 3.51 (3H, s), 3.60 (1H, d, J = 14.0 Hz), 3.73 (3H, s), 4.06-4.39 (6H, m), 6.09 (1H, s), 6.38 (1H, d, J = 2.4 Hz), 6.74-6.80 (3H, m), 7.04 (1H, dd, J = 2.4, 6.6 Hz), 7.10-7.36 (7H, m), 7.54 (1H, dd, J = 2.4, 8.8 Hz), 7.74 (1H, d, J = 8.8 Hz), 8.41 (2H, brs), 8.46 (1H, t, J = 5.9 Hz).

### Example 21

### trans-2-[7-Chloro-1-(2,2-dimethylpropyl)-5-[2-methoxy-3-(3-{[3-(2-methoxyphenyl)propyl]amino}propoxy)phenyl]-2-oxo-1,2,3,5-tetrahydro-4,1-benzoxazepin-3-yl]-N-(2-fluorobenzyl)acetamide hydrochloride

From the compound obtained in Example 2 (150 mg) and according to the same manner as that of Example 16, the title compound (75 mg) was obtained as colorless crystals.
¹H-NMR (DMSO-d₆) δ: 0.88 (9H, s), 1.76-1.90 (2H, m), 2.02-2.14 (2H, m), 2.55-2.68 (4H, m), 2.86-2.96 (2H, m), 3.02-3.12 (2H, m), 3.51 (3H, s), 3.60 (1H, d, J = 14.0 Hz), 3.77 (3H, s), 4.06-4.39 (6H, m), 6.09 (1H, s), 6.38 (1H, d, J = 2.5 Hz), 6.87 (1H, t, J = 7.3 Hz), 6.96 (1H, d, J = 7.8 Hz), 7.04 (1H, dd, J = 2.5, 6.7 Hz), 6.99-7.24 (6H, m), 7.26-7.36 (2H, m), 7.54 (1H, dd, J = 2.4, 8.7 Hz), 7.74 (1H, d, J = 8.9 Hz), 8.42 (2H, brs), 8.46 (1H, t, J = 5.8 Hz).

### Example 22

### trans-2-[7-Chloro-5-[3-(3-{[3-(3,4-dimethoxyphenyl)propyl]amino}propoxy)-2-methoxyphenyl]-1-(2,2-dimethylpropyl)-2-oxo-1,2,3,5-tetrahydro-4,1-benzoxazepin-3-yl]-N-(2-fluorobenzyl)acetamide hydrochloride

From the compound obtained in Example 2 (150 mg) and according to the same manner as that of Example 16, the title compound (60 mg) was obtained as colorless crystals.
¹H-NMR (DMSO-d₆) δ: 0.90 (9H, s), 1.82-1.96 (2H, m), 2.05-2.18 (2H, m), 2.55-2.70 (4H, m), 2.86-2.97 (2H, m), 3.03-3.13 (2H, m), 3.54 (3H, s), 3.62 (1H, d, J = 14.0 Hz), 3.73 (3H, s), 3.75 (3H, s), 4.10-4.41 (6H, m), 6.11 (1H, s), 6.41 (1H, d, J = 2.4 Hz), 6.73 (1H, dd, J = 1.7, 8.1 Hz), 6.82 (1H, d, J = 1.7 Hz), 6.88 (1H, d, J = 8.2 Hz), 7.07 (1H, dd, J = 2.6, 6.5 Hz), 7.12-7.23 (4H, m), 7.29-7.39 (2H, m), 7.56 (1H, dd, J = 2.4, 8.7 Hz), 7.77 (1H, d, J = 8.7 Hz), 8.48 (1H, t, J = 5.8 Hz), 8.52 (2H, brs).

### Example 23

### trans-2-[7-Chloro-1-(2,2-dimethylpropyl)-5-[3-(3-{[2-(1H-indol-3-yl)ethyl]amino}propoxy)-2-methoxyphenyl]-2-oxo-1,2,3,5-tetrahydro-4,1-benzoxazepin-3-yl]-N-(2-fluorobenzyl)acetamide hydrochloride

From the compound obtained in Example 2 (150 mg) and according to the same manner as that of Example 16, the title compound (70 mg) was obtained as colorless crystals.
¹H-NMR (DMSO-d₆) δ: 0.87 (9H, s), 2.06-2.19 (2H, m), 2.58-2.68 (2H, m), 3.00-3.28 (6H, m), 3.52 (3H, s), 3.59 (1H, d, J = 13.9 Hz), 4.07-4.40 (6H, m), 6.09 (1H, s), 6.38 (1H, d, J = 2.5 Hz), 6.96-7.39 (11H, m), 7.51-7.59 (2H, m), 7.74 (1H, d, J = 8.8 Hz), 8.46 (1H, t, J = 5.8 Hz), 8.68 (2H, brs), 10.95 (1H, s).

### Example 24

### trans-[2-(3-{7-Chloro-1-(2,2-dimethylpropyl)-3-[(2-fluorobenzylcarbamoyl)methyl]-2-oxo-1,2,3,5-tetrahydro-4,1-benzoxazepin-5-yl}-2-methoxyphenoxy)ethyl]carbamic acid tert-butyl ester

From the compound obtained in Reference Example 10 (1.00g) and 2-bromoethylcarbamic acid tert-butyl ester (0.48g) and according to the same manner as that of Example 1, the title compound (1.21g) was obtained as colorless crystals.
¹H-NMR (CDCl₃) δ: 0.94 (9H, s), 1.43 (9H, s), 2.69 (1H, dd, J = 6.1, 14.4 Hz), 2.87 (1H, dd, J = 6.8, 14.4 Hz), 3.36 (1H, d, J = 13.8 Hz), 3.48-3.59 (2H, m), 3.62 (3H, s), 4.02-4.10 (2H, m), 4.37-4.58 (4H, m), 4.98 (1H, brs), 6.25 (1H, s), 6.36 (1H, brs), 6.59 (1H, d, J = 2.1 Hz), 6.93-7.17 (5H, m), 7.21-7.37 (4H, m).

### Example 25

### trans-2-[5-[3-(2-Aminoethoxy)-2-methoxyphenyl]-7-chloro-1-(2,2-dimethylpropyl)-2-oxo-1,2,3,5-tetrahydro-4,1-benzoxazepin-3-yl]-N-(2-fluorobenzyl)acetamide hydrochloride

From the compound obtained in Example 24 (1.15g) and according to the same manner as that of Example 2, the title compound (0.97g) was obtained as a white powder.
¹H-NMR (DMSO-d₆) δ: 0.88 (9H, s), 2.62-2.66 (2H, m), 3.20-3.28 (2H, m), 3.51-3.63 (4H, m), 4.18-4.40 (6H, m), 6.09 (1H, s), 6.37 (1H, d, J = 2.5 Hz), 7.06-7.20 (5H, m), 7.28-7.38 (2H, m), 7.54 (1H, dd, J = 2.5, 8.8 Hz), 7.75 (1H, d, J = 8.9 Hz), 8.02 (3H, brs), 8.48 (1H, t, J = 5.6 Hz).

### Example 26

### trans-2-[7-Chloro-1-(2,2-dimethylpropyl)-5-(2-methoxy-3-{2-[(2-phenylethyl)amino]ethoxy}phenyl)-2-oxo-1,2,3,5-tetrahydro-4,1-benzoxazepin-3-yl]-N-(2-fluorobenzyl)acetamide hydrochloride

From the compound obtained in Example 25 (100 mg) and according to the same manner as that of Example 16, the title compound (35 mg) was obtained as colorless crystals.
¹H-NMR (DMSO-d₆) δ: 0.88 (9H, s), 2.62-2.66 (2H, m), 2.90-3.00 (2H, m), 3.40-3.53 (4H, m), 3.53 (3H, s), 3.60 (1H, d, J = 14.3 Hz), 4.17-4.40 (6H, m), 6.09 (1H, s), 6.38 (1H, s), 7.07-7.38 (12H, m), 7.54 (1H, d, J = 8.6 Hz), 7.75 (1H, d, J = 8.6 Hz), 8.47 (1H, t, J = 5.4 Hz) 8.81 (2H, brs).

### Example 27

### trans-2-[7-Chloro-1-(2,2-dimethylpropyl)-5-(2-methoxy-3-{2-[(3-phenylpropyl)amino]ethoxy}phenyl)-2-oxo-1,2,3,5-tetrahydro-4,1-benzoxazepin-3-yl]-N-(2-fluorobenzyl)acetamide hydrochloride

From the compound obtained in Example 25 (150 mg) and 3-phenylpropanal (34mg) and according to the same manner as that of Example 3, the title compound (60 mg) was obtained as colorless crystals.
¹H-NMR (DMSO-d₆) δ: 0.87 (9H, s), 1.85-2.00 (2H, m), 2.57-2.68 (4H, m), 2.92-3.06 (2H, m), 3.38-3.62 (6H, m), 4.16-4.40 (6H, m), 6.09 (1H, s), 6.37 (1H, d, J = 2.5 Hz), 7.05-7.38 (12H, m), 7.54 (1H, dd, J = 2.5, 8.8 Hz), 7.75 (1H, d, J = 8.8 Hz), 8.47 (1H, t, J = 5.8 Hz) 8.75 (2H, brs).

### Example 28

### trans-2-[7-Chloro-1-(2,2-dimethylpropyl)-5-(2-methoxy-3-{2-[(4-phenylbutyl)amino]ethoxy}phenyl)-2-oxo-1,2,3,5-tetrahydro-4,1-benzoxazepin-3-yl]-N-(2-fluorobenzyl)acetamide hydrochloride

From the compound obtained in Example 25 (300 mg) and 4-phenylbutanal (70 mg) and according to the same manner as that of Example 3, the title compound (135 mg) was obtained as colorless crystals.
¹H-NMR (DMSO-d₆) δ: 0.87 (9H, s), 1.53-1.67 (4H, m), 2.55-2.68 (4H, m), 2.95-3.10 (2H, m), 3.32-3.42 (2H, m), 3.54 (3H, s), 3.60 (1H, d, J = 14.0 Hz), 4.18-4.39 (6H, m), 6.09 (1H, s), 6.37 (1H, d, J = 2.5 Hz), 7.05-7.21 (8H, m), 7.23-7.37 (4H, m), 7.54 (1H, dd, J = 2.5, 8.8 Hz), 7.74 (1H, d, J = 8.8 Hz), 8.47 (1H, t, J = 5.8 Hz), 8.72 (2H, brs).

### Example 29

### trans-2-[7-Chloro-1-(2,2-dimethylpropyl)-5-(2-methoxy-3-{2-[methyl(3-phenylpropyl)amino]ethoxy}phenyl)-2-oxo-1,2,3,5-tetrahydro-4,1-benzoxazepin-3-yl]-N-(2-fluorobenzyl)acetamide hydrochloride

From the compound obtained in Example 27 (54mg) and according to the same manner as that of Example 15, the title compound (35 mg) was obtained as colorless crystals.
¹H-NMR (DMSO-d₆) δ: 0.87 (9H, s), 1.96-2.06 (2H, m), 2.55-2.69 (4H, m), 2.87 (3H, d, J = 4.4 Hz), 3.03-3.28 (2H, m), 3.42-3.70 (6H, m), 4.18-4.50 (6H, m), 6.10 (1H, s), 6.39 (1H, s), 7.07-7.38 (12H, m), 7.54 (1H, dd, J = 2.0, 8.8 Hz), 7.75 (1H, d, J = 8.8 Hz), 8.50 (1H, t, J = 5.7 Hz) 9.80 (1H, brs).

### Example 30

### trans-(3-{3-[7-Chloro-1-(2,2-dimethylpropyl)-3-{2-[(2-fluorobenzyl)amino]-2-oxoethyl}-2-oxo-1,2,3,5-tetrahydro-4,1-benzoxazepin-5-yl]-2-methoxyphenoxy}propyl)(3-phenylpropyl)carbamic acid tert-butyl ester

To a tetrahydrofuran (20 ml) solution of the compound obtained in Example 3 (1.30g) were added triethylamine (0.54g) and di(tert-butyl) dicarbonate (0.78g), and the mixture was stirred at room temperature for 1 hour. This solution was concentrated under reduced pressure, and the residue was purified by silica gel chromatography to obtain the title compound (1.30g) as a colorless oil.
¹H-NMR (CDCl₃) δ: 0.94 (9H, s), 1.43 (9H, s), 1.78-1.90 (2H, m), 1.97-2.10 (2H, m), 2.50-2.60 (2H, m), 2.68 (1H, dd, J = 6.1, 14.3 Hz), 2.86 (1H, dd, J = 6.8, 14.3 Hz), 3.13-3.43 (5H, m), 3.59 (3H, s), 3.96-4.07 (2H, m), 4.36-4.57 (4H, m), 6.24 (1H, s), 6.27 (1H, t, J = 5.8 Hz), 6.59 (1H, d, J = 2.1 Hz), 6.99-7.36 (14H, m).

### Example 31

### trans-N-(2-Fluorobenzyl)-2-[1-(2,2-dimethylpropyl)-5-(2-methoxy-3-{3-[(3-phenylpropyl)amino]propoxy}phenyl)-2-oxo-1,2,3,5-tetrahydro-4,1-benzoxazepin-3-yl]acetamide hydrochloride

To a methanol (10 ml) solution of the compound obtained in Example 30 (200 mg) were added 10% palladium carbon (100 mg) and ammonium formate (200 mg), and the mixture was stirred for 15 hours under heating and refluxing. The reaction mixture was filtered with Celite, and the filtrate was concentrated under reduced pressure. The residue was partitioned between ethyl acetate and water, and the organic layer was washed with water and an aqueous saturated sodium chloride solution, dried over anhydrous magnesium sulfate, and concentrated under reduced pressure. The residue was purified by silica gel chromatography to obtain a colorless oil (150 mg). To this oil (150 mg) was added a 4N hydrochloric acid/ethyl acetate solution (5 ml), and the mixture was stirred at room temperature for 15 hours. The reaction mixture was concentrated under reduced pressure, and the resulting residue was crystallized from methylene chloride-diethyl ether to obtain the title compound (120 mg) as colorless crystals.
¹H-NMR (DMSO-d₆) δ: 0.88 (9H, s), 1.88-1.96 (2H, m), 2.03-2.14 (2H, m), 2.60-2.70 (4H, m), 2.88-2.98 (2H, m), 3.02-3.13 (2H, m), 3.47 (3H, s), 3.62 (1H, d, J = 13.8 Hz), 4.10-4.14 (2H, m), 4.19-4.38 (4H, m), 6.14 (1H, s), 6.48 (1H, dd, J = 1.5, 7.7 Hz), 7.03-7.22 (9H, m), 7.26-7.38 (4H, m), 7.42-7.50 (1H, m), 7.66 (1H, d, J = 8.0 Hz), 8.45 (1H, t, J = 5.9 Hz), 8.55 (2H, brs).

### Example 32

### trans-N-(2-Fluorobenzyl)-2-[1-(2,2-dimethylpropyl)-5-(2-methoxy-3-{3-[(3-phenylpropyl)amino]propoxy}phenyl)-2-oxo-7-phenyl-1,2,3,5-tetrahydro-4,1-benzoxazepin-3-yl]acetamide hydrochloride

To a xylene (10 ml) suspension of the compound obtained in Example 30 (200 mg), phenylboric acid (88mg), tris(dibenzylideneacetone)dipalladium (11 mg) and cesium carbonate (235 mg) was added tri-tert-butylphosphine (10 mg), and the mixed solution was heated to reflux for 15 hours under the nitrogen atmosphere. The reaction mixture was filtered with Celite, and the filtrate was concentrated under reduced pressure. The residue was partitioned between ethyl acetate and an aqueous saturate sodium bicarbonate solution, and the organic layer was washed with water and an aqueous saturated sodium chloride solution, dried over anhydrous magnesium sulfate, and concentrated under reduced pressure. The residue was purified by silica gel chromatography to obtain a colorless oil (140 mg). To this oil (140 mg) was added a 4N hydrochloric acid/ethyl acetate solution (5 ml), and the mixture was stirred at room temperature for 15 hours. The reaction mixture was concentrated under reduced pressure, and the resulting residue was purified by reverse phase liquid chromatography to obtain a colorless oil (80 mg). To a methylene chloride (3 ml) solution of this oil (80 mg) was added 1N etheric hydrochloric acid (0.5 ml), and the mixture was stirred at room temperature for 5 minutes. The reaction mixture was concentrated under reduced pressure, and the resulting residue was crystallized from methylene chloride-diethyl ether to obtain the title compound (61 mg) as colorless crystals.
¹H-NMR (DMSO-d₆) δ: 0.91 (9H, s), 1.80-1.95 (2H, m), 2.00-2.14 (2H, m), 2.58-2.73 (4H, m), 2.85-2.95 (2H, m), 3.00-3.10 (2H, m), 3.48 (3H, s), 3.67 (1H, d, J = 13.9 Hz), 4.08-4.12 (2H, m), 4.18-4.40 (4H, m), 6.20 (1H, s), 6.71 (1H, s), 7.10-7.45 (17H, m), 7.72-7.80 (2H, m), 8.46 (1H, t, J = 5.7 Hz), 8.52 (2H, brs).

### Reference Example 12Y

### trans-[7-Chloro-1-(2,2-dimethylpropyl)-5-(3-hydroxy-2-methoxyphenyl)-2-oxo-1,2,3,5-tetrahydro-4,1-benzoxazepin-3-yl]acetic acid ethyl ester

From the compound obtained in Reference Example 7 (10.0g) and according to the same manner as that of Reference Example 10, the title compound (8.46g) was obtained as a colorless oil.
¹H-NMR (CDCl₃) δ: 0.95 (9H, s), 1.26 (3H, t, J = 7.1 Hz), 2.77 (1H, dd, J = 6.0, 16.5 Hz), 3.03 (1H, dd, J = 7.6, 16.5 Hz), 3.38 (1H, d, J = 13.9 Hz), 3.60 (3H, s), 4.12 (2H, q, J = 7.1 Hz), 4.41 (1H, dd, J = 6.0, 7.6 Hz), 4.52 (1H, d, J = 13.9 Hz), 5.36 (1H, s), 6.24 (1H, s), 6.63 (1H, d, J = 1.8 Hz), 6.95-7.03 (1H, m), 7.15-7.20 (2H, m), 7.30-7.38 (2H, m).

### Example 33

### trans-(3-{3-[7-Chloro-1-(2,2-dimethylpropyl)-3-(ethoxycarbonylmethyl)-2-oxo-1,2,3,5-tetrahydro-4,1-benzoxazepin-5-yl]-2-methoxyphenoxy}propyl)carbamic acid tert-butyl ester

From the compound obtained in Reference Example 12 (8.46g) and the compound obtained in Reference Example 11 (6.35g) and according to the same manner as that of Example 1, the title compound (9.03g) was obtained as pale yellow crystals.
¹H-NMR (CDCl₃) δ: 0.94 (9H, s), 1.24 (3H, t, J = 7.1 Hz), 1.42 (9H, s), 1.97-2.06 (2H, m), 2.79 (1H, dd, J = 6.0, 16.4 Hz), 3.03 (1H, dd, J = 7.6, 16.4 Hz), 3.30-3.36 (2H, m), 3.37 (1H, d, J = 13.9 Hz), 3.63 (3H, s), 4.04-4.17 (4H, m), 4.39 (1H, dd, J = 6.0, 7.6 Hz), 4.51 (1H, d, J = 13.9 Hz), 4.84 (1H, brs), 6.27 (1H, s), 6.61 (1H, d, J = 1.4 Hz), 6.96 (1H, dd, J = 2.1, 7.7 Hz), 7.12-7.36 (4H, m).

### Example 34

### Trans-{5-[3-(3-aminopropoxy)-2-methoxyphenyl]-7-chloro-1-(2,2-dimethylpropyl)-2-oxo-1,2,3,5-tetrahydro-4,1-benzoxazepin-3-yl}acetic acid ethyl ester hydrochloride

From the compound obtained in Example 33 (9.03g) and according to the same manner as that of Example 2, the title compound (8.07g) was obtained as a white powder.
¹H-NMR (DMSO-d₆) δ: 0.88 (9H, s), 1.15 (3H, t, J = 7.1 Hz), 1.98-2.10 (2H, m), 2.65-2.86 (2H, m), 2.92-3.03 (2H, m), 3.53 (3H, s), 3.62 (1H, d, J = 13.9 Hz), 3.97-4.17 (4H, m), 4.21-4.32 (2H, m), 6.10 (1H, s), 6.41 (1H, d, J = 2.5 Hz), 7.08-7.15 (2H, m), 7.19-7.27 (1H, m), 7.56 (1H, dd, J = 2.5, 8.7 Hz), 7.70-7.85 (4H, m).

### Example 35

### trans-[7-Chloro-1-(2,2-dimethylpropyl)-5-(2-methoxy-3-{3-[(3-phenylpropyl)amino]propoxy}phenyl)-2-oxo-1,2,3,5-tetrahydro-4,1-benzoxazepin-3-yl]acetic acid ethyl ester hydrochloride

To a dimethylformamide/methanol (50 ml/20 ml) solution of the compound obtained in Example 34 (6.00g), 3-phenylpropanal (1.41g) and acetic acid (1.89g) was added sodium cyanotrihydroborate (0.99g), and the mixture was stirred at room temperature for 15 hours. The resulting reaction mixture was diluted with an aqueous saturated sodium bicarbonate solution, and extracted with ethyl acetate. The extract was washed successively with water and an aqueous saturated sodium chloride solution, dried over anhydrous magnesium sulfate, and concentrated under reduced pressure. The residue was purified by silica gel chromatography to obtain a pale yellow oil (3.55g). To a methylene chloride (3 ml) solution of this oil (80 mg) was added 1N etheric hydrochloric acid (0.5 ml), and the mixture was stirred at room temperature for 5 hours. The reaction mixture was concentrated under reduced pressure, and the resulting residue was crystallized from methylene chloride-diethyl ether to obtain the title compound (50 mg) as colorless crystals.
¹H-NMR (DMSO-d₆) δ: 0.87 (9H, s), 1.15 (3H, t, J = 7.1 Hz), 1.80-1.94 (2H, m), 2.03-2.16 (2H, m), 2.60-2.89 (4H, m), 2.90-3.00 (2H, m), 3.05-3.15 (2H, m), 3.52 (3H, s), 3.62 (1H, d, J = 13.9 Hz), 4.00-4.19 (4H, m), 4.24 (1H, t, J = 7.0 Hz), 4.29 (1H, d, J = 13.9 Hz), 6.10 (1H, s), 6.41 (1H, d, J = 2.5 Hz), 7.10-7.35 (8H, m), 7.56 (1H, dd, J = 2.5, 8.7 Hz), 7.77 (1H, d, J = 8.7 Hz), 8.55 (2H, brs).

### Example 36

### trans-[5-(3-{3-[Bis(3-phenylpropyl)amino]propoxy}-2-methoxyphenyl)-7-chloro-1-(2,2-dimethylpropyl)-2-oxo-1,2,3,5-tetrahydro-4,1-benzoxazepin-3-yl]acetic acid ethyl ester hydrochloride

As a by-product of Example 35, the title compound (133 mg) as colorless crystals was obtained.
¹H-NMR (DMSO-d₆) δ: 0.88 (9H, s), 1.16 (3H, t, J = 7.1 Hz), 1.83-2.02 (4H, m), 2.05-2.21 (2H, m), 2.55-2.90 (6H, m), 3.03-3.30 (6H, m), 3.47 (3H, s), 3.63 (1H, d, J = 14.4 Hz), 4.00-4.35 (6H, m), 6.10 (1H, s), 6.41 (1H, d, J = 2.3 Hz), 7.10-7.32 (13H, m), 7.58 (1H, dd, J = 2.5, 8.7 Hz), 7.78 (1H, d, J = 8.8 Hz), 9.80 (1H, brs).

### Example 37

### trans-[5-(3-{3-[(tert-Butoxycarbonyl)(3-phenylpropyl)amino]propoxy}-2-methoxyphenyl)-7-chloro-1-(2,2-dimethylpropyl)-2-oxo-1,2,3,5-tetrahydro-4,1-benzoxazepin-3-yl]acetic acid ethyl ester

From the free form of the compound obtained in Example 35 and according to the same manner as that of Example 30, the title compound (3.54 g) was obtained as a colorless oil.
¹H-NMR (CDCl₃) δ: 0.95 (9H, s), 1.24 (3H, t, J = 7.2 Hz), 1.43 (9H, s), 1.78-1.90 (2H, m), 2.00-2.11 (2H, m), 2.55-2.60 (2H, m), 2.77 (1H, dd, J = 5.9, 16.4 Hz), 3.04 (1H, dd, J = 7.7, 16.4 Hz), 3.15-3.30 (2H, m), 3.32-3.45 (3H, m), 3.61 (3H, s), 4.00-4.05 (2H, m), 4.10-4.20 (2H, m), 4.39 (1H, dd, J = 6.0, 7.6 Hz), 4.52 (1H, d, J = 14.2 Hz), 6.26 (1H, s), 6.61 (1H, s), 6.94 (1H, dd, J = 1.7, 8.7 Hz), 7.10-7.28 (9H, m).

### Example 38

### trans-[5-(3-{3-[(tert-Butoxycarbonyl)(3-phenylpropyl)amino]propoxy}-2-methoxyphenyl)-7-chloro-1-(2,2-dimethylpropyl)-2-oxo-1,2,3,5-tetrahydro-4,1-benzoxazepin-3-yl]acetic acid

To an ethanol/tetrahydrofuran (20 ml/20 ml) solution of the compound obtained in Example 37 (1.03 g) was added a 1N aqueous sodium hydroxide solution (6 ml), and the mixture was heated at 60°C for 1 hour. The reaction mixture was cooled, poured into a 10% aqueous citric acid solution, and this was extracted with ethyl acetate. The extract was washed with an aqueous saturated sodium chloride solution, dried over anhydrous magnesium sulfate, and concentrated under reduced pressure to obtain the title compound (0.99g) as a pale yellow oil.
¹H-NMR (CDCl₃) δ: 0.95 (9H, s), 1.43 (9H, s), 1.78-1.90 (2H, m), 2.00-2.12 (2H, m), 2.55-2.60 (2H, m), 2.82 (1H, dd, J = 5.6, 16.5 Hz), 3.08 (1H, dd, J = 7.6, 16.5 Hz), 3.18-3.30 (2H, m), 3.32-3.43 (3H, m), 3.61 (3H, s), 4.00-4.05 (2H, m), 4.34 (1H, dd, J = 5.6, 7.6 Hz), 4.51 (1H, d, J = 13.9 Hz), 6.25 (1H, s), 6.62 (1H, d, J = 1.6 Hz), 6.94 (1H, dd, J = 1.7, 7.8 Hz), 7.12-7.39 (9H, m).

### Example 39

### trans-[7-Chloro-1-(2,2-dimethylpropyl)-5-(2-methoxy-3-{3-[(3-phenylpropyl)amino]propoxy}phenyl)-2-oxo-1,2,3,5-tetrahydro-4,1-benzoxazepin-3-yl]acetic acid hydrochloride

To the compound obtained in Example 38 (85 mg) was added a 4N hydrochloric acid/ethyl acetate solution (2 ml), and the mixture was stirred at room temperature for 4 hours. The reaction mixture was concentrated under reduced pressure, and the resulting residue was crystallized from methylene chloride-diethyl ether to obtain the title compound (60 mg) as colorless crystals.
¹H-NMR (DMSO-d₆) δ: 0.87 (9H, s), 1.82-1.96 (2H, m), 2.05-2.17 (2H, m), 2.58-2.69 (3H, m), 2.71-2.83 (1H, m), 2.86-2.98 (2H, m), 3.03-3.15 (2H, m), 3.52 (3H, s), 3.62 (1H, d, J = 13.9 Hz), 4.07-4.23 (3H, m), 4.29 (1H, d, J = 13.9 Hz), 6.10 (1H, s), 6.41 (1H, d, J = 2.4 Hz), 7.12-7.34 (8H, m), 7.55 (1H, dd, J = 2.4, 8.8 Hz), 7.77 (1H, d, J = 8.8 Hz), 8.60 (2H, brs), 12.20 (1H, brs).

### Example 40

### trans-2-[7-Chloro-1-(2,2-dimethylpropyl)-5-(2-methoxy-3-{3-[(3-phenylpropyl)amino]propoxy}phenyl)-2-oxo-1,2,3,5-tetrahydro-4,1-benzoxazepin-3-yl]acetamide hydrochloride

To a tetrahydrofuran (5 ml) solution of the compound obtained in Example 38 (100 mg) and triethylamine (17 mg) was added isobutyl chloroformate (23 mg) under ice-cooling, the mixture was stirred at room temperature for 30 minutes, a 28% aqueous ammonia solution (160 mg) was added, and this was further stirred at room temperature for 15 hours. The reaction mixture was concentrated under reduced pressured, and the residue was partitioned between ethyl acetate and water. The organic layer was washed successively with water and an aqueous saturated sodium chloride solution, dried over anhydrous magnesium sulfate, and concentrated under reduced pressure. The residue was purified by silica gel chromatography to obtain a colorless oil (40 mg). To this oil (25 mg) was added a 4N hydrochloric acid/ethyl acetate solution (5 ml), and the mixture was stirred at room temperature for 4 hours. The reaction mixture was concentrated under reduced pressure, and the resulting residue was crystallized from methylene chloride-diethyl ether to obtain the title compound (20 mg) as colorless crystals.
¹H-NMR (DMSO-d₆) δ: 0.87 (9H, s), 1.80-1.94 (2H, m), 2.02-2.15 (2H, m), 2.53-2.70 (4H, m), 2.86-2.96 (2H, m), 3.02-3.11 (2H, m), 3.52 (3H, s), 3.59 (1H, d, J = 13.9 Hz), 4.06-4.19 (2H, m), 4.21-4.35 (2H, m), 6.08 (1H, s), 6.39 (1H, d, J = 2.3 Hz), 6.82 (1H, s), 7.11-7.42 (9H, m), 7.54 (1H, dd, J = 2.3, 8.7 Hz), 7.74 (1H, d, J = 8.7 Hz), 8.43 (2H, brs).

### Example 41

### trans-N-Benzyl-2-[7-chloro-1-(2,2-dimethylpropyl)-5-(2-methoxy-3-{3-[(3-phenylpropyl)amino]propoxy}phenyl)-2-oxo-1,2,3,5-tetrahydro-4,1-benzoxazepin-3-yl]acetamide hydrochloride

To a dimethylformamide (3 ml) solution of the compound obtained in Example 38 (100 mg), 1-ethyl-3-(3-dimethylaminopropyl)carbodiimide hydrochloride (32 mg), and 1-hydroxybenzotriazole (22 mg) was added benzylamine (18mg), and the mixture was stirred at room temperature for 48 hours. Water was poured into the reaction mixture, followed by extraction with ethyl acetate. The extract was washed successively with water and an aqueous saturated sodium chloride solution, dried over anhydrous magnesium sulfate, and concentrated under reduced pressure. The residue was purified by silica gel chromatography to obtain a colorless oil (100 mg). To this oil (100 mg) was added a 4N hydrochloric acid/ethyl acetate solution (5 ml), and the mixture was stirred at room temperature for 15 hours. The reaction mixture was concentrated under reduced pressure, and the resulting residue was crystallized from methylene chloride-diethyl ether to obtain the title compound (80 mg) as colorless crystals.
¹H-NMR (DMSO-d₆) δ: 0.88 (9H, s), 1.82-1.96 (2H, m), 2.06-2.18 (2H, m), 2.57-2.70 (4H, m), 2.86-2.98 (2H, m), 3.02-3.15 (2H, m), 3.52 (3H, s), 3.60 (1H, d, J = 13.9 Hz), 4.08-4.22 (3H, m), 4.25-4.38 (3H, m), 6.10 (1H, s), 6.39 (1H, d, J = 2.5 Hz), 7.02-7.35 (13H, m), 7.54 (1H, dd, J = 2.5, 8.8 Hz), 7.75 (1H, d, J = 8.8 Hz), 8.45 (1H, t, J = 6.0 Hz) 8.60 (2H, brs).

### Example 42

### trans-2-[7-Chloro-1-(2,2-dimethylpropyl)-5-(2-methoxy-3-{3-[(3-phenylpropyl)amino]propoxy}phenyl)-2-oxo-1,2,3,5-tetrahydro-4,1-benzoxazepin-3-yl]-N-(2-phenylethyl)acetamide hydrochloride

From the compound obtained in Example 38 (96mg) and 2-phenylethylamine (19 mg) and the according to the same manner as that of Example 41, the title compound (80 mg) was obtained as colorless crystals.
¹H-NMR (DMSO-d₆) δ: 0.87 (9H, s), 1.82-1.97 (2H, m), 2.05-2.15 (2H, m), 2.66-2.77 (6H, m), 2.88-2.98 (2H, m), 3.02-3.30 (4H, m), 3.52 (3H, s), 3.59 (1H, d, J = 13.8 Hz), 4.06-4.18 (2H, m), 4.22-4.35 (2H, m), 6.09 (1H, s), 6.40 (1H, d, J = 2.4 Hz), 7.10-7.33 (13H, m), 7.55 (1H, dd, J = 2.4, 8.8 Hz), 7.75 (1H, d, J = 8.8 Hz), 8.05 (1H, t, J = 5.6 Hz) 8.56 (2H, brs).

### Example 43

### trans-N-Benzyl-2-[7-chloro-1-(2,2-dimethylpropyl)-5-(2-methoxy-3-{3-[(3-phenylpropyl)amino]propoxy}phenyl)-2-oxo-1,2,3,5-tetrahydro-4,1-benzoxazepin-3-yl]-N-methylacetamide hydrochloride

From the compound obtained in Example 38 (60 mg) and N-methylbenzylamine (12 mg) and according to the same manner as that of Example 41, the title compound (35 mg) was obtained as colorless crystals.
¹H-NMR (DMSO-d₆) δ: 0.88 (9H, s), 1.83-1.97 (2H, m), 2.03-2.18 (2H, m), 2.60-3.17 (11H, m), 3.51 (1.2H, s), 3.52 (1.8 H, s), 3.63 (1H, d, J = 13.6 Hz), 4.07-4.20 (2H, m), 4.26-4.62 (4H, m), 6.07 (0.4H, s), 6.11 (0.6H, s), 6.42 (1H, d, J = 2.1 Hz), 7.09-7.40 (13H, m), 7.52-7.60 (1H, m), 7.77 (1H, d, J = 8.7 Hz), 8.57 (2H, brs).

### Example 44

### trans-2-[7-Chloro-1-(2,2-dimethylpropyl)-5-(2-methoxy-3-(3-[(3-phenylpropyl)amino]propoxy}phenyl)-2-oxo-1,2,3,5-tetrahydro-4,1-benzoxazepin-3-yl]-N-phenylacetamide hydrochloride

From the compound obtained in Example 38 (77 mg) and aniline (12 mg) and according to the same manner as that of Example 41, the title compound (60 mg) was obtained as colorless crystals.
¹H-NMR (DMSO-d₆) δ: 0.88 (9H, s), 1.80-1.96 (2H, m), 2.02-2.17 (2H, m), 2.62-2.66 (2H, m), 2.77-2.98 (4H, m), 3.02-3.15 (2H, m), 3.52 (3H, s), 3.61 (1H, d, J = 13.9 Hz), 4.05-4.18 (2H, m), 4.26-4.38 (2H, m), 6.13 (1H, s), 6.41 (1H, d, J = 2.5 Hz), 7.00-7.35 (11H, m), 7.48-7.60 (3H, m), 7.77 (1H, d, J = 8.9 Hz), 8.53 (2H, brs), 10.03 (1H, s).

### Example 45

### trans-2-[7-Chloro-1-(2,2-dimethylpropyl)-5-(2-methoxy-3-{3-[(3-phenylpropyl)amino]propoxy}phenyl)-2-oxo-1,2,3,5-tetrahydro-4,1-benzoxazepin-3-yl]-N-2-pyridinylmethyl)acetamide dihydrochloride

From the compound obtained in Example 38 (92 mg) and 2-aminomethylpyridine (16mg) and according to the same manner as that of Example 41, the title compound (76 mg) was obtained as colorless crystals.
¹H-NMR (DMSO-d₆) δ: 0.87 (9H, s), 1.82-1.97 (2H, m), 2.03-2.18 (2H, m), 2.60-2.78 (4H, m), 2.85-2.98 (2H, m), 3.02-3.13 (2H, m), 3.48-3.78 (4H, m), 4.08-4.18 (2H, m), 4.25-4.48 (4H, m), 6.10 (1H, s), 6.38 (1H, d, J = 2.5 Hz), 7.06 (1H, dd, J = 2.1, 7.0 Hz), 7.10-7.23 (5H, m), 7.26-7.33 (2H, m), 7.35-7.45 (2H, m), 7.53 (1H, dd, J = 2.5, 8.8 Hz), 7.74 (1H, d, J = 8.8 Hz), 7.85-7.96 (1H, m), 8.52-8.67 (4H, brs).

### Example 46

### trans-2-[7-Chloro-1-(2,2-dimethylpropyl)-5-(2-methoxy-3-{3-[(3-phenylpropyl)amino]propoxy}phenyl)-2-oxo-1,2,3,5-tetrahydro-4,1-benzoxazepin-3-yl]-N-propylacetamide hydrochloride

From the compound obtained in Example 38 (100 mg) and propylamine (10 mg) and according to the same manner as that of Example 41, the title compound (20 mg) was obtained as colorless crystals.
¹H-NMR (DMSO-d₆) δ: 0.83 (3H, t, J = 7.4 Hz), 0.87 (9H, s), 1.32-1.45 (2H, m), 1.80-1.93 (2H, m), 2.01-2.14 (2H, m), 2.50-2.57 (2H, m), 2.62-2.66 (2H, m), 2.84-3.10 (6H, m), 3.51 (3H, s), 3.59 (1H, d, J = 13.8 Hz), 4.07-4.18 (2H, m), 4.23-4.33 (2H, m), 6.09 (1H, s), 6.38 (1H, d, J = 2.5 Hz), 7.08-7.24 (6H, m), 7.26-7.34 (2H, m), 7.54 (1H, dd, J = 2.5, 8.8 Hz), 7.74 (1H, d, J = 8.8 Hz), 7.90 (1H, t, J = 5.5 Hz), 8.37 (2H, brs).

### Example 47

### trans-2-[7-Chloro-1-(2,2-dimethylpropyl)-5-(2-methoxy-3-{3-[(3-phenylpropyl)amino]propoxy}phenyl)-2-oxo-1,2,3,5-tetrahydro-4,1-benzoxazepin-3-yl]-N,N-diethylacetamide hydrochloride

From the compound obtained in Example 38 (80 mg) and diethylamine (10 mg) and according to the same manner as that of Example 41, the title compound (60 mg) was obtained as colorless crystals.
¹H-NMR (DMSO-d₆) δ: 0.87 (9H, s), 0.96 (3H, t, J = 7.0 Hz), 1.12 (3H, t, J = 7.1 Hz), 1.82-1.95 (2H, m), 2.04-2.15 (2H, m), 2.60-2.70 (3H, m), 2.87-2.98 (3H, m), 3.02-3.18 (3H, m), 3.20-3.39 (3H, m), 3.40 (3H, s), 3.61 (1H, d, J = 14.0 Hz), 4.08-4.18 (2H, m), 4.23-4.35 (2H, m), 6.09 (1H, s), 6.41 (1H, d, J = 2.5 Hz), 7.12-7.34 (8H, m), 7.54 (1H, dd, J = 2.5, 8.8 Hz), 7.76 (1H, d, J = 8.8 Hz), 8.54 (2H, brs).

### Example 48

### trans-2-[7-Chloro-1-(2,2-dimethylpropyl)-5-(2-methoxy-3-{3-[(3-phenylpropyl)amino]propoxy}phenyl)-2-oxo-1,2,3,5-tetrahydro-4,1-benzoxazepin-3-yl]-N-(2-methoxybenzyl)acetamide hydrochloride

From the compound obtained in Example 38 (90 mg) and 2-methoxybenzylamine (20 mg) and according to the same manner as that of Example 41, the title compound (70 mg) was obtained as colorless crystals.
¹H-NMR (DMSO-d₆) δ: 0.88 (9H, s), 1.80-1.94 (2H, m), 2.01-2.14 (2H, m), 2.55-2.70 (4H, m), 2.85-2.96 (2H, m), 3.00-3.10 (2H, m), 3.52 (3H, s), 3.60 (1H, d, J = 13.9 Hz), 3.78 (3H, s), 4.06-4.36 (6H, m), 6.10 (1H, s), 6.38 (1H, d, J = 2.5 Hz), 6.83-6.90 (1H, m), 6.96 (1H, d, J = 8.0 Hz), 7.04-7.34 (10H, m), 7.53 (1H, dd, J = 2.5, 8.8 Hz), 7.74 (1H, d, J = 8.8 Hz), 8.27 (1H, t, J = 5.8 Hz), 8.35 (2H, brs).

### Example 49

### trans-7-Chloro-1-(2,2-dimethylpropyl)-5-(2-methoxy-3-{3-[(3-phenylpropyl)amino]propoxy}phenyl)-3-[2-oxo-2-(1-piperidinyl)ethyl]-1,5-dihydro-4,1-benzoxazepine-2(3H)-one hydrochloride

From the compound obtained in Example 38 (70 mg) and piperidine (10 mg) and according to the same manner as that of Example 41, the title compound (20 mg) was obtained as colorless crystals.
¹H-NMR (DMSO-d₆) δ: 0.87 (9H, s), 1.30-1.62 (6H, m), 1.80-1.95 (2H, m), 2.00-2.15 (2H, m), 2.58-2.70 (3H, m), 2.85-3.16 (5H, m), 3.33-3.47 (4H, m), 3.51 (3H, s), 3.61 (1H, d, J = 13.5 Hz), 4.05-4.17 (2H, m), 4.22-4.33 (2H, m), 6.09 (1H, s), 6.41 (1H, d, J = 2.3 Hz), 7.10-7.33 (8H, m), 7.54 (1H, dd, J = 2.3, 8.7 Hz), 7.76 (1H, d, J = 8.7 Hz), 8.46 (2H, brs).

### Example 50

### trans-7-Chloro-1-(2,2-dimethylpropyl)-5-(2-methoxy-3-{3-[(3-phenylpropyl)amino]propoxy}phenyl)-3-[2-oxo-2-(1-piperazinyl)ethyl]-1,5-dihydro-4,1-benzoxazepine-2(3H)-one dihydrochloride

From the compound obtained in Example 38 (100 mg) and piperazine-1-carboxylic acid-tert-butyl ester (31 mg) and according to the same manner as that of Example 41, the title compound (90 mg) was obtained as colorless crystals.
¹H-NMR (DMSO-d₆) δ: 0.87 (9H, s), 1.83-1.95 (2H, m), 2.05-2.16 (2H, m), 2.60-2.78 (3H, m), 2.88-3.12 (9H, m), 3.45-3.74 (8H, m), 4.09-4.19 (2H, m), 4.22-4.32 (2H, m), 6.09 (1H, s), 6.42 (1H, d, J = 2.5 Hz), 7.12-7.33 (8H, m), 7.55 (1H, dd, J = 2.5, 8.8 Hz), 7.77 (1H, d, J = 8.8 Hz), 8.70 (4H, brs).

### Example 51

### trans-7-Chloro-1-(2,2-dimethylpropyl)-5-(2-methoxy-3-{3-[(3-phenylpropyl)amino]propoxy}phenyl)-3-[2-(4-methyl-1-piperazinyl)-2-oxoethyl]-1,5-dihydro-4,1-benzoxazepine-2(3H)-one dihydrochloride

From the compound obtained in Example 38 (80 mg) and N-methylpiperazine (31 mg) and according to the same manner as that of Example 41, the title compound (70 mg) was obtained as colorless crystals.
¹H-NMR (DMSO-d₆) δ: 0.87 (9H, s), 1.82-1.98 (2H, m), 2.03-2.20 (2H, m), 2.58-3.13 (15H, m), 3.36-3.43 (2H, m), 3.51 (3H, s), 3.62 (1H, d, J = 13.9 Hz), 4.06-4.35 (6H, m), 6.10 (1H, s), 6.42 (1H, d, J = 2.5 Hz), 7.11-7.35 (8H, m), 7.55 (1H, dd, J = 2.5, 8.8 Hz), 7.77 (1H, d, J = 8.8 Hz), 8.74 (2H, brs), 10.59 (1H, brs).

### Example 52

### trans-7-Chloro-1-(2,2-dimethylpropyl)-5-(2-methoxy-3-{3-[(3-phenylpropyl)amino]propoxy}phenyl)-3-[2-(4-morpholinyl)-2-oxoethyl]-1,5-dihydro-4,1-benzoxazepine-2(3H)-one hydrochloride

From the compound obtained in Example 38 (80 mg) and morpholine (11 mg) and according to the same manner as that of Example 41, the title compound (60 mg) was obtained as colorless crystals.
¹H-NMR (DMSO-d₆) δ: 0.89 (9H, s), 1.74-1.80 (2H, m), 1.83-1.96 (2H, m), 2.05-2.17 (2H, m), 2.60-2.80 (3H, m), 2.89-3.12 (5H, m), 3.35-3.70 (10H, m), 4.07-4.20 (2H, m), 4.24-4.35 (2H, m), 6.10 (1H, s), 6.43 (1H, d, J = 2.5 Hz), 7.12-7.35 (8H, m), 7.56 (1H, dd, J = 2.5, 8.7 Hz), 7.78 (1H, d, J = 8.7 Hz), 8.53 (2H, brs).

### Example 53

### trans-2-[7-Chloro-1-(2,2-dimethylpropyl)-5-(2-methoxy-3-{3-[(3-phenylpropyl)amino]propoxy}phenyl)-2-oxo-1,2,3,5-tetrahydro-4,1-benzoxazepin-3-yl]-N-(4-piperidinylmethyl)acetamide dihydrochloride

From the compound obtained in Example 38 (100 mg) and 4-(aminomethyl)piperidine-1-carboxylic tert-butyl ester (35 mg) and according to the same manner as that of Example 41, the title compound (90 mg) was obtained as colorless crystals.
¹H-NMR (DMSO-d₆) δ: 0.87 (9H, s), 1.18-1.35 (2H, m), 1.56-1.78 (3H, m), 1.86-1.98 (2H, m), 2.06-2.18 (2H, m), 2.55-3.68 (4H, m), 2.72-3.10 (8H, m), 3.16-3.27 (2H, m), 3.52 (3H, s), 3.59 (1H, d, J = 14.0 Hz), 4.08-4.18 (2H, m), 4.22-4.32 (2H, m), 6.09 (1H, s), 6.38 (1H, d, J = 2.5 Hz), 7.05-7.34 (8H, m), 7.54 (1H, dd, J = 2.5, 8.8 Hz), 7.74 (1H, d, J = 8.8 Hz), 8.04 (1H, t, J = 5.9 Hz), 8.78 (4H, brs).

### Example 54

### trans-3-[2-(1,4'-Bipiperidin-1'-yl)-2-oxoethyl]-7-chloro-1-(2,2-dimethylpropyl)-5-(2-methoxy-3-{3-[(3-phenylpropyl)amino]propoxy}phenyl)-1,5-dihydro-4,1-benzoxazepine-2(3H)-one dihydrochloride

From the compound obtained in Example 38 (90 mg) and 4-piperidinopiperidine (25 mg) and according to the same manner as that of Example 41, the title compound (75 mg) was obtained as colorless crystals.
¹H-NMR (DMSO-d₆) δ: 0.87 (9H, s), 1.22-2.20 (14H, m), 2.59-3.12 (13H, m), 3.28-3.40 (2H, m), 3.51 (3H, s), 3.62 (1H, d, J = 14.0 Hz), 4.00-4.47 (6H, m), 6.09 (1H, s), 6.41 (1H, d, J = 2.2 Hz), 7.12-7.33 (8H, m), 7.55 (1H, dd, J = 2.5, 8.7 Hz), 7.76 (1H, d, J = 8.8 Hz), 8.77 (2H, brs), 9.98 (1H, brs).

### Example 55

### trans-N-(2-Aminoethyl)-2-[7-chloro-1-(2,2-dimethylpropyl)-5-(2-methoxy-3-{3-[(3-phenylpropyl)amino]propoxy}phenyl)-2-oxo-1,2,3,5-tetrahydro-4,1-benzoxazepin-3-yl]acetamide dihydrochloride

From the compound obtained in Example 38 (100 mg) and N-(2-aminoethyl)carbamic acid tert-butyl ester (27 mg) and according to the same manner as that of Example 41, the title compound (75 mg) was obtained as colorless crystals. ¹H-NMR (DMSO-d₆) δ: 0.87 (9H, s), 1.84-2.00 (2H, m), 2.05-2.20 (2H, m), 2.55-2.70 (4H, m), 2.75-2.96 (4H, m), 3.00-3.11 (2H, m), 3.18-3.30 (2H, m), 3.52 (3H, s), 3.60 (1H, d, J = 14.0 Hz), 4.07-4.34 (4H, m), 6.09 (1H, s), 6.40 (1H, d, J = 2.1 Hz), 7.06-7.34 (8H, m), 7.54 (1H, dd, J = 2.1, 8.7 Hz), 7.75 (1H, d, J = 8.7 Hz), 8.23 (1H, t, J = 5.5 Hz), 8.27 (5H, brs).

### Example 56

### trans-N-(3-Aminopropyl)-2-[7-chloro-1-(2,2-dimethylpropyl)-5-(2-methoxy-3-{3-[(3-phenylpropyl)amino]propoxy}phenyl)-2-oxo-1,2,3,5-tetrahydro-4,1-benzoxazepin-3-yl]acetamide dihydrochloride

From the compound obtained in Example 38 (100 mg) and N-(3-aminopropyl)carbamic acid tert-butyl ester (29 mg) and according to the same manner as that of Example 41, the title compound (90 mg) was obtained as colorless crystals.
¹H-NMR (DMSO-d₆) δ: 0.87 (9H, s), 1.60-1.73 (2H, m), 1.84-1.98 (2H, m), 2.05-2.68 (2H, m), 2.53-2.68 (4H, m), 2.70-2.80 (2H, m), 2.85-2.95 (2H, m), 3.00-3.13 (4H, m), 3.52 (3H, s), 3.59 (1H, d, J = 13.9 Hz), 4.02-4.32 (4H, m), 6.09 (1H, s), 6.40 (1H, d, J = 2.5 Hz), 7.07-7.33 (8H, m), 7.54 (1H, dd, J = 2.5, 8.7 Hz), 7.74 (1H, d, J = 8.8 Hz), 7.84 (3H, brs), 8.13 (1H, t, J = 5.8 Hz), 8.60 (2H, brs).

### Example 57

### trans-3-[2-(4-Amino-1-piperidinyl)-2-oxoethyl]-7-chloro-1-(2,2-dimethylpropyl)-5-(2-methoxy-3-{3-[(3-phenylpropyl)amino]propoxy}phenyl)-1,5-dihydro-4,1-benzoxazepine-2(3H)-one dihydrochloride

From the compound obtained in Example 38 (100 mg) and piperidin-4-ylcarbamic acid tert-butyl ester (33 mg) and according to the same manner as that of Example 41, the title compound (70 mg) was obtained as colorless crystals.
¹H-NMR (DMSO-d₆) δ: 0.87 (9H, s), 1.18-1.51 (2H, m), 1.79-1.97 (4H, m), 2.02-2.17 (2H, m), 2.56-2.74 (4H, m), 2.84-3.25 (7H, m), 3.51 (3H, s), 3.62 (1H, d, J = 12.6 Hz), 3.89-4.01 (1H, m), 4.08-4.33 (5H, m), 6.08 (1H, s), 6.42 (1H, d, J = 2.2 Hz), 7.10-7.35 (8H, m), 7.54 (1H, dd, J = 2.5, 8.8 Hz), 7.76 (1H, d, J = 8.8 Hz), 8.10 (5H, brs).

### Example 58

### trans-7-Chloro-3-[2-(1,4-diazepan-1-yl)-2-oxoethyl]-1-(2,2-dimethylpropyl)-5-(2-methoxy-3-3-[(3-phenylpropyl)amino]propoxy)phenyl)-1,5-dihydro-4,1-benzoxazepine-2(3H)-one dihydrochloride

From the compound obtained in Example 38 (100 mg) and 1-homopiperazinecarboxylic acid tert-butyl ester (33 mg) and according to the same manner as that of Example 41 the title compound (90 mg) was obtained as colorless crystals.
¹H-NMR (DMSO-d₆) δ: 0.87 (9H, s), 1.80-2.18 (6H, m), 2.58-2.75 (3H, m), 2.86-3.28 (9H, m), 3.40-3.80 (8H, m), 4.08-4.35 (4H, m), 6.10 (1H, s), 6.42 (1H, d, J = 2.5 Hz), 7.12-7.34 (8H, m), 7.55 (1H, dd, J = 2.2, 8.7 Hz), 7.77 (1H, d, J = 8.7 Hz), 8.78 (4H, brs).

### Example 59

### trans-2-[7-Chloro-1-(2,2-dimethylpropyl)-5-(2-methoxy-3-{3-[(3-phenylpropyl)amino]propoxy}phenyl)-2-oxo-1,2,3,5-tetrahydro-4,1-benzoxazepin-3-yl]-N-(piperidin-4-yl)acetamide dihydrochloride

From the compound obtained in Example 38 (100 mg) and 4-aminopiperidine-1-carboxylic acid tert-butyl ester (33 mg) and according to the same manner as that of Example 41, the title compound (70 mg) was obtained as colorless crystals.
¹H-NMR (DMSO-d₆) δ: 0.87 (9H, s), 1.46-1.63 (2H, m), 1.77-1.97 (4H, m), 2.04-2.18 (2H, m), 2.52-2.69 (4H, m), 2.86-3.11 (6H, m), 3.17-3.28 (2H, m), 3.51 (3H, s), 3.59 (1H, d, J = 14.0 Hz), 3.72-3.85 (1H, m), 4.08-4.34 (4H, m), 6.09 (1H, s), 6.38 (1H, d, J = 2.5 Hz), 7.05-7.34 (8H, m), 7.54 (1H, dd, J = 2.5, 8.7 Hz), 7.74 (1H, d, J = 8.8 Hz), 8.12 (1H, d, J = 7.5 Hz), 8.69 (4H, brs).

### Example 60

### trans-N-{[7-Chloro-1-(2,2-dimethylpropyl)-5-(2-methoxy-3-{3-[(3-phenylpropyl)amino]propoxy}phenyl)-2-oxo-1,2,3,5-tetrahydro-4,1-benzoxazepin-3-yl]acetyl}glycine hydrochloride

From the compound obtained in Example 38 (100 mg) and glycine tert-butyl ester hydrochloride (28 mg) and according to the same manner as that of Example 41, the title compound (80 mg) was obtained as colorless crystals.
¹H-NMR (DMSO-d₆) δ: 0.87 (9H, s), 1.80-1.94 (2H, m), 2.00-2.13 (2H, m), 2.58-2.70 (4H, m), 2.85-2.95 (2H, m), 3.00-3.10 (2H, m), 3.31 (3H, m), 3.59 (1H, J = 17.0 Hz, d), 3.70-3.82 (2H, m), 4.00-4.19 (2H, m), 4.22-4.34 (2H, m), 6.08 (1H, s), 6.39 (1H, d, J = 2.3 Hz), 7.10-7.24 (8H, m), 7.53 (1H, d, J = 8.8 Hz), 7.74 1H, d, J = 8.8 Hz), 8.26 (0.35H, t, J = 5.4 Hz)., 8.39 (0.65H, t, J = 5.9 Hz), 8.50 (2H, brs).

### Example 61

### trans-2-(7-Chloro-5-{3-[3-(dimethylamino)propoxy]-2-methoxyphenyl}-1-(2,2-dimethylpropyl)-2-oxo-1,2,3,5-tetrahydro-4,1-benzoxazepin-3-yl)-N-(2-fluorobenzyl)acetamide hydrochloride

To a dimethylformamide (5 ml) suspension of the compound obtained in Reference Example 10 (150 mg) and potassium carbonate (187 mg) was added 3-(dimethylamino)propyl chloride hydrochloride (51 mg), and the mixture was stirred at 80°C for 15 hours. The reaction mixture was poured into water, and this was extracted with ethyl acetate. The extract was washed successively with water and an aqueous saturated sodium chloride solution, dried over anhydrous magnesium sulfate, and concentrated under reduced pressure. The residue was purified by alumina chromatography to obtain a colorless oil (90 mg). To a methylene chloride (3 ml) solution of this oil (90 mg) was added 1N etheric hydrochloric acid (0.5 ml), and the mixture was stirred at room temperature for 5 minutes. The reaction mixture was concentrated under reduced pressure, and the resulting residue was crystallized from methylene chloride-diethyl ether to obtain the title compound (80 mg) as pale yellow crystals.
¹H-NMR (DMSO-d₆) δ: 0.88 (9H, s), 2.05-2.20 (2H, m), 2.56-2.65 (2H, m), 2.79 (6H, s), 3.13-3.27 (2H, m), 3.53 (3H, m), 3.60 (1H, d, J = 14.0 Hz), 4.05-4.40 (6H, m), 6.09 (1H, s), 6.37 (1H, d, J = 2.4 Hz), 7.00-7.09 (1H, m), 7.10-7.23 (4H, m), 7.28-7.37 (2H, m), 7.54 (1H, dd, J = 2.4, 8.7 Hz), 7.74 (1H, d, J = 8.8 Hz), 8.47 (1H, t, J = 5.8 Hz), 9.82 (1H, brs) .

### Example 62

### trans-2-[7-Chloro-1-(2,2-dimethylpropyl)-5-{2-methoxy-3-[2-(1-pyrrolidinyl)ethoxy]phenyl}-2-oxo-1,2,3,5-tetrahydro-4,1-benzoxazepin-3-yl]-N-(2-fluorobenzyl)acetamide hydrochloride

From the compound obtained in Reference Example 10 (150 mg) and 1-(2-chloroethyl)pyrrolidine hydrochloride (54 mg) and according to the same manner as that of Example 61, the title compound (103 mg) was obtained as pale yellow crystals.
¹H-NMR (DMSO-d₆) δ: 0.88 (9H, s), 1.80-2.12 (4H, m), 2.66-2.70 (2H, m), 3.03-3.21 (2H, m), 3.50-3.71 (8H, m), 4.15-4.47 (6H, m), 6.10 (1H, s), 6.38 (1H, d, J = 2.4 Hz), 7.04-7.38 (7H, m), 7.54 (1H, dd, J = 2.4, 8.7 Hz), 7.75 (1H, d, J = 8.8 Hz), 8.47 (1H, t, J = 5.8 Hz), 10.05 (1H, brs).

### Example 63

### trans-2-[7-Chloro-1-(2,2-dimethylpropyl)-5-{2-methoxy-3-[3-(4-methyl-1-piperazinyl)propoxy]phenyl}-2-oxo-1,2,3,5-tetrahydro-4,1-benzoxazepin-3-yl]-N-(2-fluorobenzyl)acetamide dihydrochloride

From the compound obtained in Reference Example 10 (100 mg) and 1-(3-chloropropyl)-4-methylpiperazine dihydrochloride (54 mg)and according to the same manner as that of Example 61, the title compound (90 mg) was obtained as colorless crystals.
¹H-NMR (DMSO-d₆) δ: 0.88 (9H, s), 2.05-2.25 (2H, m), 2.62-2.66 (2H, m), 2.70-2.90 (2H, m), 3.00-3.80 (15H, m), 4.07-4.39 (6H, m), 6.09 (1H, s), 6.38 (1H, d, J = 2.5 Hz), 7.02-7.08 (1H, m), 7.12-7.20 (4H, m), 7.27-7.37 (2H, m), 7.54 (1H, dd, J = 2.5, 8.8 Hz), 7.74 (1H, d, J = 8.8 Hz), 8.46 (1H, t, J = 5.7 Hz).

### Example 64

### trans-2-(7-Chloro-5-{3-[2-(dimethylamino)ethoxy]-2-methoxyphenyl}-1-(2,2-dimethylpropyl)-2-oxo-1,2,3,5-tetrahydro-4,1-benzoxazepin-3-yl)-N-(2-fluorobenzyl)acetamide hydrochloride

From the compound obtained in Reference Example 10 (150 mg) and 2-(dimethylamino)ethyl chloride hydrochloride (54 mg) and according to the same manner as that of Example 61, the title compound (73 mg) was obtained as yellow crystals.
¹H-NMR (DMSO-d₆) δ: 0.88 (9H, s), 2.56-2.70 (2H, m), 2.86 (6H, s), 3.48-3.65 (6H, m), 4.13-4.47 (6H, m), 6.10 (1H, s), 6.38 (1H, d, J = 2.5 Hz), 7.08-7.26 (5H, m), 7.28-7.37 (2H, m), 7.54 (1H, dd, J = 2.5, 8.7 Hz), 7.75 (1H, d, J = 8.8 Hz), 8.47 (1H, t, J = 5.7 Hz), 9.80 (1H, brs).

### Reference Example 13

### (3-Benzyloxy-2-methoxyphenyl)-[5-chloro-2-(2,4-dimethoxybenzylamino)phenyl]methanol

From the compound obtained in Reference Example 5 (5.95 g) and 2,4-dimethoxybenzaldehyde (2.81 g) and according to the same manner as that of Reference Example 6, the title compound (6.80 g) was obtained as colorless crystals.
¹H-NMR (CDCl₃) δ: 3.10 (1H, brs), 3.76 (3H, s), 3.77 (3H, s), 3.80 (3H, s), 4.24 (2H, s), 5.12 (2H, s), 5.26 (1H, brs), 6.02 (1H, s), 6.37 (1H, dd, J = 2.3, 8.3 Hz), 6.43 (1H, d, J = 2.3 Hz), 6.59 (1H, d, J = 8.8 Hz), 6.85 (1H, dd, J = 1.9, 7.4 Hz), 6.93-7.10 (5H, m), 7.29-7.48 (5H, m).

### Reference Example 14

### [5-(3-Benzyloxy-2-methoxyphenyl)-7-chloro-1-(2,4-dimethoxybenzyl)-2-oxo-1,2,3,5-tetrahydro-4,1-benzoxazepin-3-yl]acetic acid ethyl ester (cis/trans = 1/8.7 mixture)

From the compound obtained in Reference Example 13 (6.80 g) and ethyl (E)-4-chloro-4-oxo-2-butenoate (3.30 g) and according to the same manner as that of Reference Example 7, the title compound (7.90 g) was obtained as a colorless oil.
¹H-NMR (CDCl₃) δ: 1.18-1.30 (3H, m), 2.78 (1H, dd, J = 5.9, 16.5 Hz), 3.09 (1H, dd, J = 7.7, 16.5 Hz), 3.27 (2.7H, s), 3.32 (0.3H, s), 3.65 (2.7H, s), 3.66 (0.3H, s), 3.72 (2.7H, s), 3.75 (0.3H, s), 4.05-4.17 (2H, m), 4.81 (1H, dd, J = 5.9, 7.7 Hz), 4.89 (1H, d, J = 15.0 Hz), 5.08 (0.2H, s), 5.10 (1.8H, s), 5.44 (1H, d, J = 15.0 Hz), 5.97 (1H, s), 6.35-6.45 (2H, m), 6.55 (1H, d, J = 1.9 Hz), 6.92-7.00 (1H, m), 7.04-7.19 (2H, m), 7.22-7.43 (8H, m).

### Reference Example 15

### trans-[5-(3-Benzyloxy-2-methoxyphenyl)-7-chloro-1-(2,4-dimethoxybenzyl)-2-oxo-1,2,3,5-tetrahydro-4,1-benzoxazepin-3-yl]acetic acid

From the compound obtained in Reference Example 14 (7.90 g) and according to the same manner as that of Reference Example 8, the title compound (5.45 g) was obtained as colorless crystals.
¹H-NMR (CDCl₃) δ: 2.88 (1H, dd, J = 4.9, 16.4 Hz), 3.12 (1H, dd, J = 7.5, 16.4 Hz), 3.27 (3H, s), 3.67 (3H, s), 3.75 (3H, s), 4.42 (1H, dd, J = 4.9, 7.5 Hz), 4.88 (1H, d, J = 15.0 Hz), 5.10 (2H, s), 5.47 (1H, d, J = 15.0 Hz), 5.97 (1H, s), 6.35-6.46 (2H, m), 6.58 (1H, s), 6.95-7.01 (1H, m), 7.08-7.47 (10H, m).

### Reference Example 16

### trans-2-{5-[3-(Benzyloxy)-2-methoxyphenyl]-7-chloro-1-(2,4-dimethoxybenzyl)-2-oxo-1,2,3,5-tetrahydro-4,1-benzoxazepin-3-yl}-N-(2-fluorobenzyl)acetamide

From the compound obtained in Reference Example 15 (5.44 g) and 2-fluorobenzylamine (1.32 g) and according to the same manner as that of Reference Example 9, the title compound (5.60 g) was obtained as colorless crystals.
¹H-NMR (CDC1₃) δ: 2.71 (1H, dd, J = 6.1, 14.3 Hz), 2.91 (1H, dd, J = 6.9, 14.3 Hz), 3.23 (3H, s), 3.65 (3H, s), 3.73 (3H, s), 4.37-4.60 (3H, m), 4.83 (1H, d, J = 14.9 Hz), 5.09 (2H, s), 5.46 (1H, d, J = 14.9 Hz), 5.96 (1H, s), 6.28-6.42 (3H, m), 6.53 (1H, s), 6.93-7.40 (15H, m).

### Reference Example 17

### trans-2-[7-Chloro-1-(2,4-dimethoxybenzyl)-5-(3-hydroxy-2-methoxyphenyl)-2-oxo-1,2,3,5-tetrahydro-4,1-benzoxazepin-3-yl]-N-(2-fluorobenzyl)acetamide]

From the compound obtained in Reference Example 16 (2.00 g) and according to the same manner as that of Reference Example 10, the title compound (1.73 g) was obtained as colorless crystals.
¹H-NMR (CDCl₃) δ: 2.71 (1H, dd, J = 6.2, 14.4 Hz), 2.91 (1H, dd, J = 6.9, 14.4 Hz), 3.00 (3H, s), 3.63 (3H, s), 3.76 (3H, s), 4.38-4.59 (3H, m), 4.80 (1H, d, J = 14.8 Hz), 5.41 (1H, s), 5.51 (1H, d, J = 14.8 Hz), 5.89 (1H, s), 6.30 (1H, t, J = 5.7 Hz), 6.37-6.42 (2H, m), 6.61 (1H, d, J = 14.8 Hz), 6.90-7.38 (10H, m).

### Example 65

### [3-(3-[7-Chloro-1-(2,4-dimethoxybenzyl)-3-[(2-fluorobenzylcarbamoyl)methyl]-2-oxo-1,2,3,5-tetrahydro-4,1-benzoxazepin-5-yl]-2-methoxyphenoxy)propyl]carbamic acid tert-butyl ester

From the compound obtained in Reference Example 17 (1.68 g) and the compound obtained in Reference Example 11 (0.95 g) and according to the same manner as that of Example 1, the title compound (1.98 g) was obtained as a colorless oil.
¹H-NMR (CDCl₃) δ: 1.42 (9H, s), 1.92-2.03 (2H, m), 2.71 (1H, dd, J = 6.1, 14.3 Hz), 2.91 (1H, dd, J = 6.9, 14.3 Hz), 3.20 (3H, s), 3.23-3.36 (2H, m), 3.66 (3H, s), 3.75 (3H, s), 4.00-4.05 (2H, m), 4.36-4.57 (3H, m), 4.74 (1H, brs), 4.84 (1H, d, J = 15.0 Hz), 5.43 (1H, d, J = 15.0 Hz), 5.95 (1H, s), 6.29-6.41 (3H, m), 6.52 (1H, s), 6.86-7.35 (10H, m).

### Example 66

### trans-2-[5-[3-(3-Aminopropoxy)-2-methoxyphenyl]-7-chloro-1-(2,4-dimethoxybenzyl)-2-oxo-1,2,3,5-tetrahydro-4,1-benzoxazepin-3-yl]-N-(2-fluorobenzyl)acetamide hydrochloride

From the compound obtained in Example 65 (1.60 g) and according to the same manner as that of Example 2, the title compound (1.47 g) was obtained as colorless crystals.
¹H-NMR (DMSO-d₆) δ: 2.00-2.10 (2H, m), 2.60-2.77 (2H, m), 2.90-3.00 (2H, m), 3.10 (3H, s), 3.66 (3H, s), 3.71 (3H, s), 4.03-4.13 (2H, m), 4.19-4.40 (3H, m), 4.78 (1H, d, J = 14.8 Hz), 5.49 (1H, d, J = 14.8 Hz), 5.83 (1H, s), 6.29 (1H, d, J = 2.2 Hz), 6.41 (1H, dd, J = 2.2, 8.3 Hz), 6.52 (1H, d, J = 2.0 Hz), 6.99 (1H, dd, J = 2.0, 6.8 Hz), 7.06-7.23 (5H, m), 7.27-7.36 (2H, m), 7.50 (1H, dd, J = 2.3, 8.7 Hz), 7.65 (1H, d, J = 8.7 Hz), 7.87 (3H, brs), 8.49 (1H, t, J = 5.8 Hz) .

### Example 67

### trans-2-[7-Chloro-1-(2,4-dimethoxybenzyl)-5-(2-methoxy-3-{3-[(3-phenylpropyl)amino)propoxy}phenyl)-2-oxo-1,2,3,5-tetrahydro-4,1-benzoxazepin-3-yl]-N-(2-fluorobenzyl)acetamide hydrochloride

From the compound obtained in Example 66 (1.00 g) and 3-phenylpropanal (184 mg) and according to the same manner as that of Example 3, the title compound (270 mg) was obtained as colorless crystals.
¹H-NMR (DMSO-d₆) δ: 1.83-1.96 (2H, m), 2.02-2.14 (2H, m), 2.60-2.78 (4H, m), 2.85-2.96 (2H, m), 3.00-3.08 (2H, m), 3.10 (3H, s), 3.66 (3H, s), 3.70 (3H, s), 4.05-4.15 (2H, m), 4.20-4.40 (3H, m), 4.88 (1H, d, J = 14.9 Hz), 5.49 (1H, d, J = 14.9 Hz), 5.83 (1H, s), 6.29 (1H, d, J = 2.3 Hz), 6.40 (1H, dd, J = 2.3, 8.5 Hz), 6.50 (1H, d, J = 2.2 Hz), 7.04 (1H, dd, J = 2.2, 6.9 Hz), 7.05-7.22 (8H, m), 7.23-7.36 (4H, m), 7.49 (1H, dd, J = 2.5, 8.7 Hz), 7.65 (1H, d, J = 8.7 Hz), 8.49 (1H, t, J = 5.8 Hz), 8.62 (2H, brs).

### Reference Example 18

### trans-2-{5-[3-(Benzyloxy)-2-methoxyphenyl]-7-chloro-2-oxo-1,2,3,5-tetrahydro-4,1-benzoxazepin-3-yl}-N-(2-fluorobenzyl)acetamide

To an acetone (12 ml) solution of the compound obtained in Reference Example 16 (725 mg) was added an aqueous (3 ml) solution of cerium nitrate ammonium (1.64 g) under ice-cooling, and the mixture was stirred at room temperature for 30 minutes. The reaction mixture was concentrated under reduced pressure, diluted with an aqueous saturated sodium bicarbonate solution, and extracted with ethyl acetate. The organic layer was washed with an aqueous saturated sodium chloride solution, dried over anhydrous magnesium sulfate, and concentrated under reduced pressure. The residue was purified by silica gel chromatography to obtain the title compound (410 mg) as a colorless oil.
¹H-NMR (CDCl₃) δ: 2.70 (1H, dd, J = 6.8, 14.9 Hz), 2.89 (1H, dd, J = 6.0, 14.9 Hz), 3.66 (3H, s), 4.37-4.53 (2H, m), 4.63 (1H, dd, J = 6.0, 6.8 Hz), 5.12 (2H, s), 6.20 (1H, s), 6.30 (1H, t, J = 5.7 Hz), 6.68 (1H, d, J = 2.3 Hz), 6.98-7.48 (14H, m), 8.01 (1H, s).

### Reference Example 19

### trans-2-{5-[3-(Benzyloxy)-2-methoxyphenyl]-7-chloro-1-methyl-2-oxo-1,2,3,5-tetrahydro-4,1-benzoxazepin-3-yl}-N-(2-fluorobenzyl)acetamide

To a dimethylformamide (15 ml) suspension of the compound obtained in Reference Example 18 (410 mg) and potassium carbonate (493 mg) was added methyl iodide (304 mg), and the mixture was stirred at 60°C for 20 hours. The reaction mixture was poured into water, and this was extracted with ethyl acetate. The extract was washed successively with water and an aqueous saturated sodium chloride solution, dried over anhydrous magnesium sulfate, and concentrated under reduced pressure. The residue was purified by silica gel chromatography to obtain the title compound (420 mg) as a colorless oil.
¹H-NMR (CDCl₃) δ: 2.70 (1H, dd, J = 6.3, 14.4 Hz), 2.88 (1H, dd, J = 6.7, 14.4 Hz), 3.49 (3H, s), 3.61 (3H, s), 4.39-4.59 (3H, m), 5.12 (2H, s), 6.00 (1H, s), 6.30 (1H, t, J = 5.6 Hz), 6.63 (1H, d, J = 2.4 Hz), 6.98-7.13 (5H, m), 7.16-7.48 (9H, m).

### Reference Example 20

### trans-2-[7-Chloro-5-(3-hydroxy-2-methoxyphenyl)-1-methyl-2-oxo-1,2,3,5-tetrahydro-4,1-benzoxazepin-3-yl]-N-(2-fluorobenzyl)acetamide

From the compound obtained in Reference Example 19 (420 mg) and according to the same manner as that of Reference Example 10, the title compound (280 mg) was obtained as colorless crystals.
¹H-NMR (DMSO-d₆) δ: 2.62-2.81 (2H, m), 3.42 (3H, s), 3.46 (3H, s), 4.17-4.38 (3H, m), 5.80 (1H, s), 6.45 (1H, s), 6.86-7.05 (3H, m), 7.10-7.20 (2H, m), 7.26-7.36 (2H, m), 7.57-7.61 (2H, m), 8.49 (1H, t, J = 5.8 Hz), 9.56 (1H, s).

### Example 68

### [3-(3-[7-Chloro-3-[(2-fluorobenzylcarbamoyl)methyl]-1-methyl-2-oxo-1,2,3,5-tetrahydro-4,1-benzoxazepin-5-yl]-2-methoxyphenoxy)propyl]carbamic acid tert-butyl ester

From the compound obtained by the method of Reference Example 20 (280 mg) and the compound obtained in Reference Example 11 (165 mg) and according to the same manner as that of Reference Example 12, the title compound (370 mg) was obtained as a pale brown oil.
¹H-NMR (CDCl₃) δ: 1.42 (9H, s), 1.92-2.05 (2H, m), 2.70 (1H, dd, J = 6.4, 14.4 Hz), 2.88 (1H, dd, J = 6.7, 14.4 Hz), 3.25-3.40 (2H, m), 3.48 (3H, s), 3.57 (3H, s), 4.04-4.13 (2H, m), 4.40-4.58 (3H, m), 4.90 (1H, brs), 5.99 (1H, s), 6.35 (1H, t, J = 5.8 Hz), 6.62 (1H, d, J = 2.3 Hz), 6.92-7.12 (5H, m), 7.18-7.40 (4H, m).

### Example 69

### trans-2-{5-[3-(3-Aminopropoxy)-2-methoxyphenyl]-7-chloro-1-methyl-2-oxo-1,2,3,5-tetrahydro-4,1-benzoxazepin-3-yl}-N-(2-fluorobenzyl)acetamide hydrochloride

From the compound obtained in Example 68 (370 mg) and according to the same manner as that of Example 2, the title compound (270 mg) was obtained as a white crystal.
¹H-NMR (DMSO-d₆) δ: 1.97-2.10 (2H, m), 2.59-2.73 (2H, m), 2.90-3.01 (2H, m), 3.43 (3H, s), 3.50 (3H, s), 4.05-4.37 (5H, m), 5.83 (1H, s), 6.43 (1H, s), 7.04 (1H, dd, J = 2.4, 7.9 Hz), 7.10-7.21 (4H, m), 7.25-7.37 (2H, m), 7.54-7.63 (2H, m), 7.84 (3H, brs), 8.50 (1H, t, J = 5.9 Hz).

### Example 70

### trans-2-[7-Chloro-5-(2-methoxy-3-{3-[(3-phenylpropyl)amino]propoxy}phenyl)-1-methyl-2-oxo-1,2,3,5-tetrahydro-4,1-benzoxazepin-3-yl]-N-(2-fluorobenzyl)acetamide hydrochloride

From the compound obtained in Example 69 (190 mg) and 3-phenylpropanal (43 mg) and according to the same manner as that of Example 3, the title compound (100 mg) was obtained as colorless crystals.
¹H-NMR (DMSO-d₆) δ: 1.85-1.94 (2H, m), 2.02-2.13 (2H, m), 2.57-2.73 (4H, m), 2.86-2.96 (2H, m), 3.00-3.10 (2H, m), 3.43 (3H, s), 3.49 (3H, s), 4.07-4.40 (5H, m), 5.83 (1H, s), 6.43 (1H, s), 7.02-7.37 (12H, m), 7.55-7.63 (2H, m), 8.40 (2H, brs), 8.47 (1H, t, J = 5.9 Hz).

### Reference Example 21

### trans-[5-(3-Benzyloxy-2-methoxyphenyl)-7-chloro-1-(2,4-dimethoxybenzyl)-2-oxo-1,2,3,5-tetrahydro-4,1-benzoxazepin-3-yl]acetic acid methyl ester

To a dimethylformamide (20 ml) suspension of the compound obtained in Reference Example 15 (2.00 g) and potassium carbonate (2.23 g) was added methyl iodide (0.92 g), and the mixture was stirred at room temperature for 20 hours. The reaction mixture was poured into water, and this was extracted with ethyl acetate. The extract was washed successively with water and an aqueous saturated sodium chloride solution, dried over magnesium sulfate, and concentrated under reduced pressure. The residue was purified by silica gel chromatography to obtain the title compound (2.00 g) as a colorless oil.
¹H-NMR (CDCl₃) δ: 2.80 (1H, dd, J = 5.9, 16.5 Hz), 3.11 (1H, dd, J = 7.8, 16.5 Hz), 3.27 (3H, s), 3.66 (3H, s), 3.68 (3H, s), 3.75 (3H, s), 4.48 (1H, dd, J = 5.9, 7.8 Hz), 4.90 (1H, d, J = 15.0 Hz), 5.10 (2H, s), 5.44 (1H, d, J = 15.0 Hz), 5.97 (1H, s), 6.38-6.44 (2H, m), 6.55 (1H, d, J = 1.8 Hz), 6.98 (1H, dd, J = 1.8, 7.8 Hz), 7.07-7.18 (2H, m), 7.22-7.45 (8H, m).

### Reference Example 22

### trans-[5-3-Benzyloxy-2-methoxyphenyl]-7-chloro-2-oxo-1,2,3,5-tetrahydro-4,1-benzoxazepin-3-yl]acetic acid methyl ester

From the compound obtained in Reference Example 21 (2.00 g) and according to the same manner as that of Reference Example 18, the title compound (1.10 g) was obtained as an yellow oil.
¹H-NMR (CDCl₃) δ: 2.82 (1H, dd, J = 6.7, 16.3 Hz), 3.06 (1H, dd, J = 6.7, 16.3 Hz), 3.63 (3H, s), 3.70 (3H, s), 4.67 (1H, t, J = 6.7 Hz), 5.14 (2H, s), 6.24 (1H, s), 6.70 (1H, d, J = 2.3 Hz), 7.00-7.17 (4H, m), 7.23-7.47 (6H, m), 9.09 (1H, s) .

### Reference Example 23

### trans-[5-(3-Benzyloxy-2-methoxyphenyl)-7-chloro-3-(2-methoxy-2-oxoethyl)-2-oxo-2,3-dihydro-4,1-benzoxazepin-1(5H)-yl]acetic acid tert-butyl ester

From the compound obtained in Reference Example 22 (1.07 g) and bromoacetic acid tert-butyl ester (0.65 g) and according to the same manner as that of Reference Example 19, the title compound (1.32 g) was obtained as a pale yellow oil.
¹H-NMR (CDCl₃) δ: 1.47 (9H, s), 2.81 (1H, dd, J = 6.2, 16.6 Hz), 3.06 (1H, dd, J = 7.4, 16.6 Hz), 3.67 (3H, s), 3.73 (3H, s), 4.38 (1H, d, J = 17.0 Hz), 4.50 (1H, dd, J = 6.2, 7.4 Hz), 4.69 (1H, d, J = 17.0 Hz), 5.10 (1H, d, J = 11.8 Hz), 5.15 (1H, d, J = 11.8 Hz), 6.55 (1H, s), 6.65 (1H, d, J = 2.5 Hz), 7.01 (1H, dd, J = 2.5, 8.0 Hz), 7.10-7.23 (3H, m), 7.30-7.47 (6H, m).

### Reference Example 24

### trans-[7-Chloro-5-(3-hydroxy-2-methoxyphenyl)-3-(2-methoxy-2-oxoethyl)-2-oxo-2,3-dihydro-4,1-benzoxazepin-1(5H)-yl]acetic acid tert-butyl ester

To an ethyl acetate (45 ml) suspension of the compound obtained in Reference Example 23 (1.30 g) and 10% palladium carbon (180 mg) was added 1N hydrochloric acid (0.9ml), and the mixture was stirred at room temperature for 1 hour under the hydrogen gas atmosphere. The reaction mixture was filtered with Celite, and the filtrate was washed successively with an aqueous saturated sodium bicarbonate solution and an aqueous saturated sodium chloride solution, dried over anhydrous magnesium sulfate, and concentrated under reduced pressure. The residue was purified by silica gel chromatography to obtain the title compound (1.10 g) as a colorless oil.
¹H-NMR (CDCl₃) δ: 1.47 (9H, s), 2.80 (1H, dd, J = 6.0, 17.6 Hz), 3.07 (1H, dd, J = 7.6, 17.6 Hz), 3.64 (3H, s), 3.67 (3H, s), 4.29 (1H, d, J = 17.1 Hz), 4.51 (1H, dd, J = 6.0, 7.6 Hz), 4.84 (1H, d, J = 17.1 Hz), 5.47 (1H, s), 6.59 (1H, s), 6.69 (1H, d, J = 2.4 Hz), 7.02 (1H, dd, J = 3.3, 6.3 Hz), 7.13-7.20 (3H, m), 7.36 (1H, dd, J = 2.4, 8.5 Hz).

### Reference Example 25

### 2-Nitro-N-(3-phenylpropyl)benzenesulfonamide

To a methylene chloride (50 ml) solution of 3-phenylpropylamine (5.00 g) and pyridine (3.51 g) was added 2-nitrobenzenesulfonyl chloride (9.01 g) at 0°C, and the mixture was stirred at room temperature for 15 hours. The reaction mixture was concentrated under reduced pressure, and the residue was partitioned between ethyl acetate and water. The organic layer was washed successively with 1N hydrochloride acid, an aqueous saturated sodium bicarbonate solution, and an aqueous saturated sodium chloride solution, dried over anhydrous magnesium sulfate, and concentrated under reduced pressure. The residue was purified by silica gel chromatography to obtain the title compound (9.10 g) as an yellow oil.
¹H-NMR (CDCl₃) δ: 1.81-1.92 (2H, m), 2.62-2.67 (2H, m), 3.08-3.20 (2H, m), 5.29 (1H, t, J = 5.6 Hz), 7.04-7.30 (5H, m), 7.68-7.78 (2H, m), 7.81-7.95 (1H, m), 8.04-8.13 (1H, m).

### Reference Example 26

### N-(3-bromopropyl)-2-nitro-N-(3-phenylpropyl)benzenesulfonamide

To a dimethylformamide (110 ml) solution of the compound obtained in Reference Example 25 (8.81 g) and potassium carbonate (3.84 g) was added 1,3-dibromopropane (27.8 g), and the mixture was stirred at 60°C for 8 hours. The reaction mixture was poured into water, and extracted with ethyl acetate. The extract was washed successively with water and an aqueous saturated sodium chloride solution, dried over anhydrous magnesium sulfate, and concentrated under reduced pressure. The residue was purified by silica gel chromatography to obtain the title compound (7.23 g) as an yellow oil.
¹H-NMR (CDCl₃) δ: 1.80-1.95 (2H, m), 2.03-2.17 (2H, m), 2.51-2.65 (2H, m), 3.23-3.48 (6H, m), 7.06-7.32 (5H, m), 7.56-7.74 (3H, m), 7.94 (1H, dd, J = 1.5, 7.5 Hz).

### Example 71

### trans-[7-Chloro-3-(2-methoxy-2-oxoethyl)-5-(2-methoxy-3-{3-[(3-phenylpropyl)amino]propoxy}phenyl)-2-oxo-2,3-dihydro-4,1-benzoxazepin-1(5H)-yl]acetic acid tert-butyl ester fumarate

To a dimethylformamide (10 ml) suspension of the compound obtained in Reference Example 24 (220 mg) and potassium carbonate (180 mg) was added the compound obtained in Reference Example 26 (288 mg), and the mixture was stirred at 70°C for 15 hours. The reaction mixture was poured into water, and extracted with ethyl acetate. The extract was washed successively with water and an aqueous saturated sodium chloride solution, dried over anhydrous magnesium sulfate, and concentrated under reduced pressure. The residue was purified by silica gel chromatography to obtain an yellow oil (210 mg). To a dimethylformamide (5 ml) solution of this oil (210 mg) were added cesium carbonate (237 mg) and thiophenol (80 mg), and the mixture was stirred at room temperature for 15 hours. The reaction mixture was poured into water, and extracted with ethyl acetate. The extract was washed successively with water and an aqueous saturated sodium chloride solution, dried over anhydrous magnesium sulfate, and concentrated under reduced pressure. The residue was purified by silica gel chromatography to obtain a pale yellow oil (116 mg). To a 2-propanol (4 ml) solution of this oil (71 mg) was added a 2-propanol (3 ml) solution of fumaric acid (12 mg), and the mixture was stirred at room temperature for 5 minutes. The reaction mixture was concentrated under reduced pressure, and the resulting residue was crystallized from 2-propanol-diethyl ether to obtain the title compound (60 mg) as pale yellow crystals.
¹H-NMR (DMSO-d₆) δ: 1.42 (9H, s), 1.77-1.90 (2H, m), 1.96-2.09 (2H, m), 2.58-2.67 (2H, m), 2.70-3.00 (6H, m), 3.56 (3H, s), 3.57 (3H, s), 4.05-4.15 (2H, m), 4.32 (1H, dd, J = 6.7, 6.9 Hz), 4.56 (1H, d, J = 17.4 Hz), 4.73 (1H, d, J = 17.4 Hz), 6.42-6.49 (4H, m), 7.06-7.30 (8H, m), 7.56-7.61 (2H, m).

### Example 72

### trans-[7-Chloro-3-(2-methoxy-2-oxoethyl)-5-(2-methoxy-3-{3-[(3-phenylpropyl)amino]propoxy}phenyl)-2-oxo-2,3-dihydro-4,1-benzoxazepin-1(5H)-yl]acetic acid hydrochloride

To the free form of the compound obtained in Example 71 (40 mg) was added a 4N hydrochloric acid/ethyl acetate solution (5 ml), and the mixture was stirred at room temperature for 15 hours. The resulting reaction mixture was concentrated under reduced pressure, and the residue was crystallized from methylene chloride-diethyl ether to obtain the title compound (30 mg) as pale yellow crystals.
¹H-NMR (DMSO-d₆) δ: 1.81-1.95 (2H, m), 2.02-2.14 (2H, m), 2.59-2.96 (6H, m), 3.02-3.11 (2H, m), 3.53 (3H, s), 3.58 (3H, s), 4.07-4.16 (2H, m), 4.33 (1H, dd, J = 6.7, 6.9 Hz), 4.57 (1H, d, J = 17.4 Hz), 4.75 (1H, d, J = 17.4 Hz), 6.41 (1H, s), 6.44 (1H, d, J = 2.0 Hz), 7.07-7.34 (8H, m), 7.52-7.63 (2H, m), 8.52 (2H, brs).

### Example 73

### trans-[7-Chloro-1-(2,2-dimethylpropyl)-5-(2-methoxy-3-(3-[methyl(3-phenylpropyl)amino]propoxy}phenyl)-2-oxo-1,2,3,5-tetrahydro-4,1-benzoxazepin-3-yl]acetic acid ethyl ester hydrochloride

From the free form of the compound obtained in Example 35 (560 mg) and according to the same manner as that of Example 15, the free form of the title compound (470 mg) was obtained as a pale yellow oil and, further, from this oil (50 mg), the title compound (50 mg) was obtained as pale yellow crystals.
¹H-NMR (DMSO-d₆) δ: 0.87 (9H, s), 1.15 (3H, t, J = 7.1 Hz), 1.90-2.05 (2H, m), 2.10-2.24 (2H, m), 2.57-2.88 (7H, m), 2.97-3.26 (4H, m), 3.51 (3H, s), 3.62 (1H, d, J = 14.0 Hz), 3.97-4.18 (4H, m), 4.20-4.34 (2H, m), 6.10 (1H, s), 6.41 (1H, d, J = 2.4 Hz), 7.09-7.32 (8H, m), 7.56 (1H, dd, J = 2.4, 8.7 Hz), 7.77 (1H, d, J = 8.8 Hz), 10.30 (1H, brs).

### Example 74

### trans-[7-Chloro-1-(2,2-dimethylpropyl)-5-(2-methoxy-3-{3-[methyl(3-phenylpropyl)amino]propoxy}phenyl)-2-oxo-1,2,3,5-tetrahydro-4,1-benzoxazepin-3-yl]acetic acid hydrochloride

From the free form of the compound obtained in Example 73 (400 mg) and according to the same manner as that of Reference Example 8, the title compound (385 mg) was obtained as colorless crystals.
¹H-NMR (DMSO-d₆) δ: 0.87 (9H, s), 1.87-2.03 (2H, m), 2.07-2.23 (2H, m), 2.56-2.85 (7H, m), 2.96-3.27 (4H, m), 3.51 (3H, s), 3.62 (1H, d, J = 14.0 Hz), 4.06-4.25 (3H, m), 4.29 (1H, d, J = 14.0 Hz), 6.09 (1H, s), 6.40 (1H, d, J = 2.4 Hz), 7.10-7.33 (8H, m), 7.55 (1H, dd, J = 2.4, 8.7 Hz), 7.77 (1H, d, J = 8.8 Hz), 9.93 (1H, brs), 12.29 (1H, brs).

### Example 75

### trans-7-Chloro-1-(2,2-dimethylpropyl)-5-(2-methoxy-3-{3-[methyl(3-phenylpropyl)amino]propoxy}phenyl)-3-[2-oxo-2-(1-piperazinyl)ethyl]-1,5,-dihydro-4,1-benzoxazepin-2(3H)-one dihydrochloride

From the compound obtained in Example 74 (200 mg) and piperazine-1-carboxylic acid tert-butyl ester (70 mg) and according to the same manner as that of Example 41, the title compound (180 mg) was obtained as colorless crystals. ¹H-NMR (DMSO-d₆) δ: 0.84 (9H, s), 1.85-2.01 (2H, m), 2.05-2.20 (2H, m), 2.53-2.64 (2H, m), 2.66-2.78 (4H, m), 2.88-3.25 (9H, m), 3.42-3.75 (8H, m), 4.05-4.16 (2H, m), 4.18-4.30 (2H, m), 6.06 (1H, s), 6.38 (1H, d, J = 2.4 Hz), 7.10-7.30 (8H, m), 7.52 (1H, dd, J = 2.5, 8.7 Hz), 7.74 (1H, d, J = 8.8 Hz), 9.05 (2H, brs), 10.18 (1H, brs).

### Example 76

### trans-{7-Chloro-5-[3-(3-{[3-(2-chlorophenyl)propyl]amino}propoxy)-2-methoxyphenyl]-1-(2,2-dimethylpropyl)-2-oxo-1,2,3,5-tetrahydro-4,1-benzoxazepin-3-yl}acetic acid ethyl ester hydrochloride

From the compound obtained in Example 34 (1.00 g) and 3-(2-chlorophenyl)propanal (297 mg) and according to the same manner as that of Example 3, the free form of the title compound (850 mg) was obtained as a colorless oil, and further from this oil (80 mg), the title compound (50 mg) was obtained as colorless crystals.
¹H-NMR (DMSO-d₆) δ: 0.87 (9H, s), 1.15 (3H, t, J = 7.1 Hz), 1.86-2.02 (2H, m), 2.66-3.13 (8H, m), 3.53 (3H, s), 3.62 (1H, d, J = 13.9 Hz), 3.96-4.34 (6H, m), 6.11 (1H, s), 6.42 (1H, d, J = 2.1 Hz), 7.08-7.46 (7H, m), 7.56 (1H, dd, J = 2.0, 8.6 Hz), 7.77 (1H, d, J = 8.8 Hz), 9.09 (2H, brs).

### Example 77

### trans-{5-[3-(3-{(tert-Butoxycarbonyl)[3-(2-chlorophenyl)propyl]amino}propoxy)-2-methoxyphenyl]-7-chloro-1-(2,2-dimethylpropyl)-2-oxo-1,2,3,5-tetrahydro-4,1-benzoxazepin-3-yl}acetic acid ethyl ester

From the free form of the compound obtained in Example 76 (300 mg) and according to the same manner as that of Example 30, the title compound (340 mg) was obtained as a pale yellow oil.
¹H-NMR (CDCl₃) δ: 0.94 (9H, s), 1.24 (3H, t, J = 7.1 Hz), 1.43 (9H, s), 1.76-1.90 (2H, m), 2.00-2.13 (2H, m), 2.63-2.72 (2H, m), 2.77 (1H, dd, J = 6.0, 16.5 Hz), 3.03 (1H, dd, J = 7.6, 16.5 Hz), 3.19-3.47 (5H, m), 3.62 (3H, s), 4.00-4.19 (4H, m), 4.38 (1H, dd, J = 6.0, 7.6 Hz), 4.51 (1H, d, J = 13.9 Hz), 6.26 (1H, s), 6.61 (1H, s), 6.95 (1H, dd, J = 1.6, 7.7 Hz), 7.07-7.22 (5H, m), 7.26-7.37 (3H, m).

### Example 78

### trans-{5-[3-(3-{(tert-Butoxycarbonyl)[3-(2-chlorophenyl)propyl]amino}propoxy)-2-methoxyphenyl]-7-chloro-1-(2,2-dimethylpropyl)-2-oxo-1,2,3,5-tetrahydro-4,1-benzoxazepin-3-yl}acetic acid

From the compound obtained in Example 77 (340 mg) and according to the same manner as that of Example 38, the title compound (328 mg) was obtained as a pale yellow oil.
¹H-NMR (CDCl₃) δ: 0.95 (9H, s), 1.43 (9H, s), 1.76-1.90 (2H, m), 1.98-2.13 (2H, m), 2.63-2.73 (2H, m), 2.86 (1H, dd, J = 5.1, 16.3 Hz), 3.06 (1H, dd, J = 7.3, 16.3 Hz), 3.18-3.46 (5H, m), 3.63 (3H, s), 4.00-4.08 (2H, m), 4.33 (1H, dd, J = 5.1, 7.3 Hz), 4.52 (1H, d, J = 13.9 Hz), 6.26 (1H, s), 6.63 (1H, d, J = 1.9 Hz), 6.95 (1H, d, J = 7.7 Hz), 7.07-7.38 (8H, m).

### Example 79

### trans-{7-Chloro-5-[3-(3-{[3-(2-chlorophenyl)propyl]amino}propoxy)-2-methoxyphenyl]-1-(2,2-dimethylpropyl)-2-oxo-1,2,3,5-tetrahydro-4,1-benzoxazepin-3-yl}acetic acid hydrochloride

From the compound obtained in Example 78 (156mg) and according to the same manner as that of Example 39, the title compound (110 mg) was obtained as colorless crystals. ¹H-NMR (DMSO-d₆) δ: 0.88 (9H, s), 1.82-1.97 (2H, m), 2.03-2.18 (2H, m), 2.57-2.88 (4H, m), 2.91-3.01 (2H, s),3.02-3.13 (2H, m), 3.53 (3H, s), 3.62 (1H, d, J = 14.0 Hz), 3.97-4.35 (4H, m), 6.10 (1H, s), 6.42 (1H, d, J = 2.2 Hz), 7.09-7.47 (7H, m), 7.52-7.60 (1H, m), 7.77 (1H, d, J = 8.8 Hz), 8.60 (2H, brs).

### Example 80

### trans-7-Chloro-5-[3-(3-{[3-(2-chlorophenyl)propyl]amino}propoxy)-2-methoxyphenyl]-1-(2,2-dimethylpropyl)-3-[2-oxo-2-(1-piperazinyl)ethyl]-1,5-dihydro-4,1-benzoxazepine-2(3H)-one dihydrochloride

From the compound obtained in Example 78 (172 mg) and piperazine-1-carboxylic acid tert-butyl ester (51 mg) and according to the same manner as that of Example 41, the title compound (150 mg) was obtained as colorless crystals. ¹H-NMR (DMSO-d₆) δ: 0.87 (9H, s), 1.83-1.99 (2H, m), 2.06-2.18 (2H, m), 2.69-2.82 (3H, m), 2.90-3.20 (9H, m), 3.47-3.77 (8H, m), 4.08-4.32 (4H, m), 6.10 (1H, s), 6.42 (1H, d, J = 2.4 Hz), 7.10-7.47 (7H, m), 7.55 (1H, dd, J = 2.4, 8.7 Hz), 7.77 (1H, d, J = 8.9 Hz), 8.85 (2H, brs), 9.06 (2H, brs).

### Example 81

### trans-{7-Chloro-5-[3-(3-{[3-(2-chlorophenyl)propyl](methyl)amino}propoxy)-2-methoxyphenyl]-1-(2,2-dimethylpropyl)-2-oxo-1,2,3,5-tetrahydro-4,1-benzoxazepin-3-yl}acetic acid ethyl ester hydrochloride

From the free form of the compound obtained in Example 76 (690 mg) and according to the same manner as that of Example 15, the free form of the title compound (650 mg) was obtained as a colorless oil and, further, from this oil (60 mg), the title compound (40 mg) was obtained as pale yellow crystals.
¹H-NMR (DMSO-d₆) δ: 0.87 (9H, s), 1.15 (3H, t, J = 7.1 Hz), 1.90-2.08 (2H, m), 2.12-2.30 (2H, m), 2.51 (3H, s), 2.65-2.90 (4H, m), 3.00-3.40 (4H, m), 3.52 (3H, s), 3.62 (1H, d, J = 14.0 Hz), 3.97-4.35 (6H, m), 6.10 (1H, s), 6.41 (1H, d, J = 1.4 Hz), 7.06-7.48 (7H, m), 7.50-7.70 (2H, m), 10.65 (1H, brs).

### Example 82

### trans-{7-Chloro-5-[3-(3-{[3-(2-chlorophenyl)propyl](methyl)amino}propoxy)-2-methoxyphenyl]-1-(2,2-dimethylpropyl)-2-oxo-1,2,3,5-tetrahydro-4,1-benzoxazepin-3-yl}acetic acid hydrochloride

From the free form of the compound obtained in Example 81 (550 mg) and according to the same manner as that of Reference Example 8, the title compound (470 mg) was obtained as colorless crystals.
¹H-NMR (DMSO-d₆) δ: 0.87 (9H, s), 1.87-2.02 (2H, m), 2.08-2.22 (2H, m), 2.57-2.85 (7H, m), 3.06-3.30 (4H, m), 3.51 (3H, s), 3.62 (1H, d, J = 14.0 Hz), 4.07-4.24 (3H, m), 4.29 (1H, d, J = 14.0 Hz), 6.09 (1H, s), 6.40 (1H, d, J = 2.4 Hz), 7.12-7.45 (7H, m), 7.55 (1H, dd, J = 2.4, 8.7 Hz), 7.77 (1H, d, J = 8.8 Hz), 9.86 (1H, brs), 12.28 (1H, brs).

### Example 83

### trans-7-Chloro-5-[3-(3-{[3-(2-chlorophenyl)propyl](methyl)amino}propoxy)-2-methoxyphenyl]-1-(2,2-dimethylpropyl)-3-[2-oxo-2-(1-piperazinyl)ethyl]-1,5-dihydro-4,1-benzoxazepine-2(3H)-one dihydrochloride

From the compound obtained in Example 82 (200 mg) and piperazine-1-carboxylic acid tert-butyl ester (63 mg) and according to the same manner as that of Example 41, the tile compound (120 mg) was obtained as brown crystals.
¹H-NMR (DMSO-d₆) δ: 0.87 (9H, s), 1.89-2.04 (2H, m), 2.11-2.24 (2H, m), 2.67-2.81 (6H, m), 2.93-3.30 (9H, m), 3.50 (3H, s), 3.51-3.77 (5H, m), 4.07-4.17 (2H, m), 4.22-4.32 (2H, m), 6.09 (1H, s), 6.41 (1H, d, J = 2.4 Hz), 7.12-7.45 (7H, m), 7.55 (1H, dd, J = 2.5, 8.7 Hz), 7.77 (1H, d, J = 8.9 Hz), 9.12 (2H, brs), 10.27 (1H, brs).

### Example 84

### trans-1-{2-[7-Chloro-1-(2,2-dimethylpropyl)-5-(2-methoxy-3-{3-[(3-phenylpropyl)amino]propoxy}phenyl)-2-oxo-1,2,3,5-tetrahydro-4,1-benzoxazepin-3-yl]acetyl}piperidin-4-yl acetate hydrochloride

To a dimethylformamide (6 ml) solution of the compound obtained in Example 38 (200 mg), 1-ethyl-3-(3-dimethylaminopropyl)carbodiimide hydrochloride (32 mg), and 1-hdyroxybenzotriazole (22 mg) was added 4-acetoxypiperidine hydrochloride (60 mg), and the mixture was stirred at 50°C for 15 hours. The reaction mixture was poured into water, and extracted with ethyl acetate. The extract was washed successively with water, and an aqueous saturated sodium chloride solution, dried over anhydrous magnesium sulfate, and concentrated under reduced pressure. The residue was purified by silica gel chromatography to obtain a colorless oil (216 mg). To this oil (100 mg) was added a 4N hydrochloric acid/ethyl acetate solution (5 ml), and the mixture was stirred at room temperature for 6 hours. The reaction mixture was concentrated under reduced pressure, and the resulting residue was crystallized from methylene chloride-diethyl ether to obtain the title compound (80 mg) as colorless crystals.
¹H-NMR (DMSO-d₆) δ: 0.87 (9H, s), 1.26-1.60 (2H, m), 1.68-1.95 (4H, m), 2.01 (3H, d, J = 5.6 Hz), 2.04-2.16 (2H, m), 2.58-2.77 (3H, m), 2.84-3.22 (7H, m), 3.52 (3H, s), 3.61 (1H, d, J = 13.9 Hz), 3.62-3.88 (2H, m), 4.08-4.20 (2H, m), 4.22-4.32 (2H, m), 4.79-4.91 (1H, m), 6.09 (1H, s), 6.41 (1H, d, J = 2.5 Hz), 7.11-7.33 (8H, m), 7.54 (1H, dd, J = 2.5, 8.7 Hz), 7.76 (1H, d, J = 8.9 Hz), 8.58 (2H, brs).

### Example 85

### trans-7-Chloro-1-(2,2-dimethylpropyl)-3-[2-(4-hydroxypiperidin-1-yl)-2-oxoethyl]-5-(2-methoxy-3-{3-[(3-phenylpropyl)amino]propoxy}phenyl)-1,5-dihydro-4,1-benzoxazepine-2(3H)-one hydrochloride

To an ethanol/tetrahydrofuran (2.5 ml/2.5 ml) solution of the compound obtained in Example 84 (60 mg) was added a 1N aqueous sodium hydroxide solution (0.5 ml), and the mixture was heated at 60°C for 1 hour. The reaction mixture was concentrated under reduced pressure, and water was added, this mixture was then extracted with methylene chloride. The extract was washed with an aqueous saturated sodium chloride solution, dried over anhydrous magnesium sulfate, and concentrated under reduced pressure. The residue was purified by silica gel chromatography to obtain a colorless oil. To a methylene chloride (3 ml) solution of this oil was added 1N etheric hydrochloric acid (0.5 ml), and the mixture was stirred at room temperature for 5 hours. The reaction mixture was concentrated under reduced pressure, and the resulting residue was crystallized from methylene chloride-diethyl ether to obtain the title compound (30 mg) as colorless crystals.
¹H-NMR (DMSO-d₆) δ: 0.87 (9H, s), 1.06-1.40 (2H, m), 1.58-1.78 (2H, m), 1.80-1.96 (2H, m), 2.01-2.17 (2H, m), 2.58-3.21 (10H, m), 3.51 (3H, s), 3.58-3.90 (4H, m), 4.07-4.18 (2H, m), 4.20-4.31 (2H, m), 4.73 (1H, t, J = 4.2 Hz), 6.08 (1H, s), 6.41 (1H, d, J = 2.4 Hz), 7.10-7.35 (8H, m), 7.54 (1H, dd, J = 2.6, 8.7 Hz), 7.76 (1H, d, J = 8.7 Hz), 8.45 (2H, brs).

### Example 86

### 4-{[3,5-trans-7-Chloro-1-(2,2-dimethylpropyl)-5-(2-methoxy-3-{3-[(3-phenylpropyl)amino]propoxy}phenyl)-2-oxo-1,2,3,5-tetrahydro-4,1-benzoxazepin-3-yl]acetyl}piperazine-2-carboxylic acid dihydrochloride

To a dimethylformamide (3 ml) solution of the compound obtained in Example 38 (100 mg), 1-ethyl-3-(3-dimethylaminopropyl)carbodiimide hydrochloride (32 mg), 1-hydroxybenzotriazole (22 mg), and triethylamine (56 mg) was added 2-piperazinecarboxylic acid ethyl ester dihydrochloride (38 mg), and the mixture was stirred at room temperature for 15 hours. The reaction mixture was poured into water, and extracted with ethyl acetate. The extract was washed successively with water and an aqueous saturated sodium chloride solution, dried over anhydrous magnesium sulfate, and concentrated under reduced pressure. The residue was purified by silica gel chromatography to obtain a colorless oil (91 mg). To a tetrahydrofuran (3 ml) solution of this oil (91 mg) were added di(tert-butyl) dicarbonate (28 mg) and triethylamine (21 mg), and the mixture was stirred at room temperature for 4 hours. The reaction mixture was concentrated under reduced pressure, and the resulting residue was purified by silica gel chromatography to obtain a colorless oil (92 mg). To a tetrahydrofuran/ethanol (2 ml/2 ml) solution of this oil (92 mg) was added a 1N aqueous sodium hydroxide solution (0.4 ml), and the mixture was heated at 60°C for 1 hour. The reaction mixture was concentrated under reduced pressure, a 10% aqueous citric acid solution was added, and this was extracted with ethyl acetate. The extract was washed successively with water and an aqueous saturated sodium chloride solution, dried over anhydrous magnesium sulfate, and concentrated under reduced pressure to obtain a colorless oil (80 mg). To this oil (80 mg) was added a 4N hydrochloric acid/ethyl acetate solution (5 ml), and this mixture was stirred at room temperature for 15 hours. The reaction mixture was concentrated under reduced pressure, and the resulting residue was crystallized from methylene chloride-diethyl ether to obtain the title compound (70 mg) as colorless crystals.
¹H-NMR (DMSO-d₆) δ: 0.87 (9H, s), 1.82-1.98 (2H, m), 2.02-2.20 (2H, m), 2.58-2.67 (2H, m), 2.70-4.48 (21H, m), 6.10 (1H, s), 6.42 (1H, s), 7.07-7.33 (8H, m), 7.55 (1H, dd, J = 2.3, 8.8 Hz), 7.77 (1H, d, J = 9.0 Hz), 8.86 (2H, brs), 9.57 (1H, brs).

### Example 87

### {3-[(3-{7-Chloro-1-(2,2-dimethylpropyl)-3-[(2-fluorobenzylcarbamoyl)methyl]-2-oxo-1,2,3,5-tetrahydro-1,4-benzoxazepin-5-yl}-benzyl) (trifluoroacetyl)amino]propyl}carbamic acid tert-butyl ester

To a dichloromethane solution (10 ml) of the compound of Reference Example 12I (0.79 g) were added triethylamine (0.24 ml) and trifluoroacetic anhydride (0.24 ml) under ice-cooling, and the mixture was stirred. The reaction mixture was partitioned between chloroform and water, and the organic layer was washed with water, dried over anhydrous magnesium sulfate, and concentrated under reduced pressure. The residue was purified by silica gel chromatography to obtain the title compound (0.69 g) as a colorless oil.
¹H-NMR (CDCl₃) δ: 0.93 (9H, s), 1.28 (9H, s), 1.72-1.76 (2H, m), 2.66-2.91 (4H, m), 3.19-3.22 (2H, m), 3.35 (1H, d, J = 14 Hz), 3.80 (2H, s), 4.40-4.51 (4H, m), 5.99 (1H, s), 6.55 (1H, s), 6.59 (1H, d, J = 2.2 Hz), 6.98-7.07 (2H, m), 7.20-7.61 (10H, m).

### Example 88

### 2-[5-{3-[(Benzylaminopropylamino)methyl]phenyl}-7-chloro-1-(2,2-dimethylpropyl)-2-oxo-1,2,3,5-tetrahydro-1,4-benzoxazepin-3-yl]-N-(2-fluorobenzyl)acetamide dihydrochloride

The compound obtained in Example 87 (0.31 g), ethyl acetate (3 ml) and a 4N hydrochloric acid/ethyl acetate solution (3 ml) were added, and the mixture was stirred at room temperature for 1.5 hours. After the solvent was distilled off, the residue was dissolved in methanol (3 ml), benzaldehyde (44 mg) and sodium cyanotrihydroborate (27 mg) were added, and the mixture was stirred for 24 hours. The residue was partitioned between ethyl acetate and water, and the organic layer was washed with water, dried over anhydrous magnesium sulfate, and concentrated under reduced pressure. The residue was purified by silica gel chromatography, and the resulting residue was dissolved in THF (2 ml), methanol (2 ml) and water (1 ml). To this was added lithium hydroxide (80 mg), the mixture was stirred overnight, and this was diluted with ethyl acetate, and washed with an aqueous sodium chloride solution. The organic layer was dried over anhydrous magnesium sulfate, and the solvent was distilled off. To the residue was added a 4N hydrochloric acid/ethyl acetate solution (2 ml), the mixture was stirred at room temperature for 10 minutes, and the solvent was distilled off. The residue was washed with petroleum ether and isopropyl ether to obtain the title compound (0.03 g) as a white powder.
¹H-NMR (DMSO-d₆) δ: 0.87 (9H, s), 2.60 (6H, s), 2.84 (2H, brs), 3.33 (1H, d, J = 14 Hz), 4.00-4.09 (4H, m), 4.37-4.59 (4H, m), 5.99 (1H, s), 6.47 (1H, d, J = 2.2 Hz), 6.95-7.00 (2H, m), 7.14-7.65 (14H, m), 10.34 (4H, brs).

### Example 89

### 2-(7-Chloro-1-(2,2-dimethylpropyl)-2-oxo-5-{3-[(3-phenethylaminopropylamino)methyl]-phenyl}-1,2,3,5-tetrahydro-1,4-benzoxazepin-3-yl)-N-(2-fluorobenzyl)acetamide dihydrochloride

From the compound obtained in Example 87 (0.34 g) and phenylacetoaldehyde 56 mg) and according to the same manner as that of Example 88, the title compound (0.02 g) was obtained as a white powder.
¹H-NMR (DMSO-d₆) δ: 0.88 (9H, s), 2.03-2.08 (2H, m), 2.58-2.65 (1H, m), 2.71-2.78 (1H, m), 2.93-3.13 (10H, m), 3.61 (1H, d, J = 14 Hz), 4.18-4.38 (4H, m), 5.89 (1H, s), 6.41 (1H, d, J = 2.4 Hz), 7.10-7.19 (2H, m), 7.25-7.39 (8H, m), 7.47-7.66 (4H, m), 7.77 (2H, d, J = 8.8 Hz), 8.48 (1H, t, J = 5.6 Hz), 9.01 (2H, brs), 9.22 (2H, brs).

### Example 90

### 2-(7-Chloro-1-(2,2-dimethylpropyl)-2-oxo-5-{3-[(3-phenylpropylaminopropylamino)methyl]-phenyl}-1,2,3,5-tetrahydro-1,4-benzoxazepin-3-yl)-N-(2-fluorobenzyl)acetamide dihydrochloride

From the compound obtained in Example 8 (0.34 g) and 3-phenylpropanal (99 mg) and according to the same manner as that of Example 88, the title compound (0.09 g) was obtained as a white powder.
¹H-NMR (CDCl₃, free form) δ: 0.90 (9H, s), 1.63 (1H, brs), 1.94-2.11 (4H, m), 2.65-2.92 (6H, m), 3.07-3.11 (1H, m), 3.33 (1H, d, J = 14 Hz), 3.46 (1H, t, J = 7.3 Hz), 4.07 (2H, t, J = 6.0Hz), 4.38-4.60 (4H, m), 5.96 (1H, s), 6.29 (1H, brs), 6.63 (1H, d, J = 2.2 Hz), 6.84-6.92 (3H, m), 6.98-7.08 (2H, m), 7.17-7.33 (10H, m).

### Example 91

### [2-(3-{7-Chloro-1-(2,2-dimethylpropyl)-3-[(2-fluorobenzylcarbamoyl)methyl]-2-oxo-1,2,3,5-tetrahydro-1,4-benzoxazepin-5-yl}benzylamino)ethyl]-(3-phenylpropyl)carbamic acid tert-butyl ester

1) (2-Oxoethyl)-(3-phenylpropyl)carbamic acid tert-butyl ester
   To a methanol solution (50 mg) of 3-phenylpropionaldehyde (6.71 g) were added acetic acid (1 ml), 2-aminoethanol (3.05 g) and sodium cyanotrihydroborate (6.28 g) and the mixture was stirred at room temperature overnight. Water (10 ml) was added, and this was diluted with ethyl acetate, and washed with an aqueous sodium chloride solution. The organic layer was dried over anhydrous magnesium sulfate, the solvent was distilled off, and the residue was purified by silica gel column chromatography. The residue was dissolved in THF (100 ml), triethylamine (1.49 ml) and di(tert-butyl) dicarbonate (2.14 g) were added under ice-cooling, the mixture was stirred for 3 hours, and the solvent was distilled off. The residue was partitioned between ethyl acetate and water, and the organic layer was dried over anhydrous magnesium sulfate. After the solvent was distilled off, the residue was dissolved in dichloromethane (200 ml), sodium acetate (2.29 g) and PCC (5.98 g) were added, and the mixture was stirred at room temperature for 1 hour. Diethyl ether (150 ml) and anhydrous magnesium sulfate (10 g) were added, and the mixture was stirred for 10 minutes, filtered, and washed with ethyl acetate. The filtrate and the washing solution were combined, washed with water, and dried over anhydrous magnesium sulfate. The solvent was distilled off, followed by purification by silica gel column chromatography to obtain the title compound (1.12 g) as a colorless oil.
2) [2-(3-{7-Chloro-1-(2,2-dimethylpropyl)-3-[(2-fluorobenzylcarbamoyl)methyl]-2-oxo-1,2,3,5-tetrahydro-1,4-benzoxazepin-5-yl}benzylamino)ethyl]-(3-phenylpropyl)carbamic acid tert-butyl ester

To a methanol solution (5 ml) of the compound described in Example 6 of JP 11-209356 A (0.32 g) were added acetic acid (0.1 ml), (2-oxoethyl)-(3-phenylpropyl)carbamic acid tert-butyl ester obtained in Example 91-1) (0.17 g) and sodium cyanotrihydroborate (39 mg), and the mixture was stirred at room temperature overnight. This was diluted with ethyl acetate, washed with water, and dried over anhydrous magnesium sulfate. After the solvent was distilled off, the residue was purified by column chromatography to obtain the title compound (0.22 g) as a colorless oil.
¹H-NMR (CDCl₃) δ: 0.92 (9H, s), 1.42 (9H, s), 1.79-1.86 (2H, m), 2.55-2.61 (2H, m), 2.66-2.80 (3H, m), 2.85-2.92 (1H, m), 3.22-3.37 (5H, m), 3.80(2H, s), 4.39-4.51 (4H, m), 5.99 (1H, s), 6.31 (1H, brs), 6.58 (1H, d, J = 2.2 Hz), 7.01-7.14 (2H, m), 7.17-7.36 (14H, m).

### Example 92

### 2-[7-Chloro-1-(2,2-dimethylpropyl)-2-oxo-5-(3-{[2-(3-phenylpropylamino)ethylamino]methyl}phenyl)-1,2,3,5-tetrahydro-1,4-benzoxazepin-3-yl]-N-(2-fluorobenzyl)acetamide dihydrochloride

To an ethyl acetate (2 ml) solution of the compound obtained in Example 91-2) (0.21 g) was added a 4N hydrochloric acid/ethyl acetate solution (2 ml), and the mixture was stirred at room temperature for 1 hour. The reaction mixture was concentrated under reduced pressure, the residue was dissolved in ethanol, ethyl acetate and ether were then added, and the resulting precipitate was filtered to obtain the title compound (0.12 g) as a white powder.
¹H-NMR (CDCl₃, free) δ: 0.89 (9H, s), 1.53-1.92 (6H, m), 2.41-2.85 (10H, m), 3.34 (1H, d, J = 14 Hz), 3.46 (1H, d, J = 14 Hz), 3.67 (1H, d, J = 14 Hz), 4.38-4.49 (4H, m), 5.99 (1H, s), 5.59 (1H, d, J = 2.2Hz), 6.85 (1H, brs), 6.98 (2H, t, J = 8.5 Hz), 7.12-7.37 (13H, m).

### Example 93

### [2-(3-{7-Chloro-1-(2,2-dimethylpropyl)-3-[(2-fluorobenzylcarbamoyl)methyl]-2-oxo-1,2,3,5-tetrahydro-1,4-benzoxazepin-5-yl}benzylamino)ethyl]carbamic acid tert-butyl ester

To a methanol solution (10 ml) of the compound described in Example 41-(7) of JP 11-209356 A (0.50 g) were added acetic acid (0.5 ml), (2-aminoethyl)carbamic acid tert-butyl ester (0.16 g) and sodium cyanotrihydroborate (63 mg), and the mixture was stirred at room temperature overnight. The reaction mixture was diluted with ethyl acetate, washed with water, and dried over anhydrous magnesium sulfate. After the solvent was distilled off, the residue was purified by column chromatography to obtain the title compound (0.42 g) as a colorless oil.
¹H-NMR (CDCl₃) δ: 0.92 (9H, s), 1.27 (9H, s), 2.62-2.95 (2H, m), 2.91-3.33 (4H, m), 3.34 (1H, d, J = 14 Hz), 3.80 (2H, s), 4.39-4.51 (4H, m), 5.97 (1H, s), 6.53 (1H, s), 6.60 (1H, d, J = 2.2Hz), 6.96-7.07 (2H, m), 7.21-7.61 (10H, m).

### Reference Example 27

### 2-(7-Chloro-1-(2,2-dimethylpropyl)-2-oxo-5-{3-[(6-phenylhexylamino)methyl]phenyl}-1,2,3,5-tetrahydro-1,4-benzoxazepin-3-yl)-N-(2-fluorobenzyl)acetamide hydrochloride

To a methanol solution (3 ml) of the compound described in Example 6 of JP 11-209356 A (0.12 g) were added acetic acid (0.1 ml), 6-phenylhexanal (37 mg) and sodium cyanotrihydroborate (17 mg), and the mixture was stirred at room temperature overnight. The reaction mixture was diluted with ethyl acetate, washed with water, and dried over anhydrous magnesium sulfate. After the solvent was distilled off, the residue was purified by column chromatography. To the residue was added a 4N hydrochloric acid/ethyl acetate solution (2 ml), and the mixture was stirred at room temperature for 1 hour. The reaction mixture was concentrated under reduced pressure, the residue was dissolved in ethanol, ethyl acetate and ether were then added, and the resulting precipitate was filtered to obtain the title compound (0.08 g) as a white powder.
¹H-NMR (CDCl₃) δ: 0.88 (9H, s), 1.11-1.14 (4H, m), 1.50-1. 57 (2H, m), 1.70-1.81 (2H, m), 2.54 (2H, t, J = 7.9 Hz), 2.65-2.90 (4H, m), 3.34 (1H, d, J = 14 Hz), 3.67-3.80 (2H, m), 4.38-4.60 (4H, m), 5.97 (1H, s), 6.45 (1H, s), 6.84 (1H, brs), 6.98-7.05 (2H, m), 7.11-7.37 (10H, m), 7.41-7.49 (2H, m), 7.69 (1H, d, J = 7.7 Hz), 9.86 (2H, brs).

### Reference Example 28

### [5-(2-Benzyloxyphenyl)-7-chloro-1-(2,2-dimethylpropyl)-2-oxo-1,2,3,5-tetrahydro-1,4-benzoxazepin-3-yl]acetic acid ethyl ester

From (2-benzyloxyphenyl)-[5-chloro-2-(2,2-dimethylpropylamino)phenyl]methanol and according to the same manner as that of Reference Example 7, the title compound (1.64 g) was obtained as a white powder.
¹H-NMR (CDCl₃) δ: 0.76 (9H, s), 1.25 (3H, t, J = 7.1 Hz), 1.54 (1H, s), 2.76-2.81(1H, m), 3.01-3.06(1H, m), 3.30 (1H, d, J = 14 Hz), 4.09-4.17 (2H, m), 4.39-4.48 (2H, m), 4.97 (2H, s), 6.33 (1H, s), 6.68 (1H, d, J = 2.2 Hz), 6.83 (1H, d, J = 8.1 Hz), 7.04-7.09 (3H, m), 7.22-7.39 (5H, m), 7.61 (1H, J = 1,4 Hz, 7.5 Hz).

### Reference Example 29

### [5-(2-Benzyloxyphenyl)-7-chloro-1-(2,2-dimethylpropyl)-2-oxo-1,2,3,5-tetrahydro-1,4-benzoxazepin-3-yl]acetic acid

From the compound obtained in Reference Example 28, and according to the same manner as that of Reference Example 8, the title compound (2.73 g) was obtained as a white powder.
¹H-NMR (CDCl₃) δ: 0.77 (9H, s), 2.84-2.89 (1H, m), 3.04-3.09 (1H, m), 4.35 (1H, d, J = 7.3 Hz), 4.46 (1H, d, J = 14Hz), 4.97 (2H, s), 6.32 (1H, s), 6.70 (1H, d, J = 2.3 Hz), 6.83 (1H, d, J = 8.2 Hz), 7.05-7.10 (3H, m), 7.23-7.33 (5H, m), 7.37-7.40 (1H, m), 7.62 (1H, d, J = 8.7 Hz), 10.33 (1H, brs).

### Reference Example 30

### 2-[5-(2-Benzyloxyphenyl)-7-chloro-1-(2,2-dimethylpropyl)-2-oxo-1,2,3,5-tetrahydro-1,4-benzoxazepin-3-yl]-N-(2-fluorobenzyl)acetamide

From the compound obtained in Reference Example 29 and according to the same manner as that of Reference Example 9, the title compound (2.46 g) was obtained as a white powder.
¹H-NMR (CDCl₃) δ: 0.76 (9H, s), 2.04 (1H, s), 2.68-2.73 (1H, m), 2.85-2.90 (1H, m), 3.29 (1H, d, J = 14 Hz), 4.39-4.46 (3H, m), 4.51-4.56 (1H, m), 4.96 (2H, s), 6.31 (2H, s), 6.66 (1H, d, J = 2.2 Hz), 6.83 (1H, d, J = 8.1 Hz), 7.21-7.38 (8H, m), 7.53 (1H, d, J = 7.5 Hz).

### Reference Example 31

### 2-[7-Chloro-1-(2,2-dimethylpropyl)-5-(2-hydroxyphenyl)-2-oxo-1,2,3,5-tetrahydro-1,4-benzoxazepin-3-yl]-N-(2-fluorobenzyl)acetamide

From the compound obtained in Reference Example 30 and according to the same manner as that of Reference Example 10, the title compound 0.30 g was obtained as a white powder.
¹H-NMR (CDCl₃) δ: 0.91 (9H, s), 2.06 (1H, d, J = 16 Hz), 2.73-2.84 (2H, m), 3.14 (1H, d, J = 14 Hz), 4.44-4.58 (4H, m), 6.03 (1H, t, J = 5.9 Hz), 6.09 (1H, s), 6.83 (1H, d, J = 2.3 Hz), 6.90-7.05 (5H, m), 7.25-7.36 (5H, m).

### Reference Example 32

### [7-Chloro-1-(2,2-dimethylpropyl)-5-(2-hydroxyphenyl)-2-oxo-1,2,3,5-tetrahydro-1,4-benzoxazepin-3-yl]acetic acid ethyl ester

From the compound obtained in Reference Example 28 and according to the same manner as that of Reference Example 10, the title compound (1.92 g) was obtained as a white powder.
¹H-NMR (CDCl₃) δ: 0.92 (9H, s), 1.24 (3H, t, J = 7.1 Hz), 1.54 (1H, s), 2.81-2.99 (2H, m), 3.35 (1H, d, J = 14 Hz), 4.11-4.19 (2H, m), 4.45-4.53 (2H, m), 6.11 (1H, s), 6.85 (1H, d, J = 2.2 Hz), 6.88-6.96 (2H, m), 7.03 (1H, d, J = 8.0 Hz), 7.30-7.40 (3H, m), 7.47 (1H, brs).

### Example 94

### 2-(7-Chloro-1-(2,2-dimethylpropyl)-2-oxo-5-(2-[3-(3-phenylpropylamino)propoxy]phenyl}-1,2,3,5-tetrahydro-1,4-oxazepin-3-yl)-N-(2-fluorobenzyl)acetamide hydrochloride

From the compound obtained in Reference Example 31 and according to the same manners as those of Examples 1 to 3, the title compound (0.11 g) was obtained as a white powder. ¹H-NMR (CDCl₃, free) δ : 0.92 (9H, s), 1.61 (1H, brs), 1.91-2,10 (4H, m), 2.65-2.90 (6H, m), 3.05- 3.12 (1H, m), 3.32 (1H, d, J = 14 Hz), 3.45 (1H, t, J = 7.3 Hz), 4.07 (2H, t, J = 6.0 Hz), 4.31-4.53(4H, m), 5.92 (1H, s), 6.30 (1H, brs), 6.64 (1H, d, J = 2.2 Hz), 6.94-6.95 (3H, m), 6.99-7.06 (2H, m), 7.11-7.36 (10H, m).

### Example 95

### {5-[2-(3-tert-Butoxycarbonylaminopropoxy)phenyl]-7-chloro-1-(2,2-dimethylpropyl)-2-oxo-1,2,3,5-tetrahydro-1,4-benzoxazepin-3-yl}acetic acid ethyl ester

From the compound obtained in Reference Example 32 and according to the same manner as that of Example 1, the title compound (2.14 g) was obtained as a white powder.
¹H-NMR (CDCl₃) δ: 0.93 (9H, s), 1.22 (3H, t, J = 7.1 Hz), 1.41 (9H, s), 1.68-1.85 (2H, m), 2.75-3.06 (4H, m), 3.36 (1H, d, J = 14 Hz), 3.94 (2H, d, J = 6.5 Hz), 4.09-4.16 (2H, m), 4.15 (1H, t, J = 6.1 Hz), 4.49 (1H, d, J = 14 Hz), 6.23 (1H, s), 6.63 (1H, s), 6.89 (1H, d, J = 7.9 Hz), 7.06-7.11 (1H, m), 7.30-7.38 (3H, m), 7.60 (1H, d, J = 7.9 Hz).

### Example 96

### [7-Chloro-1-(2,2-dimethylpropyl)-2-oxo-5-{2-[3-(3-phenylpropylamino)propoxy]phenyl}-1,2,3,5-tetrahydro-1,4-benzoxazepin-3-yl]acetic acid ethyl ester hydrochloride

From the compound obtained in Example 95 and according to the same manners as those of Examples 2 and 3, the title compound (1.68 g) was obtained as a white powder.
¹H-NMR (DMSO-d6) δ: 0.86 (9H, s), 1.50 (3H, t, J = 7.1 Hz), 1.81-1.91 (4H, m), 2.60-2.81 (7H, m), 3.33 (1H, s), 3.60 (1H, d, J = 14 Hz), 4.00-4.06 (4H, m), 4.24-4.30 (2H, m), 6.10 (1H, s), 6.42 (1H, d, J = 2.2 Hz), 7.10-7.15 (2H, m), 7.19-7.23 (3H, m), 7.28-7.33 (2H, m), 7.41-7.47 (1H, m), 7.51-7.55 (1H, m), 7.75 (1H, d, J = 8.8 Hz), 8.78 (2H, brs).

### Example 97

### 2-(7-Chloro-1-(2,2-dimethylpropyl)-2-oxo-5-{2-[3-(3-phenylpropylamino)propoxy]phenyl}-1,2,3,5-tetrahydro-1,4-oxazepin-3-yl)-(1-piperazinyl)acetamide dihydrochloride

From the compound obtained in Example 96 and according to the same manner as those of Examples 38 and 41, the title compound (0.18 g) was obtained as a white powder.
¹H-NMR (DMSO-d₆): 0.91 (9H, s), 1.59-1.66 (7H, m), 2.07-2.22 (4H, m), 2.67-3.34 (8H, m), 3.64-4.16 (4H, m), 4.41-4.48 (2H, m), 6.13 (1H, s), 6.63 (1H, s), 6.99-7.60 (11H, m), 9.45 (1H, brs), 9.61 (1H, brs), 9.94 (2H, brs).

### Example 98

### 2-(7-Chloro-1-(2,2-dimethylpropyl)-2-oxo-5-{3-[3-(3-phenylpropylamino)propoxy]phenyl)-1,2,3,5-tetrahydro-1,4-benzoxazepin-3-yl)-N-(2-fluorobenzyl)acetamide hydrochloride

From 2-(7-chloro-1-(2,2-dimethylpropyl)-5-(3-hydroxyphenyl)-2-oxo-1,2,3,5-tetrahydro-1,4-benzoxazepin-3-yl)-N-(2-fluorobenzyl)acetamide and according to the same manner as those of Examples 1 to 3, the title compound (0.08 g) was obtained as a white powder.
¹H-NMR (CDCl₃, free) δ: 0.90 (9H, s), 1. 63 (1H, brs), 1.94-2.11 (4H, m), 2.65-2.92 (6H, m), 3.07-3.11 (1H, m), 3.33 (1H, d, J = 14 Hz), 3.46 (1H, t, J = 7.3 Hz), 4.07 (2H, t, J = 6.0 Hz), 4.38-4.60 (4H, m), 5.96 (1H,s), 6.29 (1H, brs), 6.63 (1H, d, J = 2.2 Hz), 6.84-6.92 (3H, m), 6.98-7.08 (2H, m), 7.17-7.33 (10H, m).

### Preparation Example 1

An RFRP receptor function modulating agent containing the compound represented by the formula (I) in the present invention or a salt thereof as an active ingredient can be prepared, for example, by the following formulation.
1. Capsule

| | | |
|---|---|---|
| (1) | Compound obtained in Example 3 | 40 mg |
| (2) | Lactose | 70 mg |
| (3) | Microcrystalline cellulose | 9 mg |
| (4) | Magnesium stearate | 1 mg |
| | one capsule | 120 mg |

| | | |
|---|---|---|
| (1), (2) and (3), and 1/2 of (4) are kneaded, and this is granulated. To this is added the remaining (4), and a whole is encapsulated into a gelatin capsule. | | |

2. Capsule

| | | |
|---|---|---|
| (1) | Compound obtained in Example 50 | 40 mg |
| (2) | Lactose | 70 mg |
| (3) | Microcrystalline cellulose | 9 mg |
| (4) | Magnesium stearate | 1 mg |
| | one capsule | 120 mg |

| | | |
|---|---|---|
| (1), (2) and (3), and 1/2 of (4) are kneaded, and this is granulated. To this is added the remaining (4), and a whole is encapsulated into a gelatin capsule. | | |

3. Tablet

| | | |
|---|---|---|
| (1) | Compound obtained in Example 3 | 40 mg |
| (2) | Lactose | 58 mg |
| (3) | Corn starch | 18 mg |
| (4) | Microcrystalline cellulose | 3.5 mg |
| (5) | Magnesium stearate | 0.5 mg |
| | one tablet | 120 mg |

| | | |
|---|---|---|
| (1), (2), (3), 2/3 of (4), and 1/2 of (5) are kneaded, and this is granulated. The remaining (4) and (5) are added to this granule, and this is pressure-molded into a tablet. | | |

4. Tablet

| | | |
|---|---|---|
| (1) | Compound obtained in Example 50 | 40 mg |
| (2) | Lactose | 58 mg |
| (3) | Corn starch | 18 mg |
| (4) | Microcrystalline cellulose | 3.5 mg |
| (5) | Magnesium stearate | 0.5 mg |
| | one tablet | 120 mg |

| | | |
|---|---|---|
| (1), (2), (3), 2/3 of (4), and 1/2 of (5) are kneaded, and this is granulated. The remaining (4) and (5) are added to this granule, and this is pressure-molded into a tablet. | | |

### Preparation Example 2

(1) After 50 mg of the compound obtained in Example 3 is dissolved in 50 ml of Japanese Pharmacopoeia distilled water for injection, Japanese Pharmacopoeia distilled water for injection is added to 100 ml. This solution is filtered under the sterilization condition, then, each 1 ml of this solution is taken, filled into a vial for injection under the sterilization condition, and this is lyophilized, and sealed.
(2) After 50 mg of the compound obtained in Example 3 is dissolved in 50 ml of Japanese Pharmacopoeia distilled water for injection, Japanese Pharmacopoeia distilled water for injection is added to 100 ml. This solution is filtered under the sterilization condition, then, each 1 ml of this solution is taken, filled into a vial for injection under the sterilization condition, and this is lyophilized, and sealed.

### Test Example 1

### Assessment of binding inhibiting activity of test compound using human-type OT7T022-expressing CHO cell

### (1) Preparation of an iodine-labeled form of human-type RFRP-3 (Y-RFRP-3)

Twenty µm of a peptide (Y-RFRP-3) (sequence: Tyr-Val-Pro-Asn-Leu-Pro-Gln-Arg-Phe-amide) (0.1 mM) in which a Tyr residue had been added to a N-terminus of hRFRP-3-8 (sequence: Val-Pro-Asn-Leu-Pro-Gln-Arg-Phe-amide) having binding inhibiting activity equivalent to that of endogenous human-type RFRP-3 (hRFRP-3-28) for human-type OT7T022-expressing CHO cell and 10 µl of distilled water were mixed, 20 µl of a lactoperoxidase solution (Sigma, prepared using 0.1 M HEPES-NaOH, pH 7.0 in 10 µg/mL), 10 µl of Idoine-125 (Amersham, IMS-30, 74 MBq), and 20 µl of 0.005% hydrogen peroxide (Wako Pure Chemical Industries, Ltd.) were successively mixed therein, the mixture was allowed to stand at room temperature for 10 minutes, 600 µl of 0.1% TFA-water was added, this was separated by reverse phase HPLC, a peak of the labeled form was collected, an equivalent amount of a buffer for a binding test (50 mM TrisHCl (pH 7.5), 0.1% BSA, 5 mM EDTA, 0.5 mM PMSF, 20 µg/mL leupeptin, 0.1 µg/mL pepstatin A, 4 µ/mL E-64) was added, and this was immediately stored on ice. A part thereof was 1/100 diluted, radioactivity was measured with a γ-counter, and the remaining authentic product was distributed, which was stored at -30°C.

### (2) Assessment of binding inhibiting activity

To a 96-well microplate were added 1 µg of a membrane fraction, a compound and Y-RFRP-3 labeled with ¹²⁵I, which had been diluted with a reaction buffer (50 mM Tris-HCl, 5 mM EDTA, 0.1% BSA, 0.5 mM PMSF, 20 µg/ml leupeptin, 0.1 µg/ml pepstatin A, 4 µg/ml E-64, 10 mM MgCl₂, pH 7.5), to 100pM to react them at room temperature for 1.5 hours. For measuring non-specific binding, non-labeled Y-RFRP-3 was further added to 100 pM. Then, a membrane fraction was transferred to a unifilter GF/C (Perkin Elmer) by filtering the reaction mixture using a cell harvester (Perkin Elmer), and this was washed with a cooled 50 mM Tris buffer (pH 7.5) five times. The filter was dried, Microsinti (Packard) was added to the filter, and radioactivity was measured with Topcount (Packard).

A binding inhibiting rate (IC₅₀ value) of a test compound is shown in [Table 1].

**[Table 1]**

| Test compound | IC₅₀ value |
|---|---|
| Compound of Example 3 | <1 µM |
| Compound Example 4 | <1 µM |
| Compound Example 14 | <1 µM |
| Compound Example 18 | <1 µM |
| Compound Example 46 | <1 µM |
| Compound Example 50 | <1 µM |
| Compound Example 85 | <1 µM |
| Compound Example 86 | <1 µM |

From this, it is seen that the compound (I) of the present invention has the excellent RFRP receptor binding activity.

### Test Example 2

### Test of antagonist activity of compound in cAMP production inhibiting test system using human-type OT7T022-expressing CHO cell

Antagonist activity of a test compound was measured in an intracellular cAMP production inhibiting test system of a CHO cell expressing human-type OT7T022. In the cAMP production inhibiting test, as an assay buffer, a Hanks' balanced salt solution (Gibco) to which 20 mM HEPES pH 7.4, 0.1% bovine serum albumin, and 0.2 mM 3-isobutyl-1-methylxanthine (Sigma) had been added, was used. A sample compound was adjusted with an assay buffer so that the final concentration became 10⁻⁵M, 10⁻⁶M, 10⁻⁷M, 10⁻⁸M, or 10⁻¹⁰M. An agonist: human-type RFRP-3-8 (Val-Pro-Asn-Leu-Pro-Gln-Arg-Phe-amide) was diluted with an assay buffer obtained by adding 4 µM (final concentration 2 µM) forscoline to 40 nM (final concentration 20 nM). A human-type OT7T022-expressing CHO cell was passaged on a 96-well plate at 4×10⁴/well, and cultured at 37°C, 5% CO₂ and 95% air for one day. The plate which had been cultured for one day was washed with an assay buffer (150 µl) two times and, after 30 minutes, this was cultured for 30 minutes at 37°C and 100% air. After washed with an assay buffer (150 µl) two times, 50 µl of a sample compound solution and, then, 50 µl of an agonist + forskolin solution were added, the mixture was stirred well and, after 30 minutes, this was cultured for 30 minutes at 37°C and 100% air. An intracellular cAMP amount was measured according to a protocol of the present kit using cAMP-Screen^{TM} system (ABI) .

The antagonist activity of the test compound is shown in [Table 2].

**[Table 2]**

| Test compound | IC₅₀ value |
|---|---|
| Compound of Example 3 | <1 µM |

From this, it is seen that the compound (I) of the present invention has the excellent RFRP receptor antagonism.

### Test Example 3

### Assessment of binding inhibiting activity of test compound using rat-type OT7T022-expressing CHO cell

### (1) Preparation of rat-type OT7T022-expressing CHO cell membrane fraction

A flask in which a rat-type OT7T022-expressing CHO cell had been cultured was washed with 5 mM EDTA/PBS, cells were peeled with 5 mM EDTA/PBS, and cells were recovered by centrifugation, suspended in 25 mL of a buffer for preparing a membrane fraction (50 mM Tris-HCl, pH 7.5, 5 mM EDTA, 0.5 mM PMSF (manufactured by Wako Pure Chemical Industries, Ltd.), 20 µg/ µL leupeptin (manufactured by Peptide Laboratory), 0.1 µg/mL pepstatin A (manufactured by Peptide Laboratory), 4 µg/mL E-64 (manufactured by Peptide Laboratory)), and homogenized on an ice using Polytron (12,000 rpm, 15 seconds × 3 times). This was centrifuged in a high speed cooling centrifuge at 4°C and 1,000 g for 10 minutes, and the supernatant was recovered. 25 mL of a buffer for preparing a membrane fraction was added to precipitates, and the supernatant was recovered by the same procedure. These supernatants were combined, subjected to a cell strainer, dispensed into tubes for a supercentrifuge, and centrifuged at 4°C and 100,000 g for 1 hour. The pellet was suspended in a small amount of a buffer for preparing a membrane fraction, this was suspended using a Teflon (registered trade mark) homogenizer, a protein amount was measured using a part, and the remaining was dispensed, and stored at -80°C.

### (2) Test of binding inhibition of a sample compound for rat-type OT7T022-expressing CHO cell membrane fraction

Using an assay buffer (50 mM Tris-HCl, pH 7.5, 5 mM EDTA, 0.5 mM PMSF, 20 µg/mL leupeptin, 0.1 µg/mL pepstatin A, 4 µg/mL E-64, 0.1% bovine serum albumin, 10 mM MgCl₂), a membrane fraction of a rat-type OT7T022-expressing CHO cell was diluted to the final concentration of 0.75 µg/well, and iodine-labeled Y-RFRP-3 was diluted to the final concentration of 100 pM. Regarding a sample compound, a 10⁻²M or 10⁻³M stock solution was diluted with an assay buffer so that the final concentration became 10⁻⁵M, 10⁻⁶M, 10⁻⁷M, 10⁻⁸M, 10⁻⁹M, 10⁻¹⁰M or 10⁻¹¹M. For non-specific binding, hRFRP-3-8 having the final concentration of 10⁻⁵M was prepared. Using a polypropylene 96-well plate, a prepared sample solution, a non-specific binding solution, and 50 µl of an assay buffer for total binding were dispensed, 25 µl of an iodine-labeled form diluent was added, the mixture was stirred, 25 µl of a rat-type OT7T022-expressing CHO cell membrane fraction solution was dispensed, and this was stirred, and incubated at room temperature for 1.5 hours. This was transferred to a unifilter (Perkin Elmer) pre-wetted with a washing buffer (50 mM Tris-HCl, pH 7.5) using a harvester for a 96-well plate (Packard), and this was washed with a washing buffer six times, and sufficiently dried. 50 µl of Microscinti O (Packard) was dispensed, radioactivity was measured with Top Count (Packard), and data were analyzed in a triplicate manner. Binding inhibiting activity (IC₅₀ value) of a test compound for rat-type OT7T022 is shown in [Table 3].

**[Table 3]**

| Test compound | IC₅₀ value |
|---|---|
| Compound of Example 3 | <1 µM |
| Compound of Example 4 | <1 µM |
| Compound of Example 14 | <1 µM |
| Compound of Example 18 | <1 µM |
| Compound of Example 46 | <1 µM |
| Compound of Example 50 | <1 µM |
| Compound of Example 85 | <1 µM |
| Compound of Example 86 | <1 µM |

From this, it is seen that the compound (I) of the present invention has the excellent antagonism also for a rat-type RFRP receptor.

### Test Example 4

### Blood glucose concentration increasing activity of RFRP

As RFRP, human RFRP-1 (37 amino acids) consisting of an amino acid sequence of 56^{th} (Ser) to 92^{nd} (Phe) of an amino acid sequence represented by SEQ ID No.: 4. Hereinafter, this peptide is abbreviated as RFRP-1.

In order to study influence of peripheral administration of RFRP-1 on a blood glucose concentration, operation for collecting blood under free moving was performed. A mature Wistar male rat (weight 310 to 350 g at operation) was anesthetized with 50 mg/kg of pentobarbital by intraperitoneal administration. The rat was fixed on an anatomic pad in a supine position, and a left jugular vein was exposed. A polyethylene tube SP35 (internal diameter 0.5 mm, external diameter 0.9 mm, Natsume Seisakusho) was cut into a length of about 30 cm, and this was filled with a 200 unit/ml heparin-containing physiological saline, inserted into a jugular vein by about 4.5 cm, and fixed therein. Another end of a tube was exposed at a neck part (back side) through a part under a skin on a back side.

Overnight after operation, 300 µl of blood was collected using a tuberculin injection syringe having a dose of 1 ml and a 25 gauge injection needle (both Telmo) before administration of RFRP-1. In order to prevent blood coagulation, 3 µl of a 300 KIU/ml aprotinin solution containing 3 mg/ml EDTA had been placed into an injection syringe in advance. An Otsuka physiological saline or RFRP-1 (Peptide Laboratory) (17, 80, 170 nmol) dissolved in 1 mL of a physiological saline was intravenously administered through a tube at 1 mL/Kg. After 0, 5, 15, 30 and 60 minutes from initiation of intravenous administration, each 300 µl of blood was collected from a jugular vein. The collected blood was centrifuged (13,000 rpm, 5 minutes) using a minor amount high speed cooling centrifuge (MR-150, Tommy Seiko), and the supernatant (plasma) was recovered. A blood glucose concentration was measured using Fuji Drichem 3500 (FUJIFILM). As shown in Fig. 1, an RFRP-1 10 nmol/kg administration group showed significant (p<0.05, n = 4) activity of increasing a blood glucose concentration 5 minutes and 15 minutes after intravenous administration as compared with a physiological saline administration group.

### Test Example 5

### Pancreatic glucagon secretion promoting activity of RFRP

In order to study mechanism of blood glucose concentration increasing activity of RFRP-1, influence of RFRP-1 on a blood concentration of glucagon and insulin which are known as a hormone imparting a variation to a blood glucose concentration was studied. A mature Wistar male rat (weight 310 to 350 g at operation) was operated for collecting blood under free moving. Overnight after operation, 300 µl of a blood was collected using a tuberculin injection syringe having a dose of 1 ml and a 25 gauge injection needle (both Telmo) before administration of RFRP-1. In order to prevent blood coagulation, 3 µl of a 300 KIU/ml aprotinin solution containing 3 mg/ml EDTA had been placed into an injection syringe in advance. An Otsuka physiological saline or RFRP-1 dissolved in a physiological saline (80 nmol/mL) was intravenously administered through a tube at 1 mL/Kg. After 1, 3, 5 and 15 minutes from initiation of intravenous administration, each 300 µl of blood was collected from a jugular vein. The collected blood was centrifuged (13,000 rpm, 5 minutes) using a minor amount high speed cooling centrifuge (MR-150, Tommy Seiko), and the supernatant (plasma) was recovered. A blood glucagon concentration was measured using a glucagons kit ("First" (Daiichi Radioisotope Laboratory), and a blood insulin concentration was measured using rat insulin [¹²⁵I], an assay system (Amersham Biosciences). As shown in Fig. 2, in an RFRP-1 administration group, significant (p<0.01) increase in a blood glucagon concentration was recognized two minutes after administration as compared with a physiological saline administration group and, also 5 minutes after administration, significant (p<0.01) increase was continued. On the other hand, a variation due to administration of RFRP-1 was not recognized in a blood insulin concentration (Fig. 3). From these results, and from the fact that, in an RFRP-1 administration group, increase in a blood glucose concentration is seen after increase in a blood glucagon concentration, it was thought that activity of increasing a blood glucose concentration due to intravenous administration of RFRP-1 is caused by glucagon secretion of stimulation by RFRP-1.

### Test Example 6

### Amnesia promoting activity of RFRP

Since an RFRP nerve is projected on amygdaloid complex, in order to study involvement of RFRP in amygdaloid complex-dependent memory•learning ability, influence under cued fear conditioning due to ventricle administration of RFRP-1 was studied. A mature Wistar male rat (weight 280 to 320 g at operation) was anesthetized with 50 mg/kg of pentobarbital by intraperitoneal administration, and a rat was fixed in a brain localization apparatus. A tooth cutting bar was set lower by 3.3 mm from an interoralline. A cranial bone was exposed, and a hole was opened in a bone using a dental drill in order to embed a guide cannulae AG-12 (internal diameter 0.4 mm, external diameter 0.5 mm, Eicom) in a ventricle. In addition, an anchor screw was embedded in four places at a periphery thereof. A stainless guide cannulae AG-12 was inserted so that its tip was positioned at an upper part of a side ventricle. A localization coordinate was set to be AP: -0.8 mm, L: 1.5 mm, H: 4.5 mm from a Blegma according to Atlas of Paxinos and Watson (1986). A guide cannulae was fixed at a cranial bone with an instant adhesive and a dental cement, and an anchor screw. A stainless dummy cannulae AD-12 (external diameter 0.35 mm, Eicon) was inserted into a guide cannulae, and fixed with a capnight (Eicom). After operation, a rat was raised in a separate cage. A recovery term was set to be one week after operation, during which sufficient handling was performed.

In cued fear conditioning, first, as a training session, a rat was placed into a shock chamber, and was acclimated for 2 minutes and, immediately after impartation of sound stimulation for 30 seconds, a cycle giving electric stimulation 2.5 mA for 2 seconds and giving rest for 28 seconds was repeated five times (total 5 minutes). After test, the rat was released in a chamber for 2 minutes, and returned to an original cage. Then, as a test session, 24 hours (1 day) and 48 hours (2 day) after the aforementioned training, the rat was placed into the same chamber as that at training, sound stimulation for 30 seconds was given at the same timing as that of training five times, the rat was placed into a chamber, and behavior for 5 minutes was observed. Behavior analysis was used using analysis software Freeze Frame (Actimetric). When behavior of a variation rate of 15 or lower was observed by sound stimulation, it was defined as freezing. RFRP-1 (3 nmol) and a physiological saline (Otsuka Seiyaku) were administered into a ventricle before and after training, and before a test. The number of experimental animals was 12 in each group. As condition of the present test, a route when a test animal is brought to a test chamber was changed every time, and the animal was made to wait in a room other than a room in which a test was performed. As shown in Fig. 4, in an RFRP-1 administration group, a rate of freezing was remarkably reduced on 2 day as compared with a physiological saline administration group (physiological saline administration group; 46.5%, RFRP administration group; 35.5%). From these results, it was seen that RFRP-1 shows amnesia promoting activity.

### Test Example 7

### Inhibitory activity of increase in blood glucose concentration and blood glucagon concentration in RFRP-1-administered rats

A mature Wistar male rat (weight 300 to 350 g at operation) was anesthetized with 50 mg/kg pentobarbital by intraperitoneal administration. The rat was fixed on an anatomic pad in a supine position, and a left jugular vein was exposed. A polyethylene tube SP35 (internal diameter 0.5 mm, external diameter 0.9 mm, Natsume Seisakusho) was cut into a length of about 30 cm, and this was filled with a 200 unit/ml heparin-containing physiological saline, inserted into a jugular vein by about 4.5 cm, and fixed therein. Another end of a tube was exposed on a neck part (back side) through a part under a skin on a back side.

Overnight after operation, 300 µl of blood was collected using a tuberculin injection syringe having a dose of 1 ml and a 25 gauge injection needle (both Telmo) before administration of the compound of Example 50. In a blood recovering tube, in order to prevent blood coagulation, 3 µl of a 300KIU/ml aprotinin solution containing 3 mg/ml EDTA was placed in an injection syringe in advance. An Otsuka physiological saline or the compound of Example 50 dissolved in an Otsuka physiological saline was intravenously administered at 0.2, 1 or 5 mg/kg through a tube. Five minutes after administration of the compound of Example 50, 30 nmol mL/kg RFRP-1 (Peptide Laboratory) was intravenously administered and, 0, 2, 5, 15 and 30 minutes after the administration, each 300 µl of blood was collected from a jugular vein. The collected blood was centrifuged (13,000 rpm, 5 minutes) using a minor amount high speed cooling centrifuge (MR-150, Tommy Seiko), and the supernatant (plasma) was recovered. A blood glucose concentration was measured using Fuji Drichem 3500 (FUJIFILM). A blood glucagon concentration was measured using a glucagons kit "First" (Daiichi Radioisotope Laboratory). As shown in Fig. 5, the Example 50 compound administration group inhibits increase in a blood glucose concentration due to RFRP-1 in a dose dependent manner as compared with a control group and, in a 5 mg/kg administration group, activity of increasing a blood glucose concentration due to RFRP-1 was inhibited significantly (p<0.01, n = 4). In addition, as shown in Fig. 6, regarding a blood glucagon concentration, the Example 50 compound administration group inhibits increase in a blood glucagon concentration due to RFRP-1 in a dose dependent manner and, in a 5 mg/kg administration group, activity of increasing a blood glucagon concentration due to RFRP-1 was significantly inhibited 2 minutes after administration of RFRP-1 (p<0.05) and 5 minutes after administration of RFRP-1 (p<0.001).

From these results, it is seen that since the compound of Example 50 significantly inhibits activity of increasing a blood glucagons concentration due to RFRP-1, and lowers a blood glucose concentration, the compound is useful as a pancreatic glucagons secretion inhibiting agent, a blood glucose lowering agent, or a urine production inhibiting agent.

### Test Example 8

### Analgesic activity in mouse (SP-it test method)

Analgesic activity of an RFRP receptor antagonist on provisional pain caused by intrathecal injection (it administration) of substance P (SP) was investigated. In a test, one group (10 animals) of an ICR mouse (♂), 5 week old, was used. A skin of a back of a mouse was excised under ether anesthesia and, after 3 hours or longer recovering time, substance P (SP) was dissolved in a physiological saline, and this was administered by intrathecal injection (between L5-L6) at 10 ng/5 µL/mouse. A physiological saline and the compound of Example 50 were administered by intrathecal injection as a mixed solution with substance P (SP) at a dose of 1, 3 or 10 µg/mouse. From immediately after administration, the number of alternate chewing at a lower abdomen by a mouse was counted for 1 minute, and the time was used as an index for a provisional pain reaction due to substance P (SP). A test was performed using two of an administrator and a measurer, and measurement was performed in the blind state in which a measurer did not know which was administered.

As shown in Table 4, the compound of Example 50 showed significant (p<0.01) decrease in the chewing time at a dose of 3 and 10 µg/mouse, and showed analgesic activity in which a pain reaction due to substance P (SP) is inhibited in a dose dependent manner.

**[Table 4]**

| Administration group | Dose (µg/mouse) | Chewing time |
|---|---|---|
| Physiological saline | | 75.9 ± 4.4 |
| Compound of Example 50 | 1 | 71.8 ± 5.1 |
| Compound of Example 50 | 3 | 18.6 ± 4.1** |
| Compound of Example 50 | 10 | 0.0 ± 0.0** |

### Industrial Applicability

Since the compound (I) of the present invention or a salt thereof or a prodrug thereof has the excellent RFRP receptor function modulating activity, and exhibits the excellent oral absorbing property, it is used as a safe and effective drug, and is used as an analgesic, an agent for promoting analgesic activity of morphine, an agent for avoiding resistance due to morphine, an agent for modulating prolactin secretion, an agent for inhibiting pancreatic glucagon secretion, a blood glucose lowering agent, a urine production inhibiting agent, or a bladder constriction inhibiting agent.

## Claims

1. An agent for modulating the function of an RFRP receptor, which comprises a compound represented by the formula: 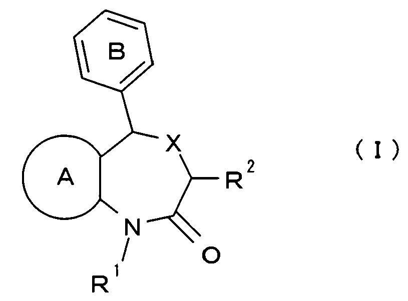
wherein a ring A represents an optionally substituted aromatic ring, a ring B represents an optionally substituted benzene ring, X represents O, S(O)n (n represents an integer of 0 to 2) or NR³ (R³ represents a hydrogen atom, an optionally substituted hydrocarbon group, or an optionally substituted heterocyclic group), and R¹ and R²each represents a hydrogen atom, an optionally substituted hydrocarbon group, or an optionally substituted heterocyclic group, or a salt thereof, or a prodrug thereof.

2. The agent according to claim 1, which comprises a compound represented by the formula: 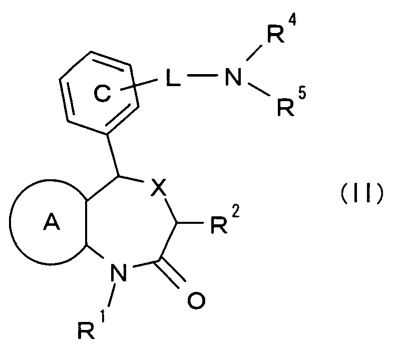
wherein L represents a linker, R⁴ and R⁵ each represents a hydrogen atom, an optionally substituted hydrocarbon group, an acyl group, an optionally substituted carbamoyl group, an esterified carboxyl group, or an optionally substituted hydrerocyclic group, R⁴ and R⁵ may be taken together to form a ring, or a R⁴ or R⁵ may be taken together with a linker represented by L to form a ring, a ring C represents an optionally further substituted benzene ring, and the other symbols are as defined in claim 1, or a salt thereof, or a prodrug thereof.

3. The agent according to claim 1, which comprises a compound represented by the formula: 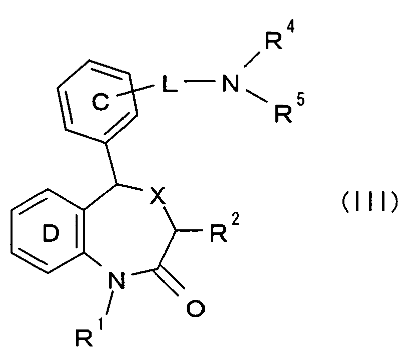
wherein a ring D represents an optionally substituted benzene ring, L represents a linker, R⁴ and R⁵ each represents a hydrogen atom, an optionally substituted hydrocarbon group, an acyl group, an optionally substituted carbamoyl group, an esterified carboxyl group, or an optionally substituted heterocyclic group, R⁴ and R⁵ may be taken together to form a ring, or R⁴ or R⁵ may be taken together with a linker represented by L to form a ring, a ring C represents an optionally further substituted benzene ring, and the other symbols are as defined in claim 1, or a salt thereof, or a prodrug thereof.

4. The agent according to claim 1, which comprises a compound represented by the formula: 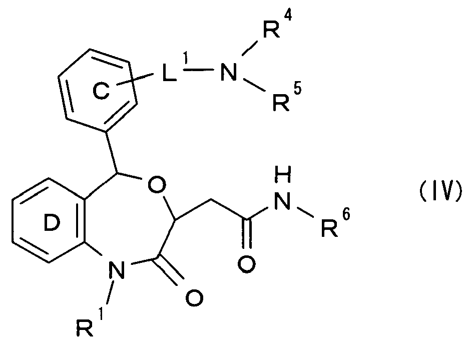
wherein a ring D represents an optionally substituted benzene ring, L¹ represents a linker represented by optionally substituted -Y-(CH₂)m- (Y represents a bond, -O-, -S(O)n¹- (n¹ represents an integer of 0 to 2) or -NR⁷- (R⁷ represents a hydrogen atom, an optionally substituted hydrocarbon group, or an optionally substituted heterocyclic group), and m represents an integer of 0 to 6), R⁴ and R⁵ each represents a hydrogen atom, an optionally substituted hydrocarbon group, an acyl group, an optionally substituted carbamoyl group, an esterified carboxyl group or an optionally substituted heterocyclic group, R⁴ and R⁵ may be taken together to form a ring, or R⁴ or R⁵ may be taken together with a linker represented by L¹ to form a ring, a ring C represents an optionally further substituted benzene ring, R⁶ represents an optionally substituted hydrocarbon group, or an optionally substituted heterocyclic group, and the other symbols are as defined in claim 1, or a salt thereof, or a prodrug thereof.

5. The agent according to claim 1, which is an analgesic, an agent for promoting analgesic activity of another analgesic drug, or an agent for avoiding resistance due to another analgesic drug.

6. The agent according to claim 1, which is an agent for modulating the prolactin secretion.

7. The agent according to claim 1, which is an agent for preventing or treating hyperprolactinemia, pituitary gland tumor, diencephalons tumor, emmeniopathy, stress, autoimmune disease, prolactinoma, infertility, impotence, amenorrhea, galactic leakage, acromegaly, Chiari-Frommel syndrome, Argonz-del Castilo syndrome, Forbes-Albright syndrome, breast cancer lymphoma, Sheehan's syndrome or spermatogenesis abnormality.

8. The agent according to claim 1, which is an agent for suppressing the pancreatic glucagon secretion, an agent for lowering a blood glucose or an agent for suppressing the urine production.

9. The agent according to claim 1, which is an agent for preventing or treating diabetes, glucose tolerance disorder, ketosis, acidosis, diabetic neuropathy, diabetic nephropathy, diabetic retinopathy, pollakiuria, nocturnal enarusis, hyperlipemia, sexual function disorder, skin disease, arthritis, osteopenia, arteriosclerosis, thrombotic disease, maldigestion or memory and learning disabilities.

10. The agent according to claim 1, which is an agent for suppressing the bladder constriction.

11. The agent according to claim 1, which is an agent for preventing or treating urine incontinence, lower uropathy, urge micturition due to excessive active bladder, or hypotonic bladder accompanied with excessive active bladder.

12. A compound represented by the formula: 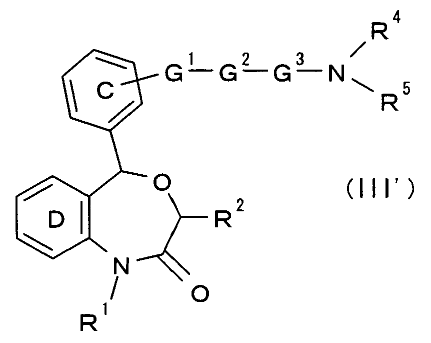
wherein a ring D represents an optionally substituted benzene ring, G¹ represents a bond, or an optionally substituted divalent hydrocarbon group, G² represents -O-, -NR⁸- (R⁸ represents a hydrogen atom, an optionally substituted hydrocarbon group, or an optionally substituted heterocyclic group) or -S(O)n²- (n² represents an integer of 0 to 2), G³ represents an optionally substituted divalent hydrocarbon group, R⁴ and R⁵ each represents a hydrogen atom, an optionally substituted hydrocarbon group, an acyl group, an optionally substituted carbamoly group, an esterified carboxyl group, or an optionally substituted heterocyclic group, a ring C represents an optionally further substituted benzene ring, R¹ and R² each represents a hydrogen atom, an optionally substituted hydrocarbon group, or an optionally substituted heterocyclic group, R⁴ may be taken together with G³ or R⁵ to form a ring and, when G² is -NR⁸-, R⁴ and R⁸ may be taken together to form a ring, provided that 3,5-trans-N-(2-fluorobenzyl)-5-[3-(3-tert-butoxycarbonylaminopropyl)aminomethylphenyl]-7-chloro-1-neopentyl-2-oxo-1,2,3,5-tetrahydro-4,1-benzooxazepine-3-acetamide, 3,5-trans-N-(2-fluorobenzyl)-5-[3-(3-aminopropyl)aminomethylphenyl]-7-chloro-1-neopentyl-2-oxo-1,2,3,5-tetrahydro-4,1-benzooxazepine-3-acetamide, 3,5-trans-N-(2-fluorobenzyl)-5-(3-aminoacetylaminomethylphenyl)-1-benzyl-7-chloro-2-oxo-1,2,3,5-tetrahydro-4,1-benzooxazepine-3-acetamide, 3,5-trans-N-(2-fluorobenzyl)-1-(4-biphenylmethyl)-7-chloro-2-oxo-5-[3-[(piperidin-4-yl)carbonylaminomethyl]phenyl]-1,2,3,5-tetrahydro-4,1-benzooxazepine-3-acetamide, 3,5-trans-N-(2-fluorobenzyl)-5-[2-(3-aminopropyloxy)phenyl]-7-chloro-1-isobutyl-2-oxo-1,2,3,5-tetrahydro-4,1-benzooxazepine-3-acetamide, 3,5-trans-N-(2-fluorobenzyl)-5-[4-(3-aminopropyloxy)-2-methoxyphenyl]-7-chloro-1-neopentyl-2-oxo-1,2,3,5-tetrahydro-4,1-benzooxazepine-3-acetamide, 7-chloro-5-[2-[3-[[(1,1-dimethylethoxy)carbonyl]amino]propoxy]phenyl]-1,2,3,5-tetrahydro-1-(2-methylpropyl)-2-oxo-4,1-benzooxazepin-3-ylacetic acid ethyl ester, and 7-chloro-5-[4-[3-[[(1,1-dimethylethoxy)carbonyl]amino]propoxy)-2-methoxyphenyl]-1-(2,2-dimethylpropyl)]-1,2,3,5-tetrahydro-2-oxo-4,1-benzooxazepin-3-ylacetic acid ethyl ester are excluded, or a salt thereof.

13. The compound according to claim 12, which is represented by the formula: 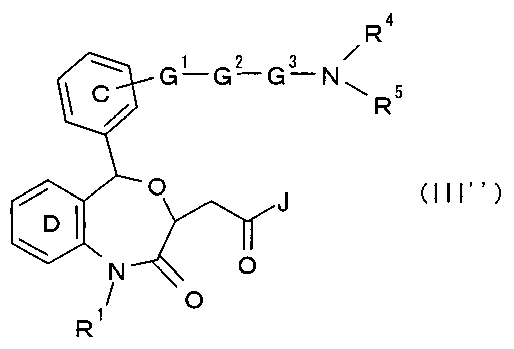
wherein J represents an optionally substituted hydroxy group, or an optionally substituted amino group, and the other symbols are as defined in claim 12.

14. The compound according to claim 12, which is represented by the formula: 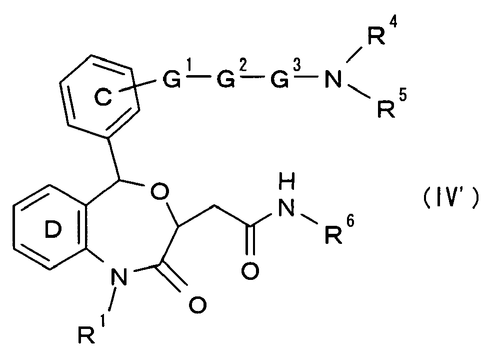
wherein R⁶ represents an optionally substituted hydrocarbon group, or an optionally substituted heterocyclic group, and the other symbols are ad defined in claim 12.

15. The compound according to claim 12, wherein G¹ is a bond, or an optionally substituted C₁₋₃ alkylene group.

16. The compound according to claim 12, wherein G³ is an optionally substituted C₂₋₆ alkylene group.

17. The compound according to claim 12, wherein G¹ is a bond, and G² is -O-.

18. The compound according to claim 12, wherein R¹ is an optionally substituted hydrocarbon group.

19. The compound according to claim 12, wherein R¹ is an optionally substituted C₁₋₈ alkyl group, or an optionally substituted C₇₋₁₆ aralkyl group.

20. The compound according to claim 12, wherein R⁴ is a hydrogen atom.

21. The compound according to claim 12, wherein R⁵ is an optionally substituted C₁₋₆ alkyl group, or an optionally substituted C₇₋₁₆ aralkyl group.

22. The compound according to claim 13, wherein J is a hydroxy group, an optionally substituted lower alkoxy group, an amino group optionally substituted with an optionally substituted alkyl group, or an optionally substituted cyclic amino group.

23. The compound according to claim 13, wherein J is an optionally substituted 5- to 8- membered cyclic amino group.

24. The compound according to claim 14, wherein R⁶ is an optionally substituted benzyl group, or an optionally substituted phenyl group.

25. The compound according to claim 12, wherein G³ is an optionally substituted divalent hydrocarbon group other than a carbonyl group and, when R⁴is a hydrogen atom, R⁵ is not a hydrogen atom or a tert-butyloxycarbonyl group.

26. A compound represented by the formula: 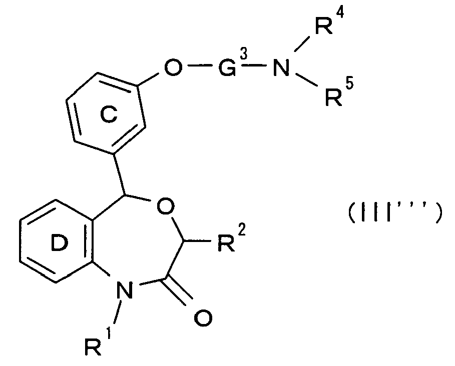
wherein a ring D represents an optionally substituted benzene ring, G³ represents an optionally substituted divalent hydrocarbon group, R⁴ and R⁵ each represents a hydrogen atom, an optionally substituted hydrocarbon group, an acyl group, an optionally substituted carbamoyl group, an esterified carboxyl group, or an optionally substituted heterocyclic group, a ring C represents an optionally further substituted benzene ring, R¹ and R² each represents an hydrogen atom, an optionally substituted hydrocarbon group, or an optionally substituted heterocyclic group, and R⁴ may be taken together with G³ or R⁵ to form a ring, or a salt thereof.

27. 2-(3,5-trans-7-Chloro-1-(2,2-dimethylpropyl)-5-{2-methoxy-3-[3-(3-phenylpropylamino)propoxy]phenyl}-2-oxo-1,2,3,5-tetrahydro-4,1-benzooxazepin-3-yl)-N-(2-fluorobenzyl)acetamide, 2-{3,5-trans-7-chloro-1-(2,2-dimethylpropyl)-5-[2-methoxy-3-(3-(pentylamino)propoxy)phenyl]-2-oxo-1,2,3,5-tetrahydro-4,1-benzooxazepin-3-yl}-N-(2-fluorobenzyl)acetamide, trans-2-{7-chloro-5-[3-(3-{[3-(2-chlorophenyl)propyl]amino}propoxy)-2-methoxyphenyl]-1-(2,2-dimethylpropyl)-2-oxo-1,2,3,5-tetrahydro-4,1-benzooxazepin-3-yl}-N-(2-fluorobenzyl)acetamide, trans-2-[7-chloro-1-(2,2-dimethylpropyl)-5-[2-methoxy-3-(3-{[(2E)-3-phenyl-2-propenyl]amino}propoxy)phenyl]-2-oxo-1,2,3,5-tetrahydro-4,1-benzooxazepin-3-yl]-N-(2-fluorobenzyl)acetamide, trans-2-[7-chloro-1-(2,2-dimethylpropyl)-5-(2-methoxy-3-{3-[(3-phenylpropyl)amino]propoxy}phenyl)-2-oxo-1,2,3,5-tetrahydro-4,1-benzooxazepin-3-yl]-N-propylacetamide, trans-7-chloro-1-(2,2-dimethylpropyl)-5-(2-methoxy-3-{3-[(3-phenylpropyl)amino]propoxy}phenyl)-3-[2-oxo-2-(1-piperazinyl)ethyl]-1,5-dihydro-4,1-benzooxazepine-2(3H)-one, trans-7-chloro-1-(2,2-dimethylpropyl)-3-[2-(4-hydroxypiperidin-1-yl)-2-oxoethyl]-5-(2-methoxy-3-{3-[(3-phenylpropyl)amino]propoxy}phenyl)-1,5-dihydro-4,1-benzooxazepine-2(3H)-one or 4-{[3,5-trans-7-chloro-1-(2,2-dimethylpropyl)-5-(2-methoxy-3-{3-[(3-phenylpropyl)amino]propoxy}phenyl)-2-oxo-1,2,3,5-tetrahydro-4,1-benzooxazepin-3-yl]acetyl}piperazine-2-carboxylic acid, or a salt thereof.

28. A prodrug of the compound according to claim 12 or 26.

29. A drug comprising the compound according to claim 12 or 26 or a prodrug thereof.

30. The drug according to claim 29, which is an agent for preventing or treating RFRP-associated with morbid state or a disease involved in RFRP.

31. A method of modulating the function of an RFRP receptor, which comprises administering an effective amount of a compound represented by the formula: 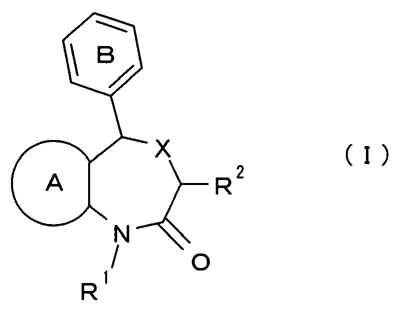
wherein a ring A represents an optionally substituted aromatic ring, a ring B represents an optionally substituted benzene ring, X represents O, S(O)n (n represents an integer of 0 to 2) or NR³ (R³ represents a hydrogen atom, an optionally substituted hydrocarbon group, or an optionally substituted heterocyclic group), and R¹ and R²each represents a hydrogen atom, an optionally substituted hydrocarbon group, or an optionally substituted heterocyclic ring, or a salt thereof, or a prodrug thereof to a mammal.

32. Use of a compound represented by the formula: 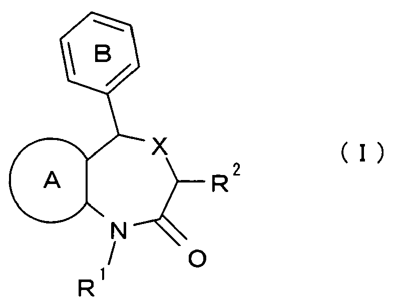
wherein a ring A represents an optionally substituted aromatic ring, a ring B represents an optionally substituted benzene ring, X represents O, S(O)n (n represents an integer of 0 to 2) or NR³ (R³ represents a hydrogen atom, an optionally substituted hydrocarbon group, or an optionally substituted heterocyclic group), and R¹ and R² each represents a hydrogen atom, an optionally substituted hydrocarbon group, or an optionally substituted heterocyclic group, or a salt thereof, or a prodrug thereof for preparing an agent for modulating the function of an RFRP receptor.
